(19) Europäisches Patentamt
European Patent Office
Office européen des brevets

(11) EP 4 745 240 A2

(12) EUROPEAN PATENT APPLICATION

(43) Date of publication:
**20.05.2026 Bulletin 2026/21**

(21) Application number: **25204758.4**

(22) Date of filing: **25.09.2025**

(51) International Patent Classification (IPC):
***C12N 15/113*** (2010.01) ***A61K 31/713*** (2006.01)

(52) Cooperative Patent Classification (CPC):
(C-Sets available)
**C12N 15/1136; A61K 31/7115; A61K 31/712; A61K 31/7125; A61K 31/713; A61P 9/12; C12N 15/113; C12N 15/1137;** C12N 2310/11; C12N 2310/14; C12N 2310/3125; C12N 2310/315; C12N 2310/321; C12N 2310/322; C12N 2310/323;
(Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **26.09.2024 CN 202411357089**

(71) Applicant: **Hangzhou Tianlong Pharmaceutical Co., Ltd.**
**Hangzhou, Zhejiang 310020 (CN)**

(72) Inventors:
- **SONG, Gengshen**
  **Zheijiang 310020 (CN)**
- **HUANG, Zeao**
  **Zheijiang 310020 (CN)**
- **YANG, Shuo**
  **Zheijiang 310020 (CN)**
- **JI, Guangshen**
  **Zheijiang 310020 (CN)**
- **TIAN, Zhikang**
  **Zheijiang 310020 (CN)**
- **WU, Yucheng**
  **Zheijiang 310020 (CN)**
- **GAO, Zhongcai**
  **Zheijiang 310020 (CN)**
- **ZHANG, Wei**
  **Zheijiang 310020 (CN)**
- **ZHANG, Man**
  **Zheijiang 310020 (CN)**

(74) Representative: **Dehns**
**10 Old Bailey**
**London EC4M 7NG (GB)**

Remarks:
The complete document including Reference Table(s) and the Sequence Listing(s) can be downloaded from the EPO website

# (54) SIRNA FOR TARGETED INHIBITION OF AGT GENE EXPRESSION AND USE THEREOF IN TREATING HYPERTENSION

(57) The present disclosure provides a modified oligonucleotide sequence and use thereof. A series of siRNAs were designed based on the angiotensinogen (AGT) messenger ribonucleic acid (mRNA) sequence, which were alternately modified or modified using a specific set of modification templates. The results from cell and animal experiments demonstrated that some oligonucleotide sequences with alternating modifications and specific template modifications can significantly inhibit AGT gene expression and may be used for the development of medicaments for treating hypertension and other related diseases.

EP 4 745 240 A2

(52) Cooperative Patent Classification (CPC): (Cont.)
C12N 2310/335; C12N 2310/336; C12N 2310/343;
C12N 2310/344; C12N 2310/346; C12N 2310/351;
C12N 2310/3521; C12N 2310/3533;
C12N 2320/11; C12N 2320/30

C-Sets
C12N 2310/321, C12N 2310/3521;
C12N 2310/322, C12N 2310/3533

## Description

### TECHNICAL FIELD

The present disclosure belongs to the technical field of nucleic acid modification, and specifically relates to a small interfering ribonucleic acid (siRNA) modified by various chemical methods and use thereof in the manufacture of a medicament for treating a disease associated with AGT gene expression, e.g., an siRNA for targeted inhibition of AGT gene expression and use thereof in treating hypertension.

### BACKGROUND

Nucleic acid drugs, particularly oligonucleotide drugs, have been widely used due to their simple synthesis and high activity. Oligonucleotide drugs typically include antisense oligonucleotides (ASOs), small interfering RNAs (siRNAs), microRNAs (miRNAs), and aptamers.

Oligonucleotides are short DNA or RNA molecules or oligomers that readily bind, in a sequence-specific manner, to their respective complementary oligonucleotides, DNA, or RNA to form duplexes or, less commonly, hybrids of a higher order. The fundamental property enables oligonucleotides to have broad applications in genetic testing, research, and medicine. In nature, oligonucleotides are typically small RNA molecules involved in regulating gene expression or intermediates derived from the degradation of larger nucleic acid molecules.

RNA interference (RNAi) is a natural defense mechanism against exogenous genes. siRNA can knock out the target gene by recognizing a specific sequence and degrading the target mRNA.

The effective molecule of classical RNAi consists of a hallmark polymer structure of 19 + 2 nucleotides (a double-helix structure consisting of a 21-nucleotide RNA molecule and a 19-nucleotide molecule with corresponding nucleobases, containing a 2-nucleotide 3' overhang). One strand of the siRNA (the guide strand or antisense strand) is complementary to the mRNA transcript of the target gene, while the other strand is designated as the passenger strand (sense strand). The siRNA (antisense strand) guides the Argonaute protein (AGO2) to complement the target transcript and becomes part of the RNA-induced silencing complex (RISC). The complete complementarity between the siRNA (antisense strand) and the target leads to cleavage of the target transcript at the opposite position of the guide strand (antisense strand) at positions 10 to 11 catalyzed by the AGO2 protein.

siRNA has inherent advantages over small molecule and antibody drugs because siRNA executes its function through Watson-Crick base pairing with mRNA, whereas small molecule and monoclonal antibody drugs require recognition of the complicated spatial conformation of specific proteins. As a result, there are many diseases that are not treatable by small molecules or monoclonal antibodies due to the high activity of their target molecules and the inability to recognize molecular structures with affinity and binding specificity thereto. The mechanism of action of siRNA drugs allows them to regulate the expression of target proteins at the genetic level, offering targeting specificity compared to small molecule or antibody drugs. The mechanism based on the principle of complementary base pairing also makes siRNA therapies broader in scope, simpler in design, and shorter in development cycles.

Oligonucleotides can bind sequence-specifically to complementary RNA strands and, upon hybridization, can induce RNase H cleavage of the target RNA. In natural oligonucleotides, nucleotides are linked by phosphodiester bonds, making them particularly susceptible to nucleases under physiological conditions. Thus, natural, unstructured, and unmodified oligonucleotide drugs are easily and rapidly degraded by nucleases *in vivo,* exhibiting low activity and poor druggability. Chemical modification of oligonucleotide structures is an effective approach to enhance their activity, which can improve their stability to nucleases and affinity for RNA, and better facilitate endocytosis and tissue targeting, thereby effectively regulating the expression of target genes.

Based on the fundamental structure of oligonucleotides, *i.e.*, bases, sugar rings, phosphate backbones, and termini, chemical modifications can be divided into four parts:

1) Base modifications: primarily categorized into purine modifications, pyrimidine modifications, and base substitutions. Purine modifications include N6-methyladenosine, N1-methyladenosine, and 7-methylguanosine modifications; pyrimidine modifications include 3-methyluridine, 5-methyluridine, 5-methylcytidine, N4-acetylcytidine, pseudouridine, thiouridine, propynyluridine, and dihydrouridine modifications.

2) Sugar ring modifications: primarily categorized into sugar ring modifications and substitutions. Sugar ring modifications include 2'-modifications, 4'-modifications, 5'-modifications, isomer modifications, and combinations thereof. The most common 2'-modifications of siRNA are 2'-OMe (2'-O-methyl) and 2'-F (2'-fluoro) modifications. Compared to natural siRNAs, siRNAs modified with both 2'-OMe and 2'-F exhibit higher Tm values, stronger serum stability, and better activity.

3) Phosphate backbone modifications: primary modification methods include phosphorothioate modifications; modifications with methylphosphonate, selenophosphate, boranophosphate, dithiophosphate, and substitution of

the bridging oxygen atom in the phosphodiester linkage with a sulfur atom; and substitution of the entire phosphate group between nucleosides with a non-phosphorus group, such as substitution of the P atom with a C, S, or N atom to form guanidinyl, S-methylthiourea, *etc.*

4) Terminal modifications: covalent conjugation of special groups at the 5' or/and 3' end of the sense strand and phosphorylation modification at the 5' end of the antisense strand.

Hypertension is a systemic disease characterized primarily by elevated blood pressure, with high prevalence but low treatment and control rates.

Angiotensinogen (AGT) protein is a secreted protein predominantly expressed in the liver in humans. AGT is also expressed in the brain, gallbladder, heart, kidney, and other tissues. AGT protein is cleaved by renin to generate angiotensin I (Ang I), which is subsequently cleaved by angiotensin-converting enzyme (ACE) to generate physiologically active angiotensin II (Ang II). Ang II binds to angiotensin receptors (ATR), triggering vasoconstriction and elevating blood pressure.

Although numerous antihypertensive medications are currently available for the treatment of hypertension, over two-thirds of patients struggle to control their blood pressure with one medication and require two or more medications to control their blood pressure, which leads to reduced therapy adherence, increased potential side effects, and compromised therapeutic outcomes. Thus, there is a need in the art for alternative and combination therapies for patients suffering from angiotensinogen-related diseases.

SUMMARY

The present disclosure relates to a double-stranded siRNA drug conjugated to N-acetylgalactosamine (GalNAc). Upon entering the bloodstream, GalNAc can bind to the asialoglycoprotein receptor (ASGPR) on the surface of hepatocytes, after which the drug is internalized into the cells and stored in endosomal structures. After being released from endosomes or lysosomes into the cytoplasm, the drug binds to the RNA-induced silencing complex (RISC) and, mediated by the antisense strand, binds to the mRNA transcribed from the AGT gene, inducing mRNA degradation and thereby inhibiting translation of AGT protein. Inhibition of the expression of AGT protein, as an upstream protein in the renin-angiotensin-aldosterone system (RAAS), will fundamentally inhibit the blood pressure-elevating effect of the RAAS system, thereby reducing blood pressure.

The present disclosure designs a series of unique siRNA sequences by targeting AGT mRNA sequences, followed by incorporating alternating modifications and specific template modifications.

Typically, siRNA employs 2'-O-methyl (2'-OMe) and 2'-fluoro (2'-F) modifications for monomers. However, even considering only the combination of these two monomer modifications, for siRNA, the sense and antisense strands collectively comprise 44 bases, resulting in $2^{44}$ possible combinations. Furthermore, with the addition of different terminal phosphorothioate modification patterns, the number of possible modification schemes becomes more extensive.

The same siRNA sequence with different modification patterns exhibits significant differences in activity, while different siRNA sequences with the same modification pattern also exhibit significant differences in activity. Although some modification principles exist for siRNA modification design, previous studies have shown that it is impossible to accurately predict the activity based on the modification pattern, meaning that there is no definitive relationship between modification pattern and activity. Therefore, screening for highly active modification schemes from a myriad of possible modification combinations is extremely challenging.

The present disclosure screens out a number of sequences with alternating modifications and special modifications that significantly inhibit AGT gene expression by chemically modifying the designed siRNA sequences.

In one aspect, the present disclosure provides a double-stranded RNAi agent comprising an oligonucleotide duplex formed by pairing of a sense strand and an antisense strand.

In another aspect, the present disclosure provides a conjugate for reducing the expression of AGT, comprising the double-stranded RNAi agent described above and a ligand conjugated thereto.

In another aspect, the present disclosure provides a nucleic acid-protein composition comprising a duplex region of the double-stranded RNAi agent described above or an antisense strand of the duplex region, and a nuclease.

In another aspect, the present disclosure provides a recombinant vector comprising a nucleic acid molecule encoding the double-stranded RNAi agent described above.

In some embodiments, the vector backbone of the recombinant vector is selected from recombinant virus-derived circular RNA vectors, tRNA, rRNA scaffolds, and chimeric tRNA/pre-miRNA vectors.

In another aspect, the present disclosure provides a recombinant cell that synthesizes and secretes the double-stranded RNAi agent described above.

In some embodiments, the recombinant cell is selected from *Thiocapsa roseopersicina* and ribonuclease III-deficient *Corynebacterium glutamicum.*

In another aspect, the present disclosure provides a method for preparing a double-stranded RNAi agent,

comprising culturing the recombinant cell described above or chemical synthesis.

In another aspect, the present disclosure provides a pharmaceutical composition comprising the double-stranded RNAi agent described above, the conjugate described above, or the nucleic acid-protein composition described above, and a pharmaceutically acceptable carrier.

In another aspect, the present disclosure provides a method for inhibiting AGT gene expression, comprising contacting the double-stranded RNAi agent described above, the conjugate described above, the nucleic acid-protein composition described above, or the pharmaceutical composition described above with a target cell.

In some embodiments, the method is for non-diagnostic or non-therapeutic purposes.

In some embodiments, the method is performed *in vivo* or *in vitro.*

In another aspect, the present disclosure provides a use of the double-stranded RNAi agent described above, the conjugate described above, the nucleic acid-protein composition described above, or the pharmaceutical composition described above in the manufacture of a medicament for treating a disease associated with AGT gene expression.

The disease associated with AGT gene expression is selected from diseases caused by overexpression of AGT protein, pathogenic mutations in the AGT gene, abnormal metabolism of AGT protein, and abnormal interaction between AGT and another substance.

In some embodiments, the disease associated with AGT gene expression is selected from hypertension, ocular hypertension, glaucoma, pulmonary hypertension, portal hypertension, systemic venous hypertension, hypertensive heart disease, atherosclerosis, arteriosclerosis, vascular disease, diabetic nephropathy, diabetic retinopathy, chronic heart failure, cardiomyopathy, diabetic cardiomyopathy, glomerulosclerosis, aortic coarctation, aortic aneurysm, ventricular fibrosis, Cushing's syndrome and other glucocorticoid excess states (including chronic steroid therapy), pheochromocytoma, reninoma, secondary hyperaldosteronism and other mineralocorticoid excess states, sleep apnea, thyroid/parathyroid diseases, heart failure, myocardial infarction, angina pectoris, stroke, diabetes, nephropathy (e.g., hypertensive nephropathy), renal failure, systemic sclerosis, intrauterine growth retardation (IUGR), and fetal growth restriction.

In another aspect, the present disclosure provides the double-stranded RNAi agent described above, the conjugate described above, the nucleic acid-protein composition described above, or the pharmaceutical composition described above for use in treating a disease associated with AGT gene expression.

In another aspect, the present disclosure provides a method for treating a disease associated with AGT gene expression, comprising administering to a subject in need thereof an effective amount of the double-stranded RNAi agent described above, the conjugate described above, the nucleic acid-protein composition described above, or the pharmaceutical composition described above.

In another aspect, the present disclosure provides a use of the double-stranded RNAi agent described above, the conjugate described above, the nucleic acid-protein composition described above, or the pharmaceutical composition described above in the manufacture of a medicament for treating hypertension and cardiovascular and cerebrovascular diseases.

In another aspect, the present disclosure provides the double-stranded RNAi agent described above, the conjugate described above, the nucleic acid-protein composition described above, or the pharmaceutical composition described above for use in treating hypertension and cardiovascular and cerebrovascular diseases.

In another aspect, the present disclosure provides a method for treating hypertension and cardiovascular and cerebrovascular diseases, comprising administering to a subject in need thereof an effective amount of the double-stranded RNAi agent described above, the conjugate described above, the nucleic acid-protein composition described above, or the pharmaceutical composition described above.

The beneficial effects achieved by the present disclosure include at least the following:

(1) The unmodified sequences, including unmodified sequences 1576s, 1578s, 1838, 1835, 1816, 1812, 1579, 1836, 1789, 1839, 1791, 1795, and 1585s, exhibit significant inhibitory effects on AGT, with an inhibition rate exceeding 50% at maximum.

(2) The multiply modified sequences exhibit an inhibition rate of up to 70% or higher. Moreover, the modified sequences conjugated with GalNAc compounds can be efficiently delivered to the liver in animals, significantly inhibiting AGT gene expression and significantly reducing blood pressure levels.

(3) The sequences modified by alternating modifications and the modification templates of the present disclosure all exhibit significantly enhanced AGT inhibitory activity compared to the unmodified sequences with minimal differences disclosed in the prior art, with a maximum increase of 91.0%.

(4) The present disclosure shows that siRNAs with similar sequences, whether unmodified sequences or alternately modified sequences, exhibit significant differences in activity. For example, the unmodified sequence 1812 exhibits a 14.3% increase in inhibition rate compared to the unmodified sequence 812P, while the alternately modified sequence B1812-AL exhibits a 20.4% increase in inhibition rate compared to the unmodified sequence 812P, indicating a significant improvement.

(5) The present disclosure also shows that the effect of alternating modifications on activity varies across different sequences. For some sequences, the inhibition rate is significantly improved, e.g., the unmodified sequence 836 exhibits a 13.3% increase in inhibition rate after alternating modification. For others, the improvement is minimal, e.g., the unmodified sequences 994, 1279, and 1591 exhibit virtually no difference in inhibition rate between alternately modified and unmodified sequences.

BRIEF DESCRIPTION OF THE DRAWINGS

To more clearly illustrate the technical solutions of the embodiments of the present disclosure, the accompanying drawings of the embodiments will be briefly described below. It is apparent that the accompanying drawings in the following description relate only to some embodiments of the present disclosure and are not intended to limit the present disclosure.

FIG. 1 shows alternately modified sequences with significant inhibitory effects on the AGT gene, all exhibiting inhibition rates above 40%.
FIG. 2 shows alternately modified sequences with inhibition rates of 25% to 40% for the AGT gene.
FIG. 3 shows alternately modified sequences with inhibition rates below 25% for the AGT gene.
FIG. 4 shows unmodified sequences with significant inhibitory effects on the AGT gene, all exhibiting inhibition rates above 45%.
FIG. 5 shows unmodified sequences with inhibition rates below 45% for the AGT gene.
FIG. 6 shows the inhibition rates of unmodified sequence 1579 for the AGT gene after different template modifications, as well as the inhibition rate of sequence 579P disclosed in the prior art for the AGT gene.
FIG. 7 shows the inhibition rates of unmodified sequences 1578s and 1835 for the AGT gene after template modification and anti-off-target modification.
FIG. 8 shows the inhibition rates of unmodified sequences 1579, 1789, 1812, 1576s, 1578s, and 1585s for AGT protein in animal serum after different modification schemes and conjugation with GalNAc compounds.
FIG. 9 shows the inhibition rates of unmodified sequences 1579, 1789, 1812, 1576s, 1578s, and 1585s for AGT protein in animal serum at different time points after different modification schemes and conjugation with GalNAc compounds.

DETAILED DESCRIPTION OF THE PREFERRED EMBODIMENT

To facilitate understanding of the present disclosure, certain terms are first defined. In addition, it should be noted that whenever a value or a range of values for a parameter is recited, it is intended that values and ranges intermediate to the recited values are also intended to be part of the present disclosure.

As used herein, the articles "a" and "an" refer to one or more than one (*i.e.,* at least one) of the grammatical object of the article. By way of example, "an element" means one element or more than one element, *e.g.,* a plurality of elements.

The term "including" is used herein to mean, and is used interchangeably with, the phrase "including, but not limited to".

The term "or" is used herein to mean, and is used interchangeably with, the term "and/or", unless the context clearly indicates otherwise.

As used herein, the term "about" or "approximately", as applied to one or more values of interest, refers to a value that is similar to a stated reference value. In certain embodiments, the term "approximately" or "about" refers to a range of values that fall within 20%, 19%, 18%, 17%, 16%, 15%, 14%, 13%, 12%, 11%, 10%, 9%, 8%, 7%, 6%, 5%, 4%, 3%, 2%, 1%, or less of the stated reference value in either direction (greater than or less than), unless otherwise specified or clearly evident from the context (except where such a number would exceed 100% of a possible value).

As used herein, "AGT" refers to the angiotensinogen gene or protein.

"G", "C", "A", and "U" each generally represent a nucleotide containing guanine, cytosine, adenine, and uracil as a base, respectively. "T", "Td", and "dT" are used interchangeably herein and refer to a deoxyribonucleotide in which the nucleobase is thymine, such as deoxyribothymine, 2'-deoxythymidine, or thymidine. However, it should be understood that the term "ribonucleotide", "nucleotide", or "deoxyribonucleotide" may also refer to a modified nucleotide (as further detailed below) or a surrogate replacement moiety. Those skilled in the art will readily appreciate that guanine, cytosine, adenine, and uracil may be replaced by other moieties without substantially altering the base pairing properties of an oligonucleotide (including a nucleotide comprising such a replacement moiety). For example, without limitation, a nucleotide containing inosine as its base may base pair with a nucleotide containing adenine, cytosine, or uracil. Hence, nucleotides containing uracil, guanine, or adenine may be replaced in the nucleotide sequences of the present disclosure by a nucleotide containing, for example, inosine. Sequences comprising such replacement moieties are embodiments of the present disclosure.

The terms "iRNA", "RNAi agent", "iRNA agent", and "RNA interference agent" are used interchangeably herein,

and the defined terms encompass RNA agents that can mediate the targeted cleavage of a transcript via an RNA-induced silencing complex (RISC) pathway. iRNA directs the sequence-specific degradation of mRNA through a process known as RNA interference (RNAi). iRNA modulates, *e.g.,* inhibits, the expression of AGT in cells such as those within a subject (*e.g.,* a mammalian subject). RNAi molecules include single-stranded RNAi molecules (Lima et al., 2012, Cell 150:883) and double-stranded siRNAs, as well as short hairpin RNAs (shRNAs).

The term "small interfering ribonucleic acid" or "siRNA" refers to small interfering ribonucleic acid RNAi molecules. It is a class of double-stranded RNA molecules, also known in the art as short interfering RNA or silencing RNA. siRNA typically comprises a sense strand (also referred to as a passenger strand) and an antisense strand (also referred to as a guide strand), wherein each strand is 17 to 30 nucleotides in length, typically 19 to 25 nucleotides in length, wherein the antisense strand is complementary *(e.g.,* at least 95% complementary, *e.g.,* fully complementary) to the target nucleic acid (suitably a mature mRNA sequence), and the sense strand is complementary to the antisense strand, such that the sense and antisense strands form a duplex or duplex region. The siRNA strands may form a blunt-ended duplex, or preferably, the 3' ends of the sense and antisense strands may form a 3' overhang, e.g., 1, 2, or 3 nucleotides, to resemble the product produced by Dicer, which may form a RISC substrate *in vivo.* Effective extended forms of Dicer substrates have been described in US 8,349,809 and US 8,513,207, which are incorporated herein by reference. In some embodiments, both the sense strand and the antisense strand have a 2-nt 3' overhang. Thus, the duplex region may be, *e.g.,* 17 to 25 nucleotides in length, such as 21 to 23 nucleotides in length.

The term "antisense strand" refers to the strand of RNAi *(e.g.,* dsRNA) which includes a region that is substantially complementary to a target sequence. As used herein, the term "region of complementarity" refers to a region on the antisense strand that is substantially complementary to a sequence *(e.g.,* a target sequence) as defined herein. Where the region of complementarity is not fully complementary to the target sequence, the mismatches may be in the internal or terminal regions of the molecule. Typically, the most tolerated mismatches are in the terminal regions, e.g., within 5, 4, 3, or 2 nucleotides of the 5' and/or 3' ends.

As used herein, the term "sense strand" refers to the strand of RNAi which includes a region that is substantially complementary to a region of the antisense strand (as the term is defined herein).

The term "alternating modification" refers to a fully modified siRNA sequence employing alternating 2'-O-methyl (2'-OMe) and 2'-fluoro (2'-F) modifications. For the antisense strand of an siRNA sequence, 2'-O-methyl modifications are introduced at odd-numbered positions *(i.e.,* positions 1, 3, 5, 7...21, 23), while 2'-fluoro modifications are introduced at even-numbered positions *(i.e.,* positions 2, 4, 6, 8...20, 22). For the sense strand complementary to the antisense strand, at positions where 2'-O-methyl modifications are introduced on the antisense strand, 2'-fluoro modifications are introduced at complementary positions on the sense strand; at positions where 2'-fluoro modifications are introduced on the antisense strand, 2'-O-methyl modifications are introduced at complementary positions on the sense strand.

As used herein, the term "inhibiting" is used interchangeably with "reducing", "silencing", "downregulating", "suppressing", and other similar terms, and includes any level of inhibition.

As used herein, the phrase "inhibiting expression of AGT" includes inhibition of expression of any AGT gene *(e.g.,* a mouse AGT gene, a rat AGT gene, a monkey AGT gene, or a human AGT gene) as well as variants (e.g., naturally occurring variants) or mutants of the AGT gene. Thus, the AGT gene may be a wild-type AGT gene, a mutant AGT gene, or a transgenic AGT gene in the context of genetically manipulated cells, cell populations, or organisms.

"Inhibiting AGT gene expression" includes any level of inhibition of the AGT gene, *e.g.,* at least partial inhibition of AGT gene expression, such as inhibition of at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99%.

AGT gene expression may be assessed based on the level of any variable associated with AGT gene expression, e.g., AGT mRNA levels or AGT protein levels. Inhibition may be assessed by a reduction in the absolute or relative level of one or more of these variables compared to a control level. The control level may be any type of control level utilized in the art, *e.g.,* a pre-dose baseline level or a level determined from a similar subject, cell, or sample that is untreated or treated with a control (e.g., buffer-only control or inert agent control).

As used herein, a "patient" or "subject" is intended to include a human or non-human animal, preferably a mammal, e.g., a monkey. Most preferably, the subject or patient is a human.

As used herein, a "AGT-associated disease" is intended to include any disease associated with the AGT gene or protein. Such a disease may be caused, for example, by overproduction of the AGT protein, by mutations in the AGT gene, by abnormal cleavage of the AGT protein, or by abnormal interaction between AGT and other proteins or other endogenous or exogenous substances. Exemplary AGT-associated diseases include hypertension, borderline hypertension, primary hypertension, secondary hypertension, hypertensive crisis, hypertensive urgency, isolated systolic and diastolic hypertension, pregnancy-associated hypertension, diabetic hypertension, resistant hypertension, refractory hypertension, paroxysmal hypertension, renovascular hypertension, Goldblatt hypertension, ocular hypertension, glaucoma, pulmonary hypertension, portal hypertension, systemic venous hypertension, systolic hypertension, labile hypertension, hypertensive heart disease, hypertensive nephropathy, atherosclerosis, arteriosclerosis, vasculopathy, diabetic nephropathy, diabetic retinopathy, chronic heart failure, cardiomyopathy, diabetic cardiomyopathy, glomerulosclerosis, aortic coarctation, aortic aneurysm, ventricular fibrosis, Cushing's syndrome and other glucocorticoid excess states (including chronic steroid therapy), pheochromocytoma, reninoma, secondary hyperaldosteronism and other mineralocorticoid excess states, sleep apnea, thyroid/parathyroid diseases, heart failure, myocardial infarction, angina, stroke, diabetes, nephropathy, renal failure, systemic sclerosis, intrauterine growth retardation (IUGR), and fetal growth restriction.

As used herein, a "therapeutically effective amount" is intended to include an amount of an RNAi agent that, when administered to a patient for treating an AGT-associated disease, is sufficient to achieve treatment of the disease (e.g., by attenuating, ameliorating, or maintaining the existing disease or one or more symptoms of the disease). The "therapeutically effective amount" may vary depending on the RNAi agent, how the agent is administered, the disease and its severity, medical history, age, weight, family history, genetic makeup, stage of pathological processes mediated by the expression of AGT, type of prior or concomitant therapy (if any), and other individual characteristics of the patient to be treated.

As used herein, a "prophylactically effective amount" is intended to include an amount of an RNAi agent that, when administered to a subject who has not yet experienced or exhibited symptoms of an AGT-associated disease, but who may be susceptible to the disease, is sufficient to prevent or ameliorate the disease or one or more symptoms of the disease. Amelioration of the disease includes slowing the progression of the disease or reducing the severity of later-onset disease. The "prophylactically effective amount" may vary depending on the RNAi agent, how the agent is administered, degree of risk of the disease, medical history, age, weight, family history, genetic makeup, type of prior or concomitant therapy (if any), and other individual characteristics of the patient to be treated.

The "therapeutically effective amount" or "prophylactically effective amount" also includes an amount of an RNAi agent that produces a desired local or systemic effect at a reasonable benefit/risk ratio applicable to any treatment. The RNAi agent used in the methods of the present disclosure may be administered in an amount sufficient to achieve a reasonable benefit/risk ratio applicable to such treatment.

As used herein, the term "sample" includes a collection of similar fluids, cells, or tissues isolated from a subject, as well as fluids, cells, or tissues present in the subject. Examples of biological fluids include blood, serum and serous fluid, plasma, cerebrospinal fluid, ocular fluid, lymph, urine, saliva, *etc.* Tissue samples may include samples from tissues, organs, or localized regions. For example, samples may be derived from specific organs, parts of organs, or fluids or cells within these organs. In certain embodiments, samples may be derived from the liver *(e.g.,* the entire liver or certain segments of the liver, or certain types of cells in the liver, such as hepatocytes). In preferred embodiments, a "sample derived from a subject" refers to blood or plasma drawn from the subject. In other embodiments, a "sample derived from a subject" refers to liver tissue (or subcomponents thereof) derived from the subject.

In one aspect, the present disclosure provides a double-stranded RNAi agent for reducing the expression of AGT, comprising any one oligonucleotide duplex selected from the following pairings of a sense strand and an antisense strand:

(1) the sense strand has the sequence shown in SEQ ID NO: 1 or a fragment thereof, or a modified sequence of the sequence or the fragment thereof; and the antisense strand has the sequence shown in SEQ ID NO: 14 or a fragment thereof, or a modified sequence of the sequence or the fragment thereof;
(2) the sense strand has the sequence shown in SEQ ID NO: 2 or a fragment thereof, or a modified sequence of the sequence or the fragment thereof; and the antisense strand has the sequence shown in SEQ ID NO: 15 or a fragment thereof, or a modified sequence of the sequence or the fragment thereof;
(3) the sense strand has the sequence shown in SEQ ID NO: 3 or a fragment thereof, or a modified sequence of the sequence or the fragment thereof; and the antisense strand has the sequence shown in SEQ ID NO: 16 or a fragment thereof, or a modified sequence of the sequence or the fragment thereof;
(4) the sense strand has the sequence shown in SEQ ID NO: 4 or a fragment thereof, or a modified sequence of the sequence or the fragment thereof; and the antisense strand has the sequence shown in SEQ ID NO: 17 or a fragment thereof, or a modified sequence of the sequence or the fragment thereof;
(5) the sense strand has the sequence shown in SEQ ID NO: 5 or a fragment thereof, or a modified sequence of the sequence or the fragment thereof; and the antisense strand has the sequence shown in SEQ ID NO: 18 or a fragment thereof, or a modified sequence of the sequence or the fragment thereof;
(6) the sense strand has the sequence shown in SEQ ID NO: 6 or a fragment thereof, or a modified sequence of the sequence or the fragment thereof; and the antisense strand has the sequence shown in SEQ ID NO: 19 or a fragment thereof, or a modified sequence of the sequence or the fragment thereof;
(7) the sense strand has the sequence shown in SEQ ID NO: 7 or a fragment thereof, or a modified sequence of the sequence or the fragment thereof; and the antisense strand has the sequence shown in SEQ ID NO: 20 or a fragment thereof, or a modified sequence of the sequence or the fragment thereof;

(8) the sense strand has the sequence shown in SEQ ID NO: 8 or a fragment thereof, or a modified sequence of the sequence or the fragment thereof; and the antisense strand has the sequence shown in SEQ ID NO: 21 or a fragment thereof, or a modified sequence of the sequence or the fragment thereof;
(9) the sense strand has the sequence shown in SEQ ID NO: 9 or a fragment thereof, or a modified sequence of the sequence or the fragment thereof; and the antisense strand has the sequence shown in SEQ ID NO: 22 or a fragment thereof, or a modified sequence of the sequence or the fragment thereof;
(10) the sense strand has the sequence shown in SEQ ID NO: 10 or a fragment thereof, or a modified sequence of the sequence or the fragment thereof; and the antisense strand has the sequence shown in SEQ ID NO: 23 or a fragment thereof, or a modified sequence of the sequence or the fragment thereof;
(11) the sense strand has the sequence shown in SEQ ID NO: 11 or a fragment thereof, or a modified sequence of the sequence or the fragment thereof; and the antisense strand has the sequence shown in SEQ ID NO: 24 or a fragment thereof, or a modified sequence of the sequence or the fragment thereof;
(12) the sense strand has the sequence shown in SEQ ID NO: 12 or a fragment thereof, or a modified sequence of the sequence or the fragment thereof; and the antisense strand has the sequence shown in SEQ ID NO: 25 or a fragment thereof, or a modified sequence of the sequence or the fragment thereof;
(13) the sense strand has the sequence shown in SEQ ID NO: 13 or a fragment thereof, or a modified sequence of the sequence or the fragment thereof; and the antisense strand has the sequence shown in SEQ ID NO: 26 or a fragment thereof, or a modified sequence of the sequence or the fragment thereof. In some embodiments, all nucleotides on the sense and antisense strands are modified nucleotides.

In some embodiments, the double-stranded RNAi agent is an RNAi agent for inhibiting AGT gene expression.
In some embodiments, the sense strand differs from any one of the sequences of SEQ ID NOs: 1 to 13 by 1 to 3 nucleotides.
In some embodiments, the antisense strand differs from any one of the sequences of SEQ ID NOs: 14 to 26 by 1 to 3 nucleotides.
In some embodiments, at least one of the modified nucleotides is selected from the group consisting of a deoxynucleotide, a 3'-deoxy-thymidine (dT) nucleotide, a 2'-O-methyl modified nucleotide, a 2'-fluoro modified nucleotide, a 2'-deoxy-modified nucleotide, a locked nucleotide, an unlocked nucleotide, a conformationally restricted nucleotide, a constrained ethyl nucleotide, an abasic nucleotide, a 2'-amino-modified nucleotide, a 2'-O-allyl-modified nucleotide, a 2'-C-alkyl-modified nucleotide, a 2'-hydroxy-modified nucleotide, a 2'-methoxyethyl modified nucleotide, a 2'-O-alkyl-modified nucleotide, a morpholino nucleotide, a phosphoramidate, a nucleotide comprising a non-natural base, a tetrahydropyran modified nucleotide, a 1,5-anhydrohexitol modified nucleotide, a cyclohexenyl modified nucleotide, a nucleotide comprising a phosphorothioate group, a nucleotide comprising a methylphosphonate group, a nucleotide comprising a 5'-phosphate group, and a nucleotide comprising a 5'-phosphate mimic.
In some embodiments, at least one strand comprises a 3' overhang of at least 1 nucleotide.
In some embodiments, at least one strand comprises a 3' overhang of at least 2 nucleotides.
In some embodiments, the duplex region is 15 to 30 nucleotide pairs in length.
In some embodiments, the duplex region is 17 to 25 nucleotide pairs in length.
In some embodiments, the duplex region is 19 to 23 nucleotide pairs in length.
In some embodiments, the duplex region is 21 nucleotide pairs in length.
In some embodiments, each strand has 15 to 30 nucleotides.
In some embodiments, each strand has 19 to 25 nucleotides.
In some embodiments, the sense strand has 21 nucleotides and the antisense strand has 23 nucleotides.
In some embodiments, all nucleotide modifications on the sense and antisense strands are chemical modifications at the 2' position of the nucleotide ribose.
In some embodiments, the chemical modification at the 2' position of the nucleotide ribose is selected from any one of 2'-O-methyl, 2'-methoxyethyl, 2'-fluoro, 2'-benzyloxy, 2'-methylcarbonylamino, and 2'-pyridinylmethoxy, or a combination thereof.
In some embodiments, the chemical modification at the 2' position of each nucleotide ribose is selected from a combination of 2'-O-methyl and 2'-fluoro.
In some embodiments, the chemical modification at the 2' position of each nucleotide ribose is selected from an alternating combination of 2'-O-methyl and 2'-fluoro.
In some embodiments, the chemical modification pattern at the 2' position of each nucleotide ribose is as follows: on the sense strand, all odd-numbered positions are modified with 2'-fluoro, and all even-numbered positions are modified with 2'-O-methyl; on the antisense strand, all odd-numbered positions are modified with 2'-O-methyl, and all even-numbered positions are modified with 2'-fluoro.
In some embodiments, nucleotide monomers are linked by a 3',5'-phosphodiester bond.
In some embodiments, the 3',5'-phosphodiester bond between nucleotide monomers is phosphorothioate-modified.

In some embodiments, the aforementioned oligonucleotide comprises the following modifications: the antisense strand is modified according to one of the modification patterns shown in Table 42:

Table 42: Modification of antisense strand

| No. | Antisense strand (AS) 5'-3' | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| A | 2'-OMe +PS+EVP | 2'-F +PS | 2'-OMe | 2'-OMe | 2'-OMe | 2'-F | 2'-OMe | 2'-OMe | 2'-OMe | 2'-OMe | 2'-OMe | 2'-OMe |
| | 13 | 14 | 15 | 16 | 17 | 18 | 19 | 20 | 21 | 22 | 23 | |
| | 2'-OMe | 2'-F | 2'-OMe | 2'-F | 2'-OMe | 2'-OMe | 2'-OMe | 2'-OMe | 2'-OMe +PS | 2'-OMe +PS | 2'-OMe | |
| B | 1 | 2 | 3 | 4 | 5 | 6 | 7 | 8 | 9 | 10 | 11 | 12 |
| | 2'-OMe +PS+EVP | 2'-F +PS | 2'-F | 2'-F | 2'-OMe | 2'-F | 2'-OMe | 2'-OMe | 2'-OMe | 2'-OMe | 2'-OMe | 2'-OMe |
| | 13 | 14 | 15 | 16 | 17 | 18 | 19 | 20 | 21 | 22 | 23 | |
| | 2'-OMe | 2'-F | 2'-OMe | 2'-F | 2'-OMe | 2'-OMe | 2'-OMe | 2'-OMe | 2'-OMe +PS | 2'-OMe +PS | 2'-OMe | |
| C | 1 | 2 | 3 | 4 | 5 | 6 | 7 | 8 | 9 | 10 | 11 | 12 |
| | 2'-OMe +PS+EVP | 2'-F +PS | 2'-OMe | 2'-F | 2'-F | 2'-F | 2'-OMe | 2'-OMe | 2'-OMe | 2'-OMe | 2'-OMe | 2'-OMe |
| | 13 | 14 | 15 | 16 | 17 | 18 | 19 | 20 | 21 | 22 | 23 | |
| | 2'-OMe | 2'-F | 2'-OMe | 2'-F | 2'-OMe | 2'-OMe | 2'-OMe | 2'-OMe | 2'-OMe +PS | 2'-OMe +PS | 2'-OMe | |
| D | 1 | 2 | 3 | 4 | 5 | 6 | 7 | 8 | 9 | 10 | 11 | 12 |
| | 2'-OMe +PS+EVP | 2'-F +PS | 2'-OMe | 2'-OMe | 2'-F | 2'-F | 2'-OMe | 2'-OMe | 2'-OMe | 2'-OMe | 2'-OMe | 2'-OMe |
| | 13 | 14 | 15 | 16 | 17 | 18 | 19 | 20 | 21 | 22 | 23 | |
| | 2'-OMe | 2'-F | 2'-OMe | 2'-F | 2'-OMe | 2'-OMe | 2'-OMe | 2'-OMe | 2'-OMe +PS | 2'-OMe +PS | 2'-OMe | |
| E | 1 | 2 | 3 | 4 | 5 | 6 | 7 | 8 | 9 | 10 | 11 | 12 |
| | 2'-OMe +PS+EVP | 2'-F +PS | 2'-F | 2'-OMe | 2'-F | 2'-F | 2'-OMe | 2'-OMe | 2'-OMe | 2'-OMe | 2'-OMe | 2'-OMe |
| | 13 | 14 | 15 | 16 | 17 | 18 | 19 | 20 | 21 | 22 | 23 | |
| | 2'-OMe | 2'-F | 2'-OMe | 2'-F | 2'-OMe | 2'-OMe | 2'-OMe | 2'-OMe | 2'-OMe +PS | 2'-OMe +PS | 2'-OMe | |
| F | 1 | 2 | 3 | 4 | 5 | 6 | 7 | 8 | 9 | 10 | 11 | 12 |
| | 2'-OMe +PS+EVP | 2'-F +PS | 2'-F | 2'-OMe | 2'-OMe | 2'-F | 2'-OMe | 2'-OMe | 2'-OMe | 2'-OMe | 2'-OMe | 2'-OMe |
| | 13 | 14 | 15 | 16 | 17 | 18 | 19 | 20 | 21 | 22 | 23 | |
| | 2'-OMe | 2'-F | 2'-OMe | 2'-F | 2'-OMe | 2'-OMe | 2'-OMe | 2'-OMe | 2'-OMe +PS | 2'-OMe +PS | 2'-OMe | |

The sense strand is modified according to one of the modification patterns shown in Table 43:

Table 43: Modification of sense strand

| No. | Sense strand (SS) 5'-3' | | | | | | | | | | |
|---|---|---|---|---|---|---|---|---|---|---|---|
| | **1** | **2** | **3** | **4** | **5** | **6** | **7** | **8** | **9** | **10** | **11** |
| **a** | 2'-OMe+PS | 2'-OMe+PS | 2'-OMe | 2'-OMe | 2'-OMe | 2'-OMe | **2'-F** | 2'-OMe | **2'-F** | **2'-F** | **2'-F** |
| | **12** | **13** | **14** | **15** | **16** | **17** | **18** | **19** | **20** | **21** | |
| | 2'-OMe | 2'-OMe | 2'-OMe | 2'-OMe | 2'-OMe | **2'-F** | 2'-OMe | 2'-OMe | 2'-OMe | 2'-OMe | |
| **b** | **1** | **2** | **3** | **4** | **5** | **6** | **7** | **8** | **9** | **10** | **11** |
| | 2'-OMe+PS | 2'-OMe+PS | 2'-OMe | 2'-OMe | 2'-OMe | 2'-OMe | **2'-F** | 2'-OMe | **2'-F** | 2'-OMe | **2'-F** |
| | **12** | **13** | **14** | **15** | **16** | **17** | **18** | **19** | **20** | **21** | |
| | 2'-OMe | 2'-OMe | 2'-OMe | 2'-OMe | 2'-OMe | **2'-F** | 2'-OMe | 2'-OMe | 2'-OMe | 2'-OMe | |

In the above tables, 2'-OMe represents 2'-O-methyl; 2'-F represents 2'-fluoro; PS represents a phosphorothioate backbone;

wherein the antisense strand is modified according to modification pattern A, and the sense strand is modified according to modification pattern a;
wherein the antisense strand is modified according to modification pattern B, and the sense strand is modified according to modification pattern a;
wherein the antisense strand is modified according to modification pattern C, and the sense strand is modified according to modification pattern a;
wherein the antisense strand is modified according to modification pattern B, and the sense strand is modified according to modification pattern b;
wherein the antisense strand is modified according to modification pattern C, and the sense strand is modified according to modification pattern b;
wherein the antisense strand is modified according to modification pattern D, and the sense strand is modified according to modification pattern b;
wherein the antisense strand is modified according to modification pattern E, and the sense strand is modified according to modification pattern b;
wherein the antisense strand is modified according to modification pattern F, and the sense strand is modified according to modification pattern b.

In some embodiments, the aforementioned oligonucleotide comprises the following modifications: the antisense strand is modified according to one of the modification patterns shown in Table 44:

Table 44: Modification of antisense strand

| No. | Antisense strand (AS) 5'-3' | | | | | | | | | | |
|---|---|---|---|---|---|---|---|---|---|---|---|
| | **1** | **2** | **3** | **4** | **5** | **6** | **7** | **8** | **9** | **10** | **11** |
| **A** | 2'-OMe +PS+EVP | **2'-F +PS** | 2'-OMe | 2'-OMe | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **B** | **1** | **2** | **3** | **4** | **5** | **6** | **7** | **8** | **9** | **10** | **11** |
| | 2'-OMe +PS+EVP | **2'-F +PS** | **2'-F** | **2'-F** | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **C** | **1** | **2** | **3** | **4** | **5** | **6** | **7** | **8** | **9** | **10** | **11** |
| | 2'-OMe +PS+EVP | **2'-F +PS** | 2'-OMe | **2'-F** | **2'-F** | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **D** | **1** | **2** | **3** | **4** | **5** | **6** | **7** | **8** | **9** | **10** | **11** |
| | 2'-OMe +PS+EVP | **2'-F +PS** | 2'-OMe | 2'-OMe | **2'-F** | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **E** | **1** | **2** | **3** | **4** | **5** | **6** | **7** | **8** | **9** | **10** | **11** |
| | 2'-OMe +PS+EVP | **2'-F +PS** | **2'-F** | 2'-OMe | **2'-F** | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| F | **1** | **2** | **3** | **4** | **5** | **6** | **7** | **8** | **9** | **10** | **11** |
| | 2'-OMe +PS+EVP | **2'-F +PS** | **2'-F** | 2'-OMe | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |

The sense strand is modified according to one of the modification patterns shown in Table 45:

Table 45: Modification of sense strand

| No. | **Sense strand (SS) 5'-3'** | | | | | | | | | |
|---|---|---|---|---|---|---|---|---|---|---|
| | **1** | **2** | **3** | **4** | **5** | **6** | **7** | **8** | **9** | **10** |
| **a** | 2'-OMe+PS | 2'-OMe+PS | 2'-OMe | 2'-OMe | 2'-OMe | **2'-F** | 2'-OMe | **2'-F** | **2'-F** | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | **2'-F** | 2'-OMe | 2'-OMe | 2'-OMe | 2'-OMe |
| **b** | **1** | **2** | **3** | **4** | **5** | **6** | **7** | **8** | **9** | **10** |
| | 2'-OMe+PS | 2'-OMe+PS | 2'-OMe | 2'-OMe | 2'-OMe | **2'-F** | 2'-OMe | **2'-F** | 2'-OMe | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | **2'-F** | 2'-OMe | 2'-OMe | 2'-OMe | 2'-OMe |

In the above tables, 2'-OMe represents 2'-O-methyl; 2'-F represents 2'-fluoro; PS represents a phosphorothioate backbone;

wherein the antisense strand is modified according to modification pattern A, and the sense strand is modified according to modification pattern a;
wherein the antisense strand is modified according to modification pattern B, and the sense strand is modified according to modification pattern a;
wherein the antisense strand is modified according to modification pattern C, and the sense strand is modified according to modification pattern a;
wherein the antisense strand is modified according to modification pattern B, and the sense strand is modified according to modification pattern b;
wherein the antisense strand is modified according to modification pattern C, and the sense strand is modified according to modification pattern b;
wherein the antisense strand is modified according to modification pattern D, and the sense strand is modified according to modification pattern b;
wherein the antisense strand is modified according to modification pattern E, and the sense strand is modified according to modification pattern b;
wherein the antisense strand is modified according to modification pattern F, and the sense strand is modified according to modification pattern b.

In some embodiments, the antisense strand comprises a modification group at positions 2 to 8 from the 5' end, wherein the modification group is selected from UNA, GNA, or DNA, wherein the structures of UNA and GNA are as follows:

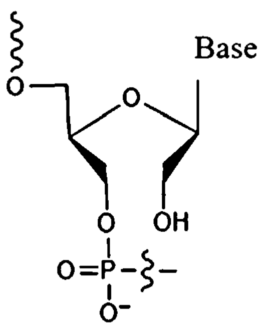

Unlocked nucleic acid (UNA)

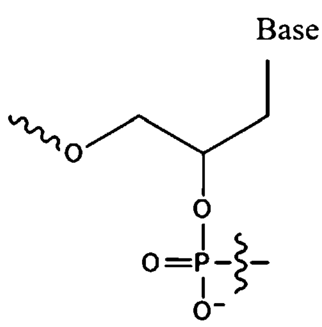

Glycol nucleic acid (GNA)

;

wherein the base is selected from adenine, guanine, cytosine, thymine, and uracil.

In some embodiments, the phosphorylation of the 5'-carbon atom of the 5'-terminal nucleotide glycoside of a

modified antisense strand includes, but is not limited to, the following 5'-phosphorylation groups: 5'-vinylphosphonate group (5'-E-VP); 5'-methylphosphonate group (5'-MP); 5'-C-methylphosphate group; 5'-phosphorothioate group (5'-PS); 5'-phosphate group (5'-P), the structures of which are as follows:

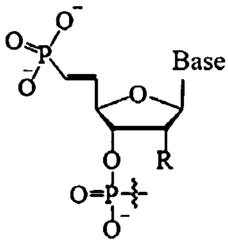
5'-vinylphosphonate group (5'-E-VP)

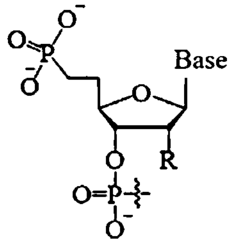
5'-methylphosphonate group (5'-MP)

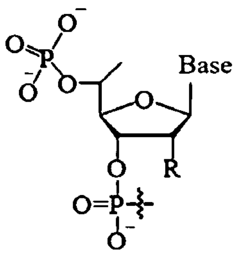
5'-C-methylphosphate group

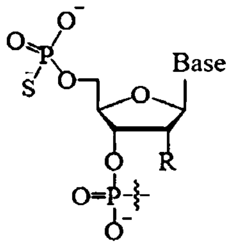
5'-phosphorothioate group (5'-PS)

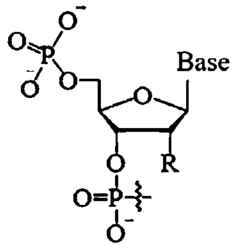
5'-phosphate group (5'-P)

;

wherein R is hydrogen, hydroxyl, amino, $C_{1-4}$ alkyl, aryl, $C_{1-4}$ alkoxy, $C_{1-4}$ alkylcarbonylamino, or halogen; the base is selected from adenine, guanine, cytosine, thymine, and uracil.

In some embodiments, the 3',5'-phosphodiester bond between nucleotide monomers at the terminus of the sequence is phosphorothioate-modified to form a chirally pure 3',5'-phosphorothioate bond, wherein the 5' ends of both the sense and antisense strands contain 1 to 3 phosphorothioate linkages, and the 3' end of the antisense strand contains 1 to 3 phosphorothioate linkages.

The double-stranded RNA (dsRNA) agent of the present disclosure may optionally be conjugated to one or more ligands. The ligand may be attached to a sense strand, an antisense strand, or both strands at the 3' end, the 5' end, or both ends. For example, the ligand may be conjugated to the sense strand. In a preferred example, the ligand is attached to the 3' end of the sense strand. In a preferred example, the ligand is a GalNAc ligand.

The present disclosure provides a conjugate for reducing the expression of AGT, comprising the double-stranded RNAi agent and a ligand conjugated thereto.

In some embodiments, the ligand is conjugated to the 3' end or the 5' end of the sense strand of the oligonucleotide.

In some embodiments, the ligand is one or more GalNAc derivatives attached via a bivalent or trivalent branched linker.

In some embodiments, the ligand is as follows:

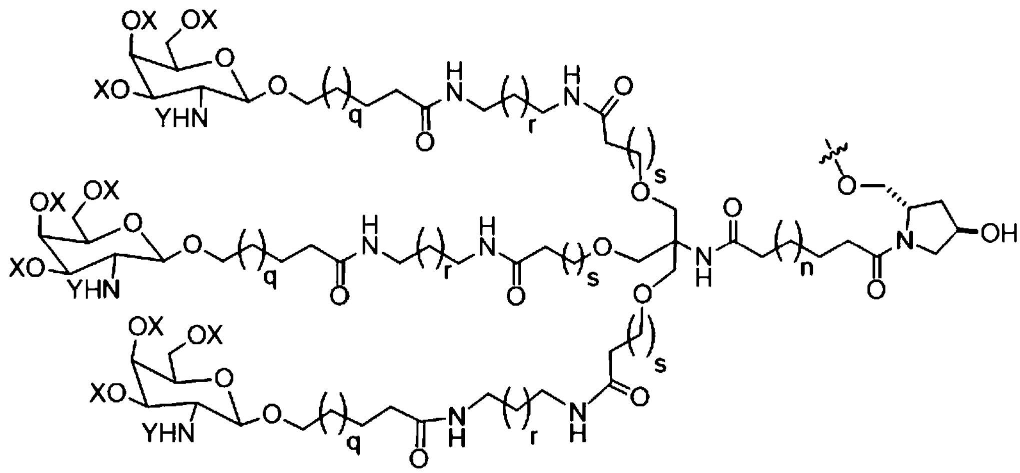

,

wherein X is hydrogen, a hydroxyl protecting group, or H, the hydroxyl protecting group including acetyl, benzoyl, or isobutyryl; Y is an amino protecting group or H, the amino protecting group being formyl, acetyl, propionyl, n-butyryl, or isobutyryl; n is an integer from 0 to 20; q, r, and s are independently integers from 1 to 7.

In some embodiments, the ligand is as follows:

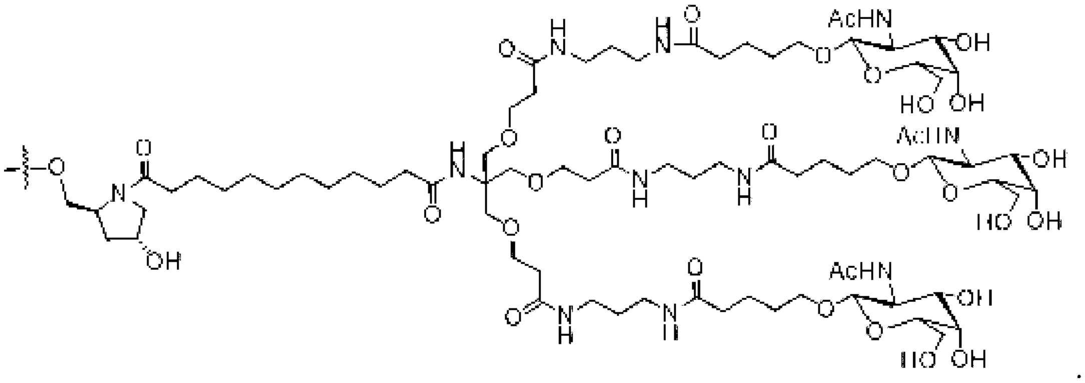

In some embodiments, the ligand is as follows:

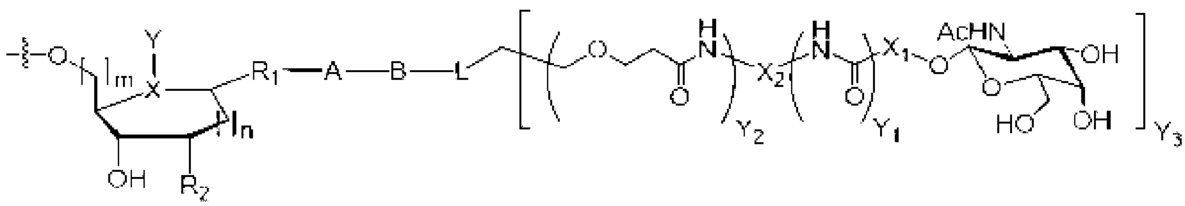

wherein X is oxygen, nitrogen, or sulfur;
Y is alkyl or aryl;
$R_1$ is oxygen or sulfur;
$R_2$ is hydrogen, amino, $C_{1-4}$ alkyl, aryl, $C_{1-4}$ alkoxy, or halogen;
A is $-(CH_2)_a-$, $-(CH_2CH_2O)_b-$, $-((CH_2)_cNHCO)_d-$, or $-((CH_2)_cCONH)_d-$, wherein a is an integer from 1 to 15, b is an integer from 1 to 7, c is an integer from 1 to 7, and d is an integer from 1 to 5;
B is $-(CH_2)_e-$, wherein e is an integer from 0 to 7;
L is -CONH- or -NHCO-;
$X_1$ is $-(CH_2)_f-$ or $-(CH_2CH_2O)_fCH_2-$, wherein f is an integer from 1 to 5;
$X_2$ is $-(CH_2)_g-$, wherein g is an integer from 1 to 6;
$Y_1$ is 0 or 1;
$Y_2$ is 0, 1, or 2;
$Y_3$ is 1, 2, or 3;
m is an integer from 0 to 4;
n is an integer from 0 to 4.

In some embodiments, the ligand is G4, G5, G6, or G7:

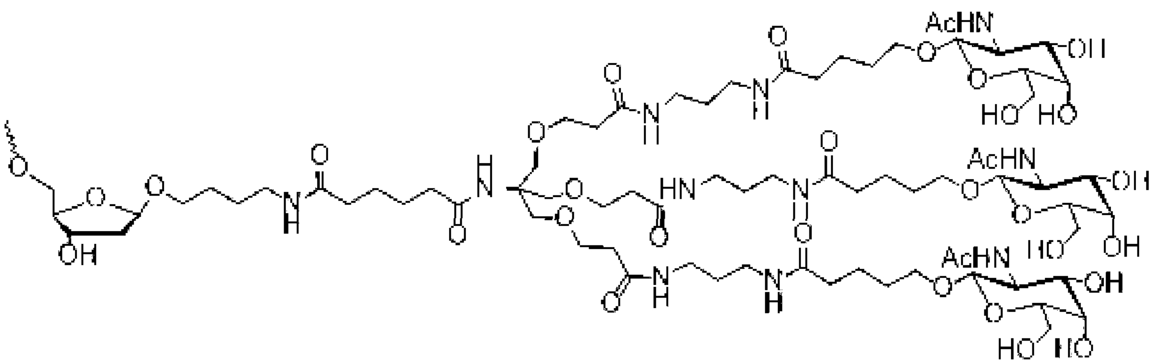

G4

,

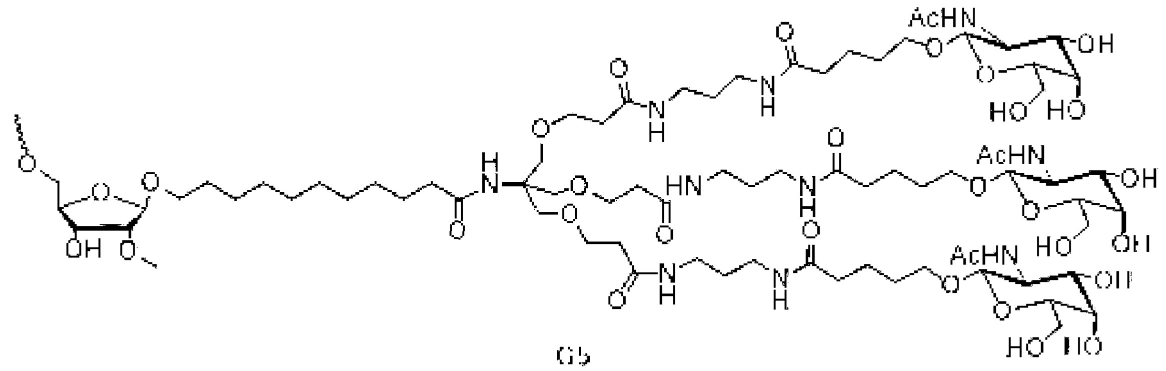

G5

,

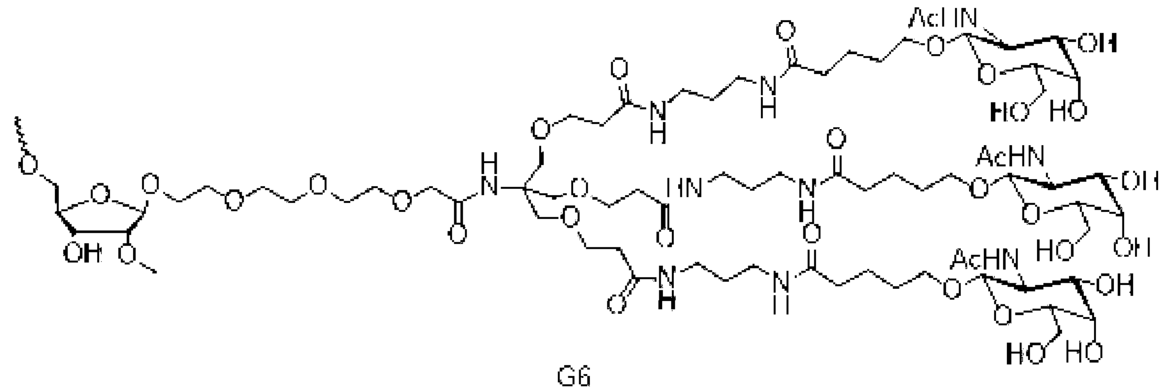

G6

,

or

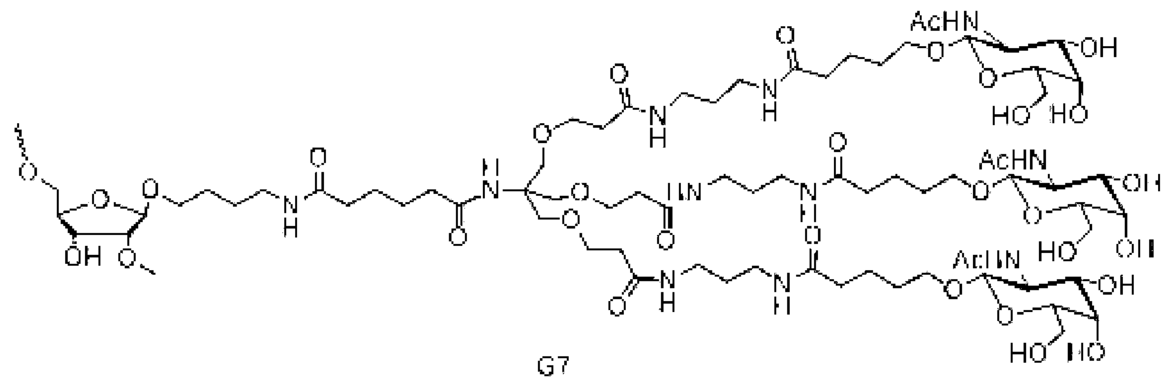

G7

.

In some embodiments, the conjugate has a structure as shown below:

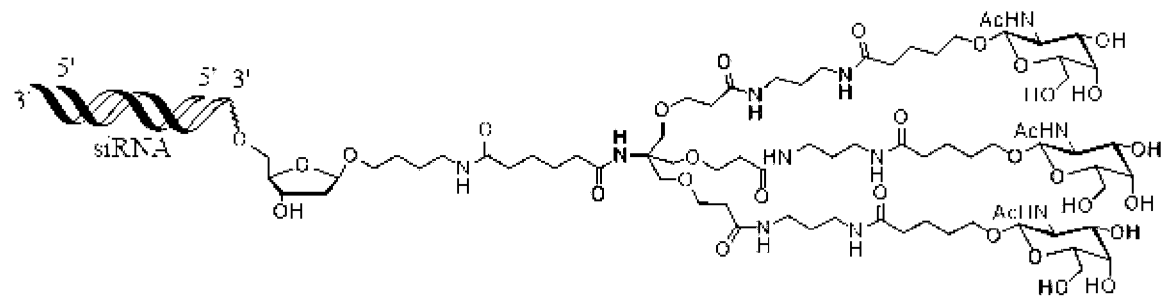

,

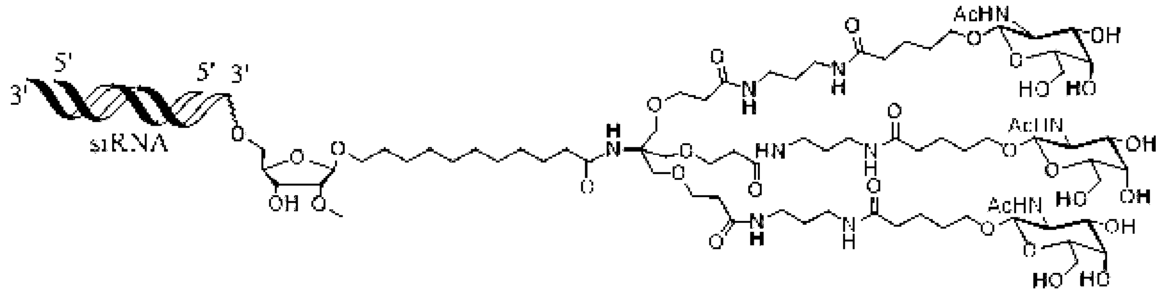

,

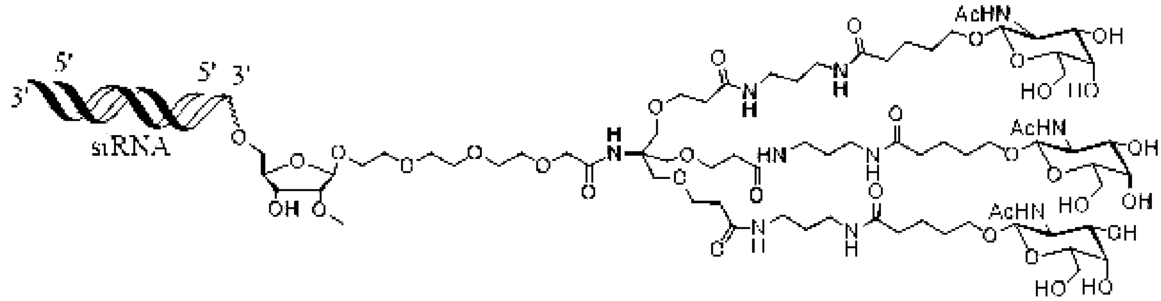

,

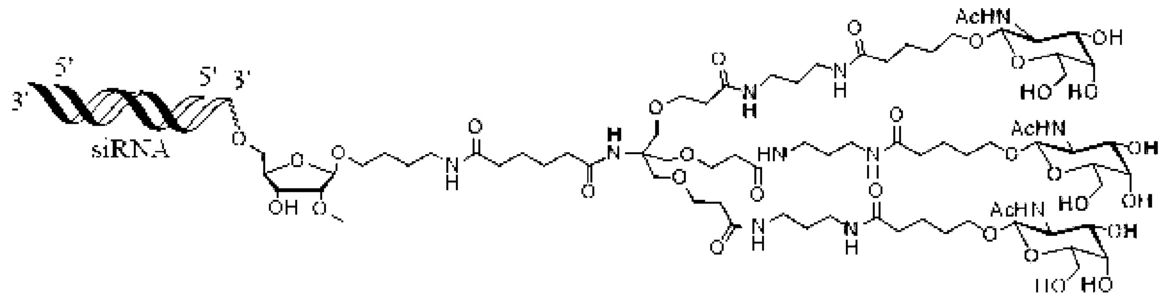

,

or

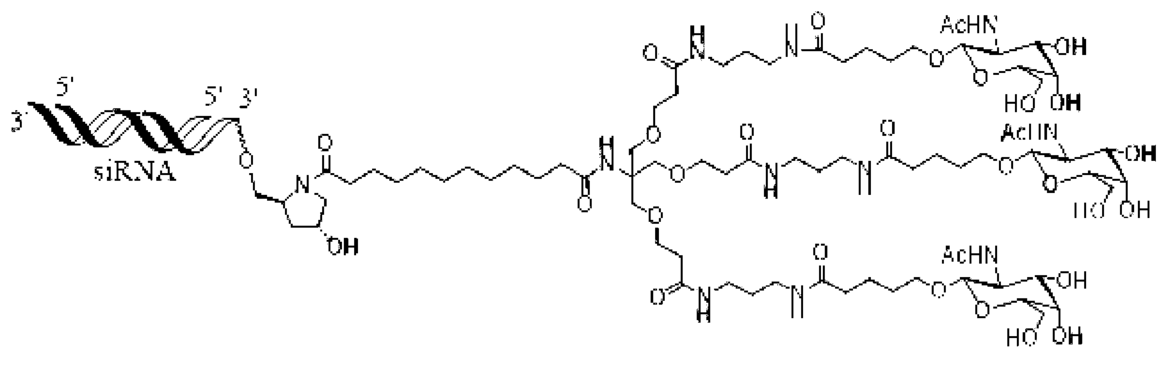

.

In some embodiments, the double-stranded RNAi agent comprises any one oligonucleotide duplex selected from the following pairings of a sense strand and an antisense strand:

(1) the sense strand has the sequence shown in SEQ ID NO: 423; and the antisense strand has the sequence shown in SEQ ID NO: 488;
(2) the sense strand has the sequence shown in SEQ ID NO: 406; and the antisense strand has the sequence shown in SEQ ID NO: 453;
(3) the sense strand has the sequence shown in SEQ ID NO: 404; and the antisense strand has the sequence shown in SEQ ID NO: 450;
(4) the sense strand has the sequence shown in SEQ ID NO: 426; and the antisense strand has the sequence shown in

SEQ ID NO: 492;
(5) the sense strand has the sequence shown in SEQ ID NO: 412; and the antisense strand has the sequence shown in SEQ ID NO: 465;
(6) the sense strand has the sequence shown in SEQ ID NO: 412; and the antisense strand has the sequence shown in SEQ ID NO: 468;
(7) the sense strand has the sequence shown in SEQ ID NO: 421; and the antisense strand has the sequence shown in SEQ ID NO: 483;
(8) the sense strand has the sequence shown in SEQ ID NO: 417; and the antisense strand has the sequence shown in SEQ ID NO: 476;
(9) the sense strand has the sequence shown in SEQ ID NO: 419; and the antisense strand has the sequence shown in SEQ ID NO: 479;
(10) the sense strand has the sequence shown in SEQ ID NO: 401; and the antisense strand has the sequence shown in SEQ ID NO: 443;
(11) the sense strand has the sequence shown in SEQ ID NO: 398; and the antisense strand has the sequence shown in SEQ ID NO: 433;
(12) the sense strand has the sequence shown in SEQ ID NO: 429; and the antisense strand has the sequence shown in SEQ ID NO: 496;
(13) the sense strand has the sequence shown in SEQ ID NO: 414; and the antisense strand has the sequence shown in SEQ ID NO: 471;

wherein the oligonucleotide duplex is conjugated with ligand G4, G5, G6, or G7.

In some embodiments, the double-stranded RNAi agent comprises any one oligonucleotide duplex selected from the following pairings of a sense strand and an antisense strand:

(1) the sense strand has the sequence shown in SEQ ID NO: 423; and the antisense strand has the sequence shown in SEQ ID NO: 488;
(2) the sense strand has the sequence shown in SEQ ID NO: 423; and the antisense strand has the sequence shown in SEQ ID NO: 489;
(3) the sense strand has the sequence shown in SEQ ID NO: 424; and the antisense strand has the sequence shown in SEQ ID NO: 489;
(4) the sense strand has the sequence shown in SEQ ID NO: 424; and the antisense strand has the sequence shown in SEQ ID NO: 490;
(5) the sense strand has the sequence shown in SEQ ID NO: 424; and the antisense strand has the sequence shown in SEQ ID NO: 488;

wherein the oligonucleotide duplex is conjugated with ligand G4, G5, G6, or G7.

In some embodiments, the double-stranded RNAi agent comprises an oligonucleotide duplex formed by pairing of the sense strand shown in SEQ ID NO: 423 and the antisense strand shown in SEQ ID NO: 488, wherein the oligonucleotide duplex is conjugated with ligand G5.

The present disclosure also provides a pharmaceutical composition comprising the double-stranded RNAi agent or conjugate and a pharmaceutically acceptable carrier.

In one example, provided herein is a pharmaceutical composition comprising the iRNA as described herein and a pharmaceutically acceptable carrier. The iRNA-containing pharmaceutical composition may be used for treating a disease or disorder associated with the expression or activity of the AGT gene, such as hypertension. Such pharmaceutical compositions are formulated based on the mode of delivery. One example is a composition formulated for systemic administration via parenteral delivery, e.g., via subcutaneous injection (S.C.). Another example is a composition formulated for direct delivery into the brain parenchyma, e.g., via infusion into the brain, such as by continuous pump infusion.

The pharmaceutical composition comprising the RNAi agent of the present disclosure may be, for example, a solution with or without a buffer or a composition comprising a pharmaceutically acceptable carrier. Such compositions include, for example, aqueous or crystalline compositions, liposomal formulations, micellar formulations, emulsions, and gene therapy vectors.

In the methods of the present disclosure, the RNAi agent may be administered in a solution. A free RNAi agent may be administered in an unbuffered solution, e.g., in saline or in water. Alternatively, the free siRNA may also be administered in a suitable buffered solution. The buffered solution may comprise acetate, citrate, prolamin, carbonate, or phosphate, or any combination thereof. In a preferred example, the buffered solution is phosphate-buffered saline (PBS). The pH and osmolarity of the buffered solution comprising the iRNA agent may be adjusted such that it is suitable for administration to a subject.

In some examples, the buffered solution further comprises an agent for controlling the osmolarity of the solution, such that the osmolarity is maintained at a desired value, *e.g.,* at the physiological value of human plasma. Solutes that may be added to the buffered solution to control the osmolarity include, but are not limited to, proteins, peptides, amino acids, non-metabolized polymers, vitamins, ions, sugars, metabolites, organic acids, lipids, or salts. In some examples, the agent for controlling the osmolarity of the solution is a salt. In certain examples, the agent for controlling the osmolarity of the solution is sodium chloride or potassium chloride.

The pharmaceutical composition of the present disclosure may be administered at a dose sufficient to inhibit AGT gene expression. Generally, suitable doses of the iRNA of the present disclosure range from about 0.001 to about 200.0 milligrams per kilogram body weight of the recipient per day, typically ranging from about 1 to 50 mg per kilogram body weight per day. For example, the RNAi agent (e.g., dsRNA) may be administered at about 0.01, 0.05, 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 31, 32, 33, 34, 34, 35, 36, 37, 38, 39, 40, 41, 42, 43, 44, 45, 46, 47, 48, 49, or about 50 mg/kg per single dose.

The pharmaceutical composition may be administered once daily, or multiple times at various intervals over 1 to 365 days, or the iRNA may be administered as two, three, or more sub-doses at appropriate intervals within a year, or even using continuous infusion or delivery via a controlled-release formulation. In such cases, the amount of iRNA contained in each sub-dose must be proportionally smaller in order to achieve the total daily dose. Dose units may also be compounded for delivery over several days, e.g., using a conventional sustained-release formulation that provides sustained release of the iRNA over a period of days. Sustained-release formulations are well known in the art and are particularly useful for delivering agents to specific sites, and thus may be used with the agents of the present disclosure. In this embodiment, the dose unit contains a corresponding plurality of daily doses.

In other embodiments, a single dose of the pharmaceutical composition may be long-lasting, such that subsequent doses are administered at intervals of no more than 3, 4, or 5 days, or no more than 1, 2, 3, or 4 weeks. In some embodiments of the present disclosure, a single dose of the pharmaceutical composition of the present disclosure is administered once weekly. In other embodiments of the present disclosure, a single dose of the pharmaceutical composition of the present disclosure is administered once every two months.

Those skilled in the art will appreciate that certain factors may influence the dose and timing required to effectively treat a subject, including, but not limited to, the severity of the disease or disorder, prior treatments, the general health and/or age of the subject, and other existing diseases. Furthermore, treating a subject with a therapeutically effective dose of the composition may involve a single treatment or a series of treatments. As described elsewhere herein, the effective dose and *in vivo* half-life for each iRNA encompassed by the present disclosure may be estimated using conventional methods or on the basis of *in vivo* testing using suitable animal models.

The pharmaceutical composition of the present disclosure may be administered in various ways depending upon whether local or systemic treatment is desired and upon the area to be treated. Administration may be topical *(e.g.,* via a transdermal patch); pulmonary; *e.g.,* via inhalation or insufflation of a powder or aerosol, including via a nebulizer; intratracheal; intranasal; epidermal and transdermal, oral, or parenteral. Parenteral administration includes intravenous, intraarterial, subcutaneous, intraperitoneal, or intramuscular injection or infusion; subdermal administration, *e.g.,* via an implantable device; or intracranial administration, *e.g.,* intraparenchymal, intrathecal, or intraventricular administration.

The iRNA for use in the compositions and methods of the present disclosure may be formulated for delivery in a membranous molecular assembly, e.g., a liposome or a micelle. As used herein, the term "liposome" refers to a vesicle composed of amphipathic lipids arranged in at least one bilayer *(e.g.,* one bilayer or a plurality of bilayers). Liposomes include unilamellar or multilamellar vesicles having a membrane formed from a lipophilic material and an aqueous interior. The aqueous portion contains the iRNA composition. The lipophilic material isolates the aqueous interior from an aqueous exterior, which typically does not contain the iRNA composition (although in some examples, it may). Liposomes are useful for transferring and delivering active ingredients to sites of action. Because liposomal membranes are structurally similar to biological membranes, when liposomes are applied to a tissue, the bilayer of the liposome fuses with the bilayer of the cell membrane. As the fusion of the liposome with the cell proceeds, the internal aqueous contents, including the iRNA, are delivered into the cell, where the iRNA can specifically bind to a target RNA and can mediate RNAi. In some cases, these liposomes are also specifically targeted, *e.g.,* to direct the iRNA to particular cell types.

A liposome containing an RNAi agent can be prepared by a variety of methods. In one example, a lipid component of the liposome is dissolved in a detergent such that micelles are formed with the lipid component. For example, the lipid component may be an amphipathic cationic lipid or a lipid conjugate. The detergent may have a high critical micelle concentration and may be nonionic. Exemplary detergents include cholate, CHAPS, octyl glucoside, deoxycholate, and lauroyl sarcosinate. The RNAi agent preparation is then added to the micelles comprising the lipid component. The cationic groups on the lipid interact with the RNAi agent and condense around the RNAi agent to form a liposome. After

condensation, the detergent is removed, e.g., by dialysis, to obtain a liposomal preparation of the RNAi agent.

The iRNA, *e.g.,* the dsRNA of the present disclosure, may be fully encapsulated in a lipid formulation (e.g., a lipid nanoparticle (LNP) or other nucleic acid-lipid particle).

As used herein, the term "LNP" refers to a stable nucleic acid-lipid particle. The LNP comprises a cationic lipid, a non-cationic lipid, and a lipid that prevents aggregation of the particle *(e.g.,* a PEG-lipid conjugate). LNPs are extremely useful for synthetic applications, as they exhibit prolonged circulation lifetimes following intravenous (i.v.) injection and accumulate at distal sites *(e.g.,* sites physically separated from the administration site).

In one example, the lipid-to-drug ratio (mass/mass ratio) (e.g., lipid-to-dsRNA ratio) ranges from about 1:1 to about 50:1, from about 1:1 to about 25:1, from about 3:1 to about 15:1, from about 4:1 to about 10:1, from about 5:1 to about 9:1, or from about 6:1 to about 9:1.

In some preferred embodiments, the lipid nanoparticle comprise a cationic lipid, a neutral lipid, a structured lipid, and a polymer-conjugated lipid.

In some preferred embodiments, the cationic lipid is a compound with the structure of formula (I), or an N-oxide, a solvate, a pharmaceutically acceptable salt, or a stereoisomer thereof, wherein $G_1$ is $C_{1-6}$ alkylene; $G_2$ is $C_{2-8}$ alkylene; $G_3$ is $C_{1-3}$ alkylene; $L_1$ is $C_{6-15}$ linear alkyl; $L_2$ is $C_{12-25}$ branched alkyl. For example, YK-009 with the structure of formula (I-I) (see patent CN114044741B).

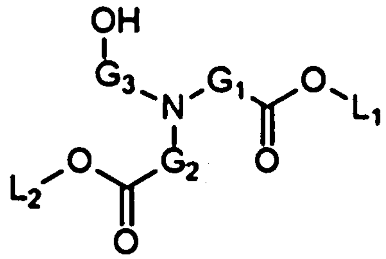

(I)

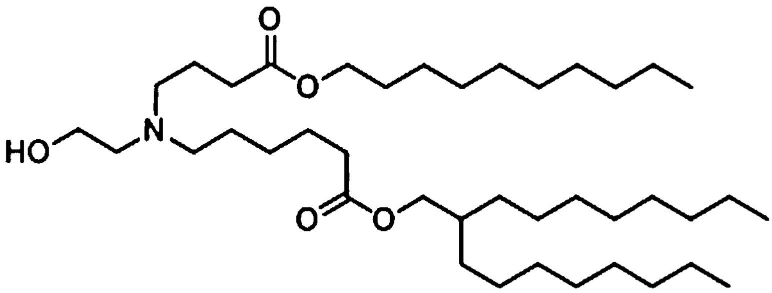

YK-009

(I-I)

In some preferred embodiments, the cationic lipid is a compound with the structure of formula (II), or an N-oxide, a solvate, a pharmaceutically acceptable salt, or a stereoisomer thereof, wherein $G_1$ is $C_{2-8}$ alkylene; $G_2$ is $C_{2-8}$ alkylene; $L_1$ is -C(O)O- or -OC(O)-; $L_2$ is -C(O)O- or - OC(O)-; $R_1$ is $C_{6-25}$ linear or branched alkyl; $R_2$ is $C_{6-25}$ linear or branched alkyl; $G_3$ is $HO(CH_2)_2$- or $HO(CH_2)_3$-; $G_4$ is $HO(CH_2)_2$- or $HO(CH_2)_3$-; L is $(CH_2)_2$-, $-(CH_2)_3$-, or $-(CH_2)_4$-. For example, YK-401 with the structure of formula (II-I) or YK-402 with the structure of formula (II-II) (see patent CN115784921B).

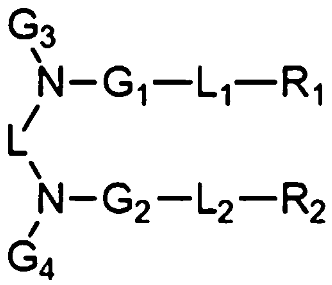

(II)

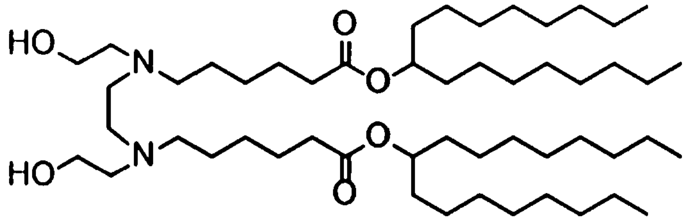

YK-401

(II-I)

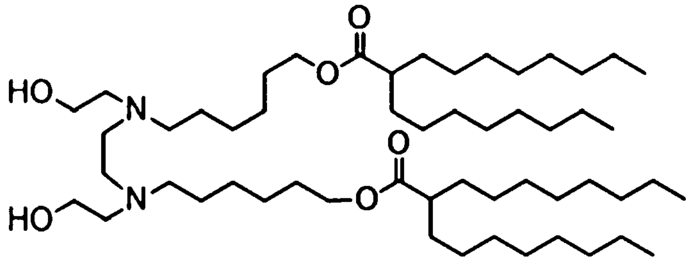

YK-402

(II-II)

In some preferred embodiments, the cationic lipid is a compound with the structure of formula (III), or an N-oxide, a solvate, a pharmaceutically acceptable salt, or a stereoisomer thereof, wherein $G_1$ is $C_{1-6}$ alkylene; $G_2$ is $C_{2-8}$ alkylene; $R_1$ is $C_{6-20}$ linear or branched alkyl; $R_2$ is $C_{12-25}$ branched alkyl; $G_3$ is $HO(CH_2)_2N(CH_3)(CH_2)_2$-, $HO(CH_2)_2N(CH_2CH_3)(CH_2)_2$-, $(HO(CH_2)_2)_2N(CH_2)_2$-, $CH_3O(CH_2)_2N(CH_3)(CH_2)_2$-, $(CH_3)_2N(CH_2)_3SC(O)O(CH_2)_2$-, $(CH_3)_2N(CH_2)_3SC(O)$-, $CH_3NH(CH_2)_2N(CH_3)(CH_2)_2$-, or $CH_3CH_2NH(CH_2)_2$-. For example, YK-201 with the structure of formula (III-I) or YK-202 with the structure of formula (III-II) (see patent CN115677518B).

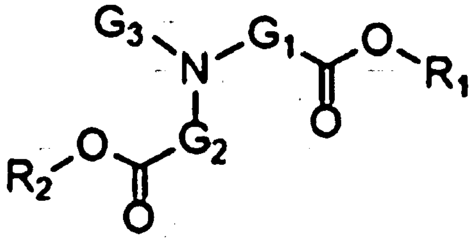

(III)

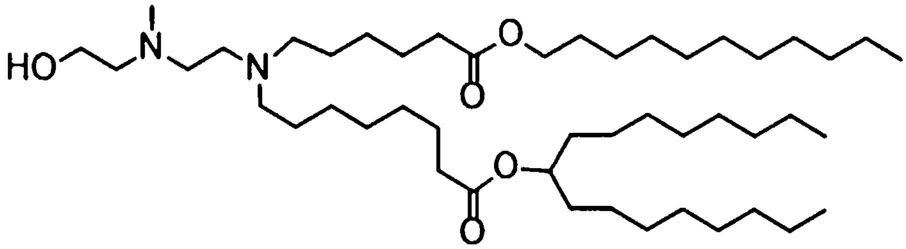

YK-201

(III-I)

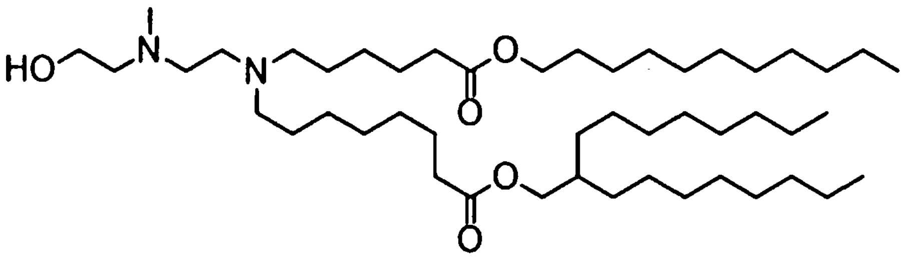

YK-202

(III-II)

In some preferred embodiments, the cationic lipid is a compound with the structure of formula (IV), or an N-oxide, a solvate, a pharmaceutically acceptable salt, or a stereoisomer thereof, wherein $G_1$ is $C_{1-8}$ alkylene; $G_2$ is $C_{2-8}$ alkylene; $R_1$ is $C_{6-25}$ linear or branched alkyl; $R_2$ is $C_{12-25}$ linear or branched alkyl; $G_3$ is $HO(CH_2)_2N(R_3)CH_2CH(OH)CH_2$-, wherein $R_3$ is $-CH_3$, $-CH_2CH_3$, or $-CH_2CH_2OH$. For example, YK-305 with the structure of formula (IV-I) or YK-310 with the structure of formula (IV-II) (see patent CN115745820B).

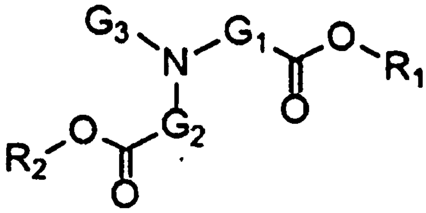

(IV)

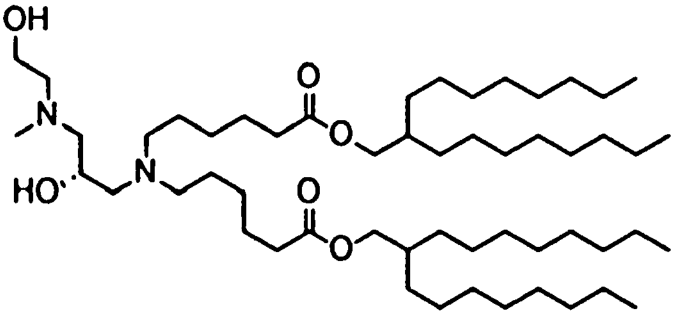

YK-305

(IV-I)

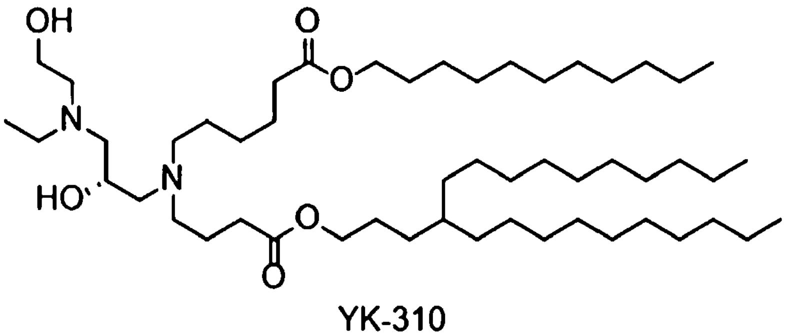

YK-310

(IV-II)

In some preferred embodiments, the cationic lipid is a compound with the structure of formula (V), or an N-oxide, a solvate, a pharmaceutically acceptable salt, or a stereoisomer thereof, wherein $G^1$ and $G^2$ are each independently unsubstituted $C_6$-$C_{10}$ alkylene; $G^3$ is unsubstituted $C_1$-$C_{12}$ alkylene; $R^1$ and $R^2$ are each independently $C_6$-$C_{24}$ alkyl or $C_6$-$C_{24}$ alkenyl; $R^3$ is $OR^5$, N, $-C(=O)OR^4$, $-OC(=O)R^4$, or $-NR^5C(=O)R^4$; $R^4$ is $C_1$-$C_{12}$ hydrocarbyl; and $R^5$ is H or $C_1$-$C_6$ hydrocarbyl; for example, ALC0315 with the structure of formula (V-I) (see patent CN108368028B).

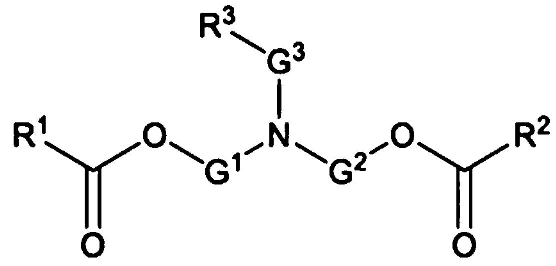

(V)

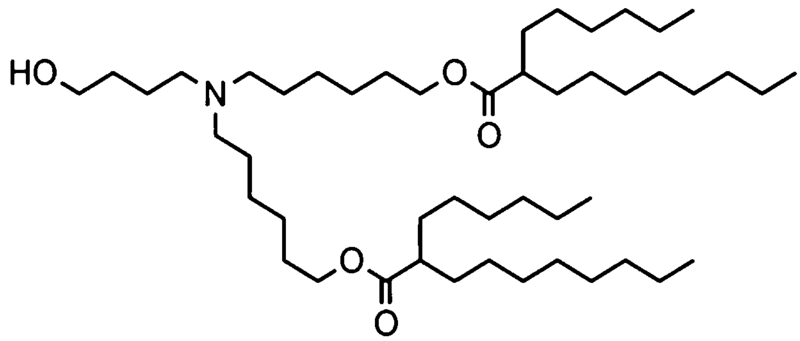

(V-I)

In some preferred embodiments, the cationic lipid is a compound with the structure of formula (VI), or an N-oxide, a solvate, a pharmaceutically acceptable salt, or a stereoisomer thereof, wherein $R_4$ is selected from $-(CH_2)_nQ$ and $-(CH_2)_nCHQR$; Q is selected from the group consisting of -OR, -OH, $-O(CH_2)_nN(R)_2$, -OC(O)R, $-CX_3$, -CN, -N(R)C(O)R, -N(H)C(O)R, $-N(R)S(O)_2R$, $-N(H)S(O)_2R$, $-N(R)C(O)N(R)_2$, $-N(H)C(O)N(R)_2$, -N(H)C(O)N(H)(R), $-N(R)C(S)N(R)_2$, $-N(H)C(S)N(R)_2$, -N(H)C(S)N(H)(R), $-N(R)S(O)_2R_8$, and heterocycle; n is 1, 2, or 3; for example, SM102 with the structure of formula (VI-I) (see patent CN110520409A).

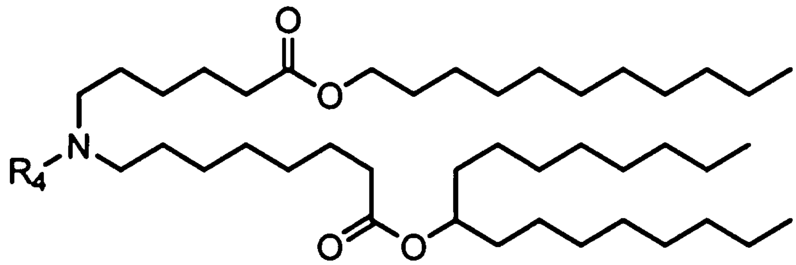

(VI)

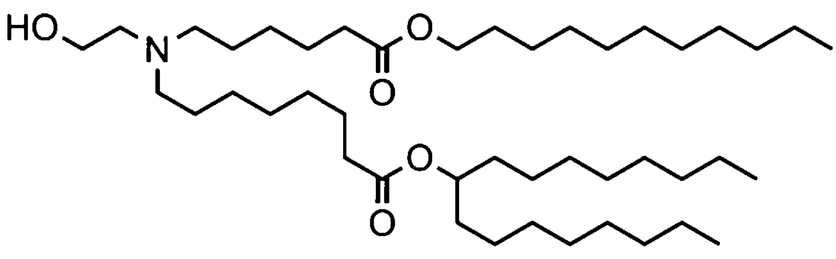

(VI-I)

In some preferred embodiments, the cationic lipid is a compound with the structure of formula (VII), or an N-oxide, a solvate, a pharmaceutically acceptable salt, or a stereoisomer thereof (see patent CN102625696B, DLIN-MC3-DMA),

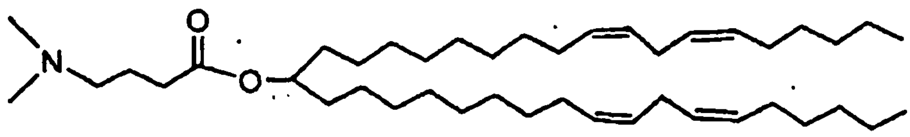

(VII).

In some more preferred embodiments, the cationic lipid comprises YK-009, YK-401, YK-305, ALC0315, SM102, and DLIN-MC3-DMA.

In some preferred embodiments, the molar ratio of the cationic lipid to the neutral lipid is 1:1 to 10:1.

In some preferred embodiments, the molar ratio of the cationic lipid to the structured lipid is 1:1 to 5:1.

In some preferred embodiments, the molar ratio of the cationic lipid, the neutral lipid, the structured lipid, and the polymer-conjugated lipid is (25 to 65):(5 to 25):(25 to 70):(0.5 to 5).

In some preferred embodiments, the molar ratio of the cationic lipid, the neutral lipid, the structured lipid, and the polymer-conjugated lipid is (25 to 65):(5 to 25):(25 to 45):(0.5 to 5).

In some more preferred embodiments, the molar ratio of the cationic lipid, the neutral lipid, the structured lipid, and the polymer-conjugated lipid is 50:10:38.5:1.5 or 49:10:39.5:1.5.

In some preferred embodiments, the neutral lipid includes one or more of phosphatidylcholine, phosphatidylethanolamine, sphingomyelin, ceramide, sterol, and derivatives thereof.

In some more preferred embodiments, the neutral lipid is selected from one or more of the following: 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), dipalmitoyl phosphatidylglycerol (DPPG), palmitoyl oleoyl phosphatidylethanolamine (POPE), distearoyl-phosphatidyl-ethanolamine (DSPE), dipalmitoyl phosphatidylethanolamine (DPPE), dimyristoyl phosphoethanolamine (DMPE), 1-stearoyl-2-oleoyl-stearoylethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyl oleoyl phosphatidylcholine, lysophosphatidylcholine, lysophosphatidylethanolamine (LPE), and mixtures thereof.

In some more preferred embodiments, the neutral lipid is DOPE and/or DSPC.

In some preferred embodiments, the structured lipid is selected from one or more of the following: cholesterol, nonsterol, sitosterol, ergosterol, campesterol, stigmasterol, brassinosterol, tomatine, ursolic acid, $\alpha$-tocopherol, or corticosteroid.

In some more preferred embodiments, the structured lipid is cholesterol.

In some preferred embodiments, the polymer-conjugated lipid is selected from one or more of PEG-modified phosphatidylethanolamine, PEG-modified phosphatidic acid, PEG-modified ceramide, PEG-modified dialkylamine, PEG-modified diacylglycerol, or PEG-modified dialkylglycerol.

In some more preferred embodiments, the polymer-conjugated lipid is selected from one or more of distearoyl phosphatidylethanolamine polyethylene glycol 2000 (DSPE-PEG2000), dimyristoylglycero-3-methoxypolyethylene glycol 2000 (DMG-PEG2000), or methoxypolyethylene glycol ditetradecylacetamide (ALC-0159).

The pharmaceutical composition of the present disclosure includes, but is not limited to, solutions, emulsions, and liposome-containing formulations. These compositions can be derived from a variety of components, including, but not limited to, preformed liquids, self-emulsifying solids, and self-emulsifying semisolids. Particularly preferred are formulations targeting the liver when treating hepatic disorders (e.g., hepatocellular carcinoma).

The pharmaceutical formulation of the present disclosure (which may conveniently be in unit dosage form) can be prepared according to conventional techniques well known in the pharmaceutical industry. Such techniques include the step of bringing into association the active ingredients with the pharmaceutical carrier(s) or excipient(s). Generally, the formulations are prepared by uniformly and intimately bringing into association the active ingredients with a liquid carrier or a finely divided solid carrier or both, and then, if necessary, shaping the product.

The composition of the present disclosure can be formulated into any of many possible dosage forms such as, but not limited to, tablets, capsules, gel capsules, liquid syrups, softgels, suppositories, and enemas. The composition of the present disclosure can also be formulated as a suspension in aqueous, non-aqueous, or mixed media. Aqueous suspensions may further comprise substances that increase the viscosity of the suspension, including, for example, sodium carboxymethylcellulose, sorbitol, and/or dextran. The suspension may also comprise a stabilizer.

Certain compositions of the present disclosure further incorporate a carrier compound into the formulation. As used herein, "carrier compound" or "carrier" may refer to a nucleic acid or analog thereof that is inert (*i.e.*, lacks biological activity per se) but is recognized as a nucleic acid by *in vivo* processes that reduce the bioavailability of a biologically active nucleic acid by, for example, degrading the biologically active nucleic acid or facilitating its removal from circulation. Co-administration of a nucleic acid and a carrier compound (typically in excess of the latter) can result in a substantial reduction in the amount of nucleic acid recovered from the liver, kidney, or other extracirculatory reservoirs, presumably due to competition between the carrier compound and the nucleic acid for a common receptor. For example, co-administration with polyinosinic acid, dextran sulfate, polycytidylic acid, or 4-acetamido-4'-isothiocyano-stilbene-2,2'-disulfonic acid can reduce the recovery of partially phosphorothioated dsRNA in hepatic tissue (Miyao et al., DsRNA Res. Dev., 1995, 5, 115-121; Takakura et al., DsRNA & Nucl. Acid Drug Dev., 1996, 6, 177-183).

In contrast to carrier compounds, a "pharmaceutical carrier" or "excipient" is a pharmaceutically acceptable solvent, suspending agent, or any other pharmacologically inert vehicle for delivering one or more nucleic acids to an animal. The excipient may be liquid or solid and is selected, in combination with a nucleic acid and other components of a particular pharmaceutical composition, to provide the desired bulk, consistency, *etc.,* with reference to the intended mode of administration. Typical pharmaceutical carriers include, but are not limited to, binders (e.g., pregelatinized corn starch, polyvinylpyrrolidone, or hydroxypropyl methylcellulose); fillers *(e.g.,* lactose and other sugars, microcrystalline cellulose, pectin, gelatin, calcium sulfate, ethyl cellulose, polyacrylates, or calcium hydrogen phosphate); lubricants (e.g., magnesium stearate, talc, silica, colloidal silicon dioxide, stearic acid, metallic stearate, hydrogenated vegetable oil, corn starch, polyethylene glycol, sodium benzoate, or sodium acetate); disintegrants *(e.g.,* starch or sodium starch glycolate); and wetting agents *(e.g.,* sodium lauryl sulfate).

Pharmaceutically acceptable organic or inorganic excipients suitable for non-parenteral administration that do not deleteriously react with nucleic acids may also be used to formulate the compositions of the present disclosure. Suitable pharmaceutically acceptable carriers include, but are not limited to, water, salt solutions, alcohols, polyethylene glycol, gelatin, lactose, amylose, magnesium stearate, talc, silicic acid, viscous paraffin, hydroxymethylcellulose, polyvinylpyrrolidone, *etc.*

Formulations for topical administration of nucleic acids may include sterile or non-sterile aqueous solutions or non-aqueous solutions in common solvents such as alcohols, or nucleic acid solutions in liquid or solid oil bases. The solutions may further include buffers, diluents, and other suitable additives. Pharmaceutically acceptable organic or inorganic excipients suitable for non-parenteral administration that do not deleteriously react with nucleic acids may be used.

Suitable pharmaceutically acceptable excipients include, but are not limited to, water, salt solutions, alcohols, polyethylene glycol, gelatin, lactose, amylose, magnesium stearate, talc, silicic acid, viscous paraffin, hydroxymethylcellulose, polyvinylpyrrolidone, *etc.*

The present disclosure also provides methods for treating or preventing diseases and disorders that can be modulated by downregulating the AGT gene expression. For example, treatment of hypertension, borderline hypertension, primary hypertension, secondary hypertension, hypertensive crisis, hypertensive urgency, isolated systolic and diastolic hypertension, pregnancy-associated hypertension, diabetic hypertension, resistant hypertension, refractory hypertension, paroxysmal hypertension, renovascular hypertension, Goldblatt hypertension, ocular hypertension, glaucoma, pulmonary hypertension, portal hypertension, systemic venous hypertension, systolic hypertension, labile hypertension, hypertensive heart disease, hypertensive nephropathy, atherosclerosis, arteriosclerosis, vasculopathy, diabetic nephropathy, diabetic retinopathy, chronic heart failure, cardiomyopathy, diabetic cardiomyopathy, glomerulosclerosis, aortic coarctation, aortic aneurysm, ventricular fibrosis, Cushing's syndrome and other glucocorticoid excess states (including chronic steroid therapy), pheochromocytoma, reninoma, secondary hyperaldosteronism and other mineralocorticoid excess states, sleep apnea, thyroid/parathyroid diseases, heart failure, myocardial infarction, angina, stroke, diabetes, nephropathy, renal failure, systemic sclerosis, intrauterine growth retardation (IUGR), and fetal growth restriction.

The RNAi agent of the present disclosure may be administered to a subject using any mode of administration known in the art, including, but not limited to, subcutaneous, intravenous, intramuscular, intraocular, intrabronchial, intrapleural, intraperitoneal, intraarterial, lymphatic, cerebrospinal, and any combination thereof. In a preferred example, the agents are administered subcutaneously.

In further examples, the siRNA is administered in combination with an additional therapeutic agent. The siRNA and an additional therapeutic agent may be administered in combination in the same composition, e.g., parenterally, or the additional therapeutic agent may be administered as part of a separate composition or by another method described herein.

Examples of additional therapeutic agents include those known to treat hypertension or those known to treat cardiovascular and cerebrovascular diseases. For example, additional antihypertensive agents are selected from angiotensin-converting enzyme inhibitors (e.g., captopril, enalapril, benazepril, perindopril), angiotensin II receptor antagonists (e.g., losartan, losartan hydrochlorothiazide, valsartan, valsartan hydrochlorothiazide, telmisartan, telmisartan hydrochlorothiazide, olmesartan medoxomil), β-blockers (e.g., propranolol, bisoprolol, metoprolol tartrate, metoprolol succinate).

In one example, the iRNA agent is administered to a patient, followed by administration of the additional therapeutic agent to the patient (or vice versa). In another example, the iRNA agent and the additional therapeutic agent

are administered simultaneously.

The following examples are provided to illustrate the present disclosure but are not intended to limit the scope thereof. Unless otherwise specified, the technical means used in the examples are conventional means well known to those skilled in the art, and all raw materials are commercially available products.

The abbreviations for nucleotides used herein are as follows:

A = adenosine-3'-phosphate
Am = 2'-O-methyladenosine-3'-phosphate
Ams = 2'-O-methyladenosine-3'-thiophosphate
Af = 2'-fluoroadenosine-3'-phosphate
Afs = 2'-fluoroadenosine-3'-thiophosphate
G = guanosine-3'-phosphate
Gm = 2'-O-methylguanosine-3'-phosphate
Gms = 2'-O-methylguanosine-3'-thiophosphate
Gf = 2'-fluoroguanosine-3'-phosphate
Gfs = 2'-fluoroguanosine-3'-thiophosphate
C = cytidine-3'-phosphate
Cm = 2'-O-methylcytidine-3'-phosphate
Cms = 2'-O-methylcytidine-3'-thiophosphate
Cf = 2'-fluorocytidine-3'-phosphate
Cfs = 2'-fluorocytidine-3'-thiophosphate
U = uridine-3'-phosphate
Um = 2'-O-methyluridine-3'-phosphate
Ums = 2'-O-methyluridine-3'-thiophosphate
Uf = 2'-fluorouridine-3'-phosphate
Ufs = 2'-fluorouridine-3'-thiophosphate
AmsEVP = 5'-ethenyl-(E)-phosphonate-2'-O-methyladenosine-3'-thiophosphate
UmsEVP = 5'-ethenyl-(E)-phosphonate-2'-O-methyluridine-3'-thiophosphate
Agna = adenosine-glycol nucleic acid
Cgna = cytidine-glycol nucleic acid
Ggna = guanosine-glycol nucleic acid
Tgna = thymidine-glycol nucleic acid
Ugna = uridine-glycol nucleic acid
The modifications used herein correspond to the following English names:
2'-O-Methyl: 2'-O-methyl
2'-Fluoro: 2'-fluoro
3'-Thiophosphate: 3'-thiophosphate
5'-Ethenyl-(E)-phosphonate: 5'-ethenyl-(E)-phosphonate
2'-Deoxy: 2'-deoxy

The numbering schemes for compounds used herein is as follows:
Unmodified sequences refer to unmodified RNA sequences. For sequences in which the sense and antisense strands are 21 and 23 nucleotides in length, respectively, the number represents the position on the AGT mRNA, such as compound 1579. For sequences in which the sense and antisense strands are 20 and 22 nucleotides in length, respectively, the suffix "s" is added after the number of the representative position for distinction, such as compound 1578s.

Sequences alternately modified with 2'-O-methyl (2'-OMe) and 2'-fluoro (2'-F) are designated by adding the prefix "B" before the number of the unmodified sequence, and appending the suffix "-AL" after the number. For example, the alternately modified sequence 1578s is designated as B1578s-AL.

For compounds in Example 4, sequences modified using the templates DV25P-29P and DV32P-34P disclosed herein or the template DV22 disclosed in the prior art are designated by adding the prefix "C" before the number of the unmodified sequence, and appending the corresponding template name after the number. For example, the sequence 1578s modified with the template DV29P is designated as C1578s-DV29P.

For compounds in Example 6, sequences modified using the templates DV25P-29P and DV32P-34P disclosed herein or the template DV22 disclosed in the prior art, and optionally containing anti-off-target modifications, are designated by removing the initial digit "1" from the number of the unmodified sequence, adding the prefix "D" before the number, and appending the corresponding template name and anti-off-target modification name in sequence. For example, the unmodified sequence 1578s modified with the template DV29P and containing the anti-off-target modification d7B is designated as D578s-DV29Pd7B.

For compounds in Example 7, sequences modified using the template DV25P-29P disclosed herein, optionally containing anti-off-target modifications, and conjugated with GalNAc at the 3' end of the sense strand for liver-targeted delivery, are designated by appending "G5" to the end based on the numbering scheme in Example 6. For example, the sequence D578s-DV29Pd7B conjugated with G5 is designated as D578s-DV29Pd7BG5.

Table 49: Numbering schemes used in examples

| Type of numbering | Unmodified sequence | Alternately modified sequence | Specifically modified sequence | Anti-off-target modified sequence | *In vivo* experimental sequence |
|---|---|---|---|---|---|
| Example of numbering | 1578s | B1578s-AL | C1578s-DV29P | D578s-DV29Pd7B | D578s-DV29Pd7BG5 |

In the following examples, the P-values of intergroup comparisons are less than 0.05, indicating statistically significant differences.

**Examples**

**Example 1: Synthesis of alternately modified small interfering oligonucleotides**

99 unmodified siRNA sequences were designed based on the AGT mRNA sequence (NM_001384479.1). To enhance the inhibitory efficiency and stability of the sequences, the unmodified sequences were alternately modified with 2'-O-methyl (2'-OMe) and 2'-fluoro (2'-F), with terminal phosphorothioate modifications. For sense strands of odd-numbered length, the odd-numbered positions were modified with 2'-F, and the even-numbered positions were modified with 2'-OMe. However, for sense strands of even-numbered length, the odd-numbered positions were modified with 2'-OMe, and the even-numbered positions were modified with 2'-F. For antisense strands, the odd-numbered positions were modified with 2'-OMe, and the even-numbered positions were modified with 2'-F. In addition, the 5' end of the sense strand contained two phosphorothioate modifications, while the 5' and 3' ends of the antisense strand each contained two phosphorothioate modifications. The siRNA sequences with alternating modifications are shown in Table 2.

**1. Synthesis of alternately modified sequence B1579-AL**

The unmodified sequence of the small interfering ribonucleic acid designated as B1579-AL in Table 1 is as follows:

Sense strand: 5'-CCUUUUCUUCUAAUGAGUCGA-3' (SEQ ID NO: 4)
Antisense strand: 5'-UCGACUCAUUAGAAGAAAAGGUG-3' (SEQ ID NO: 17)
The odd-numbered positions of the sense strand and the even-numbered positions of the antisense strand were modified with 2'-F, while all other positions were modified with 2'-OMe. In addition, the 5' end of the sense strand contained two phosphorothioate modifications, while the 5' and 3' ends of the antisense strand each contained two phosphorothioate modifications.

Instruments and reagents: A Tsingke 192 P model DNA/RNA automatic synthesizer was used. The solid-phase support was a universal support composed of cross-linked polystyrene beads, model: Primer Support 5G Unylinker 350 (manufactured by Cytiva).

Preparation method:

Nucleoside phosphoramidite monomer solutions were prepared in acetonitrile at a concentration of 0.15 M, using the following monomers: DMT-A-OMe phosphoramidite monomer (Formula 1), DMT-C-OMe phosphoramidite monomer (Formula 2), DMT-G-OMe phosphoramidite monomer (Formula 3), DMT-U-OMe phosphoramidite monomer (Formula 4), DMT-A-F phosphoramidite monomer (Formula 5), DMT-C-F phosphoramidite monomer (Formula 6), DMT-G-F phosphoramidite monomer (Formula 7), and DMT-U-F phosphoramidite monomer (Formula 8).

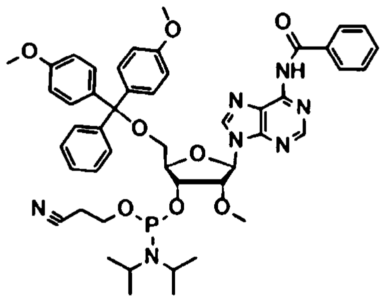

Formula 1

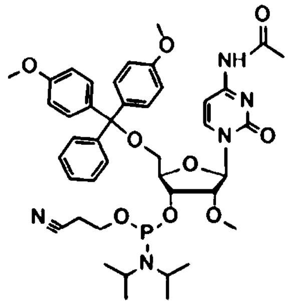

Formula 2

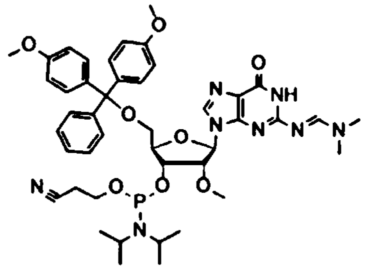

Formula 3

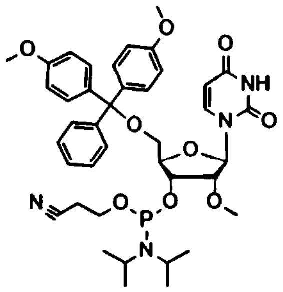

Formula 4

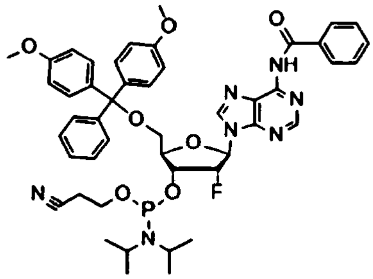

Formula 5

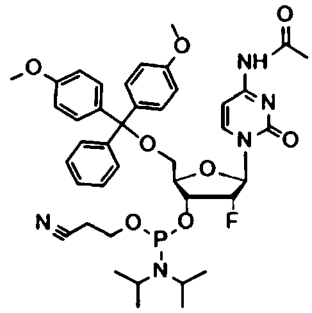

Formula 6

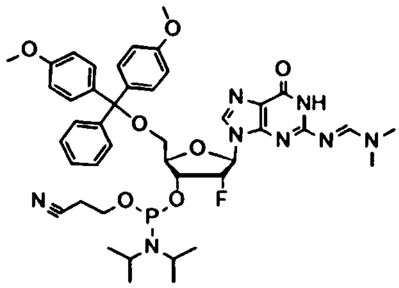

Formula 7

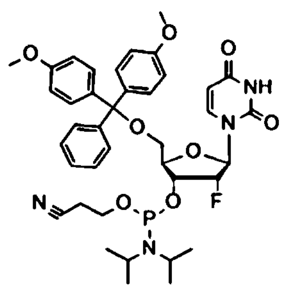

Formula 8

The preparation was carried out according to the following steps:

The solid-phase support was loaded into the designated position of the synthesizer, and a corresponding fully protected product was obtained through several synthesis cycles, each cycle including (1) deprotection, (2) coupling, (3) oxidation/sulfurization, and (4) hydroxyl protection. The cycle process and the reagents used are described as follows:

(1) Deprotection
3% dichloroacetic acid in toluene was used as a deprotection reagent to remove the DMT protecting group, followed by washing with acetonitrile.
(2) Coupling
Each nucleoside phosphoramidite monomer in acetonitrile was coupled using 0.25 M 5-ethylthio-1*H*-tetrazole as an activator, followed by rinsing with acetonitrile.
(3) Oxidation/sulfurization

Oxidation: 0.05 M iodine in pyridine/water (90/10) was used as an oxidant for oxidation, followed by rinsing with acetonitrile.
Sulfurization: 3% xanthane hydride in pyridine was used as a sulfurizing agent for sulfurization, followed by rinsing with acetonitrile.

(4) Hydroxyl protection

10% acetic anhydride in tetrahydrofuran (CAP A) and tetrahydrofuran/pyridine/N-methylimidazole (74/10/16, v/v/v) (CAP B) were used as hydroxyl protecting reagents for hydroxyl protection, followed by rinsing with acetonitrile.

The above steps were repeated according to the set sequence to obtain the fully protected product.

(5) 3% dichloroacetic acid in toluene was used as a deprotection reagent to remove the DMT protecting group from the last nucleotide, followed by washing with acetonitrile.

(6) Ammonolysis and purification

The solid-phase support was transferred to a reactor, then concentrated aqueous ammonia (25 to 28%) was added, and ammonolysis was performed at 60°C for 12 hours. The system was then cooled to room temperature, and the mixture was transferred to a pressure filtration vessel, followed by rinsing with a mixture of purified water and ethanol. The filtrates were combined, subjected to column chromatography, concentrated, and lyophilized to obtain the product.

(7) Annealing

The purified sense and antisense strands were mixed at a ratio of 1:1, heated to 95°C and maintained for 3 minutes, and slowly cooled to room temperature to form a duplex.

B1579-AL had a purity of 90.61% and a measured molecular weight of 14437.86.

**2. Synthesis of other sequences**

The other sequences listed in Table 1 were synthesized according to the method described above, totaling 99 siRNA sequences.

Table 1: Alternately modified siRNA sequences

| Sequence ID | Sense strand sequence 5'-3' | Sequence SEQ ID NO./ corresponding unmodified sequence SEQ ID NO. | Antisense strand sequence 5'-3' | Sequence SEQ ID NO./ corresponding unmodified sequence SEQ ID NO. |
|---|---|---|---|---|
| B1835-AL | Ufs-Ums-Gf-Gm-Gf-Um-Uf-Um-Uf-Am-Af-Am-Af-Um-Uf-Am-Af-Am-Gf-Um-Af | 199/1 | Ums-Afs-Cm-Uf-Um-Uf-Am-Af-Um-Uf-Um-Uf-Am-Af-Am-Af-Cm-Cf-Cm-Af-Ams-Ufs-Um | 212/14 |
| B 1816-AL | Ufs-Ums-Cf-Am-Af-Gm-Uf-Um-Gf-Am-Gf-Am-Af-Cm-Af-Am-Af-Am-Af-Um-Uf | 200/2 | Ams-Afs-Um-Uf-Um-Uf-Um-Gf-Um-Uf-Cm-Uf-Cm-Af-Am-Cf-Um-Uf-Gm-Af-Ams-Afs-Am | 213/15 |
| B1812-AL | Cfs-Cms-Uf-Um-Uf-Um-Cf-Am-Af-Gm-Uf-Um-Gf-Am-Gf-Am-Af-Cm-Af-Am-Af | 201/3 | Ums-Ufs-Um-Gf-Um-Uf-Cm-Uf-Cm-Af-Am-Cf-Um-Uf-Gm-Af-Am-Af-Am-Gf-Gms-Gfs-Am | 214/16 |
| B1579-AL | Cfs-Cms-Uf-Um-Uf-Um-Cf-Um-Uf-Cm-Uf-Am-Af-Um-Gf-Am-Gf-Um-Cf-Gm-Af | 202/4 | Ums-Cfs-Gm-Af-Cm-Uf-Cm-Af-Um-Uf-Am-Gf-Am-Af-Gm-Af-Am-Af-Am-Gf-Gms-Ufs-Gm | 215/17 |
| B1836-AL | Ufs-Gms-Gf-Gm-Uf-Um-Uf-Um-Af-Am-Af-Am-Uf-Um-Af-Am-Af-Gm-Uf-Am-Uf | 203/5 | Ams-Ufs-Am-Cf-Um-Uf-Um-Af-Am-Uf-Um-Uf-Um-Af-Am-Af-Am-Cf-Cm-Cf-Ams-Afs-Um | 216/18 |
| B 1789-AL | Gfs-Ums-Uf-Um-Gf-Um-Gf-Am-Af-Am-Cf-Am-Af-Am-Af-Am-Af-Gm-Uf-Gm-Uf | 204/6 | Ams-Cfs-Am-Cf-Um-Uf-Um-Uf-Um-Uf-Gm-Uf-Um-Uf-Cm-Af-Cm-Af-Am-Af-Cms-Afs-Am | 217/19 |
| B1839-AL | Gfs-Ums-Uf-Um-Uf-Am-Af-Am-Af-Um-Uf-Am-Af-Am-Gf-Um-Af-Um-Af-Cm-Af | 205/7 | Ums-Gfs-Um-Af-Um-Af-Cm-Uf-Um-Uf-Am-Af-Um-Uf-Um-Uf-Am-Af-Am-Af-Cms-Cfs-Cm | 218/20 |
| B1791-AL | Ufs-Ums-Gf-Um-Gf-Am-Af-Am-Cf-Am-Af-Am-Af-Am-Af-Gm-Uf-Gm-Uf-Um-Af | 206/8 | Ums-Afs-Am-Cf-Am-Cf-Um-Uf-Um-Uf-Um-Uf-Gm-Uf-Um-Uf-Cm-Af-Cm-Af-Ams-Afs-Cm | 219/21 |

(continued)

| Sequence ID | Sense strand sequence 5'-3' | Sequence SEQ ID NO./ corresponding unmodified sequence SEQ ID NO. | Antisense strand sequence 5'-3' | Sequence SEQ ID NO./ corresponding unmodified sequence SEQ ID NO. |
|---|---|---|---|---|
| B1795-AL | Gfs-Ams-Af-Am-Cf-Am-Af-Am-Af-Am-Af-Gm-Uf-Gm-Uf-Um-Cf-Cm-Cf-Um-Uf | 207/9 | Ams-Afs-Gm-Gf-Gm-Af-Am-Cf-Am-Cf-Um-Uf-Um-Uf-Um-Uf-Gm-Uf-Um-Uf-Cms-Afs-Cm | 220/22 |
| B1585sAL | Cms-Ufs-Um-Cf-Um-Af-Am-Uf-Gm-Af-Gm-Uf-Cm-Gf-Am-Cf-Um-Uf-Um-Af | 208/10 | Ums-Afs-Am-Af-Gm-Uf-Cm-Gf-Am-Cf-Um-Cf-Am-Uf-Um-Af-Gm-Af-Am-Gfs-Ams-Af | 221/23 |
| B1576sAL | Cms-Cfs-Am-Cf-Cm-Uf-Um-Uf-Um-Cf-Um-Uf-Cm-Uf-Am-Af-Um-Gf-Am-Af | 209/11 | Ums-Ufs-Cm-Af-Um-Uf-Am-Gf-Am-Af-Gm-Af-Am-Af-Am-Gf-Gm-Uf-Gm-Gfs-Gms-Af | 222/24 |
| B 1578sAL | Ams-Cfs-Cm-Uf-Um-Uf-Um-Cf-Um-Uf-Cm-Uf-Am-Af-Um-Gf-Am-Gf-Um-Af | 210/12 | Ums-Afs-Cm-Uf-Cm-Af-Um-Uf-Am-Gf-Am-Af-Gm-Af-Am-Af-Am-Gf-Gm-Ufs-Gms-Gf | 223/25 |
| B1838-AL | Gfs-Gms-Uf-Um-Uf-Um-Af-Am-Af-Am-Uf-Um-Af-Am-Af-Gm-Uf-Am-Uf-Am-Af | 211/13 | Ums-Ufs-Am-Uf-Am-Cf-Um-Uf-Um-Af-Am-Uf-Um-Uf-Um-Af-Am-Af-Am-Cf-Cms-Cfs-Am | 224/26 |
| B1008-AL | Cfs-Ums-Uf-Cm-Af-Cm-Uf-Gm-Af-Gm-Af-Gm-Cf-Gm-Cf-Cm-Uf-Gm-Cf-Cm-Uf | 225/27 | Ams-Gfs-Gm-Cf-Am-Gf-Gm-Cf-Gm-Cf-Um-Cf-Um-Cf-Am-Gf-Um-Gf-Am-Af-Gms-Gfs-Gm | 311/113 |
| B1011-AL | Cfs-Ams-Cf-Um-Gf-Am-Gf-Am-Gf-Cm-Gf-Cm-Cf-Um-Gf-Cm-Cf-Um-Gf-Cm-Uf | 226/28 | Ams-Gfs-Cm-Af-Gm-Gf-Cm-Af-Gm-Gf-Cm-Gf-Cm-Uf-Cm-Uf-Cm-Af-Gm-Uf-Gms-Afs-Am | 312/114 |
| B1014-AL | Ufs-Gms-Af-Gm-Af-Gm-Cf-Gm-Cf-Cm-Uf-Gm-Cf-Cm-Uf-Gm-Cf-Um-Gf-Cm-Uf | 227/29 | Ams-Gfs-Cm-Af-Gm-Cf-Am-Gf-Gm-Cf-Am-Gf-Gm-Cf-Gm-Cf-Um-Cf-Um-Cf-Ams-Gfs-Um | 313/115 |

(continued)

| Sequence ID | Sense strand sequence 5'-3' | Sequence SEQ ID NO./ corresponding unmodified sequence SEQ ID NO. | Antisense strand sequence 5'-3' | Sequence SEQ ID NO./ corresponding unmodified sequence SEQ ID NO. |
|---|---|---|---|---|
| B1016-AL | Afs-Gms-Af-Gm-Cf-Gm-Cf-Cm-Uf-Gm-Cf-Cm-Uf-Gm-Cf-Um-Gf-Cm-Uf-Gm-Af | 228/30 | Ums-Cfs-Am-Gf-Cm-Af-Gm-Cf-Am-Gf-Gm-Cf-Am-Gf-Gm-Cf-Gm-Cf-Um-Cf-Ums-Cfs-Am | 314/116 |
| B1017-AL | Gfs-Ams-Gf-Cm-Gf-Cm-Cf-Um-Gf-Cm-Cf-Um-Gf-Cm-Uf-Gm-Cf-Um-Gf-Am-Uf | 229/31 | Ams-Ufs-Cm-Af-Gm-Cf-Am-Gf-Cm-Af-Gm-Gf-Cm-Af-Gm-Gf-Cm-Gf-Cm-Uf-Cms-Ufs-Cm | 315/117 |
| B 1020-AL | Cfs-Gms-Cf-Cm-Uf-Gm-Cf-Cm-Uf-Gm-Cf-Um-Gf-Cm-Uf-Gm-Af-Um-Cf-Cm-Af | 230/32 | Ums-Gfs-Gm-Af-Um-Cf-Am-Gf-Cm-Af-Gm-Cf-Am-Gf-Gm-Cf-Am-Gf-Gm-Cf-Gms-Cfs-Um | 316/118 |
| B1024-AL | Ufs-Gms-Cf-Cm-Uf-Gm-Cf-Um-Gf-Cm-Uf-Gm-Af-Um-Cf-Cm-Af-Gm-Cf-Cm-Uf | 231/33 | Ams-Gfs-Gm-Cf-Um-Gf-Gm-Af-Um-Cf-Am-Gf-Cm-Af-Gm-Cf-Am-Gf-Gm-Cf-Ams-Gfs-Gm | 317/119 |
| B1026-AL | Cfs-Cms-Uf-Gm-Cf-Um-Gf-Cm-Uf-Gm-Af-Um-Cf-Cm-Af-Gm-Cf-Cm-Uf-Cm-Af | 232/34 | Ums-Gfs-Am-Gf-Gm-Cf-Um-Gf-Gm-Af-Um-Cf-Am-Gf-Cm-Af-Gm-Cf-Am-Gf-Gms-Cfs-Am | 318/120 |
| B1028-AL | Ufs-Gms-Cf-Um-Gf-Cm-Uf-Gm-Af-Um-Cf-Cm-Af-Gm-Cf-Cm-Uf-Cm-Af-Cm-Uf | 233/35 | Ams-Gfs-Um-Gf-Am-Gf-Gm-Cf-Um-Gf-Gm-Af-Um-Cf-Am-Gf-Cm-Af-Gm-Cf-Ams-Gfs-Gm | 319/121 |
| B1029-AL | Gfs-Cms-Uf-Gm-Cf-Um-Gf-Am-Uf-Cm-Cf-Am-Gf-Cm-Cf-Um-Cf-Am-Cf-Um-Af | 234/36 | Ums-Afs-Gm-Uf-Gm-Af-Gm-Gf-Cm-Uf-Gm-Gf-Am-Uf-Cm-Af-Gm-Cf-Am-Gf-Cms-Afs-Gm | 320/122 |
| B1030-AL | Cfs-Ums-Gf-Cm-Uf-Gm-Af-Um-Cf-Cm-Af-Gm-Cf-Cm-Uf-Cm-Af-Cm-Uf-Am-Uf | 235/37 | Ams-Ufs-Am-Gf-Um-Gf-Am-Gf-Gm-Cf-Um-Gf-Gm-Af-Um-Cf-Am-Gf-Cm-Af-Gms-Cfs-Am | 321/123 |

(continued)

| Sequence ID | Sense strand sequence 5'-3' | Sequence SEQ ID NO./ corresponding unmodified sequence SEQ ID NO. | Antisense strand sequence 5'-3' | Sequence SEQ ID NO./ corresponding unmodified sequence SEQ ID NO. |
|---|---|---|---|---|
| B1038-AL | Cfs-Cms-Af-Gm-Cf-Cm-Uf-Cm-Af-Cm-Uf-Am-Uf-Gm-Cf-Cm-Uf-Cm-Uf-Gm-Af | 236/38 | Ums-Cfs-Am-Gf-Am-Gf-Gm-Cf-Am-Uf-Am-Gf-Um-Gf-Am-Gf-Gm-Cf-Um-Gf-Gms-Afs-Um | 322/124 |
| B1041-AL | Gfs-Cms-Cf-Um-Cf-Am-Cf-Um-Af-Um-Gf-Cm-Cf-Um-Cf-Um-Gf-Am-Cf-Cm-Uf | 237/39 | Ams-Gfs-Gm-Uf-Cm-Af-Gm-Af-Gm-Gf-Cm-Af-Um-Af-Gm-Uf-Gm-Af-Gm-Gf-Cms-Ufs-Gm | 323/125 |
| B1044-AL | Ufs-Cms-Af-Cm-Uf-Am-Uf-Gm-Cf-Cm-Uf-Cm-Uf-Gm-Af-Cm-Cf-Um-Gf-Gm-Af | 238/40 | Ums-Cfs-Cm-Af-Gm-Gf-Um-Cf-Am-Gf-Am-Gf-Gm-Cf-Am-Uf-Am-Gf-Um-Gf-Ams-Gfs-Gm | 324/126 |
| B1046-AL | Afs-Cms-Uf-Am-Uf-Gm-Cf-Cm-Uf-Cm-Uf-Gm-Af-Cm-Cf-Um-Gf-Gm-Af-Cm-Af | 239/41 | Ums-Gfs-Um-Cf-Cm-Af-Gm-Gf-Um-Cf-Am-Gf-Am-Gf-Gm-Cf-Am-Uf-Am-Gf-Ums-Gfs-Am | 325/127 |
| B1047-AL | Cfs-Ums-Af-Um-Gf-Cm-Cf-Um-Cf-Um-Gf-Am-Cf-Cm-Uf-Gm-Gf-Am-Cf-Am-Af | 240/42 | Ums-Ufs-Gm-Uf-Cm-Cf-Am-Gf-Gm-Uf-Cm-Af-Gm-Af-Gm-Gf-Cm-Af-Um-Af-Gms-Ufs-Gm | 326/128 |
| B1053-AL | Cfs-Ums-Cf-Um-Gf-Am-Cf-Cm-Uf-Gm-Gf-Am-Cf-Am-Af-Gm-Gf-Um-Gf-Gm-Af | 241/43 | Ums-Cfs-Cm-Af-Cm-Cf-Um-Uf-Gm-Uf-Cm-Cf-Am-Gf-Gm-Uf-Cm-Af-Gm-Af-Gms-Gfs-Cm | 327/129 |
| B1057-AL | Gfs-Ams-Cf-Cm-Uf-Gm-Gf-Am-Cf-Am-Af-Gm-Gf-Um-Gf-Gm-Af-Gm-Gf-Gm-Uf | 242/44 | Ams-Cfs-Cm-Cf-Um-Cf-Cm-Af-Cm-Cf-Um-Uf-Gm-Uf-Cm-Cf-Am-Gf-Gm-Uf-Cms-Afs-Gm | 328/130 |
| B1061-AL | Ufs-Gms-Gf-Am-Cf-Am-Af-Gm-Gf-Um-Gf-Gm-Af-Gm-Gf-Gm-Uf-Cm-Uf-Cm-Af | 243/45 | Ums-Gfs-Am-Gf-Am-Cf-Cm-Cf-Um-Cf-Cm-Af-Cm-Cf-Um-Uf-Gm-Uf-Cm-Cf-Ams-Gfs-Gm | 329/131 |

(continued)

| Sequence ID | Sense strand sequence 5'-3' | Sequence SEQ ID NO./ corresponding unmodified sequence SEQ ID NO. | Antisense strand sequence 5'-3' | Sequence SEQ ID NO./ corresponding unmodified sequence SEQ ID NO. |
|---|---|---|---|---|
| B1065s-AL | Cms-Afs-Am-Gf-Gm-Uf-Gm-Gf-Am-Gf-Gm-Gf-Um-Cf-Um-Cf-Am-Cf-Um-Uf | 244/46 | Ams-Afs-Gm-Uf-Gm-Af-Gm-Af-Cm-Cf-Cm-Uf-Cm-Cf-Am-Cf-Cm-Uf-Um-Gfs-Ums-Cf | 330/132 |
| B1071-AL | Gfs-Gms-Af-Gm-Gf-Gm-Uf-Cm-Uf-Cm-Af-Cm-Uf-Um-Uf-Cm-Cf-Am-Gf-Cm-Af | 245/47 | Ums-Gfs-Cm-Uf-Gm-Gf-Am-Af-Am-Gf-Um-Gf-Am-Gf-Am-Cf-Cm-Cf-Um-Cf-Cms-Afs-Cm | 331/133 |
| B1260-AL | Cfs-Ams-Gf-Gm-Gf-Um-Gf-Gm-Gf-Gm-Gf-Am-Gf-Gm-Uf-Gm-Cf-Um-Gf-Am-Af | 246/48 | Ums-Ufs-Cm-Af-Gm-Cf-Am-Cf-Cm-Uf-Cm-Cf-Cm-Cf-Cm-Af-Cm-Cf-Cm-Uf-Gms-Afs-Um | 332/134 |
| B 1262-AL | Gfs-Gms-Gf-Um-Gf-Gm-Gf-Gm-Gf-Am-Gf-Gm-Uf-Gm-Cf-Um-Gf-Am-Af-Cm-Af | 247/49 | Ums-Gfs-Um-Uf-Cm-Af-Gm-Cf-Am-Cf-Cm-Uf-Cm-Cf-Cm-Cf-Cm-Af-Cm-Cf-Cms-Ufs-Gm | 333/135 |
| B1265-AL | Ufs-Gms-Gf-Gm-Gf-Gm-Af-Gm-Gf-Um-Gf-Cm-Uf-Gm-Af-Am-Cf-Am-Gf-Cm-Af | 248/50 | Ums-Gfs-Cm-Uf-Gm-Uf-Um-Cf-Am-Gf-Cm-Af-Cm-Cf-Um-Cf-Cm-Cf-Cm-Cf-Ams-Cfs-Cm | 334/136 |
| B1266-AL | Gfs-Gms-Gf-Gm-Gf-Am-Gf-Gm-Uf-Gm-Cf-Um-Gf-Am-Af-Cm-Af-Gm-Cf-Am-Uf | 249/51 | Ams-Ufs-Gm-Cf-Um-Gf-Um-Uf-Cm-Af-Gm-Cf-Am-Cf-Cm-Uf-Cm-Cf-Cm-Cf-Cms-Afs-Cm | 335/137 |
| B1267-AL | Gfs-Gms-Gf-Gm-Af-Gm-Gf-Um-Gf-Cm-Uf-Gm-Af-Am-Cf-Am-Gf-Cm-Af-Um-Uf | 250/52 | Ams-Afs-Um-Gf-Cm-Uf-Gm-Uf-Um-Cf-Am-Gf-Cm-Af-Cm-Cf-Um-Cf-Cm-Cf-Cms-Cfs-Am | 336/138 |
| B1268-AL | Gfs-Gms-Gf-Am-Gf-Gm-Uf-Gm-Cf-Um-Gf-Am-Af-Cm-Af-Gm-Cf-Am-Uf-Um-Uf | 251/53 | Ams-Afs-Am-Uf-Gm-Cf-Um-Gf-Um-Uf-Cm-Af-Gm-Cf-Am-Cf-Cm-Uf-Cm-Cf-Cms-Cfs-Cm | 337/139 |

(continued)

| Sequence ID | Sense strand sequence 5'-3' | Sequence SEQ ID NO./ corresponding unmodified sequence SEQ ID NO. | Antisense strand sequence 5'-3' | Sequence SEQ ID NO./ corresponding unmodified sequence SEQ ID NO. |
|---|---|---|---|---|
| B 1279-AL | Afs-Ams-Cf-Am-Gf-Cm-Af-Um-Uf-Um-Uf-Um-Uf-Um-Uf-Gm-Af-Gm-Cf-Um-Uf | 252/54 | Ams-Afs-Gm-Cf-Um-Cf-Am-Af-Am-Af-Am-Af-Am-Af-Um-Gf-Cm-Uf-Gm-Uf-Ums-Cfs-Am | 338/140 |
| B1281-AL | Cfs-Ams-Gf-Cm-Af-Um-Uf-Um-Uf-Um-Uf-Um-Uf-Gm-Af-Gm-Cf-Um-Uf-Gm-Af | 253/55 | Ums-Cfs-Am-Af-Gm-Cf-Um-Cf-Am-Af-Am-Af-Am-Af-Am-Af-Um-Gf-Cm-Uf-Gms-Ufs-Um | 339/141 |
| B1282-AL | Afs-Gms-Cf-Am-Uf-Um-Uf-Um-Uf-Um-Uf-Um-Gf-Am-Gf-Cm-Uf-Um-Gf-Am-Af | 254/56 | Ums-Ufs-Cm-Af-Am-Gf-Cm-Uf-Cm-Af-Am-Af-Am-Af-Am-Af-Am-Uf-Gm-Cf-Ums-Gfs-Um | 340/142 |
| B1362-AL | Gfs-Gms-Uf-Gm-Af-Cm-Cf-Cm-Uf-Gm-Af-Am-Cf-Cm-Gf-Cm-Cf-Cm-Af-Um-Uf | 255/57 | Ams-Afs-Um-Gf-Gm-Gf-Cm-Gf-Gm-Uf-Um-Cf-Am-Gf-Gm-Gf-Um-Cf-Am-Cf-Cms-Ufs-Cm | 341/143 |
| B1365-AL | Gfs-Ams-Cf-Cm-Cf-Um-Gf-Am-Af-Cm-Cf-Gm-Cf-Cm-Cf-Am-Uf-Um-Cf-Cm-Uf | 256/58 | Ams-Gfs-Gm-Af-Am-Uf-Gm-Gf-Gm-Cf-Gm-Gf-Um-Uf-Cm-Af-Gm-Gf-Gm-Uf-Cms-Afs-Cm | 342/144 |
| B1367-AL | Cfs-Cms-Cf-Um-Gf-Am-Af-Cm-Cf-Gm-Cf-Cm-Cf-Am-Uf-Um-Cf-Cm-Uf-Gm-Uf | 257/59 | Ams-Cfs-Am-Gf-Gm-Af-Am-Uf-Gm-Gf-Gm-Cf-Gm-Gf-Um-Uf-Cm-Af-Gm-Gf-Gms-Ufs-Cm | 343/145 |
| B1369-AL | Cfs-Ums-Gf-Am-Af-Cm-Cf-Gm-Cf-Cm-Cf-Am-Uf-Um-Cf-Cm-Uf-Gm-Uf-Um-Uf | 258/60 | Ams-Afs-Am-Cf-Am-Gf-Gm-Af-Am-Uf-Gm-Gf-Gm-Cf-Gm-Gf-Um-Uf-Cm-Af-Gms-Gfs-Gm | 344/146 |
| B1393-AL | Gfs-Ums-Gf-Um-Af-Um-Gf-Am-Uf-Cm-Af-Am-Af-Gm-Cf-Gm-Cf-Cm-Af-Cm-Uf | 259/61 | Ams-Gfs-Um-Gf-Gm-Cf-Gm-Cf-Um-Uf-Um-Gf-Am-Uf-Cm-Af-Um-Af-Cm-Af-Cms-Afs-Gm | 345/147 |

(continued)

| Sequence ID | Sense strand sequence 5'-3' | Sequence SEQ ID NO./ corresponding unmodified sequence SEQ ID NO. | Antisense strand sequence 5'-3' | Sequence SEQ ID NO./ corresponding unmodified sequence SEQ ID NO. |
|---|---|---|---|---|
| B1401-AL | Ufs-Cms-Af-Am-Af-Gm-Cf-Gm-Cf-Cm-Af-Cm-Uf-Gm-Cf-Cm-Cf-Um-Gf-Cm-Af | 260/62 | Ums-Gfs-Cm-Af-Gm-Gf-Gm-Cf-Am-Gf-Um-Gf-Gm-Cf-Gm-Cf-Um-Uf-Um-Gf-Ams-Ufs-Cm | 346/148 |
| B 1404-AL | Afs-Ams-Gf-Cm-Gf-Cm-Cf-Am-Cf-Um-Gf-Cm-Cf-Cm-Uf-Gm-Cf-Am-Cf-Um-Uf | 261/63 | Ams-Afs-Gm-Uf-Gm-Cf-Am-Gf-Gm-Gf-Cm-Af-Gm-Uf-Gm-Gf-Cm-Gf-Cm-Uf-Ums-Ufs-Gm | 347/149 |
| B 1407-AL | Cfs-Gms-Cf-Cm-Af-Cm-Uf-Gm-Cf-Cm-Cf-Um-Gf-Cm-Af-Cm-Uf-Um-Cf-Cm-Uf | 262/64 | Ams-Gfs-Gm-Af-Am-Gf-Um-Gf-Cm-Af-Gm-Gf-Gm-Cf-Am-Gf-Um-Gf-Gm-Cf-Gms-Cfs-Um | 348/150 |
| B1554-AL | Gfs-Cms-Gf-Am-Uf-Gm-Uf-Gm-Uf-Cm-Af-Cm-Cf-Cm-Cf-Cm-Af-Gm-Uf-Cm-Uf | 263/65 | Ams-Gfs-Am-Cf-Um-Gf-Gm-Gf-Gm-Gf-Um-Gf-Am-Cf-Am-Cf-Am-Uf-Cm-Gf-Cms-Ufs-Gm | 349/151 |
| B1558-AL | Ufs-Gms-Uf-Gm-Uf-Cm-Af-Cm-Cf-Cm-Cf-Cm-Af-Gm-Uf-Cm-Uf-Cm-Cf-Cm-Af | 264/66 | Ums-Gfs-Gm-Gf-Am-Gf-Am-Cf-Um-Gf-Gm-Gf-Gm-Gf-Um-Gf-Am-Cf-Am-Cf-Ams-Ufs-Cm | 350/152 |
| B1561-AL | Gfs-Ums-Cf-Am-Cf-Cm-Cf-Cm-Cf-Am-Gf-Um-Cf-Um-Cf-Cm-Cf-Am-Cf-Cm-Uf | 265/67 | Ams-Gfs-Gm-Uf-Gm-Gf-Gm-Af-Gm-Af-Cm-Uf-Gm-Gf-Gm-Gf-Gm-Uf-Gm-Af-Cms-Afs-Cm | 351/153 |
| B1562-AL | Ufs-Cms-Af-Cm-Cf-Cm-Cf-Cm-Af-Gm-Uf-Cm-Uf-Cm-Cf-Cm-Af-Cm-Cf-Um-Uf | 266/68 | Ams-Afs-Gm-Gf-Um-Gf-Gm-Gf-Am-Gf-Am-Cf-Um-Gf-Gm-Gf-Gm-Gf-Um-Gf-Ams-Cfs-Am | 352/154 |
| B1564-AL | Afs-Cms-Cf-Cm-Cf-Cm-Af-Gm-Uf-Cm-Uf-Cm-Cf-Cm-Af-Cm-Cf-Um-Uf-Um-Uf | 267/69 | Ams-Afs-Am-Af-Gm-Gf-Um-Gf-Gm-Gf-Am-Gf-Am-Cf-Um-Gf-Gm-Gf-Gm-Gf-Ums-Gfs-Am | 353/155 |

(continued)

| Sequence ID | Sense strand sequence 5'-3' | Sequence SEQ ID NO./ corresponding unmodified sequence SEQ ID NO. | Antisense strand sequence 5'-3' | Sequence SEQ ID NO./ corresponding unmodified sequence SEQ ID NO. |
|---|---|---|---|---|
| B1591-AL | Afs-Ums-Gf-Am-Gf-Um-Cf-Gm-Af-Cm-Uf-Um-Uf-Gm-Af-Gm-Cf-Um-Gf-Gm-Af | 268/70 | Ums-Cfs-Cm-Af-Gm-Cf-Um-Cf-Am-Af-Am-Gf-Um-Cf-Gm-Af-Cm-Uf-Cm-Af-Ums-Ufs-Am | 354/156 |
| B1592-AL | Ufs-Gms-Af-Gm-Uf-Cm-Gf-Am-Cf-Um-Uf-Um-Gf-Am-Gf-Cm-Uf-Gm-Gf-Am-Af | 269/71 | Ums-Ufs-Cm-Cf-Am-Gf-Cm-Uf-Cm-Af-Am-Af-Gm-Uf-Cm-Gf-Am-Cf-Um-Cf-Ams-Ufs-Um | 355/157 |
| B1593-AL | Gfs-Ams-Gf-Um-Cf-Gm-Af-Cm-Uf-Um-Uf-Gm-Af-Gm-Cf-Um-Gf-Gm-Af-Am-Af | 270/72 | Ums-Ufs-Um-Cf-Cm-Af-Gm-Cf-Um-Cf-Am-Af-Am-Gf-Um-Cf-Gm-Af-Cm-Uf-Cms-Afs-Um | 356/158 |
| B1596-AL | Ufs-Cms-Gf-Am-Cf-Um-Uf-Um-Gf-Am-Gf-Cm-Uf-Gm-Gf-Am-Af-Am-Gf-Cm-Af | 271/73 | Ums-Gfs-Cm-Uf-Um-Uf-Cm-Cf-Am-Gf-Cm-Uf-Cm-Af-Am-Af-Gm-Uf-Cm-Gf-Ams-Cfs-Um | 357/159 |
| B 1602-AL | Ufs-Ums-Gf-Am-Gf-Cm-Uf-Gm-Gf-Am-Af-Am-Gf-Cm-Af-Gm-Cf-Cm-Gf-Um-Uf | 272/74 | Ams-Afs-Cm-Gf-Gm-Cf-Um-Gf-Cm-Uf-Um-Uf-Cm-Cf-Am-Gf-Cm-Uf-Cm-Af-Ams-Afs-Gm | 358/160 |
| B1603-AL | Ufs-Gms-Af-Gm-Cf-Um-Gf-Gm-Af-Am-Af-Gm-Cf-Am-Gf-Cm-Cf-Gm-Uf-Um-Uf | 273/75 | Ams-Afs-Am-Cf-Gm-Gf-Cm-Uf-Gm-Cf-Um-Uf-Um-Cf-Cm-Af-Gm-Cf-Um-Cf-Ams-Afs-Am | 359/161 |
| B 1608-AL | Ufs-Gms-Gf-Am-Af-Am-Gf-Cm-Af-Gm-Cf-Cm-Gf-Um-Uf-Um-Cf-Um-Cf-Cm-Uf | 274/76 | Ams-Gfs-Gm-Af-Gm-Af-Am-Af-Cm-Gf-Gm-Cf-Um-Gf-Cm-Uf-Um-Uf-Cm-Cf-Ams-Gfs-Cm | 360/162 |
| B1612-AL | Afs-Ams-Gf-Cm-Af-Gm-Cf-Cm-Gf-Um-Uf-Um-Cf-Um-Cf-Cm-Uf-Um-Gf-Gm-Uf | 275/77 | Ams-Cfs-Cm-Af-Am-Gf-Gm-Af-Gm-Af-Am-Af-Cm-Gf-Gm-Cf-Um-Gf-Cm-Uf-Ums-Ufs-Cm | 361/163 |

(continued)

| Sequence ID | Sense strand sequence 5'-3' | Sequence SEQ ID NO./ corresponding unmodified sequence SEQ ID NO. | Antisense strand sequence 5'-3' | Sequence SEQ ID NO./ corresponding unmodified sequence SEQ ID NO. |
|---|---|---|---|---|
| B1775-AL | Cfs-Ams-Af-Cm-Cf-Gm-Af-Cm-Cf-Am-Gf-Cm-Uf-Um-Gf-Um-Uf-Um-Gf-Um-Af | 276/78 | Ums-Afs-Cm-Af-Am-Af-Cm-Af-Am-Gf-Cm-Uf-Gm-Gf-Um-Cf-Gm-Gf-Um-Uf-Gms-Gfs-Am | 362/164 |
| B1790-AL | Ufs-Ums-Uf-Gm-Uf-Gm-Af-Am-Af-Cm-Af-Am-Af-Am-Af-Am-Gf-Um-Gf-Um-Uf | 277/79 | Ams-Afs-Cm-Af-Cm-Uf-Um-Uf-Um-Uf-Um-Gf-Um-Uf-Um-Cf-Am-Cf-Am-Af-Ams-Cfs-Am | 363/165 |
| B 1796-AL | Afs-Ams-Af-Cm-Af-Am-Af-Am-Af-Am-Gf-Um-Gf-Um-Uf-Cm-Cf-Cm-Uf-Um-Uf | 278/80 | Ams-Afs-Am-Gf-Gm-Gf-Am-Af-Cm-Af-Cm-Uf-Um-Uf-Um-Uf-Um-Gf-Um-Uf-Ums-Cfs-Am | 364/166 |
| B1798s-AL | Ams-Cfs-Am-Af-Am-Af-Am-Af-Gm-Uf-Gm-Uf-Um-Cf-Cm-Cf-Um-Uf-Um-Uf | 279/81 | Ams-Afs-Am-Af-Gm-Gf-Gm-Af-Am-Cf-Am-Cf-Um-Uf-Um-Uf-Um-Uf-Gm-Ufs-Ums-Uf | 365/167 |
| B1799-AL | Cfs-Ams-Af-Am-Af-Am-Af-Gm-Uf-Gm-Uf-Um-Cf-Cm-Cf-Um-Uf-Um-Uf-Cm-Af | 280/82 | Ums-Gfs-Am-Af-Am-Af-Gm-Gf-Gm-Af-Am-Cf-Am-Cf-Um-Uf-Um-Uf-Um-Uf-Gms-Ufs-Um | 366/168 |
| B1810-AL | Ufs-Cms-Cf-Cm-Uf-Um-Uf-Um-Cf-Am-Af-Gm-Uf-Um-Gf-Am-Gf-Am-Af-Cm-Af | 281/83 | Ums-Gfs-Um-Uf-Cm-Uf-Cm-Af-Am-Cf-Um-Uf-Gm-Af-Am-Af-Am-Gf-Gm-Gf-Ams-Afs-Cm | 367/169 |
| B1811-AL | Cfs-Cms-Cf-Um-Uf-Um-Uf-Cm-Af-Am-Gf-Um-Uf-Gm-Af-Gm-Af-Am-Cf-Am-Af | 282/84 | Ums-Ufs-Gm-Uf-Um-Cf-Um-Cf-Am-Af-Cm-Uf-Um-Gf-Am-Af-Am-Af-Gm-Gf-Gms-Afs-Am | 368/170 |
| B1814-AL | Ufs-Ums-Uf-Um-Cf-Am-Af-Gm-Uf-Um-Gf-Am-Gf-Am-Af-Cm-Af-Am-Af-Am-Af | 283/85 | Ums-Ufs-Um-Uf-Um-Gf-Um-Uf-Cm-Uf-Cm-Af-Am-Cf-Um-Uf-Gm-Af-Am-Af-Ams-Gfs-Gm | 369/171 |

(continued)

| Sequence ID | Sense strand sequence 5'-3' | Sequence SEQ ID NO./ corresponding unmodified sequence SEQ ID NO. | Antisense strand sequence 5'-3' | Sequence SEQ ID NO./ corresponding unmodified sequence SEQ ID NO. |
|---|---|---|---|---|
| B1815-AL | Ufs-Ums-Uf-Cm-Af-Am-Gf-Um-Uf-Gm-Af-Gm-Af-Am-Cf-Am-Af-Am-Af-Am-Uf | 284/86 | Ams-Ufs-Um-Uf-Um-Uf-Gm-Uf-Um-Cf-Um-Cf-Am-Af-Cm-Uf-Um-Gf-Am-Af-Ams-Afs-Gm | 370/172 |
| B1817-AL | Ufs-Cms-Af-Am-Gf-Um-Uf-Gm-Af-Gm-Af-Am-Cf-Am-Af-Am-Af-Am-Uf-Um-Gf | 285/87 | Cms-Afs-Am-Uf-Um-Uf-Um-Uf-Gm-Uf-Um-Cf-Um-Cf-Am-Af-Cm-Uf-Um-Gf-Ams-Afs-Am | 371/173 |
| B1828-AL | Afs-Cms-Af-Am-Af-Am-Af-Um-Uf-Gm-Gf-Gm-Uf-Um-Uf-Um-Af-Am-Af-Am-Uf | 286/88 | Ams-Ufs-Um-Uf-Um-Af-Am-Af-Am-Cf-Cm-Cf-Am-Af-Um-Uf-Um-Uf-Um-Gf-Ums-Ufs-Cm | 372/174 |
| B1838s-AL | Gms-Gfs-Um-Uf-Um-Uf-Am-Af-Am-Af-Um-Uf-Am-Af-Am-Gf-Um-Af-Um-Af | 287/89 | Ums-Afs-Um-Af-Cm-Uf-Um-Uf-Am-Af-Um-Uf-Um-Uf-Am-Af-Am-Af-Cm-Cfs-Cms-Af | 373/175 |
| B2018-AL | Afs-Ums-Af-Gm-Cf-Um-Gf-Gm-Uf-Um-Af-Um-Uf-Um-Cf-Um-Cf-Cm-Cf-Um-Uf | 288/90 | Ams-Afs-Gm-Gf-Gm-Af-Gm-Af-Am-Af-Um-Af-Am-Cf-Cm-Af-Gm-Cf-Um-Af-Ums-Gfs-Gm | 374/176 |
| B2020-AL | Afs-Gms-Cf-Um-Gf-Gm-Uf-Um-Af-Um-Uf-Um-Cf-Um-Cf-Cm-Cf-Um-Uf-Gm-Uf | 289/91 | Ams-Cfs-Am-Af-Gm-Gf-Gm-Af-Gm-Af-Am-Af-Um-Af-Am-Cf-Cm-Af-Gm-Cf-Ums-Afs-Um | 375/177 |
| B2025-AL | Gfs-Ums-Uf-Am-Uf-Um-Uf-Cm-Uf-Cm-Cf-Cm-Uf-Um-Gf-Um-Gf-Um-Uf-Am-Af | 290/92 | Ums-Ufs-Am-Af-Cm-Af-Cm-Af-Am-Gf-Gm-Gf-Am-Gf-Am-Af-Am-Uf-Am-Af-Cms-Cfs-Am | 376/178 |
| B2030-AL | Ufs-Ums-Cf-Um-Cf-Cm-Cf-Um-Uf-Gm-Uf-Gm-Uf-Um-Af-Gm-Uf-Am-Af-Um-Af | 291/93 | Ums-Afs-Um-Uf-Am-Cf-Um-Af-Am-Cf-Am-Cf-Am-Af-Gm-Gf-Gm-Af-Gm-Af-Ams-Afs-Um | 377/179 |

(continued)

| Sequence ID | Sense strand sequence 5'-3' | Sequence SEQ ID NO./ corresponding unmodified sequence SEQ ID NO. | Antisense strand sequence 5'-3' | Sequence SEQ ID NO./ corresponding unmodified sequence SEQ ID NO. |
|---|---|---|---|---|
| B2031-AL | Ufs-Cms-Uf-Cm-Cf-Cm-Uf-Um-Gf-Um-Gf-Um-Uf-Am-Gf-Um-Af-Am-Uf-Am-Af | 292/94 | Ums-Ufs-Am-Uf-Um-Af-Cm-Uf-Am-Af-Cm-Af-Cm-Af-Am-Gf-Gm-Gf-Am-Gf-Ams-Afs-Am | 378/180 |
| B2032-AL | Cfs-Ums-Cf-Cm-Cf-Um-Uf-Gm-Uf-Gm-Uf-Um-Af-Gm-Uf-Am-Af-Um-Af-Am-Af | 293/95 | Ums-Ufs-Um-Af-Um-Uf-Am-Cf-Um-Af-Am-Cf-Am-Cf-Am-Af-Gm-Gf-Gm-Af-Gms-Afs-Am | 379/181 |
| B2033-AL | Ufs-Cms-Cf-Cm-Uf-Um-Gf-Um-Gf-Um-Uf-Am-Gf-Um-Af-Am-Uf-Am-Af-Am-Af | 294/96 | Ums-Ufs-Um-Uf-Am-Uf-Um-Af-Cm-Uf-Am-Af-Cm-Af-Cm-Af-Am-Gf-Gm-Gf-Ams-Gfs-Am | 380/182 |
| B729-AL | Afs-Gms-Af-Am-Cf-Um-Gf-Gm-Af-Um-Gf-Um-Uf-Gm-Cf-Um-Gf-Cm-Uf-Gm-Af | 295/97 | Ums-Cfs-Am-Gf-Cm-Af-Gm-Cf-Am-Af-Cm-Af-Um-Cf-Cm-Af-Gm-Uf-Um-Cf-Ums-Gfs-Um | 381/183 |
| B731-AL | Afs-Ams-Cf-Um-Gf-Gm-Af-Um-Gf-Um-Uf-Gm-Cf-Um-Gf-Cm-Uf-Gm-Af-Gm-Af | 296/98 | Ums-Cfs-Um-Cf-Am-Gf-Cm-Af-Gm-Cf-Am-Af-Cm-Af-Um-Cf-Cm-Af-Gm-Uf-Ums-Cfs-Um | 382/184 |
| B732-AL | Afs-Cms-Uf-Gm-Gf-Am-Uf-Gm-Uf-Um-Gf-Cm-Uf-Gm-Cf-Um-Gf-Am-Gf-Am-Af | 297/99 | Ums-Ufs-Cm-Uf-Cm-Af-Gm-Cf-Am-Gf-Cm-Af-Am-Cf-Am-Uf-Cm-Cf-Am-Gf-Ums-Ufs-Cm | 383/185 |
| B734-AL | Ufs-Gms-Gf-Am-Uf-Gm-Uf-Um-Gf-Cm-Uf-Gm-Cf-Um-Gf-Am-Gf-Am-Af-Gm-Af | 298/100 | Ums-Cfs-Um-Uf-Cm-Uf-Cm-Af-Gm-Cf-Am-Gf-Cm-Af-Am-Cf-Am-Uf-Cm-Cf-Ams-Gfs-Um | 384/186 |
| B734s-AL | Ums-Gfs-Gm-Af-Um-Gf-Um-Uf-Gm-Cf-Um-Gf-Cm-Uf-Gm-Af-Gm-Af-Am-Af | 299/101 | Ums-Ufs-Um-Cf-Um-Cf-Am-Gf-Cm-Af-Gm-Cf-Am-Af-Cm-Af-Um-Cf-Cm-Afs-Gms-Uf | 385/187 |

(continued)

| Sequence ID | Sense strand sequence 5'-3' | Sequence SEQ ID NO./ corresponding unmodified sequence SEQ ID NO. | Antisense strand sequence 5'-3' | Sequence SEQ ID NO./ corresponding unmodified sequence SEQ ID NO. |
|---|---|---|---|---|
| B736-AL | Gfs-Ams-Uf-Gm-Uf-Um-Gf-Cm-Uf-Gm-Cf-Um-Gf-Am-Gf-Am-Af-Gm-Af-Um-Uf | 300/102 | Ams-Afs-Um-Cf-Um-Uf-Cm-Uf-Cm-Af-Gm-Cf-Am-Gf-Cm-Af-Am-Cf-Am-Uf-Cms-Cfs-Am | 386/188 |
| B743-AL | Cfs-Ums-Gf-Cm-Uf-Gm-Af-Gm-Af-Am-Gf-Am-Uf-Um-Gf-Am-Cf-Am-Gf-Gm-Uf | 301/103 | Ams-Cfs-Cm-Uf-Gm-Uf-Cm-Af-Am-Uf-Cm-Uf-Um-Cf-Um-Cf-Am-Gf-Cm-Af-Gms-Cfs-Am | 387/189 |
| B753sAL | Gms-Afs-Um-Uf-Gm-Af-Cm-Af-Gm-Gf-Um-Uf-Cm-Af-Um-Gf-Cm-Af-Gm-Af | 302/104 | Ums-Cfs-Um-Gf-Cm-Af-Um-Gf-Am-Af-Cm-Cf-Um-Gf-Um-Cf-Am-Af-Um-Cfs-Ums-Uf | 388/190 |
| B963-AL | Gfs-Cms-Af-Cm-Uf-Gm-Gf-Am-Gf-Um-Gf-Am-Cf-Am-Uf-Cm-Cf-Am-Gf-Gm-Af | 303/105 | Ums-Cfs-Cm-Uf-Gm-Gf-Am-Uf-Gm-Uf-Cm-Af-Cm-Uf-Cm-Cf-Am-Gf-Um-Gf-Cms-Ufs-Gm | 389/191 |
| B966-AL | Cfs-Ums-Gf-Gm-Af-Gm-Uf-Gm-Af-Cm-Af-Um-Cf-Cm-Af-Gm-Gf-Am-Cf-Am-Af | 304/106 | Ums-Ufs-Gm-Uf-Cm-Cf-Um-Gf-Gm-Af-Um-Gf-Um-Cf-Am-Cf-Um-Cf-Cm-Af-Gms-Ufs-Gm | 390/192 |
| B969-AL | Gfs-Ams-Gf-Um-Gf-Am-Cf-Am-Uf-Cm-Cf-Am-Gf-Gm-Af-Cm-Af-Am-Cf-Um-Uf | 305/107 | Ams-Afs-Gm-Uf-Um-Gf-Um-Cf-Cm-Uf-Gm-Gf-Am-Uf-Gm-Uf-Cm-Af-Cm-Uf-Cms-Cfs-Am | 391/193 |
| B971-AL | Gfs-Ums-Gf-Am-Cf-Am-Uf-Cm-Cf-Am-Gf-Gm-Af-Cm-Af-Am-Cf-Um-Uf-Cm-Uf | 306/108 | Ams-Gfs-Am-Af-Gm-Uf-Um-Gf-Um-Cf-Cm-Uf-Gm-Gf-Am-Uf-Gm-Uf-Cm-Af-Cms-Ufs-Cm | 392/194 |
| B982-AL | Gfs-Ams-Cf-Am-Af-Cm-Uf-Um-Cf-Um-Cf-Gm-Gf-Um-Gf-Am-Cf-Um-Cf-Am-Af | 307/109 | Ums-Ufs-Gm-Af-Gm-Uf-Cm-Af-Cm-Cf-Gm-Af-Gm-Af-Am-Gf-Um-Uf-Gm-Uf-Cms-Cfs-Um | 393/195 |

(continued)

| Sequence ID | Sense strand sequence 5'-3' | Sequence SEQ ID NO./ corresponding unmodified sequence SEQ ID NO. | Antisense strand sequence 5'-3' | Sequence SEQ ID NO./ corresponding unmodified sequence SEQ ID NO. |
|---|---|---|---|---|
| B984-AL | Cfs-Ams-Af-Cm-Uf-Um-Cf-Um-Cf-Gm-Gf-Um-Gf-Am-Cf-Um-Cf-Am-Af-Gm-Uf | 308/110 | Ams-Cfs-Um-Uf-Gm-Af-Gm-Uf-Cm-Af-Cm-Cf-Gm-Af-Gm-Af-Am-Gf-Um-Uf-Gms-Ufs-Cm | 394/196 |
| B992-AL | Cfs-Gms-Gf-Um-Gf-Am-Cf-Um-Cf-Am-Af-Gm-Uf-Gm-Cf-Cm-Cf-Um-Uf-Cm-Af | 309/111 | Ums-Gfs-Am-Af-Gm-Gf-Gm-Cf-Am-Cf-Um-Uf-Gm-Af-Gm-Uf-Cm-Af-Cm-Cf-Gms-Afs-Gm | 395/197 |
| B994-AL | Gfs-Ums-Gf-Am-Cf-Um-Cf-Am-Af-Gm-Uf-Gm-Cf-Cm-Cf-Um-Uf-Cm-Af-Cm-Uf | 310/112 | Ams-Gfs-Um-Gf-Am-Af-Gm-Gf-Gm-Cf-Am-Cf-Um-Uf-Gm-Af-Gm-Uf-Cm-Af-Cms-Cfs-Gm | 396/198 |

## Example 2: Inhibition of alternately modified sequences on AGT gene expression

After the siRNA sequences alternately modified with 2'-OMe and 2'-F synthesized in Example 1 were transfected into HepG2 cells via lipid nanoparticles (LNPs), the inhibitory effects of these sequences on the AGT gene were tested using qPCR technology.

### 1. Experimental materials

Test samples: the alternately modified siRNA sequences listed in Table 1 (synthesized in Example 1).

Cell type: HepG2 cell line.
Drug vehicle: sterile nuclease-free water, Gibco Opti-MEM.

### 2. Experimental methods

The inhibitory effects of the test samples on the mRNA expression of AGT genes in HepG2 cell lines were tested by qRT-PCR.

### 2.1 Cell culture

HepG2 cells in the logarithmic growth phase were harvested from subcultured stocks and cultured in DMEM medium containing 10% fetal bovine serum (supplemented with 100× penicillin and 100× streptomycin at 10 μL/mL). The cells were incubated in a cell culture incubator at 37°C with 5% $CO_2$, with medium replaced daily. For subculture, the cells were digested with 0.25% trypsin, centrifuged at 800 rpm for 3 minutes, and the supernatant was discarded. Fresh medium was added for subculture.

### 2.2 Cell transfection

Preparation of transfection mixture: Lipofectamine RNAiMAX and Opti-MEM were mixed at a ratio of 2:98 and vortexed thoroughly.
Preparation of transfection reagent: 65 μL of siRNA solution diluted in Opti-MEM was mixed with 65 μL of the prepared transfection mixture at a ratio of 1:1 (v/v), vortexed thoroughly, and the mixture was incubated at room temperature for 15 minutes to obtain lipid nanoparticles (LNPs). A 12.5 μL aliquot was taken for encapsulation efficiency analysis.
Blank control transfection reagent: 65 μL of the prepared transfection mixture was added to 65 μL of Opti-MEM, vortexed thoroughly, and the mixture was incubated at room temperature for 15 minutes.
The prepared transfection reagent was added to a 24-well cell culture plate (100 μL per well), resulting in a final siRNA concentration of 0.07 nM per well. 500 μL of cell suspension ($1.5 \times 10^5$ cells per mL) was added per well. After mixing by crosswise method, the plate was incubated in a cell culture incubator at 37°C with 5% $CO_2$ for 40 hours.

### 2.3 AGT mRNA assay

1) RNA extraction

a. The culture medium was aspirated from the 12-well plate, and the cells were washed with 0.5 mL of 1× PBS per well, followed by aspiration of the PBS. 0.5 mL of TRIzol reagent was added per well, and the cells were fully lysed by pipetting. The lysate was transferred to a 1.5 mL RNase-free EP tube and incubated at room temperature for 5 minutes.
b. 0.1 mL of chloroform was added per tube, followed by vigorous vortexing for 15 seconds and incubation at room temperature for 5 minutes. The mixture was centrifuged at 12,000 × g for 15 minutes at 4°C, and 200 μL of the supernatant was transferred to a new EP tube.
c. An equal volume of isopropanol was added, and the liquid in the tube was gently mixed by inversion. The mixture was incubated at -20°C for 10 minutes, followed by centrifugation at 12,000 × g for 15 minutes at 4°C, and the supernatant was discarded.
d. The RNA pellet was gently washed with 0.5 mL of 75% ethanol, followed by centrifugation at 12,000 × g for 5 minutes at 4°C, and the supernatant was aspirated. The washing step was repeated once, followed by centrifugation

at 12,000 × g for 1 minute at 4°C, and residual ethanol was completely removed with a micropipette tip.

e. The RNA pellet was air-dried at room temperature for 2 to 3 minutes to remove residual ethanol, and dissolved in 40 μL of RNase-free $ddH_2O$.

2) RNA concentration measurement

RNA concentration was measured using a NanoDrop spectrophotometer. 2 μL of RNase-free $ddH_2O$ was used as a blank control, and 2 μL of each RNA sample was loaded for measurement. The sample concentration was recorded.

3) Reverse transcription of AGT mRNA

Reverse transcription reagent, RNA solution, and water were mixed at a volume ratio of 2:5:3. The mixture was reacted in a PCR instrument at 37°C for 15 minutes and 85°C for 5 seconds, and finally maintained at 4°C. The resulting cDNA was diluted 5-fold using sterile nuclease-free water.

4) Quantitative analysis of AGT mRNA

In a 15 mL centrifuge tube, qPCR reagent, forward primer, and reverse primer were mixed at a volume ratio of 5:0.1 :0.1, labeled as Solution A.

In a pre-labeled 1.5 mL EP tube, diluted cDNA and water were mixed at a volume ratio of 1:3.8, labeled as Solution B.

In a 96-well PCR plate, 5.2 μL of Solution A and 4.8 μL of Solution B were added per well. The plate was sealed with a sealing film, centrifuged at 3000 rpm for 1 minute, and loaded for qPCR analysis.

*All operations in this section were performed on ice to maintain low-temperature conditions.

The plate was placed in a qPCR instrument and run according to the following program:

Pre-denaturation: 95°C for 30 seconds;
Cycling reaction: 95°C for 5 seconds; 60°C for 34 seconds; 40 cycles;
Melting curve: 95°C for 15 seconds; 60°C for 60 seconds; 95°C for 15 seconds.

The total run time was approximately 2 hours. Experimental results were analyzed, and $2^{-\Delta\Delta Ct}$ was calculated.

**2.4 Data processing**

AGT mRNA expression rate (%) was calculated using the following formula:

Expression rate = (AGT mRNA expression level / AGT mRNA expression level in blank control group) × 100%;

$$\text{Inhibition rate of AGT gene expression} = 100\% - \text{expression rate } (\%).$$

**3. Experimental results**

In this example, the inhibition rate represents the average value of three independent experiments. The inhibition rates of AGT mRNA expression in HepG2 cells by specific sequences are shown in Tables 2 to 5.

The experimental results demonstrated that some sequences alternately modified with 2'-OMe and 2'-F, including sequence B1789-AL, exhibited significant inhibitory effects on AGT mRNA expression in HepG2 cells, with inhibition rates exceeding 45%. Specifically, B1576sAL exhibited an inhibition rate exceeding 60%.

The remaining sequences exhibited poor inhibitory effects on AGT mRNA expression, with inhibition rates below 45%.

**(1) Among the 99 designed sequences, 33 sequences exhibited significant inhibitory effects on the AGT gene, with inhibition rates exceeding 40%. Some of these sequences exhibited inhibition rates exceeding 60%.**

Table 2: Alternately modified sequences with significant inhibitory effects on AGT gene expression (inhibition rate > 40%)

| Unmodified sequence ID | Alternately modified sequence ID | Inhibition rate (%) |
|---|---|---|
| 1576s | B1576s-AL | 64.6 |
| 1578s | B1578s-AL | 63.9 |
| 1838 | B1838-AL | 63.6 |
| 1789 | B 1789-AL | 63.5 |
| 1812 | B1812-AL | 62.9 |
| 1835 | B1835-AL | 62.3 |
| 1791 | B1791-AL | 61.8 |
| 1579 | B1579-AL | 61.1 |
| 1816 | B1816-AL | 60.9 |
| 1790 | B1790-AL | 60.3 |
| 1836 | B1836-AL | 59.8 |
| 1585s | B1585s-AL | 59.3 |
| 1839 | B1839-AL | 59.1 |
| 1838s | B1838s-AL | 58.4 |
| 1796 | B1796-AL | 57.9 |
| 1795 | B1795-AL | 57.5 |
| 1817 | B1817-AL | 57.3 |
| 734s | B734s-AL | 56.4 |
| 1798s | B1798s-AL | 54.4 |
| 736 | B736-AL | 53.3 |
| 1608 | B1608-AL | 52.5 |
| 1282 | B1282-AL | 51.8 |
| 1811 | B1811-AL | 51.5 |
| 1281 | B1281-AL | 50.4 |
| 1799 | B1799-AL | 49.2 |
| 1603 | B1603-AL | 48.6 |
| 1828 | B1828-AL | 46.6 |
| 1592 | B1592-AL | 44.4 |
| 1814 | B1814-AL | 43.2 |
| 1593 | B 1593-AL | 42.4 |
| 1810 | B1810-AL | 42.1 |
| 1365 | B1365-AL | 42.0 |
| 1279 | B1279-AL | 41.4 |

As shown in Table 2, the alternately modified sequences in the above table (with specific sequences listed in Table 1) exhibited significant inhibitory effects on AGT mRNA expression, with inhibition rates of 40% or more. Specifically, the top 10 sequences exhibited inhibition rates exceeding 60% (FIG. 1).

The sequences of the compounds in Table 2 are listed in Table 3 below.

Table 3: siRNA sequences with significant inhibitory effects on AGT gene expression

| Sequence ID | Sense strand sequence 5'-3' | Sequence SEQ ID NO./ corresponding unmodified sequence SEQ ID NO. | Antisense strand sequence 5'-3' | Sequence SEQ ID NO./ corresponding unmodified sequence SEQ ID NO. |
|---|---|---|---|---|
| B1576s-AL | Cms-Cfs-Am-Cf-Cm-Uf-Um-Uf-Um-Cf-Um-Uf-Cm-Uf-Am-Af-Um-Gf-Am-Af | 209/11 | Ums-Ufs-Cm-Af-Um-Uf-Am-Gf-Am-Af-Gm-Af-Am-Af-Am-Gf-Gm-Uf-Gm-Gfs-Gms-Af | 222/24 |
| B1578s-AL | Ams-Cfs-Cm-Uf-Um-Uf-Um-Cf-Um-Uf-Cm-Uf-Am-Af-Um-Gf-Am-Gf-Um-Af | 210/12 | Ums-Afs-Cm-Uf-Cm-Af-Um-Uf-Am-Gf-Am-Af-Gm-Af-Am-Af-Am-Gf-Gm-Ufs-Gms-Gf | 223/25 |
| B1838-AL | Gfs-Gms-Uf-Um-Uf-Um-Af-Am-Af-Am-Uf-Um-Af-Am-Af-Gm-Uf-Am-Uf-Am-Af | 211/13 | Ums-Ufs-Am-Uf-Am-Cf-Um-Uf-Um-Af-Am-Uf-Um-Uf-Um-Af-Am-Af-Am-Cf-Cms-Cfs-Am | 224/26 |
| B1789-AL | Gfs-Ums-Uf-Um-Gf-Um-Gf-Am-Af-Am-Cf-Am-Af-Am-Af-Am-Af-Gm-Uf-Gm-Uf | 204/6 | Ams-Cfs-Am-Cf-Um-Uf-Um-Uf-Um-Uf-Gm-Uf-Um-Uf-Cm-Af-Cm-Af-Am-Af-Cms-Afs-Am | 217/19 |
| B1812-AL | Cfs-Cms-Uf-Um-Uf-Um-Cf-Am-Af-Gm-Uf-Um-Gf-Am-Gf-Am-Af-Cm-Af-Am-Af | 201/3 | Ums-Ufs-Um-Gf-Um-Uf-Cm-Uf-Cm-Af-Am-Cf-Um-Uf-Gm-Af-Am-Af-Am-Gf-Gms-Gfs-Am | 214/16 |
| B1835-AL | Ufs-Ums-Gf-Gm-Gf-Um-Uf-Um-Uf-Am-Af-Am-Af-Um-Uf-Am-Af-Am-Gf-Um-Af | 199/1 | Ums-Afs-Cm-Uf-Um-Uf-Am-Af-Um-Uf-Um-Uf-Am-Af-Am-Af-Cm-Cf-Cm-Af-Ams-Ufs-Um | 212/14 |
| B1791-AL | Ufs-Ums-Gf-Um-Gf-Am-Af-Am-Cf-Am-Af-Am-Af-Am-Af-Gm-Uf-Gm-Uf-Um-Af | 206/8 | Ums-Afs-Am-Cf-Am-Cf-Um-Uf-Um-Uf-Um-Uf-Gm-Uf-Um-Uf-Cm-Af-Cm-Af-Ams-Afs-Cm | 219/21 |
| B1579-AL | Cfs-Cms-Uf-Um-Uf-Um-Cf-Um-Uf-Cm-Uf-Am-Af-Um-Gf-Am-Gf-Um-Cf-Gm-Af | 202/4 | Ums-Cfs-Gm-Af-Cm-Uf-Cm-Af-Um-Uf-Am-Gf-Am-Af-Gm-Af-Am-Af-Am-Gf-Gms-Ufs-Gm | 215/17 |

(continued)

| Sequence ID | Sense strand sequence 5'-3' | Sequence SEQ ID NO./ corresponding unmodified sequence SEQ ID NO. | Antisense strand sequence 5'-3' | Sequence SEQ ID NO./ corresponding unmodified sequence SEQ ID NO. |
|---|---|---|---|---|
| B1816-AL | Ufs-Ums-Cf-Am-Af-Gm-Uf-Um-Gf-Am-Gf-Am-Af-Cm-Af-Am-Af-Am-Af-Um-Uf | 200/2 | Ams-Afs-Um-Uf-Um-Uf-Um-Gf-Um-Uf-Cm-Uf-Cm-Af-Am-Cf-Um-Uf-Gm-Af-Ams-Afs-Am | 213/15 |
| B1790-AL | Ufs-Ums-Uf-Gm-Uf-Gm-Af-Am-Af-Cm-Af-Am-Af-Am-Af-Am-Gf-Um-Gf-Um-Uf | 277/79 | Ams-Afs-Cm-Af-Cm-Uf-Um-Uf-Um-Uf-Um-Gf-Um-Uf-Um-Cf-Am-Cf-Am-Af-Ams-Cfs-Am | 363/165 |
| B1836-AL | Ufs-Gms-Gf-Gm-Uf-Um-Uf-Um-Af-Am-Af-Am-Uf-Um-Af-Am-Af-Gm-Uf-Am-Uf | 203/5 | Ams-Ufs-Am-Cf-Um-Uf-Um-Af-Am-Uf-Um-Uf-Um-Af-Am-Af-Am-Cf-Cm-Cf-Ams-Afs-Um | 216/18 |
| B1585s-AL | Cms-Ufs-Um-Cf-Um-Af-Am-Uf-Gm-Af-Gm-Uf-Cm-Gf-Am-Cf-Um-Uf-Um-Af | 208/10 | Ums-Afs-Am-Af-Gm-Uf-Cm-Gf-Am-Cf-Um-Cf-Am-Uf-Um-Af-Gm-Af-Am-Gfs-Ams-Af | 221/23 |
| B1839-AL | Gfs-Ums-Uf-Um-Uf-Am-Af-Am-Af-Um-Uf-Am-Af-Am-Gf-Um-Af-Um-Af-Cm-Af | 205/7 | Ums-Gfs-Um-Af-Um-Af-Cm-Uf-Um-Uf-Am-Af-Um-Uf-Um-Uf-Am-Af-Am-Af-Cms-Cfs-Cm | 218/20 |
| B1838s-AL | Gms-Gfs-Um-Uf-Um-Uf-Am-Af-Am-Af-Um-Uf-Am-Af-Am-Gf-Um-Af-Um-Af | 287/89 | Ums-Afs-Um-Af-Cm-Uf-Um-Uf-Am-Af-Um-Uf-Um-Uf-Am-Af-Am-Af-Cm-Cfs-Cms-Af | 373/175 |
| B1796-AL | Afs-Ams-Af-Cm-Af-Am-Af-Am-Af-Am-Gf-Um-Gf-Um-Uf-Cm-Cf-Cm-Uf-Um-Uf | 278/80 | Ams-Afs-Am-Gf-Gm-Gf-Am-Af-Cm-Af-Cm-Uf-Um-Uf-Um-Uf-Um-Gf-Um-Uf-Ums-Cfs-Am | 364/166 |
| B1795-AL | Gfs-Ams-Af-Am-Cf-Am-Af-Am-Af-Am-Af-Gm-Uf-Gm-Uf-Um-Cf-Cm-Cf-Um-Uf | 207/9 | Ams-Afs-Gm-Gf-Gm-Af-Am-Cf-Am-Cf-Um-Uf-Um-Uf-Um-Uf-Gm-Uf-Um-Uf-Cms-Afs-Cm | 220/22 |

(continued)

| Sequence ID | Sense strand sequence 5'-3' | Sequence SEQ ID NO./ corresponding unmodified sequence SEQ ID NO. | Antisense strand sequence 5'-3' | Sequence SEQ ID NO./ corresponding unmodified sequence SEQ ID NO. |
|---|---|---|---|---|
| B1817-AL | Ufs-Cms-Af-Am-Gf-Um-Uf-Gm-Af-Gm-Af-Am-Cf-Am-Af-Am-Af-Am-Uf-Um-Gf | 285/87 | Cms-Afs-Am-Uf-Um-Uf-Um-Uf-Gm-Uf-Um-Cf-Um-Cf-Am-Af-Cm-Uf-Um-Gf-Ams-Afs-Am | 371/173 |
| B734s-AL | Ums-Gfs-Gm-Af-Um-Gf-Um-Uf-Gm-Cf-Um-Gf-Cm-Uf-Gm-Af-Gm-Af-Am-Af | 299/101 | Ums-Ufs-Um-Cf-Um-Cf-Am-Gf-Cm-Af-Gm-Cf-Am-Af-Cm-Af-Um-Cf-Cm-Afs-Gms-Uf | 385/187 |
| B1798s-AL | Ams-Cfs-Am-Af-Am-Af-Am-Af-Gm-Uf-Gm-Uf-Um-Cf-Cm-Cf-Um-Uf-Um-Uf | 279/81 | Ams-Afs-Am-Af-Gm-Gf-Gm-Af-Am-Cf-Am-Cf-Um-Uf-Um-Uf-Um-Uf-Gm-Ufs-Ums-Uf | 365/167 |
| B736-AL | Gfs-Ams-Uf-Gm-Uf-Um-Gf-Cm-Uf-Gm-Cf-Um-Gf-Am-Gf-Am-Af-Gm-Af-Um-Uf | 300/102 | Ams-Afs-Um-Cf-Um-Uf-Cm-Uf-Cm-Af-Gm-Cf-Am-Gf-Cm-Af-Am-Cf-Am-Uf-Cms-Cfs-Am | 386/188 |
| B1608-AL | Ufs-Gms-Gf-Am-Af-Am-Gf-Cm-Af-Gm-Cf-Cm-Gf-Um-Uf-Um-Cf-Um-Cf-Cm-Uf | 274/76 | Ams-Gfs-Gm-Af-Gm-Af-Am-Af-Cm-Gf-Gm-Cf-Um-Gf-Cm-Uf-Um-Uf-Cm-Cf-Ams-Gfs-Cm | 360/162 |
| B1282-AL | Afs-Gms-Cf-Am-Uf-Um-Uf-Um-Uf-Um-Uf-Um-Gf-Am-Gf-Cm-Uf-Um-Gf-Am-Af | 254/56 | Ums-Ufs-Cm-Af-Am-Gf-Cm-Uf-Cm-Af-Am-Af-Am-Af-Am-Af-Am-Uf-Gm-Cf-Ums-Gfs-Um | 340/142 |
| B1811-AL | Cfs-Cms-Cf-Um-Uf-Um-Uf-Cm-Af-Am-Gf-Um-Uf-Gm-Af-Gm-Af-Am-Cf-Am-Af | 282/84 | Ums-Ufs-Gm-Uf-Um-Cf-Um-Cf-Am-Af-Cm-Uf-Um-Gf-Am-Af-Am-Af-Gm-Gf-Gms-Afs-Am | 368/170 |
| B1281-AL | Cfs-Ams-Gf-Cm-Af-Um-Uf-Um-Uf-Um-Uf-Um-Uf-Gm-Af-Gm-Cf-Um-Uf-Gm-Af | 253/55 | Ums-Cfs-Am-Af-Gm-Cf-Um-Cf-Am-Af-Am-Af-Am-Af-Am-Af-Um-Gf-Cm-Uf-Gms-Ufs-Um | 339/141 |

(continued)

| Sequence ID | Sense strand sequence 5'-3' | Sequence SEQ ID NO./ corresponding unmodified sequence SEQ ID NO. | Antisense strand sequence 5'-3' | Sequence SEQ ID NO./ corresponding unmodified sequence SEQ ID NO. |
|---|---|---|---|---|
| B1799-AL | Cfs-Ams-Af-Am-Af-Am-Af-Gm-Uf-Gm-Uf-Um-Cf-Cm-Cf-Um-Uf-Um-Uf-Cm-Af | 280/82 | Ums-Gfs-Am-Af-Am-Af-Gm-Gf-Gm-Af-Am-Cf-Am-Cf-Um-Uf-Um-Uf-Um-Uf-Gms-Ufs-Um | 366/168 |
| B1603-AL | Ufs-Gms-Af-Gm-Cf-Um-Gf-Gm-Af-Am-Af-Gm-Cf-Am-Gf-Cm-Cf-Gm-Uf-Um-Uf | 273/75 | Ams-Afs-Am-Cf-Gm-Gf-Cm-Uf-Gm-Cf-Um-Uf-Um-Cf-Cm-Af-Gm-Cf-Um-Cf-Ams-Afs-Am | 359/161 |
| B1828-AL | Afs-Cms-Af-Am-Af-Am-Af-Um-Uf-Gm-Gf-Gm-Uf-Um-Uf-Um-Af-Am-Af-Am-Uf | 286/88 | Ams-Ufs-Um-Uf-Um-Af-Am-Af-Am-Cf-Cm-Cf-Am-Af-Um-Uf-Um-Uf-Um-Gf-Ums-Ufs-Cm | 372/174 |
| B1592-AL | Ufs-Gms-Af-Gm-Uf-Cm-Gf-Am-Cf-Um-Uf-Um-Gf-Am-Gf-Cm-Uf-Gm-Gf-Am-Af | 269/71 | Ums-Ufs-Cm-Cf-Am-Gf-Cm-Uf-Cm-Af-Am-Af-Gm-Uf-Cm-Gf-Am-Cf-Um-Cf-Ams-Ufs-Um | 355/157 |
| B1814-AL | Ufs-Ums-Uf-Um-Cf-Am-Af-Gm-Uf-Um-Gf-Am-Gf-Am-Af-Cm-Af-Am-Af-Am-Af | 283/85 | Ums-Ufs-Um-Uf-Um-Gf-Um-Uf-Cm-Uf-Cm-Af-Am-Cf-Um-Uf-Gm-Af-Am-Af-Ams-Gfs-Gm | 369/171 |
| B1593-AL | Gfs-Ams-Gf-Um-Cf-Gm-Af-Cm-Uf-Um-Uf-Gm-Af-Gm-Cf-Um-Gf-Gm-Af-Am-Af | 270/72 | Ums-Ufs-Um-Cf-Cm-Af-Gm-Cf-Um-Cf-Am-Af-Am-Gf-Um-Cf-Gm-Af-Cm-Uf-Cms-Afs-Um | 356/158 |
| B1810-AL | Ufs-Cms-Cf-Cm-Uf-Um-Uf-Um-Cf-Am-Af-Gm-Uf-Um-Gf-Am-Gf-Am-Af-Cm-Af | 281/83 | Ums-Gfs-Um-Uf-Cm-Uf-Cm-Af-Am-Cf-Um-Uf-Gm-Af-Am-Af-Am-Gf-Gm-Gf-Ams-Afs-Cm | 367/169 |
| B1365-AL | Gfs-Ams-Cf-Cm-Cf-Um-Gf-Am-Af-Cm-Cf-Gm-Cf-Cm-Cf-Am-Uf-Um-Cf-Cm-Uf | 256/58 | Ams-Gfs-Gm-Af-Am-Uf-Gm-Gf-Gm-Cf-Gm-Gf-Um-Uf-Cm-Af-Gm-Gf-Gm-Uf-Cms-Afs-Cm | 342/144 |

(continued)

| Sequence ID | Sense strand sequence 5'-3' | Sequence SEQ ID NO./ corresponding unmodified sequence SEQ ID NO. | Antisense strand sequence 5'-3' | Sequence SEQ ID NO./ corresponding unmodified sequence SEQ ID NO. |
|---|---|---|---|---|
| B 1279-AL | Afs-Ams-Cf-Am-Gf-Cm-Af-Um-Uf-Um-Uf-Um-Uf-Um-Uf-Gm-Af-Gm-Cf-Um-Uf | 252/54 | Ams-Afs-Gm-Cf-Um-Cf-Am-Af-Am-Af-Am-Af-Am-Af-Um-Gf-Cm-Uf-Gm-Uf-Ums-Cfs-Am | 338/140 |

**(2) A total of 23 sequences exhibited inhibition rates ranging from 25% to 40% on the AGT gene. For example, the inhibition rates of B734-AL and B994-AL were 39.2% and 37.3%, respectively.**

Table 4: Alternately modified sequences with inhibition rates ranging from 25% to 40% on AGT gene expression

| Unmodified sequence ID | Sequence ID | Inhibition rate (%) |
|---|---|---|
| 734 | B734-AL | 39.2 |
| 1591 | B1591-AL | 37.9 |
| 969 | B969-AL | 37.3 |
| 994 | B994-AL | 37.3 |
| 984 | B984-AL | 36.9 |
| 743 | B743-AL | 33.8 |
| 1393 | B1393-AL | 33.4 |
| 2030 | B2030-AL | 33.0 |
| 1267 | B1267-AL | 31.5 |
| 2033 | B2033-AL | 31.5 |
| 2031 | B2031-AL | 31.0 |
| 1815 | B1815-AL | 30.4 |
| 2020 | B2020-AL | 30.4 |
| 753s | B753s-AL | 30.2 |
| 2032 | B2032-AL | 30.2 |
| 1602 | B1602-AL | 29.0 |
| 1029 | B1029-AL | 28.6 |
| 1612 | B1612-AL | 28.6 |
| 2018 | B2018-AL | 27.9 |
| 2025 | B2025-AL | 26.6 |
| 731 | B731-AL | 26.4 |
| 1011 | B1011-AL | 26.2 |
| 732 | B732-AL | 25.4 |

The 22 sequences listed in Table 4 exhibited poor inhibitory effects on the AGT gene, with inhibition rates below 40%, ranging from 25% to 40%. For example, the inhibition rates of B734-AL and B994-AL were 39.2% and 37.3%,

respectively (FIG. 2).

**(3) A total of 43 sequences exhibited inhibition rates below 25% on the AGT gene. For example, the inhibition rate of B1017-AL was only 23.1%.**

Table 5: Alternately modified sequences with inhibition rates below 25% on AGT gene expression

| Unmodified sequence ID | Sequence ID | Inhibition rate (%) |
|---|---|---|
| 1030 | B1030-AL | 23.5 |
| 1017 | B1017-AL | 23.1 |
| 992 | B992-AL | 20.5 |
| 1362 | B 1362-AL | 17.5 |
| 1061 | B1061-AL | 17.1 |
| 982 | B982-AL | 17.1 |
| 729 | B729-AL | 16.4 |
| 1020 | B1020-AL | 16.3 |
| 1775 | B1775-AL | 15.5 |
| 1596 | B1596-AL | 15.0 |
| 971 | B971-AL | 15.0 |
| 1057 | B1057-AL | 14.8 |
| 1268 | B1268-AL | 14.2 |
| 1564 | B1564-AL | 13.8 |
| 1028 | B1028-AL | 10.4 |
| 1065s | B1065s-AL | 10.0 |
| 1024 | B1024-AL | 9.9 |
| 1044 | B1044-AL | 9.9 |
| 963 | B963-AL | 9.8 |
| 1266 | B1266-AL | 8.2 |
| 1014 | B1014-AL | 6.5 |
| 1047 | B1047-AL | 6.4 |
| 1041 | B1041-AL | 6.0 |
| 1008 | B 1008-AL | 5.6 |
| 966 | B966-AL | 4.8 |
| 1046 | B1046-AL | 4.5 |
| 1071 | B1071-AL | 4.1 |
| 1038 | B1038-AL | 3.9 |
| 1016 | B1016-AL | 3.5 |
| 1026 | B1026-AL | 3.4 |
| 1554 | B1554-AL | 1.7 |
| 1367 | B1367-AL | 1.6 |
| 1407 | B1407-AL | 1.3 |
| 1260 | B1260-AL | 0.9 |

(continued)

| Unmodified sequence ID | Sequence ID | Inhibition rate (%) |
|---|---|---|
| 1558 | B1558-AL | 0.9 |
| 1401 | B1401-AL | -0.6 |
| 1053 | B1053-AL | -1.6 |
| 1561 | B1561-AL | -2.0 |
| 1404 | B1404-AL | -2.4 |
| 1369 | B1369-AL | -2.8 |
| 1562 | B1562-AL | -3.3 |
| 1262 | B1262-AL | -6.0 |
| 1265 | B1265-AL | -8.8 |

The 43 sequences listed in Table 5 exhibited very poor inhibitory effects on AGT mRNA expression, with inhibition rates below 25%. For example, the inhibition rate of B1017-AL was only 23.1% (FIG. 3 and FIG. 4).

**(4) siRNAs with similar sequences exhibited significant differences in activity. For example, the inhibition rate of B1365-AL was 40.4% higher than that of B1367-AL, indicating a significant improvement.**

A comparison of the inhibitory effects on AGT mRNA by some siRNAs with similar sequences is shown in Table 6.

Table 6: Comparison of inhibition rates of siRNAs with similar sequences on AGT gene expression

| Similar sequence group | Unmodified sequence ID | Sequence ID | Corresponding unmodified sense strand 5'-3' | Corresponding unmodified sequence SEQ ID NO. | Corresponding unmodified antisense strand 5'-3' | Corresponding unmodified sequence SEQ ID NO. | Inhibition rate (%) |
|---|---|---|---|---|---|---|---|
| A | 1367 | B1367-AL | **CCCUGAACCGCCCAUUCCU**GU | 59 | AC**AGGAAUGGGCGGUUCAGGGUC** | 145 | 1.6 |
| | 1365 | B1365-AL | GA**CCCUGAACCGCCCAUUCCU** | 58 | **AGGAAUGGGCGGUUCAGGGUC**AC | 144 | 42.0 |
| B | 1815 | B1815-AL | U**UUCAAGUUGAGAACAAAAAU** | 86 | **AUUUUUGUUCUCAACUUGAAAA**G | 172 | 30.4 |
| | 1816 | B1816-AL | **UUCAAGUUGAGAACAAAAAU**U | 2 | A**AUUUUUGUUCUCAACUUGAAAA** | 15 | 60.9 |
| C | 729 | B729-AL | AGAAC**UGGAUGUUGCUGCUGA** | 97 | **UCAGCAGCAACAUCCAGU**UCUGU | 183 | 16.4 |
| | 734s | B734sAL | **UGGAUGUUGCUGCUGA**GAAA | 101 | UUUC**UCAGCAGCAACAUCCAGU** | 187 | 56.4 |

As shown in Table 6, some siRNAs with similar sequences exhibited significantly different inhibitory effects on AGT mRNA expression.

Unmodified sequence 1367 differs from unmodified sequence 1365 by only 2 terminal bases. For the sense strand, the 5' end of unmodified sequence 58 is GA, while the 3' end of unmodified sequence 59 is GU, with all other sequences being identical. For the antisense strand, the 3' end of unmodified sequence 144 is AC, while the 5' end of unmodified sequence 145 is AC, with all other sequences being identical. However, the inhibition rate of alternately modified sequence B1365-AL was 40.4% higher than that of B1367-AL, indicating a significant improvement.

Unmodified sequence 1815 differs from unmodified sequence 1816 by only 1 terminal base. For the sense strand, the 5' end of unmodified sequence 86 is U, while the 3' end of sequence 2 is U, with all other sequences being identical. For the antisense strand, the 3' end of unmodified sequence 172 is G, while the 5' end of unmodified sequence 15 is A, with all other sequences being identical. However, the inhibition rate of alternately modified sequence B1816-AL was 30.5% higher than that of B1815-AL, indicating a significant improvement.

Unmodified sequence 729 differs from unmodified sequence 734s by only 5 terminal bases. For the sense strand, the 5' end of unmodified sequence 97 is AGAAC, while the 3' end of unmodified sequence 101 is GAAA, with all other sequences being identical. For the antisense strand, the 3' end of unmodified sequence 183 is UCUGU, while the 5' end of unmodified sequence 187 is UUUC, with all other sequences being identical. However, the inhibition rate of alternately modified sequence B734s-AL was 40.0% higher than that of B729-AL, indicating a significant improvement.

Therefore, screening oligonucleotide sequences with significant inhibitory activity from a vast number of candidate sequences designed for AGT mRNA sequences is not straightforward and requires substantial creative effort.

**Summary:**

(1) Among the 99 designed sequences, 33 sequences exhibited significant inhibitory effects on the AGT gene, with inhibition rates exceeding 40%. Specifically, 10 sequences exhibited inhibition rates exceeding 60%.

(2) A total of 23 sequences exhibited inhibition rates ranging from 25% to 40% on the AGT gene. For example, the inhibition rates of B734-AL and B994-AL were 39.2% and 37.3%, respectively.

(3) A total of 43 sequences exhibited inhibition rates below 25% on the AGT gene. For example, the inhibition rate of B1017-AL was only 23.1%.

(4) siRNAs with similar sequences exhibited significant differences in activity. For example, the inhibition rate of B1365-AL was 40.4% higher than that of B1367-AL, indicating a significant improvement. Therefore, screening oligonucleotide sequences with significant inhibitory activity from a vast number of candidate sequences designed for AGT mRNA sequences is not straightforward and requires substantial creative effort.

**Example 3: Inhibition of unmodified sequences on AGT gene expression**

In this example, some unmodified sequences corresponding to the modified sequences in Example 2 were synthesized and transfected into HepG2 cells via lipid nanoparticles (LNPs). The inhibitory effects of these unmodified sequences on the AGT gene were tested using qPCR technology to screen unmodified siRNA sequences with favorable inhibitory effects.

**1. Experimental materials**

Test samples:

The unmodified siRNA sequences listed in Table 7.

**2. Synthesis of unmodified sequence 1579**

The siRNA sequence designated as 1579 in Table 7 is as follows:

Sense strand: 5'-CCUUUUCUUCUAAUGAGUCGA-3' (SEQ ID NO: 4)
Antisense strand: 5'-UCGACUCAUUAGAAGAAAAGGUG-3' (SEQ ID NO: 17)
Instruments and reagents: A Tsingke 192 P model DNA/RNA automatic synthesizer was used. The solid-phase support was a universal support composed of cross-linked polystyrene beads, model: Primer Support 5G Unylinker 350 (manufactured by Cytiva).

Preparation method:

Nucleoside phosphoramidite monomer solutions were prepared in acetonitrile at a concentration of 0.15 M, using the following monomers: DMT-A-2'-O-TBDMS phosphoramidite monomer (Formula 11), DMT-C-2'-O-TBDMS phosphoramidite monomer (Formula 12), DMT-G-2'-O-TBDMS phosphoramidite monomer (Formula 13), and DMT-U-2'-O-TBDMS phosphoramidite monomer (Formula 14).

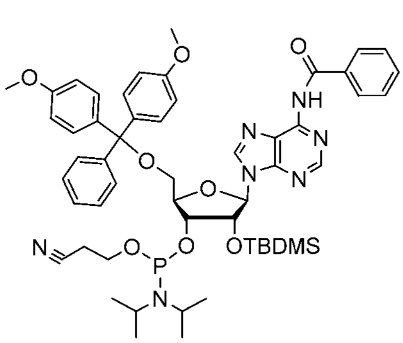

Formula 11

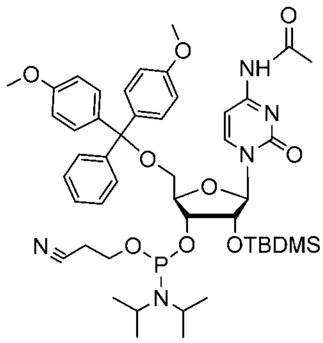

Formula 12

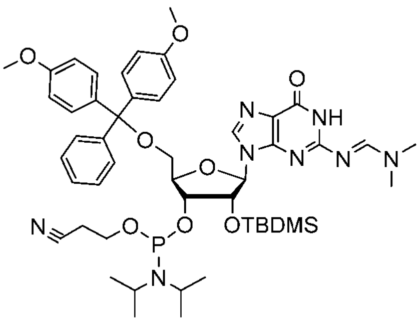

Formula 13

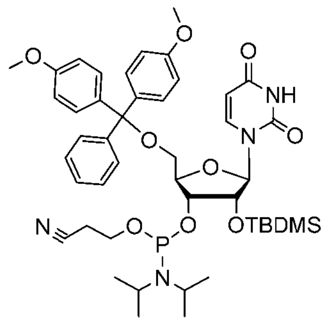

Formula 14

The preparation was carried out according to the following steps:
The solid-phase support was loaded into the designated position of the synthesizer, and a corresponding fully protected product was obtained through several synthesis cycles, each cycle including (1) deprotection, (2) coupling, (3) oxidation/sulfurization, and (4) hydroxyl protection. The cycle process and the reagents used are described as follows:

(1) Deprotection
3% dichloroacetic acid in toluene was used as a deprotection reagent to remove the DMT protecting group, followed by washing with acetonitrile.
(2) Coupling
Each nucleoside phosphoramidite monomer in acetonitrile was coupled using 0.25 M 5-ethylthio-1H-tetrazole as an activator, followed by rinsing with acetonitrile.
(3) Oxidation/sulfurization

Oxidation: 0.05 M iodine in pyridine/water (90/10) was used as an oxidant for oxidation, followed by rinsing with acetonitrile.
Sulfurization: 3% xanthane hydride in pyridine was used as a sulfurizing agent for sulfurization, followed by rinsing with acetonitrile.

(4) Hydroxyl protection

10% acetic anhydride in tetrahydrofuran (CAP A) and tetrahydrofuran/pyridine/N-methylimidazole (74/10/16, v/v/v) (CAP B) were used as hydroxyl protecting reagents for hydroxyl protection, followed by rinsing with acetonitrile.
The above steps were repeated according to the set sequence to obtain the fully protected product.

(5) 3% dichloroacetic acid in toluene was used as a deprotection reagent to remove the DMT protecting group from the last nucleotide, followed by washing with acetonitrile.

(6) Ammonolysis and purification

The reacted solid-phase support was transferred to a reactor, then concentrated aqueous ammonia (25 to 28%) was added, and ammonolysis was performed at 60°C for 12 hours. The system was then cooled to room temperature, and the mixture was transferred to a pressure filtration vessel, followed by rinsing with a mixture of purified water and ethanol. The filtrates were combined, subjected to column chromatography, concentrated, and lyophilized to obtain a 2'-O-TBDMS protected product.

(7) Removal of TBDMS protecting groups

DMSO and triethylamine trihydrofluoride were added to the obtained product, and the mixture was reacted at 60°C for 2 hours. An aqueous solution of ammonium acetate was then added to the reaction mixture and vortexed thoroughly. Anhydrous ethanol was then added, vortexed thoroughly, followed by crystallization at -20°C for 8 to 12 hours. After centrifugation, the supernatant was discarded, and the precipitate was rinsed with anhydrous ethanol to obtain the unmodified single-stranded product.

(8) Annealing

The resulting unmodified sense and antisense strands were mixed at a ratio of 1:1, heated to 95°C and maintained for 3 minutes, and slowly cooled to room temperature to form an unmodified duplex.

Unmodified sequence 1579 had a purity of 97.4% and a measured molecular weight of 13990.9.

**3. Synthesis of other sequences**

The other sequences listed in Table 7 were synthesized according to the method described above.

Table 7: Unmodified siRNA sequences

| Sequence ID | Sense strand sequence (unmodified sequence) 5'-3' | SEQ ID NO. | Antisense strand sequence (unmodified sequence) 5'-3' | SEQ ID NO. |
|---|---|---|---|---|
| 1835 | UUGGGUUUUAAAAUUAAAGUA | 1 | UACUUUAAUUUUAAAACCCAAUU | 14 |
| 1816 | UUCAAGUUGAGAACAAAAAUU | 2 | AAUUUUUGUUCUCAACUUGAAAA | 15 |
| 1812 | CCUUUUCAAGUUGAGAACAAA | 3 | UUUGUUCUCAACUUGAAAAGGGA | 16 |
| 1579 | CCUUUUCUUCUAAUGAGUCGA | 4 | UCGACUCAUUAGAAGAAAAGGUG | 17 |
| 1836 | UGGGUUUUAAAAUUAAAGUAU | 5 | AUACUUUAAUUUUAAAACCCAAU | 18 |
| 1789 | GUUUGUGAAACAAAAAAGUGU | 6 | ACACUUUUUUGUUUCACAAACAA | 19 |
| 1839 | GUUUUAAAAUUAAAGUAUACA | 7 | UGUAUACUUUAAUUUUAAAACCC | 20 |
| 1791 | UUGUGAAACAAAAAAGUGUUA | 8 | UAACACUUUUUUGUUUCACAAAC | 21 |
| 1795 | GAAACAAAAAAGUGUUCCCUU | 9 | AAGGGAACACUUUUUUGUUUCAC | 22 |
| 1585s | CUUCUAAUGAGUCGACUUUA | 10 | UAAAGUCGACUCAUUAGAAGAA | 23 |
| 1576s | CCACCUUUUCUUCUAAUGAA | 11 | UUCAUUAGAAGAAAAGGUGGGA | 24 |
| 1578s | ACCUUUUCUUCUAAUGAGUA | 12 | UACUCAUUAGAAGAAAAGGUGG | 25 |
| 1838 | GGUUUUAAAAUUAAAGUAUAA | 13 | UUAUACUUUAAUUUUAAAACCCA | 26 |
| 1008 | CUUCACUGAGAGCGCCUGCCU | 27 | AGGCAGGCGCUCUCAGUGAAGGG | 113 |
| 1011 | CACUGAGAGCGCCUGCCUGCU | 28 | AGCAGGCAGGCGCUCUCAGUGAA | 114 |
| 1014 | UGAGAGCGCCUGCCUGCUGCU | 29 | AGCAGCAGGCAGGCGCUCUCAGU | 115 |
| 1016 | AGAGCGCCUGCCUGCUGCUGA | 30 | UCAGCAGCAGGCAGGCGCUCUCA | 116 |
| 1017 | GAGCGCCUGCCUGCUGCUGAU | 31 | AUCAGCAGCAGGCAGGCGCUCUC | 117 |
| 1020 | CGCCUGCCUGCUGCUGAUCCA | 32 | UGGAUCAGCAGCAGGCAGGCGCU | 118 |
| 1024 | UGCCUGCUGCUGAUCCAGCCU | 33 | AGGCUGGAUCAGCAGCAGGCAGG | 119 |
| 1026 | CCUGCUGCUGAUCCAGCCUCA | 34 | UGAGGCUGGAUCAGCAGCAGGCA | 120 |
| 1028 | UGCUGCUGAUCCAGCCUCACU | 35 | AGUGAGGCUGGAUCAGCAGCAGG | 121 |
| 1029 | GCUGCUGAUCCAGCCUCACUA | 36 | UAGUGAGGCUGGAUCAGCAGCAG | 122 |

(continued)

| Sequence ID | Sense strand sequence (unmodified sequence) 5'-3' | SEQ ID NO. | Antisense strand sequence (unmodified sequence) 5'-3' | SEQ ID NO. |
|---|---|---|---|---|
| 1030 | CUGCUGAUCCAGCCUCACUAU | 37 | AUAGUGAGGCUGGAUCAGCAGCA | 123 |
| 1038 | CCAGCCUCACUAUGCCUCUGA | 38 | UCAGAGGCAUAGUGAGGCUGGAU | 124 |
| 1041 | GCCUCACUAUGCCUCUGACCU | 39 | AGGUCAGAGGCAUAGUGAGGCUG | 125 |
| 1044 | UCACUAUGCCUCUGACCUGGA | 40 | UCCAGGUCAGAGGCAUAGUGAGG | 126 |
| 1046 | ACUAUGCCUCUGACCUGGACA | 41 | UGUCCAGGUCAGAGGCAUAGUGA | 127 |
| 1047 | CUAUGCCUCUGACCUGGACAA | 42 | UUGUCCAGGUCAGAGGCAUAGUG | 128 |
| 1053 | CUCUGACCUGGACAAGGUGGA | 43 | UCCACCUUGUCCAGGUCAGAGGC | 129 |
| 1057 | GACCUGGACAAGGUGGAGGGU | 44 | ACCCUCCACCUUGUCCAGGUCAG | 130 |
| 1061 | UGGACAAGGUGGAGGGUCUCA | 45 | UGAGACCCUCCACCUUGUCCAGG | 131 |
| 1065s | CAAGGUGGAGGGUCUCACUU | 46 | AAGUGAGACCCUCCACCUUGUC | 132 |
| 1071 | GGAGGGUCUCACUUUCCAGCA | 47 | UGCUGGAAAGUGAGACCCUCCAC | 133 |
| 1260 | CAGGGUGGGGGAGGUGCUGAA | 48 | UUCAGCACCUCCCCCACCCUGAU | 134 |
| 1262 | GGGUGGGGGAGGUGCUGAACA | 49 | UGUUCAGCACCUCCCCCACCCUG | 135 |
| 1265 | UGGGGGAGGUGCUGAACAGCA | 50 | UGCUGUUCAGCACCUCCCCCACC | 136 |
| 1266 | GGGGGAGGUGCUGAACAGCAU | 51 | AUGCUGUUCAGCACCUCCCCCAC | 137 |
| 1267 | GGGGAGGUGCUGAACAGCAUU | 52 | AAUGCUGUUCAGCACCUCCCCCA | 138 |
| 1268 | GGGAGGUGCUGAACAGCAUUU | 53 | AAAUGCUGUUCAGCACCUCCCCC | 139 |
| 1279 | AACAGCAUUUUUUUUGAGCUU | 54 | AAGCUCAAAAAAAAUGCUGUUCA | 140 |
| 1281 | CAGCAUUUUUUUUGAGCUUGA | 55 | UCAAGCUCAAAAAAAAUGCUGUU | 141 |
| 1282 | AGCAUUUUUUUUGAGCUUGAA | 56 | UUCAAGCUCAAAAAAAAUGCUGU | 142 |
| 1362 | GGUGACCCUGAACCGCCCAUU | 57 | AAUGGGCGGUUCAGGGUCACCUC | 143 |
| 1365 | GACCCUGAACCGCCCAUUCCU | 58 | AGGAAUGGGCGGUUCAGGGUCAC | 144 |
| 1367 | CCCUGAACCGCCCAUUCCUGU | 59 | ACAGGAAUGGGCGGUUCAGGGUC | 145 |
| 1369 | CUGAACCGCCCAUUCCUGUUU | 60 | AAACAGGAAUGGGCGGUUCAGGG | 146 |
| 1393 | GUGUAUGAUCAAAGCGCCACU | 61 | AGUGGCGCUUUGAUCAUACACAG | 147 |
| 1401 | UCAAAGCGCCACUGCCCUGCA | 62 | UGCAGGGCAGUGGCGCUUUGAUC | 148 |
| 1404 | AAGCGCCACUGCCCUGCACUU | 63 | AAGUGCAGGGCAGUGGCGCUUUG | 149 |
| 1407 | CGCCACUGCCCUGCACUUCCU | 64 | AGGAAGUGCAGGGCAGUGGCGCU | 150 |
| 1554 | GCGAUGUGUCACCCCCAGUCU | 65 | AGACUGGGGGUGACACAUCGCUG | 151 |
| 1558 | UGUGUCACCCCCAGUCUCCCA | 66 | UGGGAGACUGGGGGUGACACAUC | 152 |
| 1561 | GUCACCCCCAGUCUCCCACCU | 67 | AGGUGGGAGACUGGGGGUGACAC | 153 |
| 1562 | UCACCCCCAGUCUCCCACCUU | 68 | AAGGUGGGAGACUGGGGGUGACA | 154 |
| 1564 | ACCCCCAGUCUCCCACCUUUU | 69 | AAAAGGUGGGAGACUGGGGGUGA | 155 |
| 1591 | AUGAGUCGACUUUGAGCUGGA | 70 | UCCAGCUCAAAGUCGACUCAUUA | 156 |
| 1592 | UGAGUCGACUUUGAGCUGGAA | 71 | UUCCAGCUCAAAGUCGACUCAUU | 157 |
| 1593 | GAGUCGACUUUGAGCUGGAAA | 72 | UUUCCAGCUCAAAGUCGACUCAU | 158 |
| 1596 | UCGACUUUGAGCUGGAAAGCA | 73 | UGCUUUCCAGCUCAAAGUCGACU | 159 |
| 1602 | UUGAGCUGGAAAGCAGCCGUU | 74 | AACGGCUGCUUUCCAGCUCAAAG | 160 |
| 1603 | UGAGCUGGAAAGCAGCCGUUU | 75 | AAACGGCUGCUUUCCAGCUCAAA | 161 |

(continued)

| Sequence ID | Sense strand sequence (unmodified sequence) 5'-3' | SEQ ID NO. | Antisense strand sequence (unmodified sequence) 5'-3' | SEQ ID NO. |
|---|---|---|---|---|
| 1608 | UGGAAAGCAGCCGUUUCUCCU | 76 | AGGAGAAACGGCUGCUUUCCAGC | 162 |
| 1612 | AAGCAGCCGUUUCUCCUUGGU | 77 | ACCAAGGAGAAACGGCUGCUUUC | 163 |
| 1775 | CAACCGACCAGCUUGUUUGUA | 78 | UACAAACAAGCUGGUCGGUUGGA | 164 |
| 1790 | UUUGUGAAACAAAAAAGUGUU | 79 | AACACUUUUUUGUUUCACAAACA | 165 |
| 1796 | AAACAAAAAAGUGUUCCCUUU | 80 | AAAGGGAACACUUUUUUGUUUCA | 166 |
| 1798s | ACAAAAAAGUGUUCCCUUUU | 81 | AAAAGGGAACACUUUUUUGUUU | 167 |
| 1799 | CAAAAAAGUGUUCCCUUUUCA | 82 | UGAAAAGGGAACACUUUUUUGUU | 168 |
| 1810 | UCCCUUUUCAAGUUGAGAACA | 83 | UGUUCUCAACUUGAAAAGGGAAC | 169 |
| 1811 | CCCUUUUCAAGUUGAGAACAA | 84 | UUGUUCUCAACUUGAAAAGGGAA | 170 |
| 1814 | UUUUCAAGUUGAGAACAAAAA | 85 | UUUUUGUUCUCAACUUGAAAAGG | 171 |
| 1815 | UUUCAAGUUGAGAACAAAAAU | 86 | AUUUUUGUUCUCAACUUGAAAAG | 172 |
| 1817 | UCAAGUUGAGAACAAAAAUUG | 87 | CAAUUUUUGUUCUCAACUUGAAA | 173 |
| 1828 | ACAAAAAUUGGGUUUUAAAAU | 88 | AUUUUAAAACCCAAUUUUUGUUC | 174 |
| 1838s | GGUUUUAAAAUUAAAGUAUA | 89 | UAUACUUUAAUUUUAAAACCCA | 175 |
| 2018 | AUAGCUGGUUAUUUCUCCCUU | 90 | AAGGGAGAAAUAACCAGCUAUGG | 176 |
| 2020 | AGCUGGUUAUUUCUCCCUUGU | 91 | ACAAGGGAGAAAUAACCAGCUAU | 177 |
| 2025 | GUUAUUUCUCCCUUGUGUUAA | 92 | UUAACACAAGGGAGAAAUAACCA | 178 |
| 2030 | UUCUCCCUUGUGUUAGUAAUA | 93 | UAUUACUAACACAAGGGAGAAAU | 179 |
| 2031 | UCUCCCUUGUGUUAGUAAUAA | 94 | UUAUUACUAACACAAGGGAGAAA | 180 |
| 2032 | CUCCCUUGUGUUAGUAAUAAA | 95 | UUUAUUACUAACACAAGGGAGAA | 181 |
| 2033 | UCCCUUGUGUUAGUAAUAAAA | 96 | UUUUAUUACUAACACAAGGGAGA | 182 |
| 729 | AGAACUGGAUGUUGCUGCUGA | 97 | UCAGCAGCAACAUCCAGUUCUGU | 183 |
| 731 | AACUGGAUGUUGCUGCUGAGA | 98 | UCUCAGCAGCAACAUCCAGUUCU | 184 |
| 732 | ACUGGAUGUUGCUGCUGAGAA | 99 | UUCUCAGCAGCAACAUCCAGUUC | 185 |
| 734 | UGGAUGUUGCUGCUGAGAAGA | 100 | UCUUCUCAGCAGCAACAUCCAGU | 186 |
| 734s | UGGAUGUUGCUGCUGAGAAA | 101 | UUUCUCAGCAGCAACAUCCAGU | 187 |
| 736 | GAUGUUGCUGCUGAGAAGAUU | 102 | AAUCUUCUCAGCAGCAACAUCCA | 188 |
| 743 | CUGCUGAGAAGAUUGACAGGU | 103 | ACCUGUCAAUCUUCUCAGCAGCA | 189 |
| 753s | GAUUGACAGGUUCAUGCAGA | 104 | UCUGCAUGAACCUGUCAAUCUU | 190 |
| 963 | GCACUGGAGUGACAUCCAGGA | 105 | UCCUGGAUGUCACUCCAGUGCUG | 191 |
| 966 | CUGGAGUGACAUCCAGGACAA | 106 | UUGUCCUGGAUGUCACUCCAGUG | 192 |
| 969 | GAGUGACAUCCAGGACAACUU | 107 | AAGUUGUCCUGGAUGUCACUCCA | 193 |
| 971 | GUGACAUCCAGGACAACUUCU | 108 | AGAAGUUGUCCUGGAUGUCACUC | 194 |
| 982 | GACAACUUCUCGGUGACUCAA | 109 | UUGAGUCACCGAGAAGUUGUCCU | 195 |
| 984 | CAACUUCUCGGUGACUCAAGU | 110 | ACUUGAGUCACCGAGAAGUUGUC | 196 |
| 992 | CGGUGACUCAAGUGCCCUUCA | 111 | UGAAGGGCACUUGAGUCACCGAG | 197 |
| 994 | GUGACUCAAGUGCCCUUCACU | 112 | AGUGAAGGGCACUUGAGUCACCG | 198 |

Cell type: Hepatocellular carcinoma cell line HepG2 cells.

Drug vehicle: sterile nuclease-free water, Gibco Opti-MEM.

**3. Experimental methods**

The experimental procedures and siRNA concentrations were referenced to Example 2.

**4. Experimental results**

The inhibition rates of unmodified sequences on AGT mRNA expression are shown in Tables 8 and 9.

Unmodified sequences 1576s, 1578s, 1838, 1835, 1816, 1812, 1579, 1836, 1789, 1839, 1791, 1795, and 1585s exhibited significant inhibitory effects on AGT mRNA expression in HepG2 cells, with inhibition rates of 45% or more. Specifically, 1576s, 1578s, 1838, 1835, 1812, 1579, and 1789 exhibited inhibition rates exceeding 50%. A comparison of the inhibitory effects between the unmodified sequences of the present disclosure and the sequences in the prior art is shown in Table 29.

**(1) A total of 13 unmodified sequences exhibited significant inhibitory effects on the AGT gene, with inhibition rates exceeding 45%, including 1576s, 1578s, 1838, 1835, 1816, 1812, 1579, 1836, 1789, 1839, 1791, 1795, and 1585s. Specifically, 1576s, 1578s, 1838, 1835, 1812, 1579, and 1789 exhibited inhibition rates exceeding 50%.**

Table 8: Unmodified sequences with significant inhibitory effects on AGT gene expression (inhibition rate > 45%)

| Sequence ID | Inhibition rate (%) |
|---|---|
| 1576s | 59.1 |
| 1578s | 55.2 |
| 1838 | 50.6 |
| 1835 | 51.6 |
| 1816 | 46.8 |
| 1812 | 56.8 |
| 1579 | 58.2 |
| 1836 | 46.5 |
| 1789 | 54.5 |
| 1839 | 45.7 |
| 1791 | 46.5 |
| 1795 | 49.6 |
| 1585s | 48.9 |

The unmodified sequences listed in Table 8, including 1576s, 1578s, 1838, 1835, 1816, 1812, 1579, 1836, 1789, 1839, 1791, 1795, and 1585s, exhibited significant inhibitory effects on AGT mRNA expression, with inhibition rates of 45% or more. Specifically, 1576s, 1578s, 1838, 1835, 1812, 1579, and 1789 exhibited inhibition rates exceeding 50% (FIG. 5).

**(2) Some of the unmodified sequences exhibited inhibition rates below 45% on the AGT gene. For example, the inhibition rates of 731 and 1011 were only 1.3% and 2.1%, respectively.**

Table 9: Unmodified sequences with inhibition rates below 45% on AGT gene expression

| Sequence ID | Inhibition rate (%) |
|---|---|
| 1608 | 39.3 |
| 1811 | 37.7 |

(continued)

| Sequence ID | Inhibition rate (%) |
|---|---|
| 1281 | 26.2 |
| 1799 | 34.9 |
| 1603 | 26.7 |
| 1828 | 31.5 |
| 1592 | 38.6 |
| 1593 | 41.6 |
| 1810 | 24.5 |
| 1365 | 24.3 |
| 1279 | 41.5 |
| 734 | 43.0 |
| 1591 | 37.4 |
| 969 | 27.3 |
| 994 | 36.6 |
| 984 | 31.6 |
| 743 | 29.3 |
| 1393 | 38.2 |
| 2030 | 36.3 |
| 1267 | 36.8 |
| 2033 | 34.9 |
| 2031 | 36.4 |
| 1815 | 37.1 |
| 2020 | 32.5 |
| 753s | 27.8 |
| 2032 | 17.6 |
| 1602 | 22.3 |
| 1029 | 18.5 |
| 1612 | 9.6 |
| 2018 | 14.1 |
| 2025 | 7.2 |
| 731 | 1.3 |
| 1011 | 2.1 |

The sequences listed in Table 9 exhibited very poor inhibitory effects on AGT mRNA expression, with inhibition rates below 45%. For example, the inhibition rates of 731 and 1011 were only 1.3% and 2.1%, respectively (FIG. 5).

**(3) siRNAs with similar unmodified sequences exhibited significant differences in activity.For example, the inhibition rate of unmodified sequence 1812 was 32.3% higher than that of unmodified sequence 1810, indicating a significant improvement.**

A comparison of the inhibitory effects on AGT mRNA by some siRNAs with similar sequences is shown in Table 10.

Table 10: Comparison of inhibition rates of unmodified sequences with similar sequences on AGT gene expression

| Similar sequence group | Base sequence ID | Sense strand 5'-3' | Unmodified sequence SEQ ID NO. | Antisense strand 5'-3' | Unmodified sequence SEQ ID NO. | Inhibition rate (%) |
|---|---|---|---|---|---|---|
| A | 1608 | UGGA**AAGCAGCCGUUUCUCCU** | 76 | **AGGAGAAACGGCUGCUUUC**CAGC | 162 | 39.3 |
| | 1612 | **AAGCAGCCGUUUCUCCU**UGGU | 77 | ACCA**AGGAGAAACGGCUGCUUUC** | 163 | 9.6 |
| B | 2025 | GUUAU**UUCUCCCUUGUGUUA**A | 92 | U**UAACACAAGGGAGAAAU**AACCA | 178 | 7.2 |
| | 2030 | **UUCUCCCUUGUGUUA**GUAAUA | 93 | UAUUAC**UAACACAAGGGAGAAAU** | 179 | 36.3 |
| C | 731 | AAC**UGGAUGUUGCUGCUGAGA** | 98 | **UCUCAGCAGCAACAUCCAGU**UCU | 184 | 1.3 |
| | 734 | **UGGAUGUUGCUGCUGAGA**AGA | 100 | UCU**UCUCAGCAGCAACAUCCAGU** | 186 | 43.0 |
| D | 1812 | **CCUUUUCAAGUUGAGAACA**AA | 3 | UU**UGUUCUCAACUUGAAAAGGGA** | 16 | 56.8 |
| | 1810 | UC**CCUUUUCAAGUUGAGAACA** | 83 | **UGUUCUCAACUUGAAAAGGGA**AC | 169 | 24.5 |

As shown in Table 10, some siRNAs with similar sequences exhibited significantly different inhibitory effects on AGT mRNA expression.

Unmodified sequence 1608 differs from unmodified sequence 1612 by only 4 terminal bases. For the sense strand, the 5' end of unmodified sequence 76 is UGGA, while the 3' end of unmodified sequence 77 is UGGU, with all other sequences being identical. For the antisense strand, the 3' end of unmodified sequence 162 is CAGC, while the 5' end of unmodified sequence 163 is ACCA, with all other sequences being identical. However, the inhibition rate of unmodified sequence 1608 was 29.7% higher than that of 1612, indicating a significant improvement.

Unmodified sequence 2025 differs from unmodified sequence 2030 by only 6 terminal bases. For the sense strand, the 5' end of unmodified sequence 92 is GUUAU and the 3' end is A, while the 3' end of sequence 93 is GUAAUA, with all other sequences being identical. For the antisense strand, the 3' end of unmodified sequence 178 is AACCA and the 5' end is U, while the 5' end of unmodified sequence 179 is UAUUAC, with all other sequences being identical. However, the inhibition rate of unmodified sequence 2030 was 29.1% higher than that of 2025, indicating a significant improvement.

Unmodified sequence 731 differs from unmodified sequence 734 by only 3 terminal bases. For the sense strand, the 5' end of unmodified sequence 98 is AAC, while the 3' end of unmodified sequence 100 is AGA, with all other sequences being identical. For the antisense strand, the 3' end of unmodified sequence 184 is UCU, while the 5' end of unmodified sequence 186 is UCU, with all other sequences being identical. However, the inhibition rate of unmodified sequence 734 was 41.7% higher than that of 731, indicating a significant improvement.

Unmodified sequence 1812 differs from unmodified sequence 1810 by only 2 terminal bases. For the sense strand, the 3' end of unmodified sequence 3 is AA, while the 5' end of unmodified sequence 83 is UC, with all other sequences being identical. For the antisense strand, the 5' end of unmodified sequence 16 is UU, while the 3' end of unmodified sequence 169 is AC, with all other sequences being identical. The inhibition rate of unmodified sequence 1812 was 32.3% higher than that of unmodified sequence 1810, indicating a significant improvement.

Therefore, screening unmodified oligonucleotide sequences with significant inhibitory activity from a vast number of candidate sequences designed for AGT mRNA sequences is not straightforward and requires substantial creative effort.

**(4) The effects of alternating modifications on activity enhancement varied across different sequences. For some sequences, the inhibition rate was significantly improved, *e.g.*, unmodified sequence 731 exhibited a 25.1% increase in inhibition rate after alternating modification. For others, the improvement was minimal, *e.g.*, unmodified sequences 994, 1279, and 1591 exhibited virtually no difference in inhibition rate between alternately modified and unmodified sequences.**

Table 11: Comparison of inhibition rates between alternately modified and unmodified sequences on AGT gene expression

| Sequence ID | % inhibition of unmodified sequences | Alternately modified sequence ID | % inhibition of alternately modified sequences | Increase in inhibition rate/% |
|---|---|---|---|---|
| 731 | 1.3 | B731-AL | 26.4 | 25.1 |
| 734 | 43.0 | B734-AL | 39.2 | -3.8 |
| 743 | 29.3 | B743-AL | 33.8 | 4.5 |
| 753s | 27.8 | B753s-AL | 30.2 | 2.4 |
| 969 | 27.3 | B969-AL | 37.3 | 10.0 |
| 984 | 31.6 | B984-AL | 36.9 | 5.3 |
| 994 | 36.6 | B994-AL | 37.3 | 0.7 |
| 1011 | 2.1 | B1011-AL | 26.2 | 24.1 |
| 1029 | 18.5 | B1029-AL | 28.6 | 10.1 |
| 1267 | 36.8 | B1267-AL | 31.5 | -5.3 |
| 1279 | 41.5 | B1279-AL | 41.4 | -0.1 |
| 1281 | 26.2 | B1281-AL | 50.4 | 24.2 |

(continued)

| Sequence ID | % inhibition of unmodified sequences | Alternately modified sequence ID | % inhibition of alternately modified sequences | Increase in inhibition rate/% |
|---|---|---|---|---|
| 1365 | 24.3 | B1365-AL | 42.0 | 17.7 |
| 1393 | 38.2 | B1393-AL | 33.4 | -4.8 |
| 1576s | 59.1 | B1576s-AL | 64.6 | 5.5 |
| 1578s | 55.2 | B1578s-AL | 63.9 | 8.7 |
| 1579 | 58.2 | B1579-AL | 61.1 | 2.9 |
| 1585s | 48.9 | B1585s-AL | 59.3 | 10.4 |
| 1591 | 37.4 | B1591-AL | 37.9 | 0.5 |
| 1592 | 38.6 | B1592-AL | 44.4 | 5.8 |
| 1593 | 41.6 | B1593-AL | 42.4 | 0.8 |
| 1602 | 22.3 | B1602-AL | 29.0 | 6.7 |
| 1603 | 26.7 | B1603-AL | 48.6 | 21.9 |
| 1608 | 39.3 | B1608-AL | 52.5 | 13.2 |
| 1612 | 9.6 | B1612-AL | 28.6 | 19.0 |
| 1789 | 54.5 | B 1789-AL | 63.5 | 9.0 |
| 1791 | 46.5 | B1791-AL | 61.8 | 15.3 |
| 1795 | 49.6 | B1795-AL | 57.5 | 7.9 |
| 1799 | 34.9 | B1799-AL | 49.2 | 14.3 |
| 1810 | 24.5 | B1810-AL | 42.1 | 17.6 |
| 1811 | 37.7 | B1811-AL | 51.5 | 13.8 |
| 1812 | 56.8 | B1812-AL | 62.9 | 6.1 |
| 1815 | 37.1 | B1815-AL | 30.4 | -6.7 |
| 1816 | 46.8 | B1816-AL | 60.9 | 14.1 |
| 1828 | 31.5 | B1828-AL | 46.6 | 15.1 |
| 1835 | 51.6 | B1835-AL | 62.3 | 10.7 |
| 1836 | 46.5 | B1836-AL | 59.8 | 13.3 |
| 1838 | 50.6 | B1838-AL | 63.6 | 13.0 |
| 1839 | 45.7 | B1839-AL | 59.1 | 13.4 |
| 2018 | 14.1 | B2018-AL | 27.9 | 13.8 |
| 2020 | 32.5 | B2020-AL | 30.4 | -2.1 |
| 2025 | 7.2 | B2025-AL | 26.6 | 19.4 |
| 2030 | 36.3 | B2030-AL | 33.0 | -3.3 |
| 2031 | 36.4 | B2031-AL | 31.0 | -5.4 |
| 2032 | 17.6 | B2032-AL | 30.2 | 12.6 |
| 2033 | 34.9 | B2033-AL | 31.5 | -3.4 |

As shown in Table 11, the effects of alternating modifications on AGT mRNA inhibition were not entirely consistent across different sequences. For some sequences, the inhibition rate was significantly improved, *e.g.*, unmodified sequence 731 exhibited a 25.1% increase in inhibition rate after alternating modification. For others, the improvement was minimal, *e.g.*, unmodified sequences 994, 1279, and 1591 exhibited virtually no difference in inhibition rate between

alternately modified and unmodified sequences.

Therefore, not all unmodified sequences can exhibit a substantial increase in activity after alternating modifications, and the effect of alternating modifications on activity varies across different sequences.

**Summary:**

(1) A total of 13 unmodified sequences exhibited significant inhibitory effects on the AGT gene, with inhibition rates exceeding 45%, including 1576s, 1579, 1812, 1578s, 1789, 1835, 1838, 1795, 1585s, 1816, 1791, 1836, and 1839. Specifically, 1576s, 1578s, 1812, 1579, 1789, 1835, and 1838 exhibited inhibition rates exceeding 50%.

(2) The other unmodified sequences exhibited inhibition rates below 45% on the AGT gene. For example, the inhibition rates of 731 and 1011 were only 1.3% and 2.1%, respectively.

(3) siRNAs with similar sequences exhibited significant differences in activity. For example, the inhibition rate of unmodified sequence 734 was 41.7% higher than that of unmodified sequence 731, indicating a significant improvement.

(4) The effects of alternating modifications on activity varied across different sequences. For some sequences, the inhibition rate was significantly improved, *e.g.*, unmodified sequence 731 exhibited a 25.1% increase in inhibition rate after alternating modification. For others, the improvement was minimal, *e.g.*, unmodified sequences 994, 1279, and 1591 exhibited virtually no difference in inhibition rate between alternately modified and unmodified sequences.

**Example 4: Inhibition of template-modified sequences on AGT gene expression**

In this example, a total of 13 sequences screened in Example 3, namely unmodified sequences 1576s, 1578s, 1838, 1835, 1816, 1812, 1579, 1836, 1789, 1839, 1791, 1795, and 1585s, were modified using modification templates. Specifically, DV25P, DV26P, DV27P, DV28P, DV29P, DV32P, DV33P, and DV34P are novel modification templates designed in the present disclosure, while DV22 is a previously published Advanced ESC modification template (Foster, D. J., et al. (2018). "Advanced siRNA Designs Further Improve In Vivo Performance of GalNAc-siRNA Conjugates." Mol Ther 26(3): 708-717).

Naturally 5'-phosphorylated or simply 5'-phosphorylated oligonucleotides may undergo dephosphorylation in cells. Directly 5'-phosphorylated oligonucleotide strands may undergo 90% dephosphorylation after 2 hours in circulation, and are completely dephosphorylated after 24 hours. The 5'-E-VP phosphorylation design, which utilizes E-vinylphosphonate to replace the bridging oxygen, offers improved phosphorylation effect and stability. In the modification templates DV25-29P and DV32-34P of the present disclosure, the antisense strands all incorporate the 5'-E-VP phosphorylation design.

**1. Experimental materials**

Test samples:

The sequences listed in Table 12, including sequences 1576s, 1578s, 1838, 1835, 1816, 1812, 1579, 1836, 1789, 1839, 1791, 1795, and 1585s from Example 3 modified with different templates (DV25P, DV26P, DV27P, DV28P, DV29P, DV30P, DV32P, DV33P, DV34P, and DV22), as well as their corresponding alternately modified sequences B1576s-AL, B1578s-AL, B1838-AL, B1835-AL, B1816-AL, B1812-AL, B1579-AL, B1836-AL, B1789-AL, B1839-AL, B1791-AL, B1795-AL, and B1585s-AL (synthesized in Example 1).

Synthesis of sequences:

The siRNA sequences were synthesized with reference to Example 1. During the synthesis of the base at the 5' end of the antisense strand (the last base), a monomer containing a phosphonate group at the 5' end was used, such as vinyl-(E)-phosphonate-A-OMe phosphoramidite monomer (Formula 9) or vinyl-(E)-phosphonate-U-OMe phosphoramidite monomer (Formula 10), the structures of which are as follows:

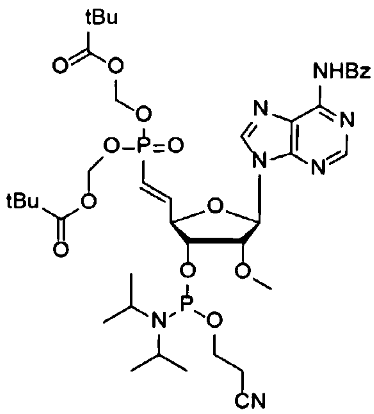

Formula 9

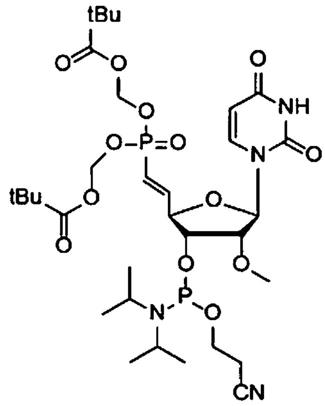

Formula 10

The modification principles for the templates in the present disclosure are as follows:

**DV22 and DV25-34P:**

The antisense strand was modified according to one of the modification patterns shown in Table 46:

Table 46: Modification of antisense strand

| No. | Antisense strand (AS) 5'-3' | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| A | 2'-OMe +PS+EVP | 2'-F +PS | 2'-OMe | 2'-OMe | 2'-OMe | 2'-F | 2'-OMe | 2'-OMe | 2'-OMe | 2'-OMe | 2'-OMe | 2'-OMe |
| | 13 | 14 | 15 | 16 | 17 | 18 | 19 | 20 | 21 | 22 | 23 | |
| | 2'-OMe | 2'-F | 2'-OMe | 2'-F | 2'-OMe | 2'-OMe | 2'-OMe | 2'-OMe | 2'-OMe +PS | 2'-OMe +PS | 2'-OMe | |
| B | 1 | 2 | 3 | 4 | 5 | 6 | 7 | 8 | 9 | 10 | 11 | 12 |
| | 2'-OMe +PS+EVP | 2'-F +PS | 2'-F | 2'-F | 2'-OMe | 2'-F | 2'-OMe | 2'-OMe | 2'-OMe | 2'-OMe | 2'-OMe | 2'-OMe |
| | 13 | 14 | 15 | 16 | 17 | 18 | 19 | 20 | 21 | 22 | 23 | |
| | 2'-OMe | 2'-F | 2'-OMe | 2'-F | 2'-OMe | 2'-OMe | 2'-OMe | 2'-OMe | 2'-OMe +PS | 2'-OMe +PS | 2'-OMe | |
| C | 1 | 2 | 3 | 4 | 5 | 6 | 7 | 8 | 9 | 10 | 11 | 12 |
| | 2'-OMe +PS+EVP | 2'-F +PS | 2'-OMe | 2'-F | 2'-F | 2'-F | 2'-OMe | 2'-OMe | 2'-OMe | 2'-OMe | 2'-OMe | 2'-OMe |
| | 13 | 14 | 15 | 16 | 17 | 18 | 19 | 20 | 21 | 22 | 23 | |
| | 2'-OMe | 2'-F | 2'-OMe | 2'-F | 2'-OMe | 2'-OMe | 2'-OMe | 2'-OMe | 2'-OMe +PS | 2'-OMe +PS | 2'-OMe | |
| D | 1 | 2 | 3 | 4 | 5 | 6 | 7 | 8 | 9 | 10 | 11 | 12 |
| | 2'-OMe +PS+EVP | 2'-F +PS | 2'-OMe | 2'-OMe | 2'-F | 2'-F | 2'-OMe | 2'-OMe | 2'-OMe | 2'-OMe | 2'-OMe | 2'-OMe |
| | 13 | 14 | 15 | 16 | 17 | 18 | 19 | 20 | 21 | 22 | 23 | |
| | 2'-OMe | 2'-F | 2'-OMe | 2'-F | 2'-OMe | 2'-OMe | 2'-OMe | 2'-OMe | 2'-OMe | 2'-OMe | 2'-OMe | |
| | | | | | | | | | +PS | +PS | | |
| E | 1 | 2 | 3 | 4 | 5 | 6 | 7 | 8 | 9 | 10 | 11 | 12 |
| | 2'-OMe +PS+EVP | 2'-F +PS | 2'-F | 2'-OMe | 2'-F | 2'-F | 2'-OMe | 2'-OMe | 2'-OMe | 2'-OMe | 2'-OMe | 2'-OMe |
| | 13 | 14 | 15 | 16 | 17 | 18 | 19 | 20 | 21 | 22 | 23 | |
| | 2'-OMe | 2'-F | 2'-OMe | 2'-F | 2'-OMe | 2'-OMe | 2'-OMe | 2'-OMe | 2'-OMe +PS | 2'-OMe +PS | 2'-OMe | |
| F | 1 | 2 | 3 | 4 | 5 | 6 | 7 | 8 | 9 | 10 | 11 | 12 |
| | 2'-OMe +PS+EVP | 2'-F +PS | 2'-F | 2'-OMe | 2'-OMe | 2'-F | 2'-OMe | 2'-OMe | 2'-OMe | 2'-OMe | 2'-OMe | 2'-OMe |
| | 13 | 14 | 15 | 16 | 17 | 18 | 19 | 20 | 21 | 22 | 23 | |
| | 2'-OMe | 2'-F | 2'-OMe | 2'-F | 2'-OMe | 2'-OMe | 2'-OMe | 2'-OMe | 2'-OMe +PS | 2'-OMe +PS | 2'-OMe | |

(continued)

| No. | Antisense strand (AS) 5'-3' | | | | | | | | | | | |
|---|---|---|---|---|---|---|---|---|---|---|---|---|
| | **1** | **2** | **3** | **4** | **5** | **6** | **7** | **8** | **9** | **10** | **11** | **12** |
| **G** | **1** | **2** | **3** | **4** | **5** | **6** | **7** | **8** | **9** | **10** | **11** | **12** |
| | 2'-OMe+PS | **2'-F +PS** | 2'-OMe | 2'-OMe | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |

The sense strand was modified according to one of the modification patterns shown in Table 47:

Table 47: Modification of sense strand

| No. | Sense strand (SS) 5'-3' | | | | | | | | | | |
|---|---|---|---|---|---|---|---|---|---|---|---|
| | **1** | **2** | **3** | **4** | **5** | **6** | **7** | **8** | **9** | **10** | **11** |
| **a** | 2'-OMe+PS | 2'-OMe+PS | 2'-OMe | 2'-OMe | 2'-OMe | 2'-OMe | **2'-F** | 2'-OMe | **2'-F** | **2'-F** | **2'-F** |
| | **12** | **13** | **14** | **15** | **16** | **17** | **18** | **19** | **20** | **21** | |
| | 2'-OMe | 2'-OMe | 2'-OMe | 2'-OMe | 2'-OMe | **2'-F** | 2'-OMe | 2'-OMe | 2'-OMe | 2'-OMe | |
| **b** | **1** | **2** | **3** | **4** | **5** | **6** | **7** | **8** | **9** | **10** | **11** |
| | 2'-OMe+PS | 2'-OMe+PS | 2'-OMe | 2'-OMe | 2'-OMe | 2'-OMe | **2'-F** | 2'-OMe | **2'-F** | 2'-OMe | 2'-F |
| | **12** | **13** | **14** | **15** | **16** | **17** | **18** | **19** | **20** | **21** | |
| | 2'-OMe | 2'-OMe | 2'-OMe | 2'-OMe | 2'-OMe | **2'-F** | 2'-OMe | 2'-OMe | 2'-OMe | 2'-OMe | |
| **c** | **1** | **2** | **3** | **4** | **5** | **6** | **7** | **8** | **9** | **10** | **11** |
| | 2'-OMe+PS | 2'-OMe+PS | 2'-OMe | 2'-OMe | 2'-OMe | 2'-OMe | **2'-F** | 2'-OMe | **2'-F** | **2'-F** | **2'-F** |
| | **12** | **13** | **14** | **15** | **16** | **17** | **18** | **19** | **20** | **21** | |
| | 2'-OMe | 2'-OMe | 2'-OMe | 2'-OMe | 2'-OMe | 2'-OMe | 2'-OMe | 2'-OMe | 2'-OMe | 2'-OMe | |

In the above tables, PS represents a phosphorothioate backbone.
The siRNA modification template, in which the antisense strand was modified according to modification pattern A and the sense strand was modified according to modification pattern a, was designated as DV25P.
The siRNA modification template, in which the antisense strand was modified according to modification pattern B and the sense strand was modified according to modification pattern a, was designated as DV26P.
The siRNA modification template, in which the antisense strand was modified according to modification pattern C and the sense strand was modified according to modification pattern a, was designated as DV27P.
The siRNA modification template, in which the antisense strand was modified according to modification pattern B and the sense strand was modified according to modification pattern b, was designated as DV28P.
The siRNA modification template, in which the antisense strand was modified according to modification pattern C and the sense strand was modified according to modification pattern b, was designated as DV29P.
The siRNA modification template, in which the antisense strand was modified according to modification pattern D and the sense strand was modified according to modification pattern b, was designated as DV32P.
The siRNA modification template, in which the antisense strand was modified according to modification pattern E and the sense strand was modified according to modification pattern b, was designated as DV33P.
The siRNA modification template, in which the antisense strand was modified according to modification pattern F and the sense strand was modified according to modification pattern b, was designated as DV34P.
The siRNA modification template, in which the antisense strand was modified according to modification pattern G and the sense strand was modified according to modification pattern c, was designated as DV22.
For compounds in which the sense and antisense strands are 20 and 22 nucleotides in length, respectively, the antisense strand was modified according to one of the modification patterns shown in Table 44:

Table 44: Modification of antisense strand

| No. | Antisense strand (AS) 5'-3' | | | | | | | | | | |
|---|---|---|---|---|---|---|---|---|---|---|---|
| | **1** | **2** | **3** | **4** | **5** | **6** | **7** | **8** | **9** | **10** | **11** |
| A | 2'-OMe +PS+EVP | **2'-F +PS** | 2'-OMe | 2'-OMe | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| | **1** | **2** | **3** | **4** | **5** | **6** | **7** | **8** | **9** | **10** | **11** |
| B | 2'-OMe +PS+EVP | **2'-F +PS** | **2'-F** | **2'-F** | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| C | **1** | **2** | **3** | **4** | **5** | **6** | **7** | **8** | **9** | **10** | **11** |
| | 2'-OMe +PS+EVP | **2'-F +PS** | 2'-OMe | **2'-F** | **2'-F** | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| D | **1** | **2** | **3** | **4** | **5** | **6** | **7** | **8** | **9** | **10** | **11** |
| | 2'-OMe +PS+EVP | **2'-F +PS** | 2'-OMe | 2'-OMe | **2'-F** | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| E | **1** | **2** | **3** | **4** | **5** | **6** | **7** | **8** | **9** | **10** | **11** |
| | 2'-OMe +PS+EVP | **2'-F +PS** | **2'-F** | 2'-OMe | **2'-F** | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| F | **1** | **2** | **3** | **4** | **5** | **6** | **7** | **8** | **9** | **10** | **11** |
| | 2'-OMe +PS+EVP | **2'-F +PS** | **2'-F** | 2'-OMe | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |

The sense strand was modified according to one of the modification patterns shown in Table 48:

Table 48: Modification of sense strand

| No. | Sense strand (SS) 5'-3' | | | | | | | | | |
|---|---|---|---|---|---|---|---|---|---|---|
| | **1** | **2** | **3** | **4** | **5** | **6** | **7** | **8** | **9** | **10** |
| **a** | 2'-OMe+PS | 2'-OMe+PS | 2'-OMe | 2'-OMe | 2'-OMe | **2'-F** | 2'-OMe | **2'-F** | **2'-F** | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | 2'-F | 2'-OMe | 2'-OMe | 2'-OMe | 2'-OMe |
| **b** | **1** | **2** | **3** | **4** | **5** | **6** | **7** | **8** | **9** | **10** |
| | 2'-OMe+PS | 2'-OMe+PS | 2'-OMe | 2'-OMe | 2'-OMe | **2'-F** | 2'-OMe | **2'-F** | 2'-OMe | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | **2'-F** | 2'-OMe | 2'-OMe | 2'-OMe | 2'-OMe |
| **c** | **1** | **2** | **3** | **4** | **5** | **6** | **7** | **8** | **9** | **10** |
| | 2'-OMe+PS | 2'-OMe+PS | 2'-OMe | 2'-OMe | 2'-OMe | **2'-F** | 2'-OMe | **2'-F** | **2'-F** | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | 2'-OMe | 2'-OMe | 2'-OMe | 2'-OMe | 2'-OMe |

In the above tables, 2'-OMe represents 2'-O-methyl; 2'-F represents 2'-fluoro; PS represents a phosphorothioate backbone.

The siRNA modification template, in which the antisense strand was modified according to modification pattern A and the sense strand was modified according to modification pattern a, was designated as DV25P.

The siRNA modification template, in which the antisense strand was modified according to modification pattern B and the sense strand was modified according to modification pattern a, was designated as DV26P.

The siRNA modification template, in which the antisense strand was modified according to modification pattern C and the sense strand was modified according to modification pattern a, was designated as DV27P.

The siRNA modification template, in which the antisense strand was modified according to modification pattern B and the sense strand was modified according to modification pattern b, was designated as DV28P.

The siRNA modification template, in which the antisense strand was modified according to modification pattern C and the sense strand was modified according to modification pattern b, was designated as DV29P. The siRNA modification template, in which the antisense strand was modified according to modification pattern D and the sense strand was modified according to modification pattern b, was designated as DV32P. The siRNA modification template, in which the antisense strand was modified according to modification pattern E and the sense strand was modified according to modification pattern b, was designated as DV33P.

The siRNA modification template, in which the antisense strand was modified according to modification pattern F and the sense strand was modified according to modification pattern b, was designated as DV34P.

The template-modified sequences are detailed in Table 12, and the synthesis methods for these sequence were the same as in Example 1.

Table 12: Sequences modified with different modification templates

| Unmodified sequence ID | Template - modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| 1835 | C1835-DV29P | Ums-Ums-Gm-Gm-Gm-Um-Uf-Um-Uf-Am-Af-Am-Am-Um-Um-Am-Af-Am-Gm-Um-Am | 397/1 | UmsEVP-Afs-Cm-Uf-Uf-Uf-Am-Am-Um-Um-Um-Um-Am-Af-Am-Af-Cm-Cm-Cm-Am-Ams-Ums-Um | 432/14 |
| | C1835-DV26P | Ums-Ums-Gm-Gm-Gm-Um-Uf-Um-Uf-Af-Af-Am-Am-Um-Um-Am-Af-Am-Gm-Um-Am | 398/1 | UmsEVP-Afs-Cf-Uf-Um-Uf-Am-Am-Um-Um-Um-Um-Am-Af-Am-Af-Cm-Cm-Cm-Am-Ams-Ums-Um | 433/14 |
| | C1835-DV27P | Ums-Ums-Gm-Gm-Gm-Um-Uf-Um-Uf-Af-Af-Am-Am-Um-Um-Am-Af-Am-Gm-Um-Am | 398/1 | UmsEVP-Afs-Cm-Uf-Uf-Uf-Am-Am-Um-Um-Um-Um-Am-Af-Am-Af-Cm-Cm-Cm-Am-Ams-Ums-Um | 432/14 |
| | C1835-DV28P | Ums-Ums-Gm-Gm-Gm-Um-Uf-Um-Uf-Am-Af-Am-Am-Um-Um-Am-Af-Am-Gm-Um-Am | 397/1 | UmsEVP-Afs-Cf-Uf-Um-Uf-Am-Am-Um-Um-Um-Um-Am-Af-Am-Af-Cm-Cm-Cm-Am-Ams-Ums-Um | 433/14 |
| | C1835-DV32P | Ums-Ums-Gm-Gm-Gm-Um-Uf-Um-Uf-Am-Af-Am-Am-Um-Um-Am-Af-Am-Gm-Um-Am | 397/1 | UmsEVP-Afs-Cm-Um-Uf-Uf-Am-Am-Um-Um-Um-Um-Am-Af-Am-Af-Cm-Cm-Cm-Am-Ams-Ums-Um | 434/14 |
| | C1835-DV34P | Ums-Ums-Gm-Gm-Gm-Um-Uf-Um-Uf-Am-Af-Am-Am-Um-Um-Am-Af-Am-Gm-Um-Am | 397/1 | UmsEVP-Afs-Cf-Um-Um-Uf-Am-Am-Um-Um-Um-Um-Am-Af-Am-Af-Cm-Cm-Cm-Am-Ams-Ums-Um | 435/14 |

(continued)

| Unmodified sequence ID | Template - modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| | C1835-DV25P | Ums-Ums-Gm-Gm-Gm-Um-Uf-Um-Uf-Af-Af-Am-Am-Um-Um-Am-Af-Am-Gm-Um-Am | 398/1 | UmsEVP-Afs-Cm-Um-Um-Uf-Am-Am-Um-Um-Um-Um-Am-Af-Am-Af-Cm-Cm-Cm-Am-Ams-Ums-Um | 436/14 |
| | C1835-DV33P | Ums-Ums-Gm-Gm-Gm-Um-Uf-Um-Uf-Am-Af-Am-Am-Um-Um-Am-Af-Am-Gm-Um-Am | 397/1 | UmsEVP-Afs-Cf-Um-Uf-Uf-Am-Am-Um-Um-Um-Um-Am-Af-Am-Af-Cm-Cm-Cm-Am-Ams-Ums-Um | 437/14 |
| | C1835-DV22 | Ums-Ums-Gm-Gm-Gm-Um-Uf-Um-Uf-Af-Af-Am-Am-Um-Um-Am-Am-Am-Gm-Um-Am | 399/1 | Ums-Afs-Cm-Um-Um-Uf-Am-Am-Um-Um-Um-Um-Am-Af-Am-Af-Cm-Cm-Cm-Am-Ams-Ums-Um | 438/14 |
| 1816 | C1816-DV29P | Ums-Ums-Cm-Am-Am-Gm-Uf-Um-Gf-Am-Gf-Am-Am-Cm-Am-Am-Af-Am-Am-Um-Um | 400/2 | AmsEVP-Afs-Um-Uf-Uf-Uf-Um-Gm-Um-Um-Cm-Um-Cm-Af-Am-Cf-Um-Um-Gm-Am-Ams-Ams-Am | 439/15 |
| | C1816-DV26P | Ums-Ums-Cm-Am-Am-Gm-Uf-Um-Gf-Af-Gf-Am-Am-Cm-Am-Am-Af-Am-Am-Um-Um | 401/2 | AmsEVP-Afs-Uf-Uf-Um-Uf-Um-Gm-Um-Um-Cm-Um-Cm-Af-Am-Cf-Um-Um-Gm-Am-Ams-Ams-Am | 440/15 |
| | C1816-DV27P | Ums-Ums-Cm-Am-Am-Gm-Uf-Um-Gf-Af-Gf-Am-Am-Cm-Am-Am-Af-Am-Am-Um-Um | 401/2 | AmsEVP-Afs-Um-Uf-Uf-Uf-Um-Gm-Um-Um-Cm-Um-Cm-Af-Am-Cf-Um-Um-Gm-Am-Ams-Ams-Am | 439/15 |

(continued)

| Unmodified sequence ID | Template - modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| | C1816-DV28P | Ums-Ums-Cm-Am-Am-Gm-Uf-Um-Gf-Am-Gf-Am-Am-Cm-Am-Am-Af-Am-Am-Um-Um | 400/2 | AmsEVP-Afs-Uf-Uf-Um-Uf-Um-Gm-Um-Um-Cm-Um-Cm-Af-Am-Cf-Um-Um-Gm-Am-Ams-Ams-Am | 440/15 |
| | C1816-DV32P | Ums-Ums-Cm-Am-Am-Gm-Uf-Um-Gf-Am-Gf-Am-Am-Cm-Am-Am-Af-Am-Am-Um-Um | 400/2 | AmsEVP-Afs-Um-Um-Uf-Uf-Um-Gm-Um-Um-Cm-Um-Cm-Af-Am-Cf-Um-Um-Gm-Am-Ams-Ams-Am | 441/15 |
| | C1816-DV34P | Ums-Ums-Cm-Am-Am-Gm-Uf-Um-Gf-Am-Gf-Am-Am-Cm-Am-Am-Af-Am-Am-Um-Um | 400/2 | AmsEVP-Afs-Uf-Um-Um-Uf-Um-Gm-Um-Um-Cm-Um-Cm-Af-Am-Cf-Um-Um-Gm-Am-Ams-Ams-Am | 442/15 |
| | C1816-DV25P | Ums-Ums-Cm-Am-Am-Gm-Uf-Um-Gf-Af-Gf-Am-Am-Cm-Am-Am-Af-Am-Am-Um-Um | 401/2 | AmsEVP-Afs-Um-Um-Um-Uf-Um-Gm-Um-Um-Cm-Um-Cm-Af-Am-Cf-Um-Um-Gm-Am-Ams-Ams-Am | 443/15 |
| | C1816-DV33P | Ums-Ums-Cm-Am-Am-Gm-Uf-Um-Gf-Am-Gf-Am-Am-Cm-Am-Am-Af-Am-Am-Um-Um | 400/2 | AmsEVP-Afs-Uf-Um-Uf-Uf-Um-Gm-Um-Um-Cm-Um-Cm-Af-Am-Cf-Um-Um-Gm-Am-Ams-Ams-Am | 444/15 |
| | C1816-DV22 | Ums-Ums-Cm-Am-Am-Gm-Uf-Um-Gf-Af-Gf-Am-Am-Cm-Am-Am-Am-Am-Am-Um-Um | 402/2 | Ams-Afs-Um-Um-Um-Uf-Um-Gm-Um-Um-Cm-Um-Cm-Af-Am-Cf-Um-Um-Gm-Am-Ams-Ams-Am | 445/15 |

(continued)

| Unmodified sequence ID | Template - modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| 1812 | C1812-DV29P | Cms-Cms-Um-Um-Um-Um-Cf-Am-Af-Gm-Uf-Um-Gm-Am-Gm-Am-Af-Cm-Am-Am-Am | 403/3 | UmsEVP-Ufs-Um-Gf-Uf-Uf-Cm-Um-Cm-Am-Am-Cm-Um-Uf-Gm-Af-Am-Am-Am-Gm-Gms-Gms-Am | 446/16 |
| | C1812-DV26P | Cms-Cms-Um-Um-Um-Um-Cf-Am-Af-Gf-Uf-Um-Gm-Am-Gm-Am-Af-Cm-Am-Am-Am | 404/3 | UmsEVP-Ufs-Uf-Gf-Um-Uf-Cm-Um-Cm-Am-Am-Cm-Um-Uf-Gm-Af-Am-Am-Am-Gm-Gms-Gms-Am | 447/16 |
| | C1812-DV27P | Cms-Cms-Um-Um-Um-Um-Cf-Am-Af-Gf-Uf-Um-Gm-Am-Gm-Am-Af-Cm-Am-Am-Am | 404/3 | UmsEVP-Ufs-Um-Gf-Uf-Uf-Cm-Um-Cm-Am-Am-Cm-Um-Uf-Gm-Af-Am-Am-Am-Gm-Gms-Gms-Am | 446/16 |
| | C1812-DV28P | Cms-Cms-Um-Um-Um-Um-Cf-Am-Af-Gm-Uf-Um-Gm-Am-Gm-Am-Af-Cm-Am-Am-Am | 403/3 | UmsEVP-Ufs-Uf-Gf-Um-Uf-Cm-Um-Cm-Am-Am-Cm-Um-Uf-Gm-Af-Am-Am-Am-Gm-Gms-Gms-Am | 447/16 |
| | C1812-DV32P | Cms-Cms-Um-Um-Um-Um-Cf-Am-Af-Gm-Uf-Um-Gm-Am-Gm-Am-Af-Cm-Am-Am-Am | 403/3 | UmsEVP-Ufs-Um-Gm-Uf-Uf-Cm-Um-Cm-Am-Am-Cm-Um-Uf-Gm-Af-Am-Am-Am-Gm-Gms-Gms-Am | 448/16 |
| | C1812-DV34P | Cms-Cms-Um-Um-Um-Um-Cf-Am-Af-Gm-Uf-Um-Gm-Am-Gm-Am-Af-Cm-Am-Am-Am | 403/3 | UmsEVP-Ufs-Uf-Gm-Um-Uf-Cm-Um-Cm-Am-Am-Cm-Um-Uf-Gm-Af-Am-Am-Am-Gm-Gms-Gms-Am | 449/16 |

(continued)

| Unmodified sequence ID | Template - modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| | C1812-DV25P | Cms-Cms-Um-Um-Um-Um-Cf-Am-Af-Gf-Uf-Um-Gm-Am-Gm-Am-Af-Cm-Am-Am-Am | 404/3 | UmsEVP-Ufs-Um-Gm-Um-Uf-Cm-Um-Cm-Am-Am-Cm-Um-Uf-Gm-Af-Am-Am-Am-Gm-Gms-Gms-Am | 450/16 |
| | C1812-DV33P | Cms-Cms-Um-Um-Um-Um-Cf-Am-Af-Gm-Uf-Um-Gm-Am-Gm-Am-Af-Cm-Am-Am-Am | 403/3 | UmsEVP-Ufs-Uf-Gm-Uf-Uf-Cm-Um-Cm-Am-Am-Cm-Um-Uf-Gm-Af-Am-Am-Am-Gm-Gms-Gms-Am | 451/16 |
| | C1812-DV22 | Cms-Cms-Um-Um-Um-Um-Cf-Am-Af-Gf-Uf-Um-Gm-Am-Gm-Am-Am-Cm-Am-Am-Am | 405/3 | Ums-Ufs-Um-Gm-Um-Uf-Cm-Um-Cm-Am-Am-Cm-Um-Uf-Gm-Af-Am-Am-Am-Gm-Gms-Gms-Am | 452/16 |
| 1579 | C1579-DV29P | Cms-Cms-Um-Um-Um-Um-Cf-Um-Uf-Cm-Uf-Am-Am-Um-Gm-Am-Gf-Um-Cm-Gm-Am | 406/4 | UmsEVP-Cfs-Gm-Af-Cf-Uf-Cm-Am-Um-Um-Am-Gm-Am-Af-Gm-Af-Am-Am-Am-Gm-Gms-Ums-Gm | 453/17 |
| | C1579-DV26P | Cms-Cms-Um-Um-Um-Um-Cf-Um-Uf-Cf-Uf-Am-Am-Um-Gm-Am-Gf-Um-Cm-Gm-Am | 407/4 | UmsEVP-Cfs-Gf-Af-Cm-Uf-Cm-Am-Um-Um-Am-Gm-Am-Af-Gm-Af-Am-Am-Am-Gm-Gms-Ums-Gm | 454/17 |
| | C1579-DV27P | Cms-Cms-Um-Um-Um-Um-Cf-Um-Uf-Cf-Uf-Am-Am-Um-Gm-Am-Gf-Um-Cm-Gm-Am | 407/4 | UmsEVP-Cfs-Gm-Af-Cf-Uf-Cm-Am-Um-Um-Am-Gm-Am-Af-Gm-Af-Am-Am-Am-Gm-Gms-Ums-Gm | 453/17 |

(continued)

| Unmodified sequence ID | Template - modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| | C1579-DV28P | Cms-Cms-Um-Um-Um-Um-Cf-Um-Uf-Cm-Uf-Am-Am-Um-Gm-Am-Gf-Um-Cm-Gm-Am | 406/4 | UmsEVP-Cfs-Gf-Af-Cm-Uf-Cm-Am-Um-Um-Am-Gm-Am-Af-Gm-Af-Am-Am-Am-Gm-Gms-Ums-Gm | 454/17 |
| | C1579-DV32P | Cms-Cms-Um-Um-Um-Um-Cf-Um-Uf-Cm-Uf-Am-Am-Um-Gm-Am-Gf-Um-Cm-Gm-Am | 406/4 | UmsEVP-Cfs-Gm-Am-Cf-Uf-Cm-Am-Um-Um-Am-Gm-Am-Af-Gm-Af-Am-Am-Am-Gm-Gms-Ums-Gm | 455/17 |
| | C1579-DV34P | Cms-Cms-Um-Um-Um-Um-Cf-Um-Uf-Cm-Uf-Am-Am-Um-Gm-Am-Gf-Um-Cm-Gm-Am | 406/4 | UmsEVP-Cfs-Gf-Am-Cm-Uf-Cm-Am-Um-Um-Am-Gm-Am-Af-Gm-Af-Am-Am-Am-Gm-Gms-Ums-Gm | 456/17 |
| | C1579-DV25P | Cms-Cms-Um-Um-Um-Um-Cf-Um-Uf-Cf-Uf-Am-Am-Um-Gm-Am-Gf-Um-Cm-Gm-Am | 407/4 | UmsEVP-Cfs-Gm-Am-Cm-Uf-Cm-Am-Um-Um-Am-Gm-Am-Af-Gm-Af-Am-Am-Am-Gm-Gms-Ums-Gm | 457/17 |
| | C1579-DV33P | Cms-Cms-Um-Um-Um-Um-Cf-Um-Uf-Cm-Uf-Am-Am-Um-Gm-Am-Gf-Um-Cm-Gm-Am | 406/4 | UmsEVP-Cfs-Gf-Am-Cf-Uf-Cm-Am-Um-Um-Am-Gm-Am-Af-Gm-Af-Am-Am-Am-Gm-Gms-Ums-Gm | 458/17 |
| | C1579-DV22 | Cms-Cms-Um-Um-Um-Um-Cf-Um-Uf-Cf-Uf-Am-Am-Um-Gm-Am-Gm-Um-Cm-Gm-Am | 408/4 | Ums-Cfs-Gm-Am-Cm-Uf-Cm-Am-Um-Um-Am-Gm-Am-Af-Gm-Af-Am-Am-Am-Gm-Gms-Ums-Gm | 459/17 |

(continued)

| Unmodified sequence ID | Template - modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| 1836 | C1836-DV29P | Ums-Gms-Gm-Gm-Um-Um-Uf-Um-Af-Am-Af-Am-Um-Um-Am-Am-Af-Gm-Um-Am-Um | 409/5 | AmsEVP-Ufs-Am-Cf-Uf-Uf-Um-Am-Am-Um-Um-Um-Um-Af-Am-Af-Am-Cm-Cm-Cm-Ams-Ams-Um | 460/18 |
| | C1836-DV26P | Ums-Gms-Gm-Gm-Um-Um-Uf-Um-Af-Af-Af-Am-Um-Um-Am-Am-Af-Gm-Um-Am-Um | 410/5 | AmsEVP-Ufs-Af-Cf-Um-Uf-Um-Am-Am-Um-Um-Um-Um-Af-Am-Af-Am-Cm-Cm-Cm-Ams-Ams-Um | 461/18 |
| | C1836-DV27P | Ums-Gms-Gm-Gm-Um-Um-Uf-Um-Af-Af-Af-Am-Um-Um-Am-Am-Af-Gm-Um-Am-Um | 410/5 | AmsEVP-Ufs-Am-Cf-Uf-Uf-Um-Am-Am-Um-Um-Um-Um-Af-Am-Af-Am-Cm-Cm-Cm-Ams-Ams-Um | 460/18 |
| | C1836-DV28P | Ums-Gms-Gm-Gm-Um-Um-Uf-Um-Af-Am-Af-Am-Um-Um-Am-Am-Af-Gm-Um-Am-Um | 409/5 | AmsEVP-Ufs-Af-Cf-Um-Uf-Um-Am-Am-Um-Um-Um-Um-Af-Am-Af-Am-Cm-Cm-Cm-Ams-Ams-Um | 461/18 |
| | C1836-DV32P | Ums-Gms-Gm-Gm-Um-Um-Uf-Um-Af-Am-Af-Am-Um-Um-Am-Am-Af-Gm-Um-Am-Um | 409/5 | AmsEVP-Ufs-Am-Cm-Uf-Uf-Um-Am-Am-Um-Um-Um-Um-Af-Am-Af-Am-Cm-Cm-Cm-Ams-Ams-Um | 462/18 |
| | C1836-DV34P | Ums-Gms-Gm-Gm-Um-Um-Uf-Um-Af-Am-Af-Am-Um-Um-Am-Am-Af-Gm-Um-Am-Um | 409/5 | AmsEVP-Ufs-Af-Cm-Um-Uf-Um-Am-Am-Um-Um-Um-Um-Af-Am-Af-Am-Cm-Cm-Cm-Ams-Ams-Um | 463/18 |

(continued)

| Unmodified sequence ID | Template - modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| | C1789-DV29P | Gms-Ums-Um-Um-Gm-Um-Gf-Am-Af-Am-Cf-Am-Am-Am-Am-Am-Af-Gm-Um-Gm-Um | 411/5 | AmsEVP-Cfs-Am-Cf-Uf-Uf-Um-Um-Um-Um-Gm-Um-Um-Uf-Cm-Af-Cm-Am-Am-Am-Cms-Ams-Am | 464/18 |
| | C1789-DV26P | Gms-Ums-Um-Um-Gm-Um-Gf-Am-Af-Af-Cf-Am-Am-Am-Am-Am-Af-Gm-Um-Gm-Um | 412/5 | AmsEVP-Cfs-Af-Cf-Um-Uf-Um-Um-Um-Um-Gm-Um-Um-Uf-Cm-Af-Cm-Am-Am-Am-Cms-Ams-Am | 465/18 |
| | C1789-DV27P | Gms-Ums-Um-Um-Gm-Um-Gf-Am-Af-Af-Cf-Am-Am-Am-Am-Am-Af-Gm-Um-Gm-Um | 412/5 | AmsEVP-Cfs-Am-Cf-Uf-Uf-Um-Um-Um-Um-Gm-Um-Um-Uf-Cm-Af-Cm-Am-Am-Am-Cms-Ams-Am | 464/18 |
| 1789 | C1789-DV28P | Gms-Ums-Um-Um-Gm-Um-Gf-Am-Af-Am-Cf-Am-Am-Am-Am-Am-Af-Gm-Um-Gm-Um | 411/6 | AmsEVP-Cfs-Af-Cf-Um-Uf-Um-Um-Um-Um-Gm-Um-Um-Uf-Cm-Af-Cm-Am-Am-Am-Cms-Ams-Am | 465/19 |
| | C1789-DV32P | Gms-Ums-Um-Um-Gm-Um-Gf-Am-Af-Am-Cf-Am-Am-Am-Am-Am-Af-Gm-Um-Gm-Um | 411/6 | AmsEVP-Cfs-Am-Cm-Uf-Uf-Um-Um-Um-Um-Gm-Um-Um-Uf-Cm-Af-Cm-Am-Am-Am-Cms-Ams-Am | 466/19 |
| | C1789-DV34P | Gms-Ums-Um-Um-Gm-Um-Gf-Am-Af-Am-Cf-Am-Am-Am-Am-Am-Af-Gm-Um-Gm-Um | 411/6 | AmsEVP-Cfs-Af-Cm-Um-Uf-Um-Um-Um-Um-Gm-Um-Um-Uf-Cm-Af-Cm-Am-Am-Am-Cms-Ams-Am | 467/19 |

(continued)

| Unmodified sequence ID | Template - modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| | C1789-DV25P | Gms-Ums-Um-Um-Gm-Um-Gf-Am-Af-Af-Cf-Am-Am-Am-Am-Am-Af-Gm-Um-Gm-Um | 412/6 | AmsEVP-Cfs-Am-Cm-Um-Uf-Um-Um-Um-Um-Gm-Um-Um-Uf-Cm-Af-Cm-Am-Am-Am-Cms-Ams-Am | 468/19 |
| | C1789-DV33P | Gms-Ums-Um-Um-Gm-Um-Gf-Am-Af-Am-Cf-Am-Am-Am-Am-Am-Af-Gm-Um-Gm-Um | 411/6 | AmsEVP-Cfs-Af-Cm-Uf-Uf-Um-Um-Um-Um-Gm-Um-Um-Uf-Cm-Af-Cm-Am-Am-Am-Cms-Ams-Am | 469/19 |
| | C1789-DV22 | Gms-Ums-Um-Um-Gm-Um-Gf-Am-Af-Af-Cf-Am-Am-Am-Am-Am-Am-Gm-Um-Gm-Um | 413/6 | Ams-Cfs-Am-Cm-Um-Uf-Um-Um-Um-Um-Gm-Um-Um-Uf-Cm-Af-Cm-Am-Am-Am-Cms-Ams-Am | 470/19 |
| 1839 | C1839-DV29P | Gms-Ums-Um-Um-Um-Am-Af-Am-Af-Um-Uf-Am-Am-Am-Gm-Um-Af-Um-Am-Cm-Am | 414/7 | UmsEVP-Gfs-Um-Af-Uf-Af-Cm-Um-Um-Um-Am-Am-Um-Uf-Um-Uf-Am-Am-Am-Am-Cms-Cms-Cm | 471/20 |
| | C1839-DV26P | Gms-Ums-Um-Um-Um-Am-Af-Am-Af-Uf-Uf-Am-Am-Am-Gm-Um-Af-Um-Am-Cm-Am | 415/7 | UmsEVP-Gfs-Uf-Af-Um-Af-Cm-Um-Um-Um-Am-Am-Um-Uf-Um-Uf-Am-Am-Am-Am-Cms-Cms-Cm | 472/20 |
| | C1839-DV27P | Gms-Ums-Um-Um-Um-Am-Af-Am-Af-Uf-Uf-Am-Am-Am-Gm-Um-Af-Um-Am-Cm-Am | 415/7 | UmsEVP-Gfs-Um-Af-Uf-Af-Cm-Um-Um-Um-Am-Am-Um-Uf-Um-Uf-Am-Am-Am-Am-Cms-Cms-Cm | 471/20 |

(continued)

| Unmodified sequence ID | Template - modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| | C1839-DV28P | Gms-Ums-Um-Um-Um-Am-Af-Am-Af-Um-Uf-Am-Am-Am-Gm-Um-Af-Um-Am-Cm-Am | 414/7 | UmsEVP-Gfs-Uf-Af-Um-Af-Cm-Um-Um-Um-Am-Am-Um-Uf-Um-Uf-Am-Am-Am-Am-Cms-Cms-Cm | 472/20 |
| | C1839-DV32P | Gms-Ums-Um-Um-Um-Am-Af-Am-Af-Um-Uf-Am-Am-Am-Gm-Um-Af-Um-Am-Cm-Am | 414/7 | UmsEVP-Gfs-Um-Am-Uf-Af-Cm-Um-Um-Um-Am-Am-Um-Uf-Um-Uf-Am-Am-Am-Am-Cms-Cms-Cm | 473/20 |
| | C1839-DV34P | Gms-Ums-Um-Um-Um-Am-Af-Am-Af-Um-Uf-Am-Am-Am-Gm-Um-Af-Um-Am-Cm-Am | 414/7 | UmsEVP-Gfs-Uf-Am-Um-Af-Cm-Um-Um-Um-Am-Am-Um-Uf-Um-Uf-Am-Am-Am-Am-Cms-Cms-Cm | 474/20 |
| 1791 | C1791-DV29P | Ums-Ums-Gm-Um-Gm-Am-Af-Am-Cf-Am-Af-Am-Am-Am-Am-Gm-Uf-Gm-Um-Um-Am | 416/8 | UmsEVP-Afs-Am-Cf-Af-Cf-Um-Um-Um-Um-Um-Um-Gm-Uf-Um-Uf-Cm-Am-Cm-Am-Ams-Ams-Cm | 475/21 |
| | C1791-DV26P | Ums-Ums-Gm-Um-Gm-Am-Af-Am-Cf-Af-Af-Am-Am-Am-Am-Gm-Uf-Gm-Um-Um-Am | 417/8 | UmsEVP-Afs-Af-Cf-Am-Cf-Um-Um-Um-Um-Um-Um-Gm-Uf-Um-Uf-Cm-Am-Cm-Am-Ams-Ams-Cm | 476/21 |
| | C1791-DV27P | Ums-Ums-Gm-Um-Gm-Am-Af-Am-Cf-Af-Af-Am-Am-Am-Am-Gm-Uf-Gm-Um-Um-Am | 417/8 | UmsEVP-Afs-Am-Cf-Af-Cf-Um-Um-Um-Um-Um-Um-Gm-Uf-Um-Uf-Cm-Am-Cm-Am-Ams-Ams-Cm | 475/21 |

(continued)

| Unmodified sequence ID | Template - modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| | C1791-DV28P | Ums-Ums-Gm-Um-Gm-Am-Af-Am-Cf-Am-Af-Am-Am-Am-Am-Gm-Uf-Gm-Um-Um-Am | 416/8 | UmsEVP-Afs-Af-Cf-Am-Cf-Um-Um-Um-Um-Um-Um-Gm-Uf-Um-Uf-Cm-Am-Cm-Am-Ams-Ams-Cm | 476/21 |
| | C1791-DV32P | Ums-Ums-Gm-Um-Gm-Am-Af-Am-Cf-Am-Af-Am-Am-Am-Am-Gm-Uf-Gm-Um-Um-Am | 416/8 | UmsEVP-Afs-Am-Cm-Af-Cf-Um-Um-Um-Um-Um-Um-Gm-Uf-Um-Uf-Cm-Am-Cm-Am-Ams-Ams-Cm | 477/21 |
| | C1791-DV34P | Ums-Ums-Gm-Um-Gm-Am-Af-Am-Cf-Am-Af-Am-Am-Am-Am-Gm-Uf-Gm-Um-Um-Am | 416/8 | UmsEVP-Afs-Af-Cm-Am-Cf-Um-Um-Um-Um-Um-Um-Gm-Uf-Um-Uf-Cm-Am-Cm-Am-Ams-Ams-Cm | 478/21 |
| 1795 | C1795-DV29P | Gms-Ams-Am-Am-Cm-Am-Af-Am-Af-Am-Af-Gm-Um-Gm-Um-Um-Cf-Cm-Cm-Um-Um | 418/9 | AmsEVP-Afs-Gm-Gf-Gf-Af-Am-Cm-Am-Cm-Um-Um-Um-Uf-Um-Uf-Gm-Um-Um-Um-Cms-Ams-Cm | 479/22 |
| | C1795-DV26P | Gms-Ams-Am-Am-Cm-Am-Af-Am-Af-Af-Af-Gm-Um-Gm-Um-Um-Cf-Cm-Cm-Um-Um | 419/9 | AmsEVP-Afs-Gf-Gf-Gm-Af-Am-Cm-Am-Cm-Um-Um-Um-Uf-Um-Uf-Gm-Um-Um-Um-Cms-Ams-Cm | 480/22 |
| | C1795-DV27P | Gms-Ams-Am-Am-Cm-Am-Af-Am-Af-Af-Af-Gm-Um-Gm-Um-Um-Cf-Cm-Cm-Um-Um | 419/9 | AmsEVP-Afs-Gm-Gf-Gf-Af-Am-Cm-Am-Cm-Um-Um-Um-Uf-Um-Uf-Gm-Um-Um-Um-Cms-Ams-Cm | 479/22 |

(continued)

| Unmodified sequence ID | Template - modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| | C1795-DV28P | Gms-Ams-Am-Am-Cm-Am-Af-Am-Af-Am-Af-Gm-Um-Gm-Um-Um-Cf-Cm-Cm-Um-Um | 418/9 | AmsEVP-Afs-Gf-Gf-Gm-Af-Am-Cm-Am-Cm-Um-Um-Um-Uf-Um-Uf-Gm-Um-Um-Um-Cms-Ams-Cm | 480/22 |
| | C1795-DV32P | Gms-Ams-Am-Am-Cm-Am-Af-Am-Af-Am-Af-Gm-Um-Gm-Um-Um-Cf-Cm-Cm-Um-Um | 418/9 | AmsEVP-Afs-Gm-Gm-Gf-Af-Am-Cm-Am-Cm-Um-Um-Um-Uf-Um-Uf-Gm-Um-Um-Um-Cms-Ams-Cm | 481/22 |
| | C1795-DV34P | Gms-Ams-Am-Am-Cm-Am-Af-Am-Af-Am-Af-Gm-Um-Gm-Um-Um-Cf-Cm-Cm-Um-Um | 418/9 | AmsEVP-Afs-Gf-Gm-Gm-Af-Am-Cm-Am-Cm-Um-Um-Um-Uf-Um-Uf-Gm-Um-Um-Um-Cms-Ams-Cm | 482/22 |
| 1585s | C1585s-DV29P | Cms-Ums-Um-Cm-Um-Af-Am-Uf-Gm-Af-Gm-Um-Cm-Gm-Am-Cf-Um-Um-Um-Am | 420/10 | UmsEVP-Afs-Am-Af-Gf-Uf-Cm-Gm-Am-Cm-Um-Cm-Am-Uf-Um-Af-Gm-Am-Am-Gms-Ams-Am | 483/23 |
| | C1585s-DV26P | Cms-Ums-Um-Cm-Um-Af-Am-Uf-Gf-Af-Gm-Um-Cm-Gm-Am-Cf-Um-Um-Um-Am | 421/10 | UmsEVP-Afs-Af-Af-Gm-Uf-Cm-Gm-Am-Cm-Um-Cm-Am-Uf-Um-Af-Gm-Am-Am-Gms-Ams-Am | 484/23 |
| | C1585s-DV27P | Cms-Ums-Um-Cm-Um-Af-Am-Uf-Gf-Af-Gm-Um-Cm-Gm-Am-Cf-Um-Um-Um-Am | 421/10 | UmsEVP-Afs-Am-Af-Gf-Uf-Cm-Gm-Am-Cm-Um-Cm-Am-Uf-Um-Af-Gm-Am-Am-Gms-Ams-Am | 483/23 |

(continued)

| Unmodified sequence ID | Template - modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| | C1585s-DV28P | Cms-Ums-Um-Cm-Um-Af-Am-Uf-Gm-Af-Gm-Um-Cm-Gm-Am-Cf-Um-Um-Um-Am | 420/10 | UmsEVP-Afs-Af-Af-Gm-Uf-Cm-Gm-Am-Cm-Um-Cm-Am-Uf-Um-Af-Gm-Am-Am-Gms-Ams-Am | 484/23 |
| | C1585s-DV32P | Cms-Ums-Um-Cm-Um-Af-Am-Uf-Gm-Af-Gm-Um-Cm-Gm-Am-Cf-Um-Um-Um-Am | 420/10 | UmsEVP-Afs-Am-Am-Gf-Uf-Cm-Gm-Am-Cm-Um-Cm-Am-Uf-Um-Af-Gm-Am-Am-Gms-Ams-Am | 485/23 |
| | C1585s-DV34P | Cms-Ums-Um-Cm-Um-Af-Am-Uf-Gm-Af-Gm-Um-Cm-Gm-Am-Cf-Um-Um-Um-Am | 420/10 | UmsEVP-Afs-Af-Am-Gm-Uf-Cm-Gm-Am-Cm-Um-Cm-Am-Uf-Um-Af-Gm-Am-Am-Gms-Ams-Am | 486/23 |
| | C1585s-DV22 | Cms-Ums-Um-Cm-Um-Af-Am-Uf-Gf-Af-Gm-Um-Cm-Gm-Am-Cm-Um-Um-Um-Am | 422/10 | Ums-Afs-Am-Am-Gm-Uf-Cm-Gm-Am-Cm-Um-Cm-Am-Uf-Um-Af-Gm-Am-Am-Gms-Ams-Am | 487/23 |
| 1576s | C1576s-DV29P | Cms-Cms-Am-Cm-Cm-Uf-Um-Uf-Um-Cf-Um-Um-Cm-Um-Am-Af-Um-Gm-Am-Am | 423/11 | UmsEVP-Ufs-Cm-Af-Uf-Uf-Am-Gm-Am-Am-Gm-Am-Am-Af-Am-Gf-Gm-Um-Gm-Gms-Gms-Am | 488/24 |
| | C1576s-DV28P | Cms-Cms-Am-Cm-Cm-Uf-Um-Uf-Um-Cf-Um-Um-Cm-Um-Am-Af-Um-Gm-Am-Am | 423/11 | UmsEVP-Ufs-Cf-Af-Um-Uf-Am-Gm-Am-Am-Gm-Am-Am-Af-Am-Gf-Gm-Um-Gm-Gms-Gms-Am | 489/24 |

(continued)

| Unmodified sequence ID | Template - modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| | C1576s-DV26P | Cms-Cms-Am-Cm-Cm-Uf-Um-Uf-Uf-Cf-Um-Um-Cm-Um-Am-Af-Um-Gm-Am-Am | 424/11 | UmsEVP-Ufs-Cf-Af-Um-Uf-Am-Gm-Am-Am-Gm-Am-Am-Af-Am-Gf-Gm-Um-Gm-Gms-Gms-Am | 489/24 |
| | C1576s-DV25P | Cms-Cms-Am-Cm-Cm-Uf-Um-Uf-Uf-Cf-Um-Um-Cm-Um-Am-Af-Um-Gm-Am-Am | 424/11 | UmsEVP-Ufs-Cm-Am-Um-Uf-Am-Gm-Am-Am-Gm-Am-Am-Af-Am-Gf-Gm-Um-Gm-Gms-Gms-Am | 490/24 |
| | C1576s-DV27P | Cms-Cms-Am-Cm-Cm-Uf-Um-Uf-Uf-Cf-Um-Um-Cm-Um-Am-Af-Um-Gm-Am-Am | 424/11 | UmsEVP-Ufs-Cm-Af-Uf-Uf-Am-Gm-Am-Am-Gm-Am-Am-Af-Am-Gf-Gm-Um-Gm-Gms-Gms-Am | 488/24 |
| | C1576s-DV22 | Cms-Cms-Am-Cm-Cm-Uf-Um-Uf-Uf-Cf-Um-Um-Cm-Um-Am-Am-Um-Gm-Am-Am | 425/11 | Ums-Ufs-Cm-Am-Um-Uf-Am-Gm-Am-Am-Gm-Am-Am-Af-Am-Gf-Gm-Um-Gm-Gms-Gms-Am | 491/24 |
| 1578s | C1578s-DV29P | Ams-Cms-Cm-Um-Um-Uf-Um-Cf-Um-Uf-Cm-Um-Am-Am-Um-Gf-Am-Gm-Um-Am | 426/12 | UmsEVP-Afs-Cm-Uf-Cf-Af-Um-Um-Am-Gm-Am-Am-Gm-Af-Am-Af-Am-Gm-Gm-Ums-Gms-Gm | 492/25 |
| | C1578s-DV28P | Ams-Cms-Cm-Um-Um-Uf-Um-Cf-Um-Uf-Cm-Um-Am-Am-Um-Gf-Am-Gm-Um-Am | 426/12 | UmsEVP-Afs-Cf-Uf-Cm-Af-Um-Um-Am-Gm-Am-Am-Gm-Af-Am-Af-Am-Gm-Gm-Ums-Gms-Gm | 493/25 |

(continued)

| Unmodified sequence ID | Template - modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| | C1578s-DV26P | Ams-Cms-Cm-Um-Um-Uf-Um-Cf-Uf-Uf-Cm-Um-Am-Am-Um-Gf-Am-Gm-Um-Am | 427/12 | UmsEVP-Afs-Cf-Uf-Cm-Af-Um-Um-Am-Gm-Am-Am-Gm-Af-Am-Af-Am-Gm-Gm-Ums-Gms-Gm | 493/25 |
| | C1578s-DV25P | Ams-Cms-Cm-Um-Um-Uf-Um-Cf-Uf-Uf-Cm-Um-Am-Am-Um-Gf-Am-Gm-Um-Am | 427/12 | UmsEVP-Afs-Cm-Um-Cm-Af-Um-Um-Am-Gm-Am-Am-Gm-Af-Am-Af-Am-Gm-Gm-Ums-Gms-Gm | 494/25 |
| | C1578s-DV27P | Ams-Cms-Cm-Um-Um-Uf-Um-Cf-Uf-Uf-Cm-Um-Am-Am-Um-Gf-Am-Gm-Um-Am | 427/12 | UmsEVP-Afs-Cm-Uf-Cf-Af-Um-Um-Am-Gm-Am-Am-Gm-Af-Am-Af-Am-Gm-Gm-Ums-Gms-Gm | 492/25 |
| | C1578s-DV22 | Ams-Cms-Cm-Um-Um-Uf-Um-Cf-Uf-Uf-Cm-Um-Am-Am-Um-Gm-Am-Gm-Um-Am | 428/12 | Ums-Afs-Cm-Um-Cm-Af-Um-Um-Am-Gm-Am-Am-Gm-Af-Am-Af-Am-Gm-Gm-Ums-Gms-Gm | 495/25 |
| 1838 | C1838-DV29P | Gms-Gms-Um-Um-Um-Um-Af-Am-Af-Am-Uf-Um-Am-Am-Am-Gm-Uf-Am-Um-Am-Am | 429/13 | UmsEVP-Ufs-Am-Uf-Af-Cf-Um-Um-Um-Am-Am-Um-Um-Uf-Um-Af-Am-Am-Am-Cm-Cms-Cms-Am | 496/26 |
| | C1838-DV28P | Gms-Gms-Um-Um-Um-Um-Af-Am-Af-Am-Uf-Um-Am-Am-Am-Gm-Uf-Am-Um-Am-Am | 429/13 | UmsEVP-Ufs-Af-Uf-Am-Cf-Um-Um-Um-Am-Am-Um-Um-Uf-Um-Af-Am-Am-Am-Cm-Cms-Cms-Am | 497/26 |

(continued)

| Unmodified sequence ID | Template - modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| | C1838-DV26P | Gms-Gms-Um-Um-Um-Um-Af-Am-Af-Af-Uf-Um-Am-Am-Am-Gm-Uf-Am-Um-Am-Am | 430/13 | UmsEVP-Ufs-Af-Uf-Am-Cf-Um-Um-Um-Am-Am-Um-Um-Uf-Um-Af-Am-Am-Am-Cm-Cms-Cms-Am | 497/26 |
| | C1838-DV25P | Gms-Gms-Um-Um-Um-Um-Af-Am-Af-Af-Uf-Um-Am-Am-Am-Gm-Uf-Am-Um-Am-Am | 430/13 | UmsEVP-Ufs-Am-Um-Am-Cf-Um-Um-Um-Am-Am-Um-Um-Uf-Um-Af-Am-Am-Am-Cm-Cms-Cms-Am | 498/26 |
| | C1838-DV27P | Gms-Gms-Um-Um-Um-Um-Af-Am-Af-Af-Uf-Um-Am-Am-Am-Gm-Uf-Am-Um-Am-Am | 430/13 | UmsEVP-Ufs-Am-Uf-Af-Cf-Um-Um-Um-Am-Am-Um-Um-Uf-Um-Af-Am-Am-Am-Cm-Cms-Cms-Am | 496/26 |
| | C1838-DV22 | Gms-Gms-Um-Um-Um-Um-Af-Am-Af-Af-Uf-Um-Am-Am-Am-Gm-Um-Am-Um-Am-Am | 431/13 | Ums-Ufs-Am-Um-Am-Cf-Um-Um-Um-Am-Am-Um-Um-Uf-Um-Af-Am-Am-Am-Cm-Cms-Cms-Am | 499/26 |

Cell type: HepG2 cell line.

Drug vehicle: sterile nuclease-free water, Gibco Opti-MEM.

## 2. Experimental methods

The inhibitory effects of the test samples on the mRNA expression of AGT genes in HepG2 cell lines were tested by qRT-PCR.

### 2.1 Cell culture

HepG2 cells in the logarithmic growth phase were harvested from subcultured stocks and cultured in DMEM medium containing 10% fetal bovine serum (supplemented with 100 μL/mL of penicillin and streptomycin). The cells were incubated in a cell culture incubator at 37°C with 5% $CO_2$, with medium replaced daily. For subculture, the cells were digested with 0.25% trypsin, centrifuged at 1000 rpm for 5 minutes, and the supernatant was discarded. Fresh medium was added for subculture.

### 2.2 Cell transfection

Preparation of transfection mixture: Lipofectamine RNAiMAX and Opti-MEM were mixed at a ratio of 2:98 and vortexed thoroughly.

Preparation of transfection reagent: 60 μL of siRNA solution diluted in Opti-MEM was mixed with 60 μL of the prepared transfection mixture at a ratio of 1:1 (v/v), vortexed thoroughly, and the mixture was incubated at room temperature for 15 minutes to obtain lipid nanoparticles (LNPs). A 12.5 μL aliquot was taken for encapsulation efficiency analysis.

Blank control transfection reagent: 60 μL of the prepared transfection mixture was added to 60 μL of Opti-MEM, vortexed thoroughly, and the mixture was incubated at room temperature for 15 minutes.

The prepared transfection reagent was added to a 24-well cell culture plate (100 μL per well), resulting in a final siRNA concentration of 0.02 nM per well. 500 μL of cell suspension ($1.5 \times 10^5$ cells per mL) was added per well. After mixing by crosswise method, the plate was incubated in a cell culture incubator at 37°C with 5% $CO_2$ for 40 hours.

### 2.3 AGT mRNA assay

The procedure was the same as that described in "2.3 AGT mRNA assay" in Example 2.

### 2.4 Data processing

AGT mRNA expression rate (%) was calculated using the following formula:

Expression rate = (AGT mRNA expression level / AGT mRNA expression level in blank control group) × 100%;

$$\text{Inhibition rate of AGT gene expression} = 1 - \text{expression rate (\%)}.$$

### 2.5 $IC_{50}$ assay

In this experiment, the siRNA concentrations for the $EC_{50}$ assay started from 1.0 nM, with 4-fold dilutions, totaling 8 concentration points (1.0 nM, 0.25 nM, 0.0625 nM, 15.6 pM, 3.9 pM, 0.977 pM, 0.244 pM, and 0.061 pM). The inhibition rate of each sequence at each concentration was measured and plotted to calculate the $IC_{50}$ values.

## 3. Experimental results

Following three independent experiments, the inhibition rates of modified sequence on AGT gene expression in HepG2 cells are shown in Tables 13 to 25.

The activity assay results demonstrated that all 13 candidate sequences (unmodified sequences 1576s, 1578s, 1838, 1835, 1816, 1812, 1579, 1836, 1789, 1839, 1791, 1795, and 1585s), when modified using the modification templates DV25P-29P and DV32P-34P designed in the present disclosure, exhibited significant inhibitory effects on AGT

gene expression, with inhibition rates of 40% or more. Specifically, C1812-DV25P, C1579-DV33P, and C1789-DV26P achieved inhibition rates of 74.4%, 74.0%, and 73.6%, respectively.

The sequences modified using the modification templates DV25P-29P and DV32P-34P of the present disclosure showed a significant improvement in inhibition rate on AGT gene expression as compared to the alternately modified sequences. For example, the inhibition rate of unmodified sequence 1579 modified with template DV33P (C1579-DV33P) was 12.9% higher than that of the alternately modified sequence, while the inhibition rate of unmodified sequence 1812 modified with template DV25P (C1812-DV25P) was 11.5% higher than that of the alternately modified sequence.

Moreover, the same siRNA sequence exhibited significant differences in activity when modified with different modification templates. For example, unmodified sequence 1579 modified with template DV33P of the present disclosure exhibited a 29.8% increase in inhibition rate compared to the sequence modified with template DV28P of the present disclosure, and a 18.4% increase in inhibition rate compared to the sequence modified with the previously published Advanced ESC template DV22.

$IC_{50}$ assays demonstrated that sequences modified using the modification templates designed in the present disclosure exhibited $IC_{50}$ values ranging from 0.0019 to 0.0615 nM. For example, the $IC_{50}$ values of C1789-DV25P, C1812-DV25P, and C1789-DV26P were 0.0019 nM, 0.0022 nM, and 0.0026 nM, respectively. These sequences effectively inhibited AGT gene expression at low concentrations.

### (i) Inhibitory effect of unmodified sequences on AGT gene expression after template modification

### (1) Unmodified sequence 1576s

Table 13: Inhibition rates of unmodified sequence 1576s on AGT gene expression after template modification

| Unmodified sequence ID | Template-modified sequence ID | Inhibition rate (%) of template-modified sequence | Inhibition rate (%) of corresponding alternately modified sequence | Increase in inhibition rate relative to alternately modified sequence (%) |
|---|---|---|---|---|
| 1576s | C1576s-DV26P | 73.4 | 64.6 | 8.8 |
| | C1576s-DV28P | 58.8 | | -5.8 |
| | C1576s-DV29P | 79.3 | | 14.7 |
| | C1576s-DV25P | 73.3 | | 8.7 |
| | C1576s-DV27P | 69.7 | | 5.1 |
| | C1576s-DV22 | 63.9 | | -0.7 |

### I. Templates DV25P-29P of the present disclosure

When modified with templates DV25, DV26, and DV29 designed in the present disclosure, unmodified sequence 1576s exhibited inhibition rates exceeding 70% on AGT gene expression, indicating a significant improvement as compared to 2'-O-methyl and 2'-fluoro alternating modifications. For example, after modification with templates DV25P and DV29P, the inhibition rates of C1576s-DV25P and C1576s-DV29P increased by 8.7% and 14.7%, respectively.

### II. Advanced ESC template DV22 disclosed in the prior art (fluorine-substituted sites: positions 2, 6, 14, and 16 on the antisense strand; positions 7, 9, 10, and 11 on the sense strand)

When unmodified sequence 1576s was modified with template DV22, the resulting sequence C1576s-DV22 exhibited an inhibition rate of 63.9% on AGT gene expression, which was 0.7% lower than that of the alternately modified sequence.

After modification with templates DV25P and DV29P of the present disclosure, the inhibition rates of C1576s-DV25P and C1576s-DV29P were 9.4% and 15.4% higher, respectively, than that of C1576s-DV22, indicating a significant improvement.

Thus, it can be concluded that:

1) When modified with templates DV25-29 of the present disclosure, unmodified sequence 1576s exhibited significantly enhanced inhibitory effect on AGT gene expression compared to the alternately modified sequence. For example, when unmodified sequence 1576s was modified with DV26, the inhibition rate increased by 8.8% compared to the alternately modified sequence.
2) The same siRNA sequence exhibited significant differences in activity when modified with different modification templates. For example, when unmodified sequence 1576s was modified with templates DV25P and DV29P of the present disclosure, the inhibition rate increased by up to 20.5% compared to modification with template DV28P of the present disclosure, indicating a significant improvement; the inhibition rate increased by up to 15.4% compared to modification with the previously published Advanced ESC template DV22, indicating a significant improvement.
3) The activity of siRNA sequences modified with different templates varied greatly, with differences of up to 20.5%. Thus, it is uncertain which modification template would confer high activity to an siRNA sequence.

**(2) Unmodified sequence 1578s**

Table 14: Inhibition rates of unmodified sequence 1579 on AGT gene expression after template modification

| Unmodified sequence ID | Template-modified sequence ID | Inhibition rate (%) of template-modified sequence | Inhibition rate (%) of corresponding alternately modified sequence | Increase in inhibition rate relative to alternately modified sequence (%) |
|---|---|---|---|---|
| 1578s | C1578s-DV26P | 69.7 | 63.9 | 5.8 |
| | C1578s-DV28P | 56.9 | | -7.0 |
| | C1578s-DV29P | 77.1 | | 13.2 |
| | C1578s-DV25P | 75.8 | | 11.9 |
| | C1578s-DV27P | 73.1 | | 9.2 |
| | C1578s-DV22 | 61.1 | | -2.8 |

**I. Templates DV25P-29P of the present disclosure .**

When modified with templates DV25P, DV27P, and DV29P designed in the present disclosure, unmodified sequence 1578s exhibited inhibition rates exceeding 70% on AGT gene expression, indicating a significant improvement as compared to 2'-O-methyl and 2'-fluoro alternating modifications. For example, after modification with templates DV25P and DV29P, the inhibition rates of C1578s-DV25P and C1578s-DV29P increased by 11.9% and 13.2%, respectively.

**II. Advanced ESC template DV22 disclosed in the prior art (fluorine-substituted sites: positions 2, 6, 14, and 16 on the antisense strand; positions 7, 9, 10, and 11 on the sense strand)**

When unmodified sequence 1578s was modified with template DV22, the resulting sequence C1578s-DV22 exhibited an inhibition rate of 61.1% on AGT gene expression, which was 2.8% lower than that of the alternately modified sequence.
After modification with templates DV25P and DV29P of the present disclosure, the inhibition rates of C1578s-DV25P and C1578s-DV29P were 14.7% and 16.0% higher, respectively, than that of C1578s-DV22, indicating a significant improvement.
Thus, it can be concluded that:

1) When modified with templates DV25P-29P of the present disclosure, unmodified sequence 1578s exhibited significantly enhanced inhibitory effect on AGT gene expression compared to the alternately modified sequence. For example, when unmodified sequence 1578s was modified with DV27P, the inhibition rate increased by 9.2% compared to the alternately modified sequence.
2) The same siRNA sequence exhibited significant differences in activity when modified with different modification templates. For example, when unmodified sequence 1578s was modified with templates DV25P and DV29P of the present disclosure, the inhibition rate increased by up to 20.2% compared to modification with template DV28P of the present disclosure, indicating a significant improvement; the inhibition rate increased by up to 16.0% compared to modification with the previously published Advanced ESC template DV22, indicating a significant improvement.

3) The activity of siRNA sequences modified with different templates varied greatly, with differences of up to 20.2%. Thus, it is uncertain which modification template would confer high activity to an siRNA sequence.

**(3) Unmodified sequence 1838**

Table 15: Inhibition rates of unmodified sequence 1579 on AGT gene expression after template modification

| Unmodified sequence ID | Template-modified sequence ID | Inhibition rate (%) of template-modified sequence | Inhibition rate (%) of corresponding alternately modified sequence | Increase in inhibition rate relative to alternately modified sequence (%) |
|---|---|---|---|---|
| 1838 | C1838-DV26P | 67.2 | 63.6 | 3.6 |
| | C1838-DV28P | 60.3 | | -3.3 |
| | C1838-DV29P | 75.6 | | 12.0 |
| | C1838-DV25P | 69.3 | | 5.7 |
| | C1838-DV27P | 72.3 | | 8.7 |
| | C1838-DV22 | 62.7 | | -0.9 |

**I. Templates DV25P-29P of the present disclosure**

When modified with templates DV27P and DV29P designed in the present disclosure, unmodified sequence 1838 exhibited inhibition rates exceeding 70% on AGT gene expression, indicating a significant improvement as compared to 2'-O-methyl and 2'-fluoro alternating modifications. For example, after modification with templates DV27P and DV29P, the inhibition rates of C1838-DV27P and C1838-DV29P increased by 8.7% and 12.0%, respectively.

**II. Advanced ESC template DV22 disclosed in the prior art (fluorine-substituted sites: positions 2, 6, 14, and 16 on the antisense strand; positions 7, 9, 10, and 11 on the sense strand)**

When unmodified sequence 1838 was modified with template DV22, the resulting sequence C1838-DV22 exhibited an inhibition rate of 62.7% on AGT gene expression, which was 0.9% lower than that of the alternately modified sequence.

After modification with templates DV27P and DV29P of the present disclosure, the inhibition rates of C1838-DV27P and C1838-DV29P were 9.6% and 12.9% higher, respectively, than that of C1838-DV22, indicating a significant improvement.

Thus, it can be concluded that:

1) When modified with templates DV25P-29P of the present disclosure, unmodified sequence 1838 exhibited significantly enhanced inhibitory effect on AGT gene expression compared to the alternately modified sequence. For example, when unmodified sequence 1838 was modified with DV27P, the inhibition rate increased by 8.7% compared to the alternately modified sequence.
2) The same siRNA sequence exhibited significant differences in activity when modified with different modification templates. For example, when unmodified sequence 1838 was modified with templates DV27P and DV29P of the present disclosure, the inhibition rate increased by up to 15.3% compared to modification with template DV28P of the present disclosure, indicating a significant improvement; the inhibition rate increased by up to 12.9% compared to modification with the previously published Advanced ESC template DV22, indicating a significant improvement.
3) The activity of siRNA sequences modified with different templates varied greatly, with differences of up to 15.3%. Thus, it is uncertain which modification template would confer high activity to an siRNA sequence.

**(4) Unmodified sequence 1579**

Table 16: Inhibition rates of unmodified sequence 1579 on AGT gene expression after template modification

| Unmodified sequence ID | Template-modified sequence ID | Inhibition rate (%) of template-modified sequence | Inhibition rate (%) of corresponding alternately modified sequence | Increase (%) |
|---|---|---|---|---|
| 1579 | C1579-DV25P | 72.2 | 61.1 | 11.1 |
| | C1579-DV26P | 70.8 | | 9.7 |
| | C1579-DV27P | 63.3 | | 2.2 |
| | C1579-DV28P | 44.2 | | -16.9 |
| | C1579-DV29P | 71.7 | | 10.6 |
| | C1579-DV32P | 68.9 | | 7.8 |
| | C1579-DV33P | 74.0 | | 12.9 |
| | C1579-DV34P | 63.9 | | 2.8 |
| | C1579-DV22 | 55.6 | | -5.5 |

**I. Templates DV25P-29P of the present disclosure**

When modified with templates DV25P, DV26P, and DV29P designed in the present disclosure, unmodified sequence 1579 exhibited inhibition rates exceeding 70% on AGT gene expression, indicating a significant improvement as compared to 2'-O-methyl and 2'-fluoro alternating modifications. For example, after modification with templates DV25P and DV29P, the inhibition rates of C1579-DV25P and C1579-DV29P increased by 11.1% and 10.6%, respectively (FIG. 7).

**II. Other modification templates of the present disclosure**

When unmodified sequence 1579 was modified with templates DV32P, DV33P, and DV34P, the resulting sequences C1579-DV32P, C1579-DV33P, and C1579-DV34P exhibited inhibition rates of 68.9%, 74.0%, and 63.9% on AGT gene expression, respectively, which were 7.8%, 12.9%, and 2.8% higher than those of the corresponding alternately modified sequences, respectively.

After modification with templates DV25P and DV29P of the present disclosure, the inhibition rates of C1579-DV25P and C1579-DV29P increased by 3.3% and 2.8%, respectively, compared to C1579-DV32P, decreased by 1.8% and 2.3%, respectively, compared to C1579-DV33P, and increased by 8.3% and 7.8%, respectively, compared to C1579-DV34P.

**III. Advanced ESC template DV22 disclosed in the prior art (fluorine-substituted sites: positions 2, 6, 14, and 16 on the antisense strand; positions 7, 9, 10, and 11 on the sense strand)**

When unmodified sequence 1579 was modified with template DV22, the resulting sequence C1579-DV22 exhibited an inhibition rate of 55.6% on AGT gene expression, which was 5.5% lower than that of the alternately modified sequence.

After modification with templates DV25P and DV29P of the present disclosure, the inhibition rates of C1579-DV25P and C1579-DV29P were 16.6% and 16.1% higher, respectively, than that of C1579-DV22, indicating a significant improvement.

Thus, it can be concluded that:

1) When modified with templates DV25-29 of the present disclosure, unmodified sequence 1579 exhibited significantly enhanced inhibitory effect on AGT gene expression compared to the alternately modified sequence. For example, when unmodified sequence 1579 was modified with DV26, the inhibition rate increased by 9.7% compared to the alternately modified sequence.
2) The same siRNA sequence exhibited significant differences in activity when modified with different modification templates. For example, when unmodified sequence 1579 was modified with templates DV25P and DV29P of the present disclosure, the inhibition rate increased by up to 28.0% compared to modification with template DV28P of the present disclosure, indicating a significant improvement; the inhibition rate increased by up to 16.6% compared to modification with the previously published Advanced ESC template DV22, indicating a significant improvement.

3) The activity of siRNA sequences modified with different templates varied greatly, with differences of up to 28.0%. Thus, it is uncertain which modification template would confer high activity to an siRNA sequence.

**(5) Unmodified sequence 1585s**

Table 17: Inhibition rates of unmodified sequence 1585s on AGT gene expression after template modification

| Unmodified sequence ID | Template-modified sequence ID | Inhibition rate (%) of template-modified sequence | Inhibition rate (%) of corresponding alternately modified sequence | Increase (%) |
|---|---|---|---|---|
| 1585s | C1585s-DV26P | 62.6 | 59.3 | 3.3 |
| | C1585s-DV27P | 64.6 | | 5.3 |
| | C1585s-DV28P | 49.5 | | -9.8 |
| | C1585s-DV29P | 59.2 | | -0.1 |
| | C1585s-DV32P | 61.8 | | 2.5 |
| | C1585s-DV34P | 54.1 | | -5.2 |
| | C1585s-DV22 | 55.2 | | -4.1 |

**I. Templates DV26P-29P of the present disclosure**

When modified with templates DV26P, DV27P, and DV29P designed in the present disclosure, unmodified sequence 1585s exhibited inhibition rates exceeding 50% on AGT gene expression, indicating a significant improvement as compared to 2'-O-methyl and 2'-fluoro alternating modifications. For example, after modification with templates DV26P and DV27P, the inhibition rates of C1585s-DV26P and C1585s-DV27P increased by 3.3% and 5.3%, respectively.

**II. Other modification templates of the present disclosure**

When unmodified sequence 1585s was modified with templates DV32P and DV34P, the resulting sequences C1585s-DV32P and C1585s-DV34P exhibited inhibition rates of 61.8% and 54.1% on AGT gene expression, respectively, which were 2.5% and -5.2% higher than those of the corresponding alternately modified sequences, respectively.
After modification with templates DV26P and DV27P of the present disclosure, the inhibition rates of C1585s-DV26P and C1585s-DV27P increased by 0.8% and 2.8%, respectively, compared to C1585s-DV32P, and increased by 8.5% and 10.5%, respectively, compared to C1585s-DV34P.

**III. Advanced ESC template DV22 disclosed in the prior art (fluorine-substituted sites: positions 2, 6, 14, and 16 on the antisense strand; positions 7, 9, 10, and 11 on the sense strand)**

When unmodified sequence 1585s was modified with template DV22, the resulting sequence C1585s-DV22 exhibited an inhibition rate of 55.2% on AGT gene expression, which was 4.1% lower than that of the alternately modified sequence.
After modification with templates DV26P and DV27P of the present disclosure, the inhibition rates of C1585s-DV26P and C1585s-DV27P were 7.4% and 9.4% higher, respectively, than that of C1585s-DV22, indicating a significant improvement.
Thus, it can be concluded that:

1) When modified with templates DV26P-29P of the present disclosure, unmodified sequence 1585s exhibited significantly enhanced inhibitory effect on AGT gene expression compared to the alternately modified sequence. For example, when unmodified sequence 1585s was modified with DV27P, the inhibition rate increased by 5.3% compared to the alternately modified sequence.
2) The same siRNA sequence exhibited significant differences in activity when modified with different modification templates. For example, when unmodified sequence 51 was modified with template DV27P of the present disclosure, the inhibition rate increased by 15.1% compared to modification with template DV28P of the present disclosure,

indicating a significant improvement; the inhibition rate increased by up to 9.4% compared to modification with the previously published Advanced ESC template DV22, indicating a significant improvement.
3) The activity of siRNA sequences modified with different templates varied greatly, with differences of up to 15.1%. Thus, it is uncertain which modification template would confer high activity to an siRNA sequence.

**(6) Unmodified sequence 1789**

Table 18: Inhibition rates of unmodified sequence 1789 on AGT gene expression after template modification

| Unmodified sequence ID | Template-modified sequence ID | Inhibition rate (%) of template-modified sequence | Inhibition rate (%) of corresponding alternately modified sequence | Increase (%) |
|---|---|---|---|---|
| 1789 | C1789-DV25P | 69.0 | 63.5 | 5.5 |
| | C1789-DV26P | 73.6 | | 10.1 |
| | C1789-DV27P | 66.3 | | 2.8 |
| | C1789-DV28P | 63.1 | | -0.4 |
| | C1789-DV29P | 64.5 | | 1 |
| | C1789-DV32P | 61.8 | | -1.7 |
| | C1789-DV33P | 57.9 | | -5.6 |
| | C1789-DV34P | 56.9 | | -6.6 |
| | C1789-DV22 | 60.3 | | -3.2 |

**I. Modification with templates DV25P-29P of the present disclosure**

When modified with templates DV25P-29P designed in the present disclosure, unmodified sequence 1789 exhibited inhibition rates exceeding 60% on AGT gene expression, indicating an improvement as compared to 2'-O-methyl and 2'-fluoro alternating modifications. For example, after modification with templates DV25P and DV26P, the inhibition rates of C1789-DV25P and C1789-DV26P increased by 5.5% and 10.1%, respectively.

**II. Other modification templates of the present disclosure**

When unmodified sequence 1789 was modified with templates DV32P-34P, the resulting sequences C1789-DV32P, C1789-DV33P, and C1789-DV34P exhibited inhibition rates of 61.8%, 57.9%, and 56.9% on AGT gene expression, respectively, which were 1.7%, 5.6%, and 6.6% lower than those of the corresponding alternately modified sequences, respectively.
After modification with templates DV25P and DV26P of the present disclosure, the inhibition rates of C1789-DV25P and C1789-DV26P increased by 7.2% and 11.8%, respectively, compared to C1789-DV32P, increased by 11.1% and 15.7%, respectively, compared to C1789-DV33P, and increased by 12.1% and 16.7%, respectively, compared to C1789-DV34P.

**III. Advanced ESC template DV22 disclosed in the prior art (fluorine-substituted sites: positions 2, 6, 14, and 16 on the antisense strand; positions 7, 9, 10, and 11 on the sense strand)**

When unmodified sequence 1789 was modified with template DV22, the resulting sequence C1789-DV22 exhibited an inhibition rate of 60.3% on AGT gene expression, which was 3.2% lower than that of the alternately modified sequence.
After modification with templates DV25P and DV26P of the present disclosure, the inhibition rates of C1789-DV25P and C1789-DV26P were 8.7% and 13.3% higher, respectively, than that of C1789-DV22, indicating a significant improvement.
Thus, it can be concluded that:

1) When modified with templates DV25P-29P of the present disclosure, unmodified sequence 1789 exhibited significantly enhanced inhibitory effect on AGT gene expression compared to the alternately modified sequence. For example, when unmodified sequence 81 was modified with DV26P, the inhibition rate increased by 10.1% compared to the alternately modified sequence.
2) The same siRNA sequence exhibited significant differences in activity when modified with different modification templates. For example, when unmodified sequence 1789 was modified with templates DV25P and DV26P of the present disclosure, the inhibition rate increased by up to 16.7% compared to modification with templates DV32P-34P of the present disclosure, indicating a significant improvement; the inhibition rate increased by up to 13.3% compared to modification with the previously published Advanced ESC template DV22, indicating a significant improvement.
3) The activity of siRNA sequences modified with different templates varied greatly, with differences of up to 16.7%. Thus, it is uncertain which modification template would confer high activity to an siRNA sequence.

**(7) Unmodified sequence 1791**

Table 19: Inhibition rates of unmodified sequence 1791 on AGT gene expression after template modification

| Unmodified sequence ID | Template-modified sequence ID | Inhibition rate (%) of template-modified sequence | Inhibition rate (%) of corresponding alternately modified sequence | Increase (%) |
|---|---|---|---|---|
| 1791 | C1791-DV26P | 70.3 | 61.8 | 8.5 |
| | C1791-DV27P | 63.6 | | 1.8 |
| | C1791-DV28P | 64.6 | | 2.8 |
| | C1791-DV29P | 59.4 | | -2.4 |
| | C1791-DV32P | 65.9 | | 4.1 |
| | C1791-DV34P | 66.7 | | 4.9 |

**I. Templates DV26P-29P of the present disclosure**

When modified with templates DV26-29P designed in the present disclosure, unmodified sequence 1791 exhibited inhibition rates exceeding 55% on AGT gene expression, indicating a significant improvement as compared to 2'-O-methyl and 2'-fluoro alternating modifications. For example, after modification with templates DV26P and DV28P, the inhibition rates of C1791-DV26P and C1791-DV28P increased by 8.5% and 2.8%, respectively.

**II. Other modification templates of the present disclosure**

When unmodified sequence 1791 was modified with templates DV32P and DV34P, the resulting sequences C1791-DV32P and C1791-DV34P exhibited inhibition rates of 65.9% and 66.7% on AGT gene expression, respectively, which were 4.1% and 4.9% higher than those of 2'-O-methyl and 2'-fluoro alternating modifications, respectively.
After modification with template DV26P of the present disclosure, the inhibition rate of C1791-DV26P increased by 4.4% and 3.6% compared to C1791-DV32P and C1791-DV34P, respectively.
Thus, it can be concluded that:

1) When modified with templates DV26-29P of the present disclosure, unmodified sequence 1791 exhibited significantly enhanced inhibitory effect on AGT gene expression compared to the alternately modified sequence. For example, when unmodified sequence 1791 was modified with DV26P, the inhibition rate increased by 8.5% compared to the alternately modified sequence.
2) The same siRNA sequence exhibited significant differences in activity when modified with different modification templates. For example, unmodified sequence 1791 modified with template DV26P of the present disclosure exhibited a 10.9% increase in inhibition rate compared to the sequence modified with template DV29P of the present disclosure, indicating a significant improvement.
3) The activity of siRNA sequences modified with different templates varied greatly, with differences of up to 10.9%. Thus, it is uncertain which modification template would confer high activity to an siRNA sequence.

**(8) Unmodified sequence 1795**

Table 20: Inhibition rates of unmodified sequence 1795 on AGT gene expression after template modification

| Unmodified sequence ID | Template-modified sequence ID | Inhibition rate (%) of template-modified sequence | Inhibition rate (%) of corresponding alternately modified sequence Increase (%) | Increase (%) |
|---|---|---|---|---|
| 1795 | C1795-DV26P | 59.3 | 57.5 | 1.8 |
| | C1795-DV27P | 63.3 | | 5.8 |
| | C1795-DV28P | 51.9 | | -5.6 |
| | C1795-DV29P | 61.6 | | 4.1 |
| | C1795-DV32P | 55.8 | | -1.7 |
| | C1795-DV34P | 55.0 | | -2.5 |

**I. Templates DV25-29P of the present disclosure**

When modified with templates DV26-29P designed in the present disclosure, unmodified sequence 1795 exhibited inhibition rates exceeding 50% on AGT gene expression, indicating an improvement as compared to 2'-O-methyl and 2'-fluoro alternating modifications. For example, after modification with templates DV27P and DV29P, the inhibition rates of C1795-DV27P and C1795-DV29P increased by 5.8% and 4.1%, respectively.

**II. Other modification templates of the present disclosure**

When unmodified sequence 1795 was modified with templates DV32P and DV34P, the resulting sequences C1795-DV32P and C1795-DV34P exhibited inhibition rates of 55.8% and 55.0% on AGT gene expression, respectively, which were 1.7% and 2.5% lower than those of the corresponding alternately modified sequences, respectively.

After modification with templates DV27P and DV29P of the present disclosure, the inhibition rates of C1795-DV27P and C1795-DV29P increased by 7.5% and 5.8%, respectively, compared to C1795-DV32P, and increased by 8.3% and 6.6%, respectively, compared to C1795-DV34P.

Thus, it can be concluded that:

1) When modified with templates DV25-29P of the present disclosure, unmodified sequence 1795 exhibited significantly enhanced inhibitory effect on AGT gene expression compared to the alternately modified sequence. For example, when unmodified sequence 84 was modified with DV27P, the inhibition rate increased by 5.8% compared to the alternately modified sequence.
2) The same siRNA sequence exhibited significant differences in activity when modified with different modification templates. For example, unmodified sequence 1795 modified with template DV27P of the present disclosure exhibited a 11.4% increase in inhibition rate compared to the sequence modified with template DV28P of the present disclosure, indicating a significant improvement.
3) The activity of siRNA sequences modified with different templates varied greatly, with differences of up to 11.4%. Thus, it is uncertain which modification template would confer high activity to an siRNA sequence.

**(9) Unmodified sequence 1812**

Table 21: Inhibition rates of unmodified sequence 1812 on AGT gene expression after template modification

| Unmodified sequence ID | Template-modified sequence ID | Inhibition rate (%) of template-modified sequence | Inhibition rate (%) of corresponding alternately modified sequence | Increase (%) |
|---|---|---|---|---|
| 1812 | C1812-DV25P | 74.4 | 62.9 | 11.5 |
| | C1812-DV26P | 58.0 | | -4.9 |
| | C1812-DV27P | 63.9 | | 1.0 |
| | C1812-DV28P | 55.3 | | -7.6 |
| | C1812-DV29P | 66.9 | | 4.0 |
| | C1812-DV32P | 63.6 | | 0.7 |
| | C1812-DV33P | 54.2 | | -8.7 |
| | C1812-DV34P | 60.7 | | -2.2 |
| | C1812-DV22 | 63.6 | | 0.7 |

**I. Modification with templates DV25P-29P of the present disclosure**

When modified with templates DV25P-29P designed in the present disclosure, unmodified sequence 1812 exhibited inhibition rates exceeding 55% on AGT gene expression, indicating an improvement as compared to 2'-O-methyl and 2'-fluoro alternating modifications. For example, after modification with template DV25P, the inhibition rate of C1812-DV25P increased by 11.5%.

**II. Other modification templates of the present disclosure**

When unmodified sequence 1812 was modified with templates DV32P-34P, the resulting sequences C1812-DV32P, C1812-DV33P, and C1812-DV34P exhibited inhibition rates of 63.6%, 54.2%, and 60.7% on AGT gene expression, respectively, which were 0.7%, -8.7%, and -2.2% higher than those of the corresponding alternately modified sequences, respectively.

After modification with template DV25P of the present disclosure, the inhibition rate of C1812-DV25P increased by 10.8%, 20.2%, and 13.7% compared to C1812-DV32P, C1812-DV33P, and C1812-DV34P, respectively, indicating a significant improvement.

**III. Advanced ESC template DV22 disclosed in the prior art (fluorine-substituted sites: positions 2, 6, 14, and 16 on the antisense strand; positions 7, 9, 10, and 11 on the sense strand)**

When unmodified sequence 1812 was modified with template DV22, the resulting sequence C812-DV22 exhibited an inhibition rate of 63.6% on AGT gene expression, which was 0.7% higher than that of the alternately modified sequence.

After modification with template DV25P of the present disclosure, the inhibition rate of C1812-DV25P increased by 10.8% compared to C1812-DV22, indicating a significant improvement.

Thus, it can be concluded that:

1) When modified with templates DV25-29P of the present disclosure, unmodified sequence 1812 exhibited significantly enhanced inhibitory effect on AGT gene expression compared to the alternately modified sequence. For example, when unmodified sequence 1812 was modified with DV25P, the inhibition rate increased by 11.5% compared to the alternately modified sequence.
2) The same siRNA sequence exhibited significant differences in activity when modified with different modification templates. For example, when unmodified sequence 1812 was modified with template DV25P of the present disclosure, the inhibition rate increased by 20.2% compared to modification with template DV33P of the present disclosure, indicating a significant improvement; the inhibition rate increased by up to 10.8% compared to modification with the previously published Advanced ESC template DV22, indicating a significant improvement.
3) The activity of siRNA sequences modified with different templates varied greatly, with differences of up to 20.2%. Thus, it is uncertain which modification template would confer high activity to an siRNA sequence.

**(10) Unmodified sequence 1816**

Table 22: Inhibition rates of unmodified sequence 1816 on AGT gene expression after template modification

| Unmodified sequence ID | Template-modified sequence ID | Inhibition rate (%) of template-modified sequence | Inhibition rate (%) of corresponding alternately modified sequence | Increase (%) |
|---|---|---|---|---|
| 1816 | C1816-DV25P | 68.1 | 60.9 | 7.2 |
| | C1816-DV26P | 58.5 | | -2.4 |
| | C1816-DV27P | 64.5 | | 3.6 |
| | C1816-DV28P | 49.2 | | -11.7 |
| | C1816-DV29P | 68.5 | | 7.6 |
| | C1816-DV32P | 58.6 | | -2.3 |
| | C1816-DV33P | 48.4 | | -12.5 |
| | C1816-DV34P | 38.5 | | -22.4 |
| | C1816-DV22 | 66.7 | | 5.8 |

**I. Modification with templates DV25P-29P of the present disclosure**

When modified with templates DV25P, DV26P, DV27P, and DV29P designed in the present disclosure, unmodified sequence 1816 exhibited inhibition rates exceeding 55% on AGT gene expression, indicating an improvement as compared to 2'-O-methyl and 2'-fluoro alternating modifications. For example, after modification with template DV29P, the inhibition rate of C1812-DV25P increased by 7.6%.

**II. Other modification templates of the present disclosure**

When unmodified sequence 1816 was modified with templates DV32P-34P, the resulting sequences C1816-DV32P, C1816-DV33P, and C1816-DV34P exhibited inhibition rates of 58.6%, 48.4%, and 38.5% on AGT gene expression, respectively, which were 2.3%, 12.5%, and 22.4% lower than those of the corresponding alternately modified sequences, respectively.

After modification with template DV29P of the present disclosure, the inhibition rate of C1816-DV25P increased by 9.9%, 20.1%, and 30.0% compared to C1816-DV32P, C1816-DV33P, and C1816-DV34P, respectively, indicating a significant improvement.

**III. Advanced ESC template DV22 disclosed in the prior art (fluorine-substituted sites: positions 2, 6, 14, and 16 on the antisense strand; positions 7, 9, 10, and 11 on the sense strand)**

When unmodified sequence 1816 was modified with template DV22, the resulting sequence C1816-DV22 exhibited an inhibition rate of 66.7% on AGT gene expression, which was 5.8% higher than that of the alternately modified sequence.

After modification with template DV29P of the present disclosure, the inhibition rate of C1816-DV29P increased by 1.8% compared to C1816-DV22.

Thus, it can be concluded that:

1) When modified with templates DV25P-29P of the present disclosure, unmodified sequence 1816 exhibited significantly enhanced inhibitory effect on AGT gene expression compared to the alternately modified sequence. For example, when unmodified sequence 1816 was modified with DV29P, the inhibition rate increased by 7.6% compared to the alternately modified sequence.

2) The same siRNA sequence exhibited significant differences in activity when modified with different modification templates. For example, when unmodified sequence 1816 was modified with template DV29P of the present disclosure, the inhibition rate increased by 30.0% compared to modification with template DV34P of the present

disclosure, indicating a significant improvement; the inhibition rate increased by 1.8% compared to modification with the previously published Advanced ESC template DV22.

3) The activity of siRNA sequences modified with different templates varied greatly, with differences of up to 30.0%. Thus, it is uncertain which modification template would confer high activity to an siRNA sequence.

**(11) Unmodified sequence 1835**

Table 23: Inhibition rates of unmodified sequence 1835 on AGT gene expression after template modification

| Unmodified sequence ID | Template-modified sequence ID | Inhibition rate (%) of template-modified sequence | Inhibition rate (%) of corresponding alternately modified sequence | Increase (%) |
|---|---|---|---|---|
| 1835 | C1835-DV25P | 68.2 | 62.3 | 5.9 |
| | C1835-DV26P | 71.8 | | 9.5 |
| | C1835-DV27P | 51.2 | | -11.1 |
| | C1835-DV28P | 70.0 | | 7.7 |
| | C1835-DV29P | 64.9 | | 2.6 |
| | C1835-DV32P | 53.3 | | -9.0 |
| | C1835-DV33P | 64.9 | | 2.6 |
| | C1835-DV34P | 71.8 | | 9.5 |
| | C1835-DV22 | 58.2 | | -4.1 |

**I. Modification with templates DV25P-29P of the present disclosure**

When modified with templates DV25P-29P designed in the present disclosure, unmodified sequence 1835 exhibited inhibition rates exceeding 50% on AGT gene expression, except for DV27P, indicating an improvement as compared to 2'-O-methyl and 2'-fluoro alternating modifications. For example, after modification with template DV26P, the inhibition rate of C1835-DV26P increased by 9.5%.

**II. Other modification templates of the present disclosure**

When unmodified sequence 1835 was modified with templates DV32P-34P, the resulting sequences C1835-DV32P, C1835-DV33P, and C1835-DV34P exhibited inhibition rates of 53.3%, 64.9%, and 71.8% on AGT gene expression, respectively, which were -9.0%, 2.6%, and 9.5% higher than those of the corresponding alternately modified sequences, respectively.

After modification with template DV26P of the present disclosure, the inhibition rate of C1835-DV26P increased by 18.5%, 10.7%, and 0.0% compared to C1835-DV32P, C1835-DV33P, and C1835-DV34P, respectively.

**III. Advanced ESC template DV22 disclosed in the prior art (fluorine-substituted sites: positions 2, 6, 14, and 16 on the antisense strand; positions 7, 9, 10, and 11 on the sense strand)**

When unmodified sequence 1835 was modified with template DV22, the resulting sequence C1835-DV22 exhibited an inhibition rate of 58.2% on AGT gene expression, which was 4.1% lower than that of the alternately modified sequence.

After modification with template DV25P of the present disclosure, the inhibition rate of C1835-DV26P increased by 13.6% compared to C1835-DV22, indicating a significant improvement.

Thus, it can be concluded that:

1) When modified with templates DV25-29P of the present disclosure, unmodified sequence 1835 exhibited significantly enhanced inhibitory effect on AGT gene expression compared to the alternately modified sequence. For example, when unmodified sequence 1835 was modified with DV26P, the inhibition rate increased by 9.5%

compared to the alternately modified sequence.

2) The same siRNA sequence exhibited significant differences in activity when modified with different modification templates. For example, when unmodified sequence 1835 was modified with template DV26P of the present disclosure, the inhibition rate increased by 20.6% compared to modification with template DV27P of the present disclosure, indicating a significant improvement; the inhibition rate increased by up to 13.6% compared to modification with the previously published Advanced ESC template DV22, indicating a significant improvement.

3) The activity of siRNA sequences modified with different templates varied greatly, with differences of up to 20.6%. Thus, it is uncertain which modification template would confer high activity to an siRNA sequence.

**(12) Unmodified sequence 1836**

Table 24: Inhibition rates of unmodified sequence 1836 on AGT gene expression after template modification

| Unmodified sequence ID | Template-modified sequence ID | Inhibition rate (%) of template-modified sequence | Inhibition rate (%) of corresponding alternately modified sequence | Increase (%) |
|---|---|---|---|---|
| 1836 | C1836-DV26P | 63.8 | 59.8 | 4.0 |
| | C1836-DV27P | 58.9 | | -0.9 |
| | C1836-DV28P | 63.8 | | 4.0 |
| | C1836-DV29P | 56.9 | | -2.9 |
| | C1836-DV32P | 49.2 | | -10.6 |
| | C1836-DV34P | 63.0 | | 3.2 |

**I. Modification with templates DV26-29P of the present disclosure**

When modified with templates DV26P-29P designed in the present disclosure, unmodified sequence 1836 exhibited inhibition rates exceeding 55% on AGT gene expression, indicating an improvement as compared to 2'-O-methyl and 2'-fluoro alternating modifications. For example, after modification with templates DV26P and DV28P, the inhibition rates of C1836-DV26P and C1836-DV28P increased by 4.0%.

**II. Other modification templates of the present disclosure**

When unmodified sequence 1836 was modified with templates DV32P and DV34P, the resulting sequences C1836-DV32P and C1836-DV34P exhibited inhibition rates of 49.2% and 63.0% on AGT gene expression, respectively, which were 2.9% and 10.6% lower than those of the corresponding alternately modified sequences, respectively.

After modification with templates DV26P and DV28P of the present disclosure, the inhibition rates of C1836-DV26P and C1836-DV28P increased by 14.6% and 0.8% compared to C1836-DV32P and C1836-DV34P, respectively.

Thus, it can be concluded that:

1) When modified with templates DV26-29P of the present disclosure, unmodified sequence 1836 exhibited significantly enhanced inhibitory effect on AGT gene expression compared to the alternately modified sequence. For example, when unmodified sequence 1836 was modified with DV26P and DV28P, the inhibition rate increased by 4.0% compared to the alternately modified sequence.

2) The same siRNA sequence exhibited significant differences in activity when modified with different modification templates. For example, unmodified sequence 1836 modified with templates DV26P and DV28P of the present disclosure exhibited a 14.6% increase in inhibition rate compared to the sequence modified with template DV32P of the present disclosure, indicating a significant improvement.

3) The activity of siRNA sequences modified with different templates varied greatly, with differences of up to 14.6%. Thus, it is uncertain which modification template would confer high activity to an siRNA sequence.

**(13) Unmodified sequence 1839**

Table 25: Inhibition rates of unmodified sequence 1839 on AGT gene expression after template modification

| Unmodified sequence ID | Template-modified sequence ID | Inhibition rate (%) of template-modified sequence | Inhibition rate (%) of corresponding alternately modified sequence | Increase (%) |
|---|---|---|---|---|
| 1839 | C1839-DV26P | 48.6 | 59.1 | -10.5 |
| | C1839-DV27P | 63.9 | | 4.8 |
| | C1839-DV28P | 51.4 | | -7.7 |
| | C1839-DV29P | 64.2 | | 5.1 |
| | C1839-DV32P | 63.7 | | 4.6 |
| | C1839-DV34P | 55.9 | | -3.2 |

**I. Modification with templates DV26-29P of the present disclosure**

When modified with templates DV26-29P designed in the present disclosure, unmodified sequence 1839 exhibited inhibition rates exceeding 50% on AGT gene expression, indicating an improvement as compared to 2'-O-methyl and 2'-fluoro alternating modifications. For example, after modification with template DV29P, the inhibition rate of C1839-DV29P increased by 5.1%.

**II. Other modification templates of the present disclosure**

When unmodified sequence 1839 was modified with templates DV32P and DV34P, the resulting sequences C1839-DV32P and C1839-DV34P exhibited inhibition rates of 63.7% and 55.9% on AGT gene expression, respectively, which were 4.6% and -3.2% higher than those of the corresponding alternately modified sequences, respectively.

After modification with template DV29P of the present disclosure, the inhibition rate of C1839-DV29P increased by 0.5% and 8.3% compared to C1839-DV32P and C1839-DV34P, respectively.

Thus, it can be concluded that:

1) When modified with templates DV26-29P of the present disclosure, unmodified sequence 1839 exhibited significantly enhanced inhibitory effect on AGT gene expression compared to the alternately modified sequence. For example, when unmodified sequence 1839 was modified with DV29P, the inhibition rate increased by 5.1% compared to the alternately modified sequence.
2) The same siRNA sequence exhibited significant differences in activity when modified with different modification templates. For example, unmodified sequence 1839 modified with template DV29P of the present disclosure exhibited a 15.6% increase in inhibition rate compared to the sequence modified with template DV26P of the present disclosure, indicating a significant improvement.
3) The activity of siRNA sequences modified with different templates varied greatly, with differences of up to 15.6%. Thus, it is uncertain which modification template would confer high activity to an siRNA sequence.

**Summary:**

(1) The screened unmodified sequences 1576s, 1578s, 1838, 1835, 1816, 1812, 1579, 1836, 1789, 1839, 1791, 1795, and 1585s, when modified with templates DV25P-29P designed in the present disclosure, exhibited significant inhibitory effects on AGT gene expression, with inhibition rates of 40% or more. Specifically, C1812-DV25P, C1579-DV33P, and C1789-DV26P achieved inhibition rates of 74.4%, 74.0%, and 73.6%, respectively.
(2) The 12 sequences modified with templates DV25P-29P of the present disclosure showed a significant improvement in inhibition rate on AGT gene expression as compared to the alternately modified sequences. For example, the inhibition rate of unmodified sequence 1812 modified with template DV25P (C1579-DV33P) was 11.5% higher than

that of the alternately modified sequence, while the inhibition rate of unmodified sequence 1791 modified with template DV26P (C1791-DV26P) was 8.5% higher than that of the alternately modified sequence.

(3) The same sequences modified with templates DV25-29P of the present disclosure exhibited significantly higher inhibition rates on AGT gene expression compared to modification with the templates disclosed in the prior art. For example, unmodified sequence 1579 modified with template DV25P of the present disclosure exhibited a 16.6% increase in inhibition rate compared to the sequence modified with the previously published Advanced ESC template DV22.

(4) The same sequences modified with templates DV25-29P of the present disclosure exhibited significantly higher inhibition rates on AGT gene expression compared to modification with templates DV32-34P of the present disclosure. For example, unmodified sequence 1812 modified with template DV25P of the present disclosure exhibited a 20.2% increase in inhibition rate compared to the sequence modified with template DV33P of the present disclosure.

(5) The activity of sequences modified with different templates varied greatly. For example, when unmodified sequence 1816 was modified with DV29P, the inhibition rate increased by 30.0% compared to modification with DV34; when unmodified sequence 1579 was modified with DV33P, the inhibition rate increased by 29.8% compared to modification with DV28P. Thus, it is uncertain which modification template would confer high activity to an siRNA sequence.

**(ii) $IC_{50}$ assay**

A total of 24 template-modified sequences, including C1816-DV29P, C1816-DV25P, C1812-DV25P, C1579-DV32P, C1789-DV26P, C1789-DV25P, C1839-DV29P, C1795-DV27P, C1585s-DV27P, C1835-DV26P, C1835-DV25P, C1579-DV25P, C1836-DV26P, C1791-DV26P, C1585s-DV29P, C1604-DV29P, C1606-DV29P, C1607-DV29P, C1835-DV29P, C1838-DV29P, C1579-DV29P, C1576s-DV29P, C1578s-DV29P, and C1579s-DV29P, were selected for $IC_{50}$ assays, and the $IC_{50}$ values of these sequences were determined. The experimental results are shown in Table 26.

Table 26: $IC_{50}$ values of modified sequences

| Sequence ID | Sequence $IC_{50}$ (pM) |
|---|---|
| C1816-DV29P | 27.925 |
| C1816-DV25P | 3.530 |
| C1812-DV25P | 2.160 |
| C1579-DV32P | 4.712 |
| C1789-DV26P | 2.643 |
| C1789-DV25P | 1.884 |
| C1839-DV29P | 3.301 |
| C1795-DV27P | 3.570 |
| C1585s-DV27P | 2.863 |
| C1835-DV26P | 14.565 |
| C1835-DV25P | 14.683 |
| C1579-DV25P | 10.532 |
| C1836-DV26P | 12.838 |
| C1791-DV26P | 7.812 |
| C1585s-DV29P | 13.333 |
| C1604-DV29P | 23.160 |
| C1606-DV29P | 46.237 |
| C1607-DV29P | 61.498 |
| C1835-DV29P | 53.143 |
| C1838-DV29P | 15.393 |
| C1579-DV29P | 15.837 |

(continued)

| Sequence ID | Sequence $IC_{50}$ (pM) |
|---|---|
| C1576s-DV29P | 11.214 |
| C1578s-DV29P | 8.568 |
| C1579s-DV29P | 30.601 |

As shown in Table 26, the $IC_{50}$ values of these 24 sequences ranged from 1.884 pM to 61.498 pM. Specifically, the $IC_{50}$ values of C1789-DV25P, C1812-DV25P, and C1789-DV26P were 1.884 pM, 2.160 pM, and 2.643 pM, respectively. The results demonstrated that these sequences effectively inhibited AGT gene expression at low concentrations.

**Example 5: Comparison of inhibitory effect of sequences disclosed in the prior art on AGT gene expression**

In this example, unmodified sequences 1579, 1789, 1812, 1576s, 1578s, 1585s, 1816, 1835, 1836, 1838, 1839, and 1791 of the present disclosure were compared with structurally similar unmodified sequences disclosed in the prior art in terms of their inhibition efficiency on AGT gene expression, when modified using alternating modifications of the present disclosure and modified with templates DV25P-29P.

**1. Experimental materials**

Test samples:

(1) The sequences disclosed in the prior art, as shown in Table 27.

Table 27: Sequences disclosed in the prior art

| Sequence ID | Sense strand | **SEQ ID NO.** | Antisense strand | **SEQ ID NO.** | Source |
|---|---|---|---|---|---|
| 579P | ACCUUUUCUUCUAAUGAGUCU | 500 | AGACUCAUUAGAAGAAAAGGUGG | 513 | CN117751189A |
| 789P | UUUGUGAAACAAAAAAGUGA | 501 | UCACUUUUUUGUUUCACAAACA | 514 | WO2024031101A1 |
| 812P | UUGUUCUCAACUUGAAAAGGGA | 502 | CCUUUUCAAGUUGAGAACAA | 515 | WO2024031101A1 |
| 576P | CCACCUUUUCUUCUAAUGAGU | 503 | ACUCAUUAGAAGAAAAGGUGGGA | 516 | CN112313335A |
| 578P | CACCUUUUCUUCUAAUGAGUA | 504 | UACUCAUUAGAAGAAAAGGUGGG | 517 | CN112313335A |
| 585P | UUCUAAUGAGUCGACUUUA | 505 | UAAAGUCGACUCAUUAGAA | 518 | CN112852809A |
| AD-60771 | AGAACAAAAAUUGGGUUUUAA | 506 | UUAAAACCCAAUUUUUGUUCUCA | 519 | CN112852809A |
| AD-85481 | GUCAUCCACAAUGAGAGUACA | 507 | UGUACUCUCAUUGUGGAUGACGA | 520 | CN112852809A |
| 816P | UCAAGUUGAGAACAAAAAUA | 508 | UAUUUUUGUUCUCAACUUGAAA | 521 | WO2024031101A1 |
| 835P | GGGUUUUAAAAUUAAAGUAUA | 509 | UACUUUAAUUUUAAAACCCAA | 522 | CN113862268A |
| 836P | GGUUUUAAAAUUAAAGUAUAC | 510 | AUACUUUAAUUUUAAAACCCA | 523 | CN113862268A |
| 839P | GUUUUAAAAUUAAAGUAUACA | 511 | UAUACUUUAAUUUUAAAACCC | 524 | CN113862268A |

(continued)

| Sequence ID | Sense strand | **SEQ ID NO.** | Antisense strand | **SEQ ID NO.** | Source |
|---|---|---|---|---|---|
| 791P | UUGUGAAACAAAAAAGU GUUU | 512 | ACACUUUUUUGUUUCAC AAUU | 525 | CN106574268B |

(2) The siRNA sequences alternately modified with 2'-OMe and 2'-F along with terminal phosphorothioate modification in Example 2.

(3) The siRNA sequences modified with modification templates DV25-29P, with specific sequences listed in Table 28.

Table 28: Sequences modified with templates DV25P-29P

| Unmodified sequence ID | Template-modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| 1579 | C1579-DV25P | Cms-Cms-Um-Um-Um-Um-Cf-Um-Uf-Cf-Uf-Am-Am-Um-Gm-Am-Gf-Um-Cm-Gm-Am | 407/4 | UmsEVP-Cfs-Gm-Am-Cm-Uf-Cm-Am-Um-Um-Am-Gm-Am-Af-Gm-Af-Am-Am-Am-Gm-Gms-Ums-Gm | 457/17 |
| 1579 | C1579-DV26P | Cms-Cms-Um-Um-Um-Um-Cf-Um-Uf-Cf-Uf-Am-Am-Um-Gm-Am-Gf-Um-Cm-Gm-Am | 407/4 | UmsEVP-Cfs-Gf-Af-Cm-Uf-Cm-Am-Um-Um-Am-Gm-Am-Af-Gm-Af-Am-Am-Am-Gm-Gms-Ums-Gm | 454/17 |
| 1579 | C1579-DV27P | Cms-Cms-Um-Um-Um-Um-Cf-Um-Uf-Cf-Uf-Am-Am-Um-Gm-Am-Gf-Um-Cm-Gm-Am | 407/4 | UmsEVP-Cfs-Gm-Af-Cf-Uf-Cm-Am-Um-Um-Am-Gm-Am-Af-Gm-Af-Am-Am-Am-Gm-Gms-Ums-Gm | 453/17 |
| 1579 | C1579-DV28P | Cms-Cms-Um-Um-Um-Um-Cf-Um-Uf-Cm-Uf-Am-Am-Um-Gm-Am-Gf-Um-Cm-Gm-Am | 406/4 | UmsEVP-Cfs-Gf-Af-Cm-Uf-Cm-Am-Um-Um-Am-Gm-Am-Af-Gm-Af-Am-Am-Am-Gm-Gms-Ums-Gm | 454/17 |

(continued)

| Unmodified sequence ID | Template-modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| 1579 | C1579-DV29P | Cms-Cms-Um-Um-Um-Um-Cf-Um-Uf-Cm-Uf-Am-Am-Um-Gm-Am-Gf-Um-Cm-Gm-Am | 406/4 | UmsEVP-Cfs-Gm-Af-Cf-Uf-Cm-Am-Um-Um-Am-Gm-Am-Af-Gm-Af-Am-Am-Am-Gm-Gms-Ums-Gm | 453/17 |
| 1789 | C1789-DV25P | Gms-Ums-Um-Um-Gm-Um-Gf-Am-Af-Af-Cf-Am-Am-Am-Am-Am-Af-Gm-Um-Gm-Um | 412/6 | AmsEVP-Cfs-Am-Cm-Um-Uf-Um-Um-Um-Um-Gm-Um-Um-Uf-Cm-Af-Cm-Am-Am-Am-Cms-Ams-Am | 468/19 |
| 1789 | C1789-DV26P | Gms-Ums-Um-Um-Gm-Um-Gf-Am-Af-Af-Cf-Am-Am-Am-Am-Am-Af-Gm-Um-Gm-Um | 412/5 | AmsEVP-Cfs-Af-Cf-Um-Uf-Um-Um-Um-Um-Gm-Um-Um-Uf-Cm-Af-Cm-Am-Am-Am-Cms-Ams-Am | 465/18 |
| 1789 | C1789-DV27P | Gms-Ums-Um-Um-Gm-Um-Gf-Am-Af-Af-Cf-Am-Am-Am-Am-Am-Af-Gm-Um-Gm-Um | 412/5 | AmsEVP-Cfs-Am-Cf-Uf-Uf-Um-Um-Um-Um-Gm-Um-Um-Uf-Cm-Af-Cm-Am-Am-Am-Cms-Ams-Am | 464/18 |

(continued)

| Unmodified sequence ID | Template-modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| 1789 | C1789-DV28P | Gms-Ums-Um-Um-Gm-Um-Gf-Am-Af-Am-Cf-Am-Am-Am-Am-Am-Af-Gm-Um-Gm-Um | 411/6 | AmsEVP-Cfs-Af-Cf-Um-Uf-Um-Um-Um-Um-Gm-Um-Um-Uf-Cm-Af-Cm-Am-Am-Am-Cms-Ams-Am | 465/19 |
| 1789 | C1789-DV29P | Gms-Ums-Um-Um-Gm-Um-Gf-Am-Af-Am-Cf-Am-Am-Am-Am-Am-Af-Gm-Um-Gm-Um | 411/5 | AmsEVP-Cfs-Am-Cf-Uf-Uf-Um-Um-Um-Um-Gm-Um-Um-Uf-Cm-Af-Cm-Am-Am-Am-Cms-Ams-Am | 464/18 |
| 1812 | C1812-DV25P | Cms-Cms-Um-Um-Um-Um-Cf-Am-Af-Gf-Uf-Um-Gm-Am-Gm-Am-Af-Cm-Am-Am-Am | 404/3 | UmsEVP-Ufs-Um-Gm-Um-Uf-Cm-Um-Cm-Am-Am-Cm-Um-Uf-Gm-Af-Am-Am-Am-Gm-Gms-Gms-Am | 450/16 |
| 1812 | C1812-DV26P | Cms-Cms-Um-Um-Um-Um-Cf-Am-Af-Gf-Uf-Um-Gm-Am-Gm-Am-Af-Cm-Am-Am-Am | 404/3 | UmsEVP-Ufs-Uf-Gf-Um-Uf-Cm-Um-Cm-Am-Am-Cm-Um-Uf-Gm-Af-Am-Am-Am-Gm-Gms-Gms-Am | 447/16 |

(continued)

| Unmodified sequence ID | Template-modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| 1812 | C1812-DV27P | Cms-Cms-Um-Um-Um-Um-Cf-Am-Af-Gf-Uf-Um-Gm-Am-Gm-Am-Af-Cm-Am-Am-Am | 404/3 | UmsEVP-Ufs-Um-Gf-Uf-Uf-Cm-Um-Cm-Am-Am-Cm-Um-Uf-Gm-Af-Am-Am-Am-Gm-Gms-Gms-Am | 446/16 |
| 1812 | C1812-DV28P | Cms-Cms-Um-Um-Um-Um-Cf-Am-Af-Gm-Uf-Um-Gm-Am-Gm-Am-Af-Cm-Am-Am-Am | 403/3 | UmsEVP-Ufs-Uf-Gf-Um-Uf-Cm-Um-Cm-Am-Am-Cm-Um-Uf-Gm-Af-Am-Am-Am-Gm-Gms-Gms-Am | 447/16 |
| 1812 | C1812-DV29P | Cms-Cms-Um-Um-Um-Um-Cf-Am-Af-Gm-Uf-Um-Gm-Am-Gm-Am-Af-Cm-Am-Am-Am | 403/3 | UmsEVP-Ufs-Um-Gf-Uf-Uf-Cm-Um-Cm-Am-Am-Cm-Um-Uf-Gm-Af-Am-Am-Am-Gm-Gms-Gms-Am | 446/16 |
| 1576s | C1576s-DV29P | Cms-Cms-Am-Cm-Cm-Uf-Um-Uf-Um-Cf-Um-Um-Cm-Um-Am-Af-Um-Gm-Am-Am | 423/11 | UmsEVP-Ufs-Cm-Af-Uf-Uf-Am-Gm-Am-Am-Gm-Am-Am-Af-Am-Gf-Gm-Um-Gm-Gms-Gms-Am | 488/24 |

(continued)

| Unmodified sequence ID | Template-modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| 1576s | C1576s-DV28P | Cms-Cms-Am-Cm-Cm-Uf-Um-Uf-Um-Cf-Um-Um-Cm-Um-Am-Af-Um-Gm-Am-Am | 423/11 | UmsEVP-Ufs-Cf-Af-Um-Uf-Am-Gm-Am-Am-Gm-Am-Am-Af-Am-Gf-Gm-Um-Gm-Gms-Gms-Am | 489/24 |
| 1576s | C1576s-DV26P | Cms-Cms-Am-Cm-Cm-Uf-Um-Uf-Uf-Cf-Um-Um-Cm-Um-Am-Af-Um-Gm-Am-Am | 424/11 | UmsEVP-Ufs-Cf-Af-Um-Uf-Am-Gm-Am-Am-Gm-Am-Am-Af-Am-Gf-Gm-Um-Gm-Gms-Gms-Am | 489/24 |
| 1578s | C1578s-DV29P | Ams-Cms-Cm-Um-Um-Uf-Um-Cf-Um-Uf-Cm-Um-Am-Am-Um-Gf-Am-Gm-Um-Am | 426/12 | UmsEVP-Afs-Cm-Uf-Cf-Af-Um-Um-Am-Gm-Am-Am-Gm-Af-Am-Af-Am-Gm-Gm-Ums-Gms-Gm | 492/25 |
| 1578s | C1578s-DV28P | Ams-Cms-Cm-Um-Um-Uf-Um-Cf-Um-Uf-Cm-Um-Am-Am-Um-Gf-Am-Gm-Um-Am | 426/12 | UmsEVP-Afs-Cf-Uf-Cm-Af-Um-Um-Am-Gm-Am-Am-Gm-Af-Am-Af-Am-Gm-Gm-Ums-Gms-Gm | 493/25 |

(continued)

| Unmodified sequence ID | Template-modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| 1578s | C1578s-DV26P | Ams-Cms-Cm-Um-Um-Uf-Um-Cf-Uf-Uf-Cm-Um-Am-Am-Um-Gf-Am-Gm-Um-Am | 427/12 | UmsEVP-Afs-Cf-Uf-Cm-Af-Um-Um-Am-Gm-Am-Am-Gm-Af-Am-Af-Am-Gm-Gm-Ums-Gms-Gm | 493/25 |
| 1585s | C1585s-DV26P | Cms-Ums-Um-Cm-Um-Af-Am-Uf-Gf-Af-Gm-Um-Cm-Gm-Am-Cf-Um-Um-Um-Am | 421/10 | UmsEVP-Afs-Af-Af-Gm-Uf-Cm-Gm-Am-Cm-Um-Cm-Am-Uf-Um-Af-Gm-Am-Am-Gms-Ams-Am | 484/23 |
| 1585s | C1585s-DV27P | Cms-Ums-Um-Cm-Um-Af-Am-Uf-Gf-Af-Gm-Um-Cm-Gm-Am-Cf-Um-Um-Um-Am | 421/10 | UmsEVP-Afs-Am-Af-Gf-Uf-Cm-Gm-Am-Cm-Um-Cm-Am-Uf-Um-Af-Gm-Am-Am-Gms-Ams-Am | 483/23 |
| 1585s | C1585s-DV28P | Cms-Ums-Um-Cm-Um-Af-Am-Uf-Gm-Af-Gm-Um-Cm-Gm-Am-Cf-Um-Um-Um-Am | 420/10 | UmsEVP-Afs-Af-Af-Gm-Uf-Cm-Gm-Am-Cm-Um-Cm-Am-Uf-Um-Af-Gm-Am-Am-Gms-Ams-Am | 484/23 |

(continued)

| Unmodified sequence ID | Template-modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| 1585s | C1585s-DV29P | Cms-Ums-Um-Cm-Um-Af-Am-Uf-Gm-Af-Gm-Um-Cm-Gm-Am-Cf-Um-Um-Um-Am | 420/10 | UmsEVP-Afs-Am-Af-Gf-Uf-Cm-Gm-Am-Cm-Um-Cm-Am-Uf-Um-Af-Gm-Am-Am-Gms-Ams-Am | 483/23 |
| 1789 | C1789-DV26P | Gms-Ums-Um-Um-Gm-Um-Gf-Am-Af-Af-Cf-Am-Am-Am-Am-Am-Af-Gm-Um-Gm-Um | 412/5 | AmsEVP-Cfs-Af-Cf-Um-Uf-Um-Um-Um-Um-Gm-Um-Um-Uf-Cm-Af-Cm-Am-Am-Am-Cms-Ams-Am | 465/18 |
| 1789 | C1789-DV27P | Gms-Ums-Um-Um-Gm-Um-Gf-Am-Af-Af-Cf-Am-Am-Am-Am-Am-Af-Gm-Um-Gm-Um | 412/5 | AmsEVP-Cfs-Am-Cf-Uf-Uf-Um-Um-Um-Um-Gm-Um-Um-Uf-Cm-Af-Cm-Am-Am-Am-Cms-Ams-Am | 464/18 |
| 1816 | C 1816-DV25P | Ums-Ums-Cm-Am-Am-Gm-Uf-Um-Gf-Af-Gf-Am-Am-Cm-Am-Am-Af-Am-Am-Um-Um | 401/2 | AmsEVP-Afs-Um-Um-Um-Uf-Um-Gm-Um-Um-Cm-Um-Cm-Af-Am-Cf-Um-Um-Gm-Am-Ams-Ams-Am | 443/15 |

(continued)

| Unmodified sequence ID | Template-modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| 1816 | C 1816-DV26P | Ums-Ums-Cm-Am-Am-Gm-Uf-Um-Gf-Af-Gf-Am-Am-Cm-Am-Am-Af-Am-Am-Um-Um | 401/2 | AmsEVP-Afs-Uf-Uf-Um-Uf-Um-Gm-Um-Um-Cm-Um-Cm-Af-Am-Cf-Um-Um-Gm-Am-Ams-Ams-Am | 440/15 |
| 1816 | C 1816-DV27P | Ums-Ums-Cm-Am-Am-Gm-Uf-Um-Gf-Af-Gf-Am-Am-Cm-Am-Am-Af-Am-Am-Um-Um | 401/2 | AmsEVP-Afs-Um-Uf-Uf-Uf-Um-Gm-Um-Um-Cm-Um-Cm-Af-Am-Cf-Um-Um-Gm-Am-Ams-Ams-Am | 439/15 |
| 1816 | C 1816-DV28P | Ums-Ums-Cm-Am-Am-Gm-Uf-Um-Gf-Am-Gf-Am-Am-Cm-Am-Am-Af-Am-Am-Um-Um | 400/2 | AmsEVP-Afs-Uf-Uf-Um-Uf-Um-Gm-Um-Um-Cm-Um-Cm-Af-Am-Cf-Um-Um-Gm-Am-Ams-Ams-Am | 440/15 |
| 1816 | C 1816-DV29P | Ums-Ums-Cm-Am-Am-Gm-Uf-Um-Gf-Am-Gf-Am-Am-Cm-Am-Am-Af-Am-Am-Um-Um | 400/2 | AmsEVP-Afs-Um-Uf-Uf-Uf-Um-Gm-Um-Um-Cm-Um-Cm-Af-Am-Cf-Um-Um-Gm-Am-Ams-Ams-Am | 439/15 |

(continued)

| Unmodified sequence ID | Template-modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| 1835 | C1835-DV25P | Ums-Ums-Gm-Gm-Gm-Um-Uf-Um-Uf-Af-Af-Am-Am-Um-Um-Am-Af-Am-Gm-Um-Am | 398/1 | UmsEVP-Afs-Cm-Um-Um-Uf-Am-Am-Um-Um-Um-Um-Am-Af-Am-Af-Cm-Cm-Cm-Am-Ams-Ums-Um | 436/14 |
| 1835 | C1835-DV26P | Ums-Ums-Gm-Gm-Gm-Um-Uf-Um-Uf-Af-Af-Am-Am-Um-Um-Am-Af-Am-Gm-Um-Am | 398/1 | UmsEVP-Afs-Cf-Uf-Um-Uf-Am-Am-Um-Um-Um-Um-Am-Af-Am-Af-Cm-Cm-Cm-Am-Ams-Ums-Um | 433/14 |
| 1835 | C1835-DV27P | Ums-Ums-Gm-Gm-Gm-Um-Uf-Um-Uf-Af-Af-Am-Am-Um-Um-Am-Af-Am-Gm-Um-Am | 398/1 | UmsEVP-Afs-Cm-Uf-Uf-Uf-Am-Am-Um-Um-Um-Um-Am-Af-Am-Af-Cm-Cm-Cm-Am-Ams-Ums-Um | 432/14 |
| 1835 | C1835-DV28P | Ums-Ums-Gm-Gm-Gm-Um-Uf-Um-Uf-Am-Af-Am-Am-Um-Um-Am-Af-Am-Gm-Um-Am | 397/1 | UmsEVP-Afs-Cf-Uf-Um-Uf-Am-Am-Um-Um-Um-Um-Am-Af-Am-Af-Cm-Cm-Cm-Am-Ams-Ums-Um | 433/14 |

(continued)

| Unmodified sequence ID | Template-modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| 1835 | C1835-DV29P | Ums-Ums-Gm-Gm-Gm-Um-Uf-Um-Uf-Am-Af-Am-Am-Um-Um-Am-Af-Am-Gm-Um-Am | 397/1 | UmsEVP-Afs-Cm-Uf-Uf-Uf-Am-Am-Um-Um-Um-Um-Am-Af-Am-Af-Cm-Cm-Cm-Am-Ams-Ums-Um | 432/14 |
| 1838 | C1838-DV29P | Gms-Gms-Um-Um-Um-Um-Af-Am-Af-Am-Uf-Um-Am-Am-Am-Gm-Uf-Am-Um-Am-Am | 429/13 | UmsEVP-Ufs-Am-Uf-Af-Cf-Um-Um-Um-Am-Am-Um-Um-Uf-Um-Af-Am-Am-Am-Cm-Cms-Cms-Am | 496/26 |
| 1838 | C1838-DV28P | Gms-Gms-Um-Um-Um-Um-Af-Am-Af-Am-Uf-Um-Am-Am-Am-Gm-Uf-Am-Um-Am-Am | 429/13 | UmsEVP-Ufs-Af-Uf-Am-Cf-Um-Um-Um-Am-Am-Um-Um-Uf-Um-Af-Am-Am-Am-Cm-Cms-Cms-Am | 497/26 |
| 1838 | C1838-DV26P | Gms-Gms-Um-Um-Um-Um-Af-Am-Af-Af-Uf-Um-Am-Am-Am-Gm-Uf-Am-Um-Am-Am | 430/13 | UmsEVP-Ufs-Af-Uf-Am-Cf-Um-Um-Um-Am-Am-Um-Um-Uf-Um-Af-Am-Am-Am-Cm-Cms-Cms-Am | 497/26 |

(continued)

| Unmodified sequence ID | Template-modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| 1836 | C1836-DV26P | Ums-Gms-Gm-Gm-Um-Um-Uf-Um-Af-Af-Af-Am-Um-Um-Am-Am-Af-Gm-Um-Am-Um | 410/5 | AmsEVP-Ufs-Af-Cf-Um-Uf-Um-Am-Am-Um-Um-Um-Um-Af-Am-Af-Am-Cm-Cm-Cm-Ams-Ams-Um | 461/18 |
| 1836 | C1836-DV27P | Ums-Gms-Gm-Gm-Um-Um-Uf-Um-Af-Af-Af-Am-Um-Um-Am-Am-Af-Gm-Um-Am-Um | 410/5 | AmsEVP-Ufs-Am-Cf-Uf-Uf-Um-Am-Am-Um-Um-Um-Um-Af-Am-Af-Am-Cm-Cm-Cm-Ams-Ams-Um | 460/18 |
| 1836 | C1836-DV28P | Ums-Gms-Gm-Gm-Um-Um-Uf-Um-Af-Am-Af-Am-Um-Um-Am-Am-Af-Gm-Um-Am-Um | 409/5 | AmsEVP-Ufs-Af-Cf-Um-Uf-Um-Am-Am-Um-Um-Um-Um-Af-Am-Af-Am-Cm-Cm-Cm-Ams-Ams-Um | 461/18 |
| 1836 | C1836-DV29P | Ums-Gms-Gm-Gm-Um-Um-Uf-Um-Af-Am-Af-Am-Um-Um-Am-Am-Af-Gm-Um-Am-Um | 409/5 | AmsEVP-Ufs-Am-Cf-Uf-Uf-Um-Am-Am-Um-Um-Um-Um-Af-Am-Af-Am-Cm-Cm-Cm-Ams-Ams-Um | 460/18 |

(continued)

| Unmodified sequence ID | Template-modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| 1839 | C1839-DV26P | Gms-Ums-Um-Um-Um-Am-Af-Am-Af-Uf-Uf-Am-Am-Am-Gm-Um-Af-Um-Am-Cm-Am | 415/7 | UmsEVP-Gfs-Uf-Af-Um-Af-Cm-Um-Um-Um-Am-Am-Um-Uf-Um-Uf-Am-Am-Am-Am-Cms-Cms-Cm | 472/20 |
| 1839 | C1839-DV27P | Gms-Ums-Um-Um-Um-Am-Af-Am-Af-Uf-Uf-Am-Am-Am-Gm-Um-Af-Um-Am-Cm-Am | 415/7 | UmsEVP-Gfs-Um-Af-Uf-Af-Cm-Um-Um-Um-Am-Am-Um-Uf-Um-Uf-Am-Am-Am-Am-Cms-Cms-Cm | 471/20 |
| 1839 | C1839-DV28P | Gms-Ums-Um-Um-Um-Am-Af-Am-Af-Um-Uf-Am-Am-Am-Gm-Um-Af-Um-Am-Cm-Am | 414/7 | UmsEVP-Gfs-Uf-Af-Um-Af-Cm-Um-Um-Um-Am-Am-Um-Uf-Um-Uf-Am-Am-Am-Am-Cms-Cms-Cm | 472/20 |
| 1839 | C1839-DV29P | Gms-Ums-Um-Um-Um-Am-Af-Am-Af-Um-Uf-Am-Am-Am-Gm-Um-Af-Um-Am-Cm-Am | 414/7 | UmsEVP-Gfs-Um-Af-Uf-Af-Cm-Um-Um-Um-Am-Am-Um-Uf-Um-Uf-Am-Am-Am-Am-Cms-Cms-Cm | 471/20 |

(continued)

| Unmodified sequence ID | Template-modified sequence ID | Sense strand sequence 5'-3' | Modified sequence SEQ ID NO./ corresponding unmodified sequence SEQ ID NO. | Antisense strand sequence 5'-3' | Modified sequence SEQ ID NO./ corresponding unmodified sequence SEQ ID NO. |
|---|---|---|---|---|---|
| 1791 | C1791-DV26P | Ums-Ums-Gm-Um-Gm-Am-Af-Am-Cf-Af-Af-Am-Am-Am-Am-Gm-Uf-Gm-Um-Um-Am | 417/8 | UmsEVP-Afs-Af-Cf-Am-Cf-Um-Um-Um-Um-Um-Um-Gm-Uf-Um-Uf-Cm-Am-Cm-Am-Ams-Ams-Cm | 476/21 |
| 1791 | C1791-DV27P | Ums-Ums-Gm-Um-Gm-Am-Af-Am-Cf-Af-Af-Am-Am-Am-Am-Gm-Uf-Gm-Um-Um-Am | 417/8 | UmsEVP-Afs-Am-Cf-Af-Cf-Um-Um-Um-Um-Um-Um-Gm-Uf-Um-Uf-Cm-Am-Cm-Am-Ams-Ams-Cm | 475/21 |
| 1791 | C1791-DV28P | Ums-Ums-Gm-Um-Gm-Am-Af-Am-Cf-Am-Af-Am-Am-Am-Am-Gm-Uf-Gm-Um-Um-Am | 416/8 | UmsEVP-Afs-Af-Cf-Am-Cf-Um-Um-Um-Um-Um-Um-Gm-Uf-Um-Uf-Cm-Am-Cm-Am-Ams-Ams-Cm | 476/21 |
| 1791 | C1791-DV29P | Ums-Ums-Gm-Um-Gm-Am-Af-Am-Cf-Am-Af-Am-Am-Am-Am-Gm-Uf-Gm-Um-Um-Am | 416/8 | UmsEVP-Afs-Am-Cf-Af-Cf-Um-Um-Um-Um-Um-Um-Gm-Uf-Um-Uf-Cm-Am-Cm-Am-Ams-Ams-Cm | 475/21 |

Cell type: HepG2 cells, provided by Cyagen (H1-1701).
HepG2 cells were cultured in DMEM medium (ATCC-30-2003) containing 10% fetal bovine serum (FBS, ExCell Bio-FSP500) and 1% penicillin-streptomycin (HyClone-SV30010).
Drug vehicle: sterile nuclease-free water, Gibco Opti-MEM.

**2. Experimental methods**

The inhibitory effects of the test samples on the mRNA expression of AGT genes in HepG2 cell lines were tested by qRT-PCR.

**2.1 Cell culture**

HepG2 cells in the logarithmic growth phase were harvested from subcultured stocks and cultured in DMEM medium containing 10% fetal bovine serum (supplemented with 100 μL/mL of penicillin and streptomycin). The cells were incubated in a cell culture incubator at 37°C with 5% $CO_2$, with medium replaced daily. For subculture, the cells were digested with 0.25% trypsin, centrifuged at 1000 rpm for 5 minutes, and the supernatant was discarded. Fresh medium was added for subculture.

**2.2 Cell transfection**

Preparation of transfection mixture: Lipofectamine RNAiMAX and Opti-MEM were mixed at a ratio of 2:98 and vortexed thoroughly.
Preparation of transfection reagent: 60 μL of siRNA solution diluted in Opti-MEM was mixed with 60 μL of the prepared transfection mixture at a ratio of 1:1 (v/v), vortexed thoroughly, and the mixture was incubated at room temperature for 15 minutes to obtain lipid nanoparticles (LNPs). A 12.5 μL aliquot was taken for encapsulation efficiency analysis.
Blank control transfection reagent: 60 μL of the prepared transfection mixture was added to 60 μL of Opti-MEM, vortexed thoroughly, and the mixture was incubated at room temperature for 15 minutes.
The prepared transfection reagent was added to a 24-well cell culture plate (100 μL per well), resulting in a final siRNA concentration of 0.02 nM per well. 500 μL of cell suspension ($1.5 \times 10^5$ cells per mL) was added per well. After mixing by crosswise method, the plate was incubated in a cell culture incubator at 37°C with 5% $CO_2$ for 40 hours.

**2.3 AGT mRNA assay**

1) RNA extraction

The procedure was the same as that described in "1) RNA extraction" under "2.3 AGT mRNA assay" in Example 2.

2) RNA concentration measurement

The procedure was the same as that described in "2) RNA concentration measurement" under "2.3 AGT mRNA assay" in Example 2.

3) Quantitative analysis of AGT mRNA

In a 15 mL centrifuge tube, all components except primers and templates were added in $7.5 \times 48 = 360$ aliquots, labeled as A.
In a pre-labeled 1.5 mL EP tube, 77 μL of A and 420 ng of total RNA were added in 7 aliquots per tube, followed by mixing for later use.
The forward and reverse primers for both the internal reference gene GAPDH and the target gene AGT were mixed for later use.
In a PCR plate, 11 μL of B and 1 μL of C were added per well. The plate was sealed with a sealing film, centrifuged at 3000 rpm for 1 minute, and loaded for qPCR analysis.
*All operations in this section were performed on ice to maintain low-temperature conditions.
The plate was placed in a qPCR instrument and run according to the following program:

Reverse transcription: 55°C for 15 minutes;

Pre-denaturation: 95°C for 30 seconds;
Cycling reaction: 95°C for 10 seconds; 60°C for 35 seconds; 40 cycles;
Melting curve: 95°C for 15 seconds; 60°C for 60 seconds; 95°C for 15 seconds.

The total run time was approximately 2 hours. Experimental results were analyzed, and $2^{-\Delta\Delta Ct}$ was calculated.

**2.4 Data processing**

AGT mRNA expression rate (%) was calculated using the following formula:

Expression rate = (AGT mRNA expression level in experimental group / AGT mRNA expression level in blank control group) × 100%;

$$\text{Inhibition rate of AGT gene expression} = 1 - \text{expression rate (\%)}.$$

**3. Experimental results**

The specific experimental results are shown in Table 29.
The experimental results demonstrated that both the alternately modified sequences and the sequences modified with specific modification templates in the present disclosure exhibited significantly enhanced AGT inhibitory activity compared to the structurally similar unmodified sequences disclosed in the prior art. For example, compared to sequence 1812 of the present disclosure, sequence 812P disclosed in the prior art lacks one base A at the 3' end of the sense strand and one base U at the 5' end of the antisense strand, with all other bases being identical. However, unmodified sequence 1812 of the present disclosure exhibited a 14.3% increase in inhibition rate compared to 812P. The alternately modified sequence exhibited an inhibition rate of 62.9%, which was 20.4% higher than that of 812P. After modification with template DV25 of the present disclosure, the inhibition rate reached 74.4%, which was 31.9% higher than that of 31P.

**(1) Compared to the structurally similar unmodified sequences disclosed in the prior art, the unmodified sequences, alternately modified sequences, and sequences modified with specific modification templates of the present disclosure exhibited significantly higher inhibition rates on AGT gene expression, with an increase of up to 91.0%.**

Compared to the structurally similar sequences disclosed in the prior art, the unmodified sequences, alternately modified sequences, and sequences modified with specific modification templates in the present disclosure exhibited significantly higher inhibition rates on AGT gene expression. For example, unmodified sequence 812P exhibited an inhibition rate of 42.5%. The structurally similar unmodified sequence 1812 of the present disclosure exhibited an inhibition rate of 56.8%, which was 14.3% higher than that of 812P. The alternately modified sequence exhibited an inhibition rate of 62.9%, which was 20.4% higher than that of 812P. After modification with template DV25 of the present disclosure, the inhibition rate reached 74.4%, which was 31.9% higher than that of 812P.

Table 29: Inhibition rates of similar sequences in the prior art and the present disclosure on AGT gene expression

| Sequences disclosed in the prior art | | Unmodified sequences of the present disclosure | | | Alternately modified sequences of the present disclosure | | | Template-modified sequences of the present disclosure | | |
|---|---|---|---|---|---|---|---|---|---|---|
| Name | Inhibition rate (%) | Seq ID | Inhibition rate (%) | Increase compared to prior art (%) | Seq ID | Inhibition rate (%) | Increase compared to prior art (%) | Seq ID | Inhibition rate (%) | Increase compared to prior art (%) |
| 579P | 47 | 1579 | 58.2 | 11.2 | B1579-AL | 61.1 | 14.1 | C1579-DV25P | 55.6 | 8.6 |
| | | | | | | | | C1579-DV26P | 72.2 | 25.2 |
| | | | | | | | | C1579-DV27P | 70.8 | 23.8 |
| | | | | | | | | C1579-DV28P | 63.3 | 16.3 |
| | | | | | | | | C1579-DV29P | 44.2 | -2.8 |
| 789P | 51.9 | 1789 | 54.5 | 2.6 | B1789-AL | 63.5 | 11.6 | C1789-DV25P | 69 | 17.1 |
| | | | | | | | | C1789-DV26P | 73.6 | 21.7 |
| | | | | | | | | C1789-DV27P | 66.3 | 14.4 |
| | | | | | | | | C1789-DV28P | 63.1 | 11.2 |
| | | | | | | | | C1789-DV29P | 64.5 | 12.6 |
| 812P | 42.5 | 1812 | 56.8 | 14.3 | B1812-AL | 62.9 | 20.4 | C1812-DV25P | 74.4 | 31.9 |
| | | | | | | | | C1812-DV26P | 58 | 15.5 |
| | | | | | | | | C1812-DV27P | 63.9 | 21.4 |
| | | | | | | | | C1812-DV28P | 55.3 | 12.8 |
| | | | | | | | | C1812-DV29P | 66.9 | 24.4 |
| 576P | 38.2 | 1576s | 59.1 | 20.9 | B1576sAL | 64.6 | 26.4 | C1576s-DV26P | 73.4 | 35.2 |
| | | | | | | | | C1576s-DV28P | 58.8 | 20.6 |
| | | | | | | | | C1576s-DV29P | 79.3 | 41.1 |
| | | | | | | | | C1576s-DV25P | 73.3 | 35.1 |
| | | | | | | | | C1576s-DV27P | 69.7 | 31.5 |

(continued)

| Sequences disclosed in the prior art | | Unmodified sequences of the present disclosure | | | Alternately modified sequences of the present disclosure | | | Template-modified sequences of the present disclosure | | |
|---|---|---|---|---|---|---|---|---|---|---|
| Name | Inhibition rate (%) | Seq ID | Inhibition rate (%) | Increase compared to prior art (%) | Seq ID | Inhibition rate (%) | Increase compared to prior art (%) | Seq ID | Inhibition rate (%) | Increase compared to prior art (%) |
| 578P | 33.6 | 1578s | 55.2 | 21.6 | B 1578sAL | 63.9 | 30.3 | C1578s-DV26P | 69.7 | 36.1 |
| | | | | | | | | C1578s-DV28P | 56.9 | 23.3 |
| | | | | | | | | C1578s-DV29P | 77.1 | 43.5 |
| | | | | | | | | C1578s-DV25P | 75.8 | 42.2 |
| | | | | | | | | C1578s-DV27P | 73.1 | 39.5 |
| 585P | 47.33 | 1585s | 48.9 | 1.57 | B1585sAL | 59.3 | 11.97 | C1585s-DV26P | 62.6 | 15.27 |
| | | | | | | | | C1585s-DV27P | 64.6 | 17.27 |
| | | | | | | | | C1585s-DV28P | 49.5 | 2.17 |
| | | | | | | | | C1585s-DV29P | 59.2 | 11.87 |
| 816P | 64.5 | 1816 | 46.8 | -17.7 | B1816-AL | 60.9 | -3.6 | C1816-DV25P | 68.1 | 3.6 |
| | | | | | | | | C1816-DV26P | 58.5 | -6 |
| | | | | | | | | C1816-DV27P | 64.5 | 0 |
| | | | | | | | | C1816-DV28P | 49.2 | -15.3 |
| | | | | | | | | C1816-DV29P | 68.5 | 4 |
| 835P | 13.9 | 1835 | 51.6 | 37.7 | B1835-AL | 62.3 | 48.4 | C1835-DV25P | 68.2 | 54.3 |
| | | | | | | | | C1835-DV26P | 71.8 | 57.9 |
| | | | | | | | | C1835-DV27P | 51.2 | 37.3 |
| | | | | | | | | C1835-DV28P | 70 | 56.1 |
| | | | | | | | | C1835-DV29P | 64.9 | 51 |

(continued)

| Sequences disclosed in the prior art | | Unmodified sequences of the present disclosure | | | Alternately modified sequences of the present disclosure | | | Template-modified sequences of the present disclosure | | |
|---|---|---|---|---|---|---|---|---|---|---|
| Name | Inhibition rate (%) | Seq ID | Inhibition rate (%) | Increase compared to prior art (%) | Seq ID | Inhibition rate (%) | Increase compared to prior art (%) | Seq ID | Inhibition rate (%) | Increase compared to prior art (%) |
| 836P | 41.9 | 1836 | 46.5 | 4.6 | B1836-AL | 59.8 | 17.9 | C1836-DV26P | 63.8 | 21.9 |
| | | | | | | | | C1836-DV27P | 58.9 | 17 |
| | | | | | | | | C1836-DV28P | 63.8 | 21.9 |
| | | | | | | | | C1836-DV29P | 56.9 | 15 |
| 839P | -15.4 | 1839 | 45.7 | 61.1 | B1839-AL | 59.1 | 74.5 | C1839-DV26P | 48.6 | 64 |
| | | | | | | | | C1839-DV27P | 63.9 | 79.3 |
| | | | | | | | | C1839-DV28P | 51.4 | 66.8 |
| | | | | | | | | C1839-DV29P | 64.2 | 79.6 |
| | | 1838 | 50.6 | 66.0 | B1838-AL | 63.6 | 79.0 | C1838-DV26P | 67.2 | 82.6 |
| | | | | | | | | C1838-DV28P | 60.3 | 75.7 |
| | | | | | | | | C1838-DV29P | 75.6 | 91 |
| | | | | | | | | C1838-DV25P | 69.3 | 84.7 |
| | | | | | | | | C1838-DV27P | 72.3 | 87.7 |
| 791P | 50.8 | 1791 | 46.5 | -4.3 | B1791-AL | 61.8 | 11 | C1791-DV26P | 70.3 | 19.5 |
| | | | | | | | | C1791-DV27P | 63.6 | 12.8 |
| | | | | | | | | C1791-DV28P | 64.6 | 13.8 |
| | | | | | | | | C1791-DV29P | 59.4 | 8.6 |

**I. Sequence structure comparison**

As shown in Table 30, the sequences disclosed in the prior art are highly similar to the sequences of the present disclosure, with only minor differences. Detailed comparisons are provided in the table below:

Table 30: Comparison between sequences disclosed in the prior art and sequences of the present disclosure

| Sequence ID | Sense strand 5'-3' | **SEQ ID NO.** | Antisense strand 5'-3' | **SEQ ID NO.** | Source |
|---|---|---|---|---|---|
| 579P | ACCUUUUCUUCUAAUGAGUCU | 500 | AGACUCAUUAGAAGAAAAGGUGG | 513 | CN117751189A |
| 1579 | CCUUUUCUUCUAAUGAGUCGA | 4 | UCGACUCAUUAGAAGAAAAGGUG | 17 | Sequence of the present disclosure |
| 789P | UUUGUGAAACAAAAAAGUGA | 501 | UCACUUUUUUGUUUCACAAACA | 514 | WO2024031101A1 |
| 1789 | GUUUGUGAAACAAAAAAGUGU | 6 | ACACUUUUUUGUUUCACAAACAA | 19 | Sequence of the present disclosure |
| 812P | CCUUUUCAAGUUGAGAACAA | 502 | UUGUUCUCAACUUGAAAAGGGA | 515 | WO2024031101A1 |
| 1812 | CCUUUUCAAGUUGAGAACAAA | 3 | UUUGUUCUCAACUUGAAAAGGGA | 16 | Sequence of the present disclosure |
| 576P | CCACCUUUUCUUCUAAUGAGU | 503 | ACUCAUUAGAAGAAAAGGUGGGA | 516 | CN112313335A |
| 1576s | CCACCUUUUCUUCUAAUGAA | 11 | UUCAUUAGAAGAAAAGGUGGGA | 24 | Sequence of the present disclosure |
| 578P | CACCUUUUCUUCUAAUGAGUA | 504 | UACUCAUUAGAAGAAAAGGUGGG | 517 | CN112313335A |
| 1578s | ACCUUUUCUUCUAAUGAGUA | 12 | UACUCAUUAGAAGAAAAGGUGG | 25 | Sequence of the present disclosure |
| 585P | UUCUAAUGAGUCGACUUUA | 505 | UAAAGUCGACUCAUUAGAA | 518 | CN112852809A |
| 1585s | CUUCUAAUGAGUCGACUUUA | 10 | UAAAGUCGACUCAUUAGAAGAA | 23 | Sequence of the present disclosure |

(continued)

| Sequence ID | Sense strand 5'-3' | **SEQ ID NO.** | Antisense strand 5'-3' | **SEQ ID NO.** | Source |
|---|---|---|---|---|---|
| 816P | UCAAGUUGAGAACAAAAAUA | 508 | UAUUUUUGUUCUCAACUUGAAA | 521 | WO2024031101A1 |
| 1816 | UUCAAGUUGAGAACAAAAAUU | 2 | AAUUUUUGUUCUCAACUUGAAAA | 15 | Sequence of the present disclosure |
| 835P | GGGUUUUAAAAUUAAAGUAUA | 509 | UACUUUAAUUUUAAAACCCAA | 522 | CN113862268A |
| 1835 | UUGGGUUUUAAAAUUAAAGUA | 1 | UACUUUAAUUUUAAAACCCAAUU | 14 | Sequence of the present disclosure |
| 836P | GGUUUUAAAAUUAAAGUAUAC | 510 | AUACUUUAAUUUUAAAACCCA | 523 | CN113862268A |
| 1836 | UGGGUUUUAAAAUUAAAGUAU | 5 | AUACUUUAAUUUUAAAACCCAAU | 18 | Sequence of the present disclosure |
| 839P | GUUUUAAAAUUAAAGUAUACA | 511 | UAUACUUUAAUUUUAAAACCC | 524 | CN113862268A |
| 1839 | GUUUUAAAAUUAAAGUAUACA | 7 | UGUAUACUUUAAUUUUAAAACCC | 20 | Sequence of the present disclosure |
| 1838 | GGUUUUAAAAUUAAAGUAUAA | 13 | UUAUACUUUAAUUUUAAAACCCA | 26 | Sequence of the present disclosure |
| 791P | UUGUGAAACAAAAAAGUGUUU | 512 | ACACUUUUUUGUUUCACAAUU | 525 | CN106574268B |
| 1791 | UUGUGAAACAAAAAAGUGUUA | 8 | UAACACUUUUUUGUUUCACAAAC | 21 | Sequence of the present disclosure |

As shown in Table 30:
Compared to sequence 1579 of the present disclosure, sequence 579P disclosed in the prior art has one additional base A at the 5' end of the sense strand, differs in one base U at the 3' end of the sense strand, differs in one base A at the 5' end of the antisense strand, and has one additional base G at the 3' end of the antisense strand.

Compared to sequence 1789 of the present disclosure, sequence 789P disclosed in the prior art lacks one base G at the 5' end of the sense strand, differs in one base A at the 3' end of the sense strand, differs in one base U at the 5' end of the antisense strand, and lacks one base A at the 3' end of the antisense strand.

Compared to sequence 1812 of the present disclosure, sequence 812P disclosed in the prior art lacks one base A at the 3' end of the sense strand and lacks one base U at the 5' end of the antisense strand.

Compared to sequence 1576s of the present disclosure, sequence 576P disclosed in the prior art differs in two bases GU at the 3' end of the sense strand and differs in two bases AC at the 5' end of the antisense strand.

Compared to sequence 1578s of the present disclosure, sequence 578P disclosed in the prior art has one additional base C at the 5' end of the sense strand and has one additional base G at the 3' end of the antisense strand.

Compared to sequence 1585s of the present disclosure, sequence 585P disclosed in the prior art lacks one base C at the 5' end of the sense strand and lacks three bases GAA at the 3' end of the antisense strand.

Compared to sequence 1816 of the present disclosure, sequence 816P disclosed in the prior art lacks one base U at the 5' end of the sense strand, differs in one base U at the 5' end of the antisense strand, and lacks one base A at the 3' end of the antisense strand.

Compared to sequence 1835 of the present disclosure, sequence 835P disclosed in the prior art lacks two bases UU at the 5' end of the sense strand and lacks two bases UU at the 3' end of the antisense strand.

Compared to sequence 1836 of the present disclosure, sequence 836P disclosed in the prior art lacks two bases UG at the 5' end of the sense strand and lacks two bases AU at the 3' end of the antisense strand.

Compared to sequence 1839 of the present disclosure, sequence 839P disclosed in the prior art has an identical sense strand but lacks two bases UG at the 5' end of the antisense strand. Compared to sequence 1838 of the present disclosure, sequence 839P disclosed in the prior art lacks one base G at the 5' end of the sense strand, differs in two bases CA at the 3' end of the sense strand, lacks one base U at the 5' end of the antisense strand, and lacks one base A at the 3' end of the antisense strand.

Compared to sequence 1791 of the present disclosure, sequence 791P disclosed in the prior art differs in one base U at the 3' end of the sense strand, lacks two bases UA at the 5' end of the antisense strand, and differs in two bases UU at the 3' end of the antisense strand.

**II. Activity comparison**

**1) The unmodified sequences of the present disclosure exhibited significantly enhanced activity compared to the structurally similar unmodified sequences disclosed in the prior art. For example, unmodified sequence 1576s of the present disclosure exhibited a 20.9% increase in inhibition rate compared to the structurally similar 576P (FIG. 6).**

Unmodified sequence 1579 of the present disclosure exhibited an inhibition rate of 58.2% on AGT gene expression, which was 11.2% higher than that of the structurally similar sequence 579P disclosed in the prior art (inhibition rate of 47%), indicating a significant improvement.

Unmodified sequence 1789 of the present disclosure exhibited an inhibition rate of 54.5% on AGT gene expression, which was 2.6% higher than that of the structurally similar sequence 789P disclosed in the prior art (inhibition rate of 51.9%).

Unmodified sequence 1812 of the present disclosure exhibited an inhibition rate of 56.8% on AGT gene expression, which was 14.3% higher than that of the structurally similar sequence 812P disclosed in the prior art (inhibition rate of 42.5%), indicating a significant improvement.

Unmodified sequence 1576s of the present disclosure exhibited an inhibition rate of 59.1% on AGT gene expression, which was 20.9% higher than that of the structurally similar sequence 576P disclosed in the prior art (inhibition rate of 38.2%), indicating a significant improvement.

Unmodified sequence 1578s of the present disclosure exhibited an inhibition rate of 55.2% on AGT gene expression, which was 21.6% higher than that of the structurally similar sequence 578P disclosed in the prior art (inhibition rate of 33.6%), indicating a significant improvement.

Unmodified sequence 1585s of the present disclosure exhibited an inhibition rate of 48.9% on AGT gene expression, which was 1.57% higher than that of the structurally similar sequence 585P disclosed in the prior art (inhibition rate of 47.33%).

Unmodified sequence 1816 of the present disclosure exhibited an inhibition rate of 46.8% on AGT gene expression, which was -17.7% higher than that of the structurally similar sequence 816P disclosed in the prior art

(inhibition rate of 64.5%), indicating a significant improvement.

Unmodified sequence 1835 of the present disclosure exhibited an inhibition rate of 51.6% on AGT gene expression, which was 37.7% higher than that of the structurally similar sequence 835P disclosed in the prior art (inhibition rate of 13.9%), indicating a significant improvement.

Unmodified sequence 1836 of the present disclosure exhibited an inhibition rate of 46.5% on AGT gene expression, which was 4.6% higher than that of the structurally similar sequence 836P disclosed in the prior art (inhibition rate of 41.9%).

Unmodified sequence 1839 of the present disclosure exhibited an inhibition rate of 45.7% on AGT gene expression, which was 61.1% higher than that of the structurally similar sequence 839P disclosed in the prior art (inhibition rate of -15.4%), indicating a significant improvement.

Unmodified sequence 1838 of the present disclosure exhibited an inhibition rate of 50.6% on AGT gene expression, which was 66.0% higher than that of the structurally similar sequence 839P disclosed in the prior art (inhibition rate of -15.4%), indicating a significant improvement.

Unmodified sequence 1791 of the present disclosure exhibited an inhibition rate of 46.5% on AGT gene expression, which was 4.3% lower than that of the structurally similar sequence 791P disclosed in the prior art (inhibition rate of 50.8%).

**2) The alternately modified sequences of the present disclosure exhibited significantly enhanced activity compared to the structurally similar unmodified sequences disclosed in the prior art. For example, the alternately modified sequence B1579-AL of the present disclosure exhibited a 14.1% increase in inhibition rate compared to sequence 579P.**

After unmodified sequence 1579 of the present disclosure was alternately modified, the resulting sequence B1579-AL exhibited an inhibition rate of 61.1% on AGT gene expression, which was 14.1% higher than that of unmodified sequence 579P disclosed in the prior art, indicating a significant improvement.

After unmodified sequence 1789 of the present disclosure was alternately modified, the resulting sequence B1789-AL exhibited an inhibition rate of 63.5% on AGT gene expression, which was 11.6% higher than that of unmodified sequence 789P disclosed in the prior art, indicating a significant improvement.

After unmodified sequence 1812 of the present disclosure was alternately modified, the resulting sequence B1812-AL exhibited an inhibition rate of 62.9% on AGT gene expression, which was 20.4% higher than that of unmodified sequence 812P disclosed in the prior art, indicating a significant improvement.

After unmodified sequence 1576s of the present disclosure was alternately modified, the resulting sequence B1576s-AL exhibited an inhibition rate of 64.6% on AGT gene expression, which was 26.4% higher than that of unmodified sequence 576P disclosed in the prior art, indicating a significant improvement.

After unmodified sequence 1578s of the present disclosure was alternately modified, the resulting sequence B1578s-AL exhibited an inhibition rate of 63.9% on AGT gene expression, which was 30.3% higher than that of unmodified sequence 578P disclosed in the prior art, indicating a significant improvement.

After unmodified sequence 1585s of the present disclosure was alternately modified, the resulting sequence B1585s-AL exhibited an inhibition rate of 59.3% on AGT gene expression, which was 11.97% higher than that of unmodified sequence 585P disclosed in the prior art, indicating a significant improvement.

After unmodified sequence 1816 of the present disclosure was alternately modified, the resulting sequence B1816-AL exhibited an inhibition rate of 60.9% on AGT gene expression, which was 3.6% lower than that of unmodified sequence 816P disclosed in the prior art.

After unmodified sequence 1835 of the present disclosure was alternately modified, the resulting sequence B1835-AL exhibited an inhibition rate of 62.3% on AGT gene expression, which was 48.4% higher than that of unmodified sequence 835P disclosed in the prior art, indicating a significant improvement.

After unmodified sequence 1836 of the present disclosure was alternately modified, the resulting sequence B1836-AL exhibited an inhibition rate of 59.8% on AGT gene expression, which was 17.9% higher than that of unmodified sequence 836P disclosed in the prior art, indicating a significant improvement.

After unmodified sequence 1839 of the present disclosure was alternately modified, the resulting sequence B1839-AL exhibited an inhibition rate of 59.1% on AGT gene expression, which was 74.5% higher than that of unmodified sequence 839P disclosed in the prior art, indicating a significant improvement.

After unmodified sequence 1838 of the present disclosure was alternately modified, the resulting sequence B1838-AL exhibited an inhibition rate of 63.6% on AGT gene expression, which was 79% higher than that of unmodified sequence 839P disclosed in the prior art, indicating a significant improvement.

After unmodified sequence 1791 of the present disclosure was alternately modified, the resulting sequence B1791-AL exhibited an inhibition rate of 61.8% on AGT gene expression, which was 11% higher than that of unmodified sequence 791P disclosed in the prior art, indicating a significant improvement.

**3) The sequences modified using the modification templates of the present disclosure exhibited significantly enhanced activity compared to the structurally similar unmodified sequences disclosed in the prior art. For example, the modified sequence C1579-DV26P of the present disclosure exhibited a 25.2% increase in inhibition rate compared to sequence 579P.**

After modification with templates DV25P-29P, unmodified sequence 1579 of the present disclosure exhibited a 20% or more increase in inhibition rate compared to unmodified sequence 579P disclosed in the prior art. For example, C1579-DV26P exhibited a 25.2% increase, indicating a significant improvement.

After modification with templates DV25P-29P, unmodified sequence 1789 of the present disclosure exhibited a 20% or more increase in inhibition rate compared to unmodified sequence 789P disclosed in the prior art. For example, C1789-DV26P exhibited a 21.7% increase, indicating a significant improvement.

After modification with templates DV25P-29P, unmodified sequence 1812 of the present disclosure exhibited a 30% or more increase in inhibition rate compared to unmodified sequence 812P disclosed in the prior art. For example, C1812-DV25P exhibited a 31.9% increase, indicating a significant improvement.

After modification with templates DV25P-29P, unmodified sequence 1576s of the present disclosure exhibited a 40% or more increase in inhibition rate compared to unmodified sequence 576P disclosed in the prior art. For example, C1576s-DV29P exhibited a 41.1% increase, indicating a significant improvement.

After modification with templates DV25P-29P, unmodified sequence 1578s of the present disclosure exhibited a 40% or more increase in inhibition rate compared to unmodified sequence 578P disclosed in the prior art. For example, C1578s-DV29P exhibited a 43.5% increase, indicating a significant improvement.

After modification with templates DV25P-29P, unmodified sequence 1585s of the present disclosure exhibited a 10% or more increase in inhibition rate compared to unmodified sequence 585P disclosed in the prior art. For example, C1585s-DV27P exhibited a 17.27% increase, indicating a significant improvement.

After modification with templates DV25P-29P, unmodified sequence 1816 of the present disclosure exhibited a non-significant increase in inhibition rate compared to unmodified sequence 816P disclosed in the prior art.

After modification with templates DV25P-29P, unmodified sequence 1835 of the present disclosure exhibited a 50% or more increase in inhibition rate compared to unmodified sequence 835P disclosed in the prior art. For example, C1835-DV26P exhibited a 57.9% increase, indicating a significant improvement.

After modification with templates DV25P-29P, unmodified sequence 1836 of the present disclosure exhibited a 20% or more increase in inhibition rate compared to unmodified sequence 836P disclosed in the prior art. For example, C1836-DV26P exhibited a 21.9% increase, indicating a significant improvement.

After modification with templates DV25P-29P, unmodified sequence 1839 of the present disclosure exhibited a 70% or more increase in inhibition rate compared to unmodified sequence 839P disclosed in the prior art. For example, C1839-DV29P exhibited a 79.6% increase, indicating a significant improvement.

After modification with templates DV25P-29P, unmodified sequence 1838 of the present disclosure exhibited a 90% or more increase in inhibition rate compared to unmodified sequence 839P disclosed in the prior art. For example, C1838-DV29P exhibited a 91% increase, indicating a significant improvement.

After modification with templates DV25P-29P, unmodified sequence 1791 of the present disclosure exhibited a 10% or more increase in inhibition rate compared to unmodified sequence 791P disclosed in the prior art. For example, C1791-DV26P exhibited a 19.5% increase, indicating a significant improvement.

Thus, compared to the structurally similar unmodified sequences in the prior art, the unmodified sequences of the present disclosure, as well as the alternately modified and template-modified sequences thereof, exhibited significantly enhanced AGT inhibitory activity, with an increase of up to 91% in inhibition rate.

**Summary:**

Compared to the structurally similar sequences disclosed in the prior art, the unmodified sequences, alternately modified sequences, and sequences modified with specific modification templates in the present disclosure exhibited significantly enhanced inhibitory effects on AGT gene expression. For example, unmodified sequence 1839 of the present disclosure exhibited a 61.1% increase in inhibition rate compared to the structurally similar sequence 839P disclosed in the prior art. The alternately modified sequence B1835-AL of the present disclosure, derived from sequence 1835, exhibited a 48.4% increase in inhibition rate compared to unmodified sequence 835P disclosed in the prior art, which was structurally similar to sequence 1835. After sequence 1812 was modified with template DV25P of the present disclosure, the resulting sequence C1812-DV25P exhibited a 31.9% increase in inhibition rate compared to unmodified sequence 812P disclosed in the prior art, which was structurally similar to sequence 1812.

**Example 6: Off-target effect assay of modified sequences**

In practical applications of siRNA, there are numerous instances where the expression of non-target mRNAs, which are only partially complementary to the guide strand (antisense strand), is inhibited. Research by Alnylam Pharmaceuticals has demonstrated that the hepatotoxicity of N-acetylgalactosamine (GalNAc)-conjugated siRNAs is primarily attributed to off-target effects resulting in gene suppression of incorrect targets through a microRNA (miRNA)-like recognition mechanism.

To investigate whether the sequences of the present disclosure would produce off-target effects, in this example, the inhibitory effects of the high-activity sequences from Example 4 on potential off-target genes in HepG2 cells were examined, and compared with Zilebesiran, a drug currently in Phase II clinical trials. For 13 sequences, including C1576s-DV29P, C1578s-DV29P, C1579-DV25P, C1789-DV25P, C1789-DV26P, C1791-DV26P, C1795-DV27P, C1812-DV25P, C1816-DV25P, C1835-DV26P, C1838-DV29P, and C1585s-DV27P, potential off-target genes with high sequence similarity were identified via BLAST analysis. C1585s-DV27P showed no significant sequence similarity to any known genes and was therefore excluded from off-target testing. The remaining 12 sequences exhibited significant inhibitory effects on AGT expression at concentrations ranging from 16 pM to 10 nM; all of which except C1578s-DV29P and C1835-DV26P showed no significant inhibitory effects on potential off-target genes ($IC_{50}$ values differed by 200-fold or more).

**1. Experimental materials**

1) Test samples:

The 12 high-activity sequences from Example 4, along with the positive control sequence APC-ZL (Table 31). APC-ZL is the Zilebesiran sequence without a GalNAc delivery vector.

Table 31: Template-modified sequences

| Sequence ID | Sense strand sequence 5'-3' | Seq ID | Antisense strand sequence 5'-3' | Seq ID |
|---|---|---|---|---|
| C1576s-DV29P | Cms-Cms-Am-Cm-Cm-Uf-Um-Uf-Um-Cf-Um-Um-Cm-Um-Am-Af-Um-Gm-Am-Am | 423 | UmsEVP-Ufs-Cm-Af-Uf-Uf-Am-Gm-Am-Am-Gm-Am-Am-Af-Am-Gf-Gm-Um-Gm-Gms-Gms-Am | 488 |
| C1578s-DV29P | Ams-Cms-Cm-Um-Um-Uf-Um-Cf-Um-Uf-Cm-Um-Am-Am-Um-Gf-Am-Gm-Um-Am | 426 | UmsEVP-Afs-Cm-Uf-Cf-Af-Um-Um-Am-Gm-Am-Am-Gm-Af-Am-Af-Am-Gm-Gm-Ums-Gms-Gm | 492 |
| C1579-DV25P | Cms-Cms-Um-Um-Um-Um-Cf-Um-Uf-Cf-Uf-Am-Am-Um-Gm-Am-Gf-Um-Cm-Gm-Am | 407 | UmsEVP-Cfs-Gm-Am-Cm-Uf-Cm-Am-Um-Um-Am-Gm-Am-Af-Gm-Af-Am-Am-Am-Gm-Gms-Ums-Gm | 457 |
| C1789-DV25P | Gms-Ums-Um-Um-Gm-Um-Gf-Am-Af-Af-Cf-Am-Am-Am-Am-Am-Af-Gm-Um-Gm-Um | 412 | AmsEVP-Cfs-Am-Cm-Um-Uf-Um-Um-Um-Um-Gm-Um-Um-Uf-Cm-Af-Cm-Am-Am-Am-Cms-Ams-Am | 468 |
| C1789-DV26P | Gms-Ums-Um-Um-Gm-Um-Gf-Am-Af-Af-Cf-Am-Am-Am-Am-Am-Af-Gm-Um-Gm-Um | 412 | AmsEVP-Cfs-Af-Cf-Um-Uf-Um-Um-Um-Um-Gm-Um-Um-Uf-Cm-Af-Cm-Am-Am-Am-Cms-Ams-Am | 465 |
| C1791-DV26P | Ums-Ums-Gm-Um-Gm-Am-Af-Am-Cf-Af-Af-Am-Am-Am-Am-Gm-Uf-Gm-Um-Um-Am | 417 | UmsEVP-Afs-Af-Cf-Am-Cf-Um-Um-Um-Um-Um-Um-Gm-Uf-Um-Uf-Cm-Am-Cm-Am-Ams-Ams-Cm | 476 |
| C1795-DV27P | Gms-Ams-Am-Am-Cm-Am-Af-Am-Af-Af-Af-Gm-Um-Gm-Um-Um-Cf-Cm-Cm-Um-Um | 419 | AmsEVP-Afs-Gm-Gf-Gf-Af-Am-Cm-Am-Cm-Um-Um-Um-Uf-Um-Uf-Gm-Um-Um-Um-Cms-Ams-Cm | 479 |

(continued)

| Sequence ID | Sense strand sequence 5'-3' | Seq ID | Antisense strand sequence 5'-3' | Seq ID |
|---|---|---|---|---|
| C1812-DV25P | Cms-Cms-Um-Um-Um-Um-Cf-Am-Af-Gf-Uf-Um-Gm-Am-Gm-Am-Af-Cm-Am-Am-Am | 404 | UmsEVP-Ufs-Um-Gm-Um-Uf-Cm-Um-Cm-Am-Am-Cm-Um-Uf-Gm-Af-Am-Am-Am-Gm-Gms-Gms-Am | 450 |
| C 1816-DV25P | Ums-Ums-Cm-Am-Am-Gm-Uf-Um-Gf-Af-Gf-Am-Am-Cm-Am-Am-Af-Am-Am-Um-Um | 401 | AmsEVP-Afs-Um-Um-Um-Uf-Um-Gm-Um-Um-Cm-Um-Cm-Af-Am-Cf-Um-Um-Gm-Am-Ams-Ams-Am | 443 |
| C1835-DV26P | Ums-Ums-Gm-Gm-Gm-Um-Uf-Um-Uf-Af-Af-Am-Am-Um-Um-Am-Af-Am-Gm-Um-Am | 398 | UmsEVP-Afs-Cf-Uf-Um-Uf-Am-Am-Um-Um-Um-Um-Am-Af-Am-Af-Cm-Cm-Cm-Am-Ams-Ums-Um | 433 |
| C1838-DV29P | Gms-Gms-Um-Um-Um-Um-Af-Am-Af-Am-Uf-Um-Am-Am-Am-Gm-Uf-Am-Um-Am-Am | 429 | UmsEVP-Ufs-Am-Uf-Af-Cf-Um-Um-Um-Am-Am-Um-Um-Uf-Um-Af-Am-Am-Am-Cm-Cms-Cms-Am | 496 |
| C1839-DV29P | Gms-Ums-Um-Um-Um-Am-Af-Am-Af-Um-Uf-Am-Am-Am-Gm-Um-Af-Um-Am-Cm-Am | 414 | UmsEVP-Gfs-Um-Af-Uf-Af-Cm-Um-Um-Um-Am-Am-Um-Uf-Um-Uf-Am-Am-Am-Am-Cms-Cms-Cm | 471 |
| APC-ZL | Gms-Ums-Cm-Am-Um-Cm-Cf-Am-Cf-Af-Af-Um-Gm-Am-Gm-Am-Gm-Um-Am-Cm-Am | 563 | Ums-Gfs-Um-Am-Cm-Tgna-Cm-Um-Cm-Am-Um-Um-Gm-Uf-Gm-Gf-Am-Um-Gm-Am-Cms-Gms-Am | 587 |

Cell type: HepG2 cells, provided by Cyagen (H1-1701).

HepG2 cells were cultured in DMEM medium (ATCC-30-2003) containing 10% fetal bovine serum (FBS, ExCell Bio-FSP500) and 1% penicillin-streptomycin (HyClone-SV30010).

Drug vehicle: sterile nuclease-free water, Gibco Opti-MEM.

### 2. Experimental methods

The inhibitory effects of the test samples on the mRNA expression of AGT genes in HepG2 cell lines were tested by qRT-PCR.

### 2.1 Cell culture

HepG2 cells in the logarithmic growth phase were harvested from subcultured stocks and cultured in DMEM medium containing 10% fetal bovine serum (supplemented with 100 μL/mL of penicillin and streptomycin). The cells were incubated in a cell culture incubator at 37°C with 5% $CO_2$, with medium replaced daily. For subculture, the cells were digested with 0.25% trypsin, centrifuged at 1000 rpm for 5 minutes, and the supernatant was discarded. Fresh medium was added for subculture.

### 2.2 Cell transfection

Preparation of transfection mixture: Lipofectamine RNAiMAX and Opti-MEM were mixed at a ratio of 2:98 and vortexed thoroughly.

Preparation of transfection reagent: 60 μL of siRNA solution diluted in Opti-MEM was mixed with 60 μL of the prepared transfection mixture at a ratio of 1:1 (v/v), vortexed thoroughly, and the mixture was incubated at room temperature for 15 minutes to obtain lipid nanoparticles (LNPs). A 12.5 μL aliquot was taken for encapsulation efficiency analysis.

Blank control transfection reagent: 60 μL of the prepared transfection mixture was added to 60 μL of Opti-MEM,

vortexed thoroughly, and the mixture was incubated at room temperature for 15 minutes.

The prepared transfection reagent was added to a 24-well cell culture plate (100 μL per well), resulting in a final siRNA concentration of 0.016 nM/0.08 nM/0.4 nM/2 nM/10 nM per well. 500 μL of cell suspension (1.5 × $10^5$ cells per mL) was added per well. After mixing by crosswise method, the plate was incubated in a cell culture incubator at 37°C with 5% $CO_2$ for 40 hours.

**2.3 RNA extraction and reverse transcription**

After 24 hours of transfection, the medium was removed and the cells were collected for RNA extraction. Total RNA was extracted using the RNeasy® 96 Kit (QIAGEN-74182) according to the instructions provided by the manufacturer. Subsequently, cDNA was synthesized using the FastKing RT Kit (With gDNase) (TIANGEN-KR116-02) according to the instructions provided by the manufacturer.

**2.4 RT-qPCR**

Refer to "Quantitative analysis of AGT mRNA" under Section 2.3 in Example 2.

**2.5 Data analysis**

The RNA expression levels of target genes in samples were calculated based on Ct values using the ΔΔCt relative quantification method. The relative expression levels of target genes were expressed as $2^{-\Delta\Delta Ct}$.

The calculation formula is as follows:

$$\Delta Ct = \text{Average Ct value of target gene - Average Ct value of reference gene;}$$

$$\Delta\Delta Ct = \Delta Ct\ (\text{treatment group}) - \Delta Ct\ (\text{RNAiMAX control group});$$

Relative mRNA expression level of target gene = $2^{-\Delta\Delta Ct}$;

Inhibition rate = (1 - relative expression level of sample / average expression level of RNAiMAX control) × 100%.

**3. Experimental results**

**3.1 All sequences exhibited significant inhibitory effects on AGT gene expression. For example, C1576s-DV29P exhibited an inhibition rate of 83.8% against AGT at 10 nM.**

The inhibition rates of these sequence against AGT at various concentrations are shown in Table 32 below. All sequences exhibited significant inhibitory effects against AGT. For example, C1576s-DV29P exhibited an inhibition rate of 83.8% against AGT at 10 nM.

Table 32: Inhibition rates of sequences against AGT at various concentrations

| Sequence name | AGT inhibition rate (%) | | | | |
|---|---|---|---|---|---|
| | 10.0 nM | 2.0 nM | 0.4 nM | 0.08 nM | 0.016 nM |
| C1576s-DV29P | 83.8 | 81.3 | 82.4 | 68.9 | 56.1 |
| C1578s-DV29P | 85.7 | 84.9 | 82.6 | 77.5 | 66.7 |
| C1579-DV25P | 85.0 | 84.9 | 82.7 | 77.9 | 58.3 |
| C1789-DV25P | 82.6 | 84.2 | 82.1 | 79.6 | 66.1 |
| C1789-DV26P | 79.3 | 82.8 | 81.3 | 78.8 | 64.5 |
| C1791-DV26P | 86.3 | 87.4 | 83.7 | 76.0 | 57.9 |
| C1795-DV27P | 87.4 | 84.1 | 83.1 | 74.2 | 57.8 |
| C1812-DV25P | 88.2 | 88.0 | 86.8 | 82.5 | 66.4 |
| C1816-DV25P | 84.5 | 83.8 | 80.2 | 73.5 | 56.3 |

(continued)

| Sequence name | AGT inhibition rate (%) | | | | |
|---|---|---|---|---|---|
| | 10.0 nM | 2.0 nM | 0.4 nM | 0.08 nM | 0.016 nM |
| C1835-DV26P | 89.2 | 88.3 | 88.4 | 82.4 | 72.2 |
| C1838-DV29P | 80.0 | 79.3 | 80.8 | 74.1 | 52.4 |
| C1839-DV29P | 82.2 | 83.2 | 81.8 | 73.8 | 64.9 |
| APC-ZL | 90.7 | 87.4 | 76.5 | 76.4 | 53.9 |

**3.2 C1578s-DV29P and C1835-DV26P exhibited certain off-target effects, while other sequences showed no significant off-target activity.**

The inhibition rates and $IC_{50}$ values of these sequences against AGT and potential off-target genes at various concentrations are shown in Table 33 below.

Table 33: Inhibition rates and $IC_{50}$ values of sequences against potential off-target genes at various concentrations

| Sequence name | Gene name | Inhibition rate (%) | | | | | | |
|---|---|---|---|---|---|---|---|---|
| | | 10.0 nM | 2.0 nM | 0.4 nM | 0.08 nM | 0.016 nM | $IC_{50}$/nM | Ratio |
| C1576s-DV29P | AGT | 83.8 | 81.3 | 82.4 | 68.9 | 56.1 | <0.016 | 1.0 |
| | DSE | -138.9 | -89.7 | -79.0 | -7.1 | -70.7 | >10.0 | >625.0 |
| C1578s-DV29P | AGT | 85.7 | 84.9 | 82.6 | 77.5 | 66.7 | 0.004 | 1.0 |
| | MIA2 | -12.9 | -4.3 | -13.5 | 4.0 | -3.8 | >10.0 | >2500.0 |
| | CHST15 | 57.9 | 63.1 | 64.0 | 63.6 | 5.0 | 0.019 | 4.8 |
| | ERRFI1 | 66.9 | 65.9 | 69.9 | 74.1 | 16.3 | 0.021 | 5.3 |
| C1579-DV25P | AGT | 85.0 | 84.9 | 82.7 | 77.9 | 58.3 | 0.011 | 1.0 |
| | GLTP | 42.3 | 17.4 | -43.6 | -91.0 | -136.7 | >10.0 | >909.1 |
| C1789-DV25P | AGT | 82.6 | 84.2 | 82.1 | 79.6 | 66.1 | 0.008 | 1.0 |
| | MRPL42 | 2.7 | -2.0 | -19.6 | 3.6 | -16.6 | >10.0 | >1250.0 |
| | NSD3 | 15.0 | 9.1 | -15.4 | 8.5 | -4.5 | >10.0 | >1250.0 |
| | LUC7L | 15.9 | 13.0 | -2.2 | 1.8 | -8.9 | >10.0 | >1250.0 |
| C1789-DV26P | AGT | 79.3 | 82.8 | 81.3 | 78.8 | 64.5 | <0.016 | 1.0 |
| | MRPL42 | 14.6 | -7.5 | -21.2 | -22.6 | -21.4 | >10.0 | >625.0 |
| | NSD3 | 28.2 | 16.7 | -7.0 | -25.8 | -24.4 | >10.0 | >625.0 |
| | LUC7L | 21.8 | 10.7 | -0.5 | -13.4 | -12.8 | >10.0 | >625.0 |
| C1791-DV26P | AGT | 86.3 | 87.4 | 83.7 | 76.0 | 57.9 | 0.008 | 1.0 |
| | ORC2 | -58.0 | -69.3 | -105.6 | -54.9 | -4.4 | >10.0 | >1250.0 |
| | RBM23 | -24.7 | -81.8 | -97.9 | -48.2 | 1.3 | >10.0 | >1250.0 |
| | SLC2A13 | 2.1 | -2.0 | -8.7 | 8.0 | -5.9 | >10.0 | >1250.0 |
| | KANK1 | 21.8 | 25.7 | 1.1 | 20.7 | -11.8 | >10.0 | >1250.0 |
| | LUC7L | 25.5 | 22.0 | 14.9 | 8.8 | -13.6 | >10.0 | >1250.0 |

(continued)

| Sequence name | Gene name | Inhibition rate (%) | | | | | | |
|---|---|---|---|---|---|---|---|---|
| | | 10.0 nM | 2.0 nM | 0.4 nM | 0.08 nM | 0.016 nM | $IC_{50}$/nM | Ratio |
| C1795-DV27P | AGT | 87.4 | 84.1 | 83.1 | 74.2 | 57.8 | 0.010 | 1.0 |
| | BCKDHB | 16.6 | -2.3 | -5.8 | -14.2 | -7.6 | >10.0 | >1000.0 |
| | ACBD5 | 25.7 | 5.5 | 1.4 | -1.7 | -1.0 | >10.0 | > 1000.0 |
| | MPDZ | 12.0 | -8.2 | 7.9 | -10.9 | 1.6 | >10.0 | >1000.0 |
| | TRIP 11 | 2.7 | -23.4 | -30.1 | -23.8 | -0.7 | >10.0 | >1000.0 |
| | WDPCP | 14.4 | 6.2 | -16.2 | -6.2 | -3.2 | >10.0 | >1000.0 |
| C1812-DV25P | AGT | 88.2 | 88.0 | 86.8 | 82.5 | 66.4 | 0.008 | 1.0 |
| | ISOC1 | -11.1 | -15.4 | -34.8 | -20.6 | -14.5 | >10.0 | >1250.0 |
| | ATXN10 | -21.9 | -19.0 | -30.1 | -11.7 | -10.3 | >10.0 | >1250.0 |
| | PARP14 | -58.2 | -102.4 | -110.3 | -20.2 | -24.9 | >10.0 | >1250.0 |
| | SRGAP 1 | -53.4 | -45.6 | -46.1 | -14.9 | -3.2 | >10.0 | >1250.0 |
| C1816-DV25P | AGT | 84.5 | 83.8 | 80.2 | 73.5 | 56.3 | 0.011 | 1.0 |
| | FOXN3 | 48.6 | 40.8 | 25.8 | 16.7 | 18.0 | >10.0 | >909.1 |
| C1835-DV26P | AGT | 89.2 | 88.3 | 88.4 | 82.4 | 72.2 | <0.016 | 1.0 |
| | MTF2 | 15.3 | -5.6 | 0.2 | -8.1 | -1.8 | 12.227 | 764.2 |
| | CCNA2 | 74.6 | 50.3 | 22.0 | -9.8 | 4.3 | 1.766 | 110.4 |
| | SMNDC1 | -24.5 | -30.7 | -22.1 | -30.3 | -12.9 | >10.0 | >625.0 |
| | ZNF107 | 14.1 | 3.3 | -4.6 | -19.6 | 0.2 | >10.0 | >625.0 |
| | RFC3 | 43.3 | 18.3 | 5.2 | -8.7 | 7.7 | >10.0 | >625.0 |
| C1838-DV29P | AGT | 80.0 | 79.3 | 80.8 | 74.1 | 52.4 | <0.016 | 1.0 |
| | AKAP9 | -45.3 | -55.3 | -37.0 | -9.6 | -13.0 | >10.0 | >625.0 |
| | DENR | 19.2 | -3.6 | -26.3 | -7.9 | -31.1 | >10.0 | >625.0 |
| | PGRMC1 | 40.9 | 6.1 | -27.2 | -13.9 | -18.9 | >10.0 | >625.0 |
| | ZNF626 | 20.3 | -0.6 | -5.2 | -6.8 | 3.1 | >10.0 | >625.0 |
| C1839-DV29P | AGT | 82.2 | 83.2 | 81.8 | 73.8 | 64.9 | <0.016 | 1.0 |
| | GRB14 | 5.8 | -11.9 | -4.7 | -12.4 | -42.1 | >10.0 | >625.0 |
| | JADE1 | -38.2 | -26.1 | -40.0 | -46.0 | -43.5 | >10.0 | >625.0 |
| | STAT5B | 13.2 | 0.3 | -6.5 | -25.4 | -0.4 | >10.0 | >625.0 |
| | ABCD3 | 35.4 | 18.2 | -0.8 | -3.2 | -0.8 | >10.0 | >625.0 |
| APC-ZL | AGT | 90.7 | 87.4 | 76.5 | 76.4 | 53.9 | 0.012 | 1.0 |
| | BAP1 | 24.4 | 14.1 | 14.0 | 2.1 | 21.7 | >10.0 | >833.3 |
| | NFAT5 | 2.4 | -8.0 | 10.4 | 0.6 | -17.5 | >10.0 | >833.3 |
| | ZNF460 | 40.6 | 42.1 | 30.3 | 27.1 | 12.8 | >10.0 | >833.3 |

As shown in the above table:

(1) Some sequences exhibited minimal inhibitory effects on potential off-target genes. For example, C1789-DV25P exhibited an inhibition rate of only 2.7% against MRPL42 at 10 nM, indicating no significant inhibitory effects.
(2) Some sequences exhibited certain inhibitory effects on potential off-target genes at high concentrations, but the $IC_{50}$ for inhibiting off-target genes was significantly higher than the $IC_{50}$ for inhibiting AGT. For example, C1579-DV25P exhibited an inhibition rate of 42.3% against GLTP at 10 nM, but the $IC_{50}$ against GLTP was greater than 10 nM, while the $IC_{50}$ against AGT was 0.011 nM, with a ratio far exceeding 200, indicating no significant off-target effects. (3) C1578s-DV29P exhibited an inhibition rate of 66.9% against ERRFI1 at 10 nM with an $IC_{50}$ of 0.021 nM, while the $IC_{50}$ against AGT was 0.004 nM, with an $IC_{50}$ ratio of only 5.3; C1835-DV26P exhibited an inhibition rate of 74.6% against CCNA2 at 10 nM with an $IC_{50}$ of 1.766 nM, while the $IC_{50}$ against AGT was less than 0.016 nM, with a minimum $IC_{50}$ ratio of 110.4. These two sequences were considered to have certain off-target effects, requiring anti-off-target modifications to reduce their inhibition of off-target genes.

**Summary:**

(1) All sequences exhibited significant inhibitory effects against AGT. For example, C1576s-DV29P exhibited an inhibition rate of 83.8% against AGT at 10 nM.
(2) C1578s-DV29P and C1835-DV26P exhibited certain off-target effects, while other sequences showed no significant off-target activity.

**Example 7: Inhibition of sequences with specific anti-off-target designs on AGT and off-target genes**

In practical applications of siRNA, there are numerous instances where the expression of non-target mRNAs, which are only partially complementary to the guide strand (antisense strand), is inhibited. Research by Alnylam Pharmaceuticals has demonstrated that the hepatotoxicity of N-acetylgalactosamine (GalNAc)-conjugated siRNAs is primarily attributed to off-target effects resulting in gene suppression of incorrect targets through a microRNA (miRNA)-like recognition mechanism.

To address the issue, Alnylam Pharmaceuticals incorporated a glycol nucleic acid (GNA) modification at position 7 of the antisense strand in their latest fifth-generation siRNA template design to disrupt the seed region of the antisense strand, thereby significantly reducing off-target effects and mitigating hepatotoxicity. Such modifications can interfere with the binding of siRNA to unintended targets via seed region recognition, thereby inhibiting off-target effects.

In addition, a study in 2008 revealed that replacing all eight base pairs at the 5' end of the antisense strand of double-stranded siRNA with DNA significantly reduced off-target effects without compromising the activity of siRNA. Research on mRNA cleavage sites revealed that DNA substitution did not interfere with RISC-mediated mRNA cleavage between nucleotides at positions 10 and 11 of the antisense strand but did interfere with cleavage at non-canonical sites, possibly by inhibiting non-specific cleavage of double-stranded RNA by RNase.

Based on the above findings, in order to reduce off-target effects for sequences, DV25-29P were used in this example to modify unmodified sequences 1578s and 1835, by replacing the nucleotides at position 7 or positions 6 and 7 of the antisense strand with DNA, while also replacing the nucleotides at complementary positions of the sense strand with DNA to reduce off-target effects. These two anti-off-target modifications were compared with an anti-off-target design using an (S)-GNA substitution at position 7 of the antisense strand.

The experimental results demonstrated that the sequences with anti-off-target designs exhibited significant inhibitory effects on the target gene AGT at concentrations ranging from 10 nM to 16 pM, while some sequences exhibited significantly reduced inhibitory effects on off-target genes. The findings indicate that introducing anti-off-target designs does not compromise the inhibitory effects of the alternately modified and template-modified sequences of the present disclosure on AGT gene expression, but can effectively inhibit off-target effects for some sequences.

**1. Experimental materials**

1) Test samples:

Template-modified versions of unmodified sequences 1578s, 1835, and 1838, as well as sequences using template modification and anti-off-target design (Table 34).

2) Synthesis of sequences:

**I. Template-modified sequences**

The sequences were synthesized with reference to Example 1, under the section of "Synthesis of alternately modified sequence".

**II. siRNA sequences with DNA-based anti-off-target design**

The siRNA sequences were synthesized with reference to Example 1. During the synthesis of the 6th or 7th nucleotide from the 5' end of the antisense strand, the following DNA monomers were used: DMT-dA phosphoramidite monomer (Formula 15), DMT-dT phosphoramidite monomer (Formula 16), DMT-dC phosphoramidite monomer (Formula 17), and DMT-dG phosphoramidite monomer (Formula 18), the structures of which are as follows:

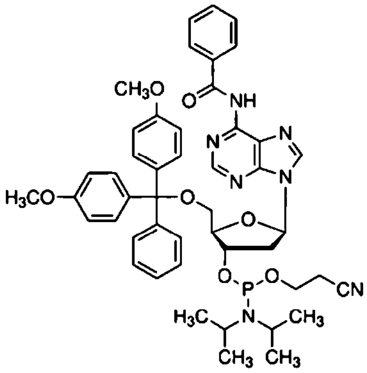

Formula 15

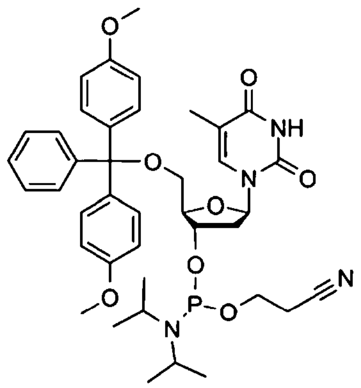

Formula 16

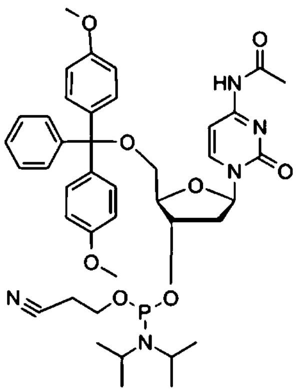

Formula 17

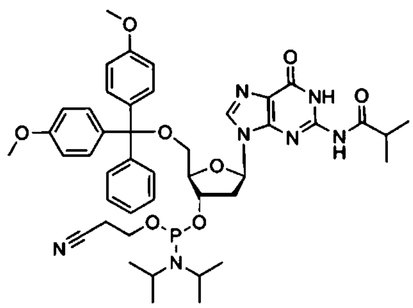

Formula 18

**III. siRNA sequences with GNA-based anti-off-target design**

The siRNA sequences were synthesized with reference to Example 1. During the synthesis of the 7th base from the 5' end of the antisense strand, a GNA monomer was used, whose structure is as follows:

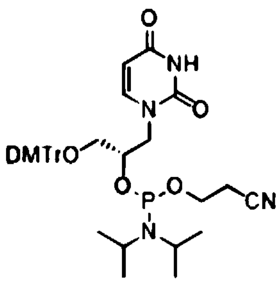

**(S)-GNA-U**

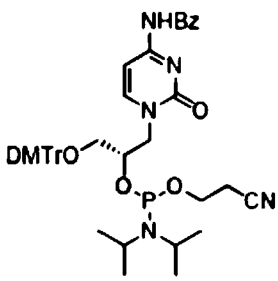

**(S)-GNA-C**

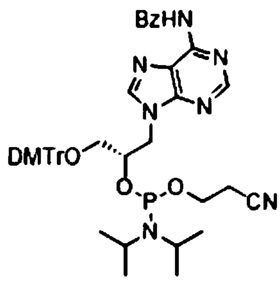

**(S)-GNA-A**

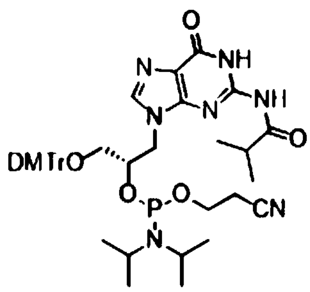

**(S)-GNA-G**

Table 34: Sequences using template modification and anti-off-target design

| Sequence ID | Sense strand 5'-3' | Modified sequence SEQ ID NO./ corresponding unmodified sequence SEQ ID NO. | Antisense strand 5'-3' | Modified sequence SEQ ID NO./ corresponding unmodified sequence SEQ ID NO. |
|---|---|---|---|---|
| D835-DV26P | Ums-Ums-Gm-Gm-Gm-Um-Uf-Um-Uf-Af-Af-Am-Am-Um-Um-Am-Af-Am-Gm-Um-Am | 398/1 | UmsEVP-Afs-Cf-Uf-Um-Uf-Am-Am-Um-Um-Um-Um-Am-Af-Am-Af-Cm-Cm-Cm-Am-Ams-Ums-Um | 433/14 |
| D835-DV26P+ | Ums-Ums-Gm-Gm-Gm-Um-Uf-Um-Uf-Af-Af-Am-Am-Um-Um-Am-Af-Am-Gm-Um-Am | 398/1 | UmsEVP-Afs-Cf-Uf-Um-Tgna-Am-Am-Um-Um-Um-Um-Am-Af-Am-Af-Cm-Cm-Cm-Am-Ams-Ums-Um | 564/14 |
| D835-DV26Pd7B | Ums-Ums-Gm-Gm-Gm-Um-Uf-Um-Uf-Af-Af-Am-Am-Um-Td-Am-Af-Am-Gm-Um-Am | 526/1 | UmsEVP-Afs-Cf-Uf-Um-Uf-Ad-Am-Um-Um-Um-Um-Am-Af-Am-Af-Cm-Cm-Cm-Am-Ams-Ums-Um | 565/14 |
| D835-DV26Pd67B | Ums-Ums-Gm-Gm-Gm-Um-Uf-Um-Uf-Af-Af-Am-Am-Um-Td-Ad-Af-Am-Gm-Um-Am | 527/1 | UmsEVP-Afs-Cf-Uf-Um-Td-Ad-Am-Um-Um-Um-Um-Am-Af-Am-Af-Cm-Cm-Cm-Am-Ams-Ums-Um | 566/14 |
| D578s-DV29P+ | Ams-Cms-Cm-Um-Um-Uf-Um-Cf-Um-Uf-Cm-Um-Am-Am-Um-Gf-Am-Gm-Um-Am | 426/12 | UmsEVP-Afs-Cm-Uf-Cf-Agna-Um-Um-Am-Gm-Am-Am-Gm-Af-Am-Af-Am-Gm-Gm-Ums-Gms-Gm | 567/25 |
| D578s-DV29Pd67B | Ams-Cms-Cm-Um-Um-Uf-Um-Cf-Um-Uf-Cm-Um-Am-Ad-Td-Gf-Am-Gm-Um-Am | 528/12 | UmsEVP-Afs-Cm-Uf-Cf-Ad-Td-Um-Am-Gm-Am-Am-Gm-Af-Am-Af-Am-Gm-Gm-Ums-Gms-Gm | 568/25 |
| D578s-DV29Pd7B | Ams-Cms-Cm-Um-Um-Uf-Um-Cf-Um-Uf-Cm-Um-Am-Ad-Um-Gf-Am-Gm-Um-Am | 529/12 | UmsEVP-Afs-Cm-Uf-Cf-Af-Td-Um-Am-Gm-Am-Am-Gm-Af-Am-Af-Am-Gm-Gm-Ums-Gms-Gm | 569/25 |
| D578s-DV29P | Ams-Cms-Cm-Um-Um-Uf-Um-Cf-Um-Uf-Cm-Um-Am-Am-Um-Gf-Am-Gm-Um-Am | 426/12 | UmsEVP-Afs-Cm-Uf-Cf-Af-Um-Um-Am-Gm-Am-Am-Gm-Af-Am-Af-Am-Gm-Gm-Ums-Gms-Gm | 492/25 |

Cell type: HepG2 cells, provided by Cyagen (H1-1701).

HepG2 cells were cultured in DMEM medium (ATCC-30-2003) containing 10% fetal bovine serum (FBS, ExCell

Bio-FSP500) and 1% penicillin-streptomycin (HyClone-SV30010).

Drug vehicle: sterile nuclease-free water, Gibco Opti-MEM.

**2. Experimental methods**

The inhibitory effects of the test samples on the mRNA expression of AGT and potential off-target genes in HepG2 cell lines were tested by qRT-PCR.

**2.1 Cell culture**

HepG2 cells in the logarithmic growth phase were harvested from subcultured stocks and cultured in DMEM medium containing 10% fetal bovine serum (supplemented with 100 μL/mL of penicillin and streptomycin). The cells were incubated in a cell culture incubator at 37°C with 5% $CO_2$, with medium replaced daily. For subculture, the cells were digested with 0.25% trypsin, centrifuged at 1000 rpm for 5 minutes, and the supernatant was discarded. Fresh medium was added for subculture.

**2.2 Cell transfection**

Preparation of transfection mixture: Lipofectamine RNAiMAX and Opti-MEM were mixed at a ratio of 2:98 and vortexed thoroughly.

Preparation of transfection reagent: 60 μL of siRNA solution diluted in Opti-MEM was mixed with 60 μL of the prepared transfection mixture at a ratio of 1:1 (v/v), vortexed thoroughly, and the mixture was incubated at room temperature for 15 minutes to obtain lipid nanoparticles (LNPs). A 12.5 μL aliquot was taken for encapsulation efficiency analysis.

Blank control transfection reagent: 60 μL of the prepared transfection mixture was added to 60 μL of Opti-MEM, vortexed thoroughly, and the mixture was incubated at room temperature for 15 minutes.

The prepared transfection reagent was added to a 24-well cell culture plate (100 μL per well), resulting in a final siRNA concentration of 16 pM/80 pM/400 pM/2 nM/10 nM per well. 500 μL of cell suspension ($1.5 \times 10^5$ cells per mL) was added per well. After mixing by crosswise method, the plate was incubated in a cell culture incubator at 37°C with 5% $CO_2$ for 40 hours.

**2.3 RNA extraction and reverse transcription**

After 24 hours of transfection, the medium was removed and the cells were collected for RNA extraction. Total RNA was extracted using the RNeasy® 96 Kit (QIAGEN-74182) according to the instructions provided by the manufacturer. Subsequently, cDNA was synthesized using the FastKing RT Kit (With gDNase) (TIANGEN-KR116-02) according to the instructions provided by the manufacturer.

**2.4 RT-qPCR**

Target cDNA was tested by qPCR, with GAPDH cDNA used as an internal control in parallel. 8 μL of prepared qPCR reaction mixture and 2 μL of sample cDNA were added to a 384-well plate. TaqMan qPCR was performed according to the following program: 95°C for 10 minutes, followed by 40 cycles of 95°C for 15 seconds and 60°C for 1 minute. SYBR qPCR was performed according to the following program: 50°C for 2 minutes, 95°C for 10 minutes, followed by 40 cycles of 95°C for 15 seconds and 60°C for 1 minute; finally, a melting curve analysis was performed at 95°C for 15 seconds, 60°C for 1 minute, and 95°C for 15 seconds.

**2.5 Data analysis**

The RNA expression levels of target genes in samples were calculated based on Ct values using the ΔΔCt relative quantification method. The relative expression levels of target genes were expressed as $2^{-\Delta\Delta Ct}$.

The calculation formula is as follows:

$$\Delta Ct = \text{Average Ct value of target gene - Average Ct value of reference gene;}$$

$$\Delta\Delta Ct = \Delta Ct\ \text{(treatment group)} - \Delta Ct\ \text{(RNAiMAX control group);}$$

Relative mRNA expression level of target gene = $2^{-\Delta\Delta Ct}$;

Inhibition rate = (1 - relative expression level of sample / average expression level of RNAiMAX control) × 100%.

**3. Experimental results**

**(1) All modified sequences exhibited significant inhibitory effects on the AGT gene. For example, the template-modified sequence D578s-DV29P exhibited an inhibition rate of 85.7%, while the sequence D578s-DV29Pd7B with both template and anti-off-target modifications exhibited an inhibition rate of 81.3%.**

The inhibition rates of all test samples on the AGT gene are listed in Table 35.
All template-modified sequences except D838-DV29P+ exhibited significant inhibitory effects on AGT at a concentration of 10 nM. For example, the template-modified sequence D578s-DV29P exhibited an inhibition rate of 85.7%.

Table 35: Inhibition rates of sequences with template and anti-off-target modifications on AGT gene expression

| Unmodified sequence | Sequence ID | Modification pattern | Inhibition rate (%) |
|---|---|---|---|
| 1578s | D578s-DV29P | Template modification | 85.7 |
| | D578s-DV29P+ | Template modification and GNA anti-off-target | 78.2 |
| | D578s-DV29Pd67B | Template modification and d67B anti-off-target | 79.3 |
| | D578s-DV29Pd7B | Template modification and d7B anti-off-target | 81.3 |
| 1835 | D835-DV26P | Template modification | 89.2 |
| | D835-DV26P+ | Template modification and GNA anti-off-target | 84.4 |
| | D835-DV26Pd67B | Template modification and d67B anti-off-target | 85.4 |
| | D835-DV26Pd7B | Template modification and d7B anti-off-target | 84.6 |

After template modification and anti-off-target design, the sequence exhibited significant inhibitory activity on AGT gene expression. For example, when the unmodified sequence 1578s was modified with template DV29P, followed by anti-off-target design, the resulting sequence D578s-DV29Pd7B exhibited an inhibition rate of 81.3%.

**(2) After using any one or more of the various modification patterns of the present disclosure, followed by anti-off-target design, the sequences exhibited significant anti-off-target effects on certain off-target genes, thereby reducing the inhibition of off-target genes.**

As shown in Table 36, the experimental results demonstrated that:
The template-modified sequences of the present disclosure, when incorporating anti-off-target designs, reduced the inhibition of off-target genes, thereby exhibiting anti-off-target effects. The sequences using the modification templates of the present disclosure, when incorporating anti-off-target designs, exhibited anti-off-target effects, with a decrease of up to 72.535% in inhibition rate on off-target genes.

Table 36: Inhibition rates of template-modified sequences and their anti-off-target modifications on off-target genes

| Unmodified sequence ID | Sequence ID | Off-target gene | Inhibition rate on off-target gene (%) | Decrease in inhibition rate compared to no anti-off-target design (%) |
|---|---|---|---|---|
| 1578s | D578s-DV29P | ERRFI1 | 66.894 | 0.000 |
| | D578s-DV29P+ | | 19.447 | 47.447 |
| | D578s-DV29Pd67B | | 12.678 | 54.216 |
| | D578s-DV29Pd7B | | -5.641 | 72.535 |

(continued)

| Unmodified sequence ID | Sequence ID | Off-target gene | Inhibition rate on off-target gene (%) | Decrease in inhibition rate compared to no anti-off-target design (%) |
|---|---|---|---|---|
| 1835 | D835-DV26P | CCNA2 | 74.619 | 0.000 |
| | D835-DV26P+ | | 35.133 | 39.486 |
| | D835-DV26Pd67B | | 33.215 | 41.404 |
| | D835-DV26Pd7B | | 20.953 | 53.666 |

**1) Template modification alone**

Some template-modified sequences exhibited certain off-target effects. For example, the template-modified sequence D578s-DV29P (unmodified sequence 1578s modified with template DV29P) exhibited an inhibition rate of 66.894% against the potential off-target gene ERRFI1; the template-modified sequence D835-DV26P (unmodified sequence 1835 modified with template DV26P) exhibited an inhibition rate of 74.619% against the potential off-target gene CCNA2.

**2) Simultaneous anti-off-target design (denoted as "d67B" or "d7B" in sequence ID)**

Some of the sequences with both template and anti-off-target modifications demonstrated significantly reduced inhibition rates against off-target genes, thereby exhibiting significant anti-off-target effects. For example, D578s-DV25Pd7B exhibited a 72.535% reduction in inhibition rate against ERRFI1, indicating a significant decrease; D835-DV26Pd7B exhibited a 53.666% reduction in inhibition rate against CCNA2, indicating a significant decrease.

**Summary:**

1. All modified sequences exhibited significant inhibitory effects on the AGT gene. For example, the template-modified sequence D578s-DV29P exhibited an inhibition rate of 85.7%, while the sequence D578s-DV29Pd7B with both template and anti-off-target modifications exhibited an inhibition rate of 81.3%.
2. After using any one or more of the various modification patterns of the present disclosure, followed by anti-off-target design, the sequences exhibited significant anti-off-target effects on certain off-target genes, thereby reducing the inhibition of off-target genes.

1) Some template-modified sequences exhibited certain off-target effects. For example, the template-modified sequence D578s-DV29P (unmodified sequence 1578s modified with template DV29P) exhibited an inhibition rate of 66.894% against the potential off-target gene ERRFI1; the template-modified sequence D835-DV26P (unmodified sequence 1835 modified with template DV26P) exhibited an inhibition rate of 74.619% against the potential off-target gene CCNA2.
2) The template-modified sequences of the present disclosure, when incorporating anti-off-target designs, reduced the inhibition of off-target genes, thereby exhibiting anti-off-target effects, with a decrease of up to 72.535% in inhibition rate on off-target genes.

**Example 8: Inhibition of sequences modified using modification templates of the present disclosure on AGT in primary human hepatocytes**

In this example, the inhibitory effects of the high-activity sequences from Example 4 on potential off-target genes in primary human hepatocytes were examined, and compared with Zilebesiran, a drug currently in Phase II clinical trials. Five sequences, namely C1576s-DV29P, C1578s-DV29P, C1579-DV25P, C1812-DV25P, and C1585s-DV27P, were synthesized using their respective modification templates or both template and anti-off-target modifications, and conjugated with GalNAc to enable free uptake by hepatocytes. The sequences listed in Table 37 were applied to primary human hepatocytes at concentrations ranging from 0.0064 nM to 100 nM via free uptake. Dose-response curves were fitted, and the $IC_{50}$ values of these sequences were calculated. The results demonstrated that the $IC_{50}$ values of some sequences were superior to that of the positive control Zilebesiran. For example, D576s-DV29PG5 exhibited an $IC_{50}$ of

4.698 nM, outperforming that of the positive control Zilebesiran at 7.071 nM.

**1. Experimental materials**

1) Test samples:

The sequences listed in Table 37, which were synthesized using template modification alone or both template and anti-off-target modifications, and conjugated with GalNAc ligand G5 at the 3' end of the sense strand:

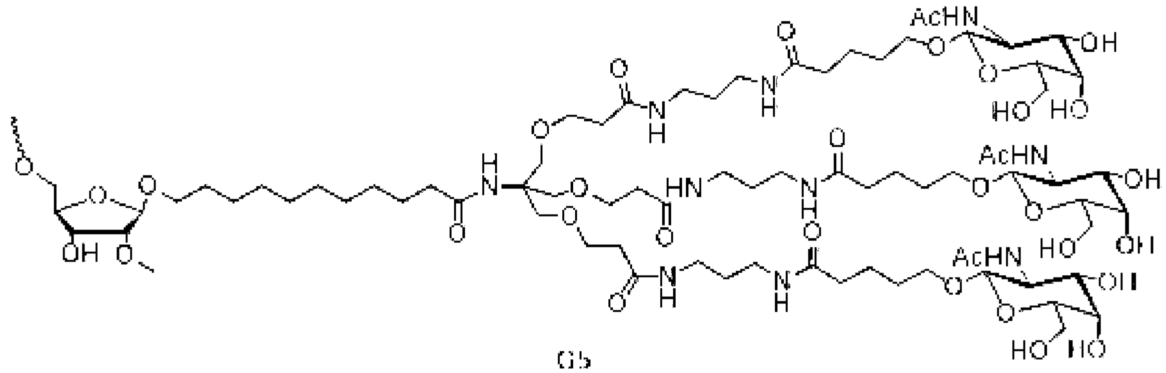

G5

The conjugation method of oligonucleotides with ligand G5 was the same as the preparation methods of conjugates 4, 5, 6, and 7 in Example 3 of Chinese Patent CN116854754A, *i.e.,* conjugating YK-GAL-304, YK-GAL-305, YK-GAL-306, and YK-GAL-307 with oligonucleotides. YK-GAL-304, YK-GAL-305, YK-GAL-306, and YK-GAL-307 were synthesized as in Example 1 of CN116854754A.

The resulting oligonucleotide-ligand conjugate is shown below:

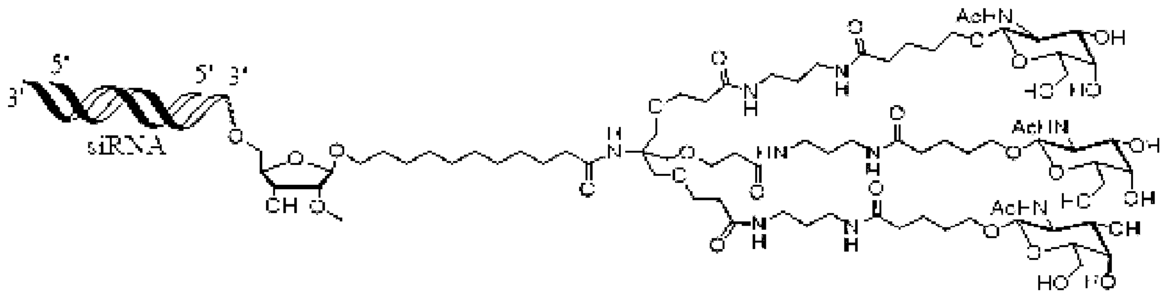


The specific sequences are listed in Table 7, where "G5" in the sequence ID indicates conjugation with GalNAc ligand G5, and "GL" indicates conjugation with GalNAc ligand L96.

Table 37: Template-modified sequences

| Sequence ID | Sense strand 5'-3' | Modified sequence SEQ ID NO./ corresponding unmodified sequence SEQ ID NO. | Antisense strand 5'-3' | Modified sequence SEQ ID NO./ corresponding unmodified sequence SEQ ID NO. |
|---|---|---|---|---|
| D579-DV25PG5 | Cms-Cms-Um-Um-Um-Um-Cf-Um-Uf-Cf-Uf-Am-Am-Um-Gm-Am-Gf-Um-Cm-Gm-Am-G5 | 530/4 | UmsEVP-Cfs-Gm-Am-Cm-Uf-Cm-Am-Um-Um-Am-Gm-Am-Af-Gm-Af-Am-Am-Am-Gm-Gms-Ums-Gm | 457/17 |
| D579-DV25Pd7BG5 | Cms-Cms-Um-Um-Um-Um-Cf-Um-Uf-Cf-Uf-Am-Am-Um-Gd-Am-Gf-Um-Cm-Gm-Am-G5 | 531/4 | UmsEVP-Cfs-Gm-Am-Cm-Uf-Cd-Am-Um-Um-Am-Gm-Am-Af-Gm-Af-Am-Am-Am-Gm-Gms-Ums-Gm | 570/17 |
| D579-DV25Pd67BG5 | Cms-Cms-Um-Um-Um-Um-Cf-Um-Uf-Cf-Uf-Am-Am-Um-Gd-Ad-Gf-Um-Cm-Gm-Am-G5 | 532/4 | UmsEVP-Cfs-Gm-Am-Cm-Td-Cd-Am-Um-Um-Am-Gm-Am-Af-Gm-Af-Am-Am-Am-Gm-Gms-Ums-Gm | 571/17 |
| D812-DV25PG5 | Cms-Cms-Um-Um-Um-Um-Cf-Am-Af-Gf-Uf-Um-Gm-Am-Gm-Am-Af-Cm-Am-Am-Am-G5 | 541/3 | UmsEVP-Ufs-Um-Gm-Um-Uf-Cm-Um-Cm-Am-Am-Cm-Um-Uf-Gm-Af-Am-Am-Am-Gm-Gms-Gms-Am | 450/16 |
| D576s-DV29PG5 | Cms-Cms-Am-Cm-Cm-Uf-Um-Uf-Um-Cf-Um-Um-Cm-Um-Am-Af-Um-Gm-Am-Am-G5 | 554/11 | UmsEVP-Ufs-Cm-Af-Uf-Uf-Am-Gm-Am-Am-Gm-Am-Am-Af-Am-Gf-Gm-Um-Gm-Gms-Gms-Am | 488/24 |
| D578s-DV29Pd7BG5 | Ams-Cms-Cm-Um-Um-Uf-Um-Cf-Um-Uf-Cm-Um-Am-Ad-Um-Gf-Am-Gm-Um-Am-G5 | 558/12 | UmsEVP-Afs-Cm-Uf-Cf-Af-Td-Um-Am-Gm-Am-Am-Gm-Af-Am-Af-Am-Gm-Gm-Ums-Gms-Gm | 569/25 |

(continued)

| Sequence ID | Sense strand 5'-3' | Modified sequence SEQ ID NO./ corresponding unmodified sequence SEQ ID NO. | Antisense strand 5'-3' | Modified sequence SEQ ID NO./ corresponding unmodified sequence SEQ ID NO. |
|---|---|---|---|---|
| D585s-DV27PG5 | Cms-Ums-Um-Cm-Um-Af-Am-Uf-Gf-Af-Gm-Um-Cm-Gm-Am-Cf-Um-Um-Um-Am-G5 | 560/10 | UmsEVP-Afs-Am-Af-Gf-Uf-Cm-Gm-Am-Cm-Um-Cm-Am-Uf-Um-Af-Gm-Am-Am-Gms-Ams-Am | 483/23 |
| Zilebesiran | Gms-Ums-Cm-Am-Um-Cm-Cf-Am-Cf-Af-Af-Um-Gm-Am-Gm-Am-Gm-Um-Am-Cm-Am-GL | 561/507 | Ums-Gfs-Um-Am-Cm-Tgna-Cm-Um-Cm-Am-Um-Um-Gm-Uf-Gm-Gf-Am-Um-Gm-Am-Cms-Gms-Am | 587/520 |

Cell type: primary human hepatocytes, provided by Liver Biotechnology Co., Ltd. (LV-PHH001).
Drug vehicle: sterile nuclease-free water, primary human hepatocyte maintenance medium (LV-WEM001).

### 2. Experimental methods

The inhibitory effects of the test samples on the mRNA expression of AGT genes in primary human hepatocytes were tested by qRT-PCR.

### 2.1 Thawing and culturing of primary human hepatocytes

The medium was pre-warmed at a constant temperature of 37°C for at least 30 minutes. 300 μL of PBS was added per well in a collagen-coated 24-well plate, which was shaken several times, and the PBS was aspirated. The cryovial containing the hepatocytes was removed from liquid nitrogen storage and placed in a 37°C water bath, gently shaken until only a small amount of ice crystals remained in the vial, and the cell suspension was poured into thawing medium at once. 1 mL of thawing medium was pipetted to rinse the inner wall of the cryovial 2 to 3 times. The cell suspension in thawing medium was mixed by inversion, followed by centrifugation at 50 g for 5 minutes at room temperature, with acceleration set to 5 and deceleration set to 3. The cells were diluted to $3 \times 10^5$ cells/mL in plating medium, then inoculated into a 24-well plate at 0.5 mL per well, and incubated in an incubator for 24 hours.

### 2.2 Free uptake by primary human hepatocytes

The test samples were diluted to 100 to 0.0064 nM in primary human hepatocyte maintenance medium. After incubation, the primary human hepatocytes were removed, the plating medium was aspirated, and 500 μL of the diluted test sample was added per well. The plate was returned to the cell culture incubator at 37°C with 5% $CO_2$ and incubated for an additional 40 hours.

### 2.3 RNA extraction and reverse transcription

After 24 hours of transfection, the medium was removed and the cells were collected for RNA extraction. Total RNA was extracted using the RNeasy® 96 Kit (QIAGEN-74182) according to the instructions provided by the manufacturer. Subsequently, cDNA was synthesized using the FastKing RT Kit (With gDNase) (TIANGEN-KR116-02) according to the instructions provided by the manufacturer.

### 2.4 RT-qPCR

Refer to "Quantitative analysis of AGT mRNA" under Section 2.3 in Example 2.

### 2.5 Data analysis

The RNA expression levels of target genes in samples were calculated based on Ct values using the ΔΔCt relative quantification method. The relative expression levels of target genes were expressed as $2^{-\Delta\Delta Ct}$.
The calculation formula is as follows:

$$\Delta Ct = \text{Average Ct value of target gene - Average Ct value of reference gene};$$

$$\Delta\Delta Ct = \Delta Ct\ (\text{treatment group}) - \Delta Ct\ (\text{RNAiMAX control group});$$

Relative mRNA expression level of target gene = $2^{-\Delta\Delta Ct}$;

Inhibition rate = (1 - relative expression level of sample / average expression level of RNAiMAX control) × 100%.

### 3. Experimental results

The inhibition rates of these sequence against AGT at various concentrations are shown in Table 38 below. All sequences exhibited significant inhibitory effects against AGT. For example, D576s-DV29PG5 exhibited an inhibition rate of 83.1% against AGT at 100 nM. In addition, the $IC_{50}$ values of some sequences were superior to that of the positive control

Zilebesiran. For example, D576s-DV29PG5 exhibited an $IC_{50}$ of 4.698 nM, outperforming that of the positive control Zilebesiran at 7.071 nM.

Table 38: Inhibition rates and $IC_{50}$ values of sequences against AGT at various concentrations

| Sequence name | AGT inhibition rate (%) | | | | | | | $IC_{50}$ (nM) |
|---|---|---|---|---|---|---|---|---|
| | 100 nM | 20 nM | 4 nM | 0.8 nM | 0.16 nM | 0.032 nM | 0.0064 nM | |
| Zilebesiran | 65.5 | 57.8 | 42.8 | 22.6 | 9.9 | 5.2 | 14.9 | 7.071 |
| D579-DV25Pd7BG5 | 71.7 | 58.8 | 63.2 | 12.7 | 2.1 | -5.5 | -6.2 | 1.998 |
| D576s-DV29PG5 | 83.1 | 66.8 | 46.4 | -0.5 | 3.0 | -1.2 | -2.8 | 4.698 |
| D812-DV25PG5 | 74.4 | 63.3 | 45.4 | 10.9 | 7.8 | 1.4 | 1.1 | 5.659 |
| D578s-DV29Pd7BG5 | 61.5 | 43.0 | 17.3 | -7.7 | -12.5 | -27.5 | -13.0 | 30.720 |
| D585s-DV27PG5 | 56.9 | 47.5 | 33.9 | 13.5 | -3.8 | -8.7 | 2.5 | 20.470 |
| D579-DV25Pd67BG5 | 76.1 | 73.3 | 42.0 | 19.9 | 24.4 | 2.3 | -12.2 | 5.719 |
| D579-DV25PG5 | 72.6 | 60.2 | 32.9 | 12.5 | -0.9 | 6.3 | 8.6 | 9.818 |

**Example 9: Inhibition of sequences modified using modification templates of the present disclosure on AGT in mouse serum**

In this example, certain sequences were exemplarily selected, including unmodified sequences 1576s, 1578s, 1579, 1585s, 1789, 1791, 1795, 1812, 1816, 1835, 1838, and 1839, which were then modified, e.g., using template modification alone or both template and anti-off-target modifications. The positive control drug used in this study was Zilebesiran, an siRNA drug currently in Phase II clinical trials. The inhibitory effects of these sequences on AGT in serum at different time points were tested by ELISA using transgenic mice expressing the human AGT gene.

**1. Experimental materials**

**Test articles:**

The sequences listed in Table 39, which were synthesized using template modification alone or both template and anti-off-target modifications, and conjugated with GalNAc ligand G5 at the 3' end of the sense strand:

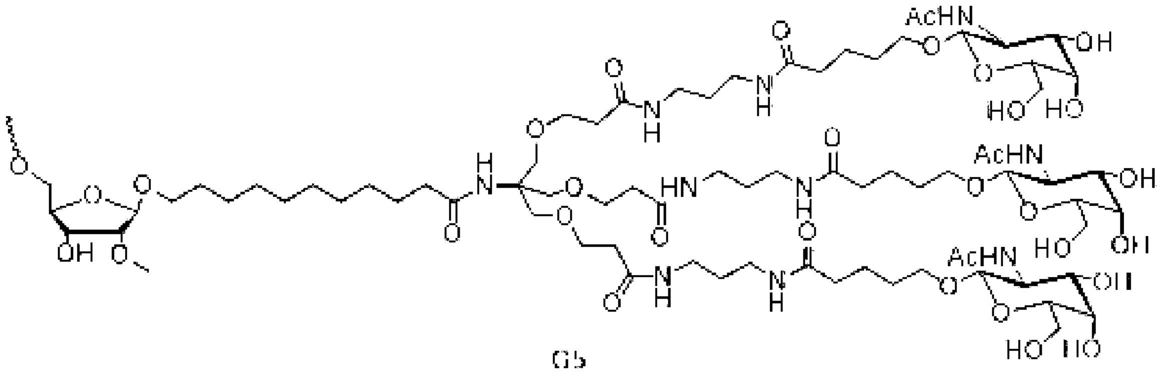

G5

The conjugation method of oligonucleotides with ligand G5 was the same as the preparation methods of conjugates 4, 5, 6, and 7 in Example 3 of Chinese Patent CN116854754A, *i.e.,* conjugating YK-GAL-304, YK-GAL-305, YK-GAL-306, and YK-GAL-307 with oligonucleotides. YK-GAL-304, YK-GAL-305, YK-GAL-306, and YK-GAL-307 were synthesized as in Example 1 of CN116854754A.

The resulting oligonucleotide-ligand conjugate is shown below:

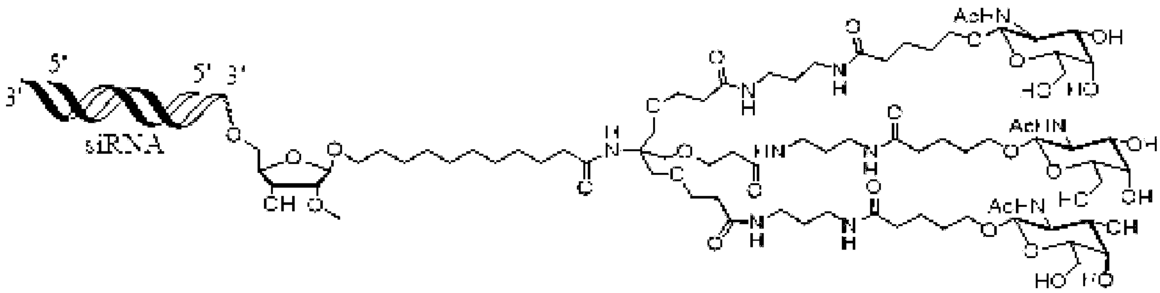

The specific sequences are listed in Table 39, where "G5" in the sequence ID indicates conjugation with GalNAc ligand G5, and "GL" indicates conjugation with GalNAc ligand L96.

The structure of G5 is as follows:

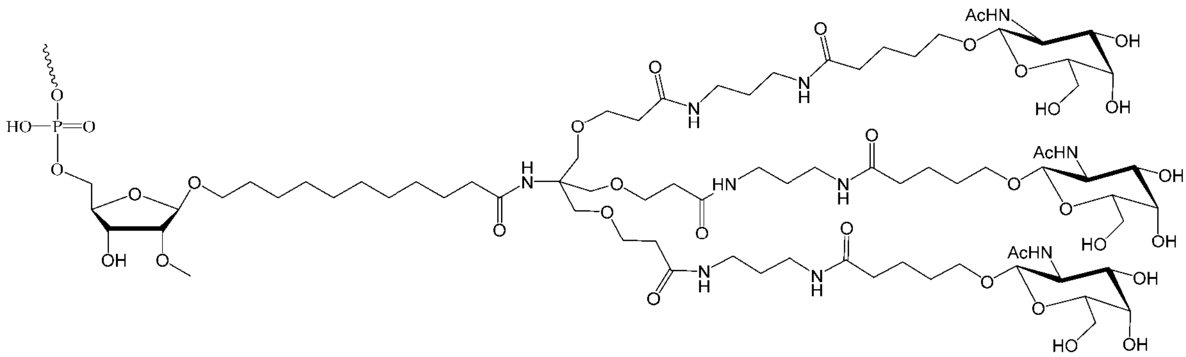

[[(1R,2R,3R,4R)-1-[[28-[[2-(acetylamino)-2-deoxy-β-D-galactopyranosyl]oxy]-13,13-bis[[3-[[3-[[5-[[2-(acetylamino)-2-deoxy-β-D-galactopyranosyl]oxy]-1-oxopentyl]amino]propyl]amino]-3-oxopropoxy]methyl]-11,18,24-trioxo-15-oxa-12,19,23-triazaoctacosan-1-yl]oxy]-2-O-methyl-5-β-D-ribofuranosyl]hydrogen phosphate]

[[(1R,2R,3R,4R)-1-[[28-[[2-(acetylamino)-2-deoxy-β-D-galactopyranosyl]oxy]-13,13-bis[[3-[[3-[[5-[[2-(acetylamino)-2-deoxy-β-D-galactopyranosyl]oxy]-1-oxopentyl]amino]propyl]amino]-3-oxopropoxy]methyl]-11,18,24-trioxo-15-oxa-12,19,23-triazaoctacosan-1-yl]oxy]-2-O-methyl-5-β-D-ribofuranosyl]hydrogen phosphate]

The structure of GL is as follows:

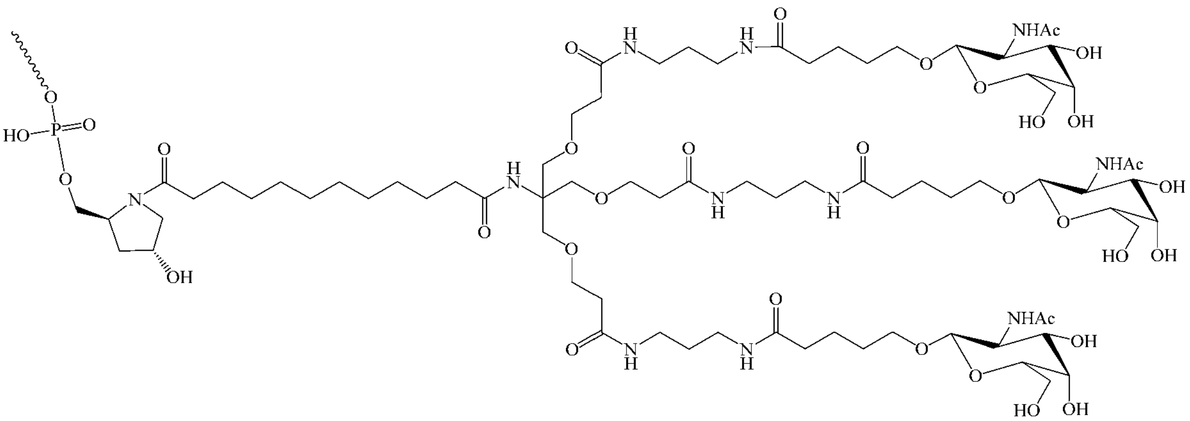

[(2S,4R)-1-[29-[[2-(acetylamino)-2-deoxy-β-D-galactopyranosyl]oxy]-14,14-bis[[3-[[3-[[5-[[2-(acetylamino)-2-deoxy-β-D-galactopyranosyl]oxy]-1-oxopentyl]amino]propyl]amino]-3-oxopropoxy]methyl]-1,12,19,25-tetraoxo-16-oxa-13,20,24-triazanonacos-1-yl]-4-hydroxy-2-pyrrolidinyl]methyl hydrogen phosphate]

[(2S,4R)-1-[29-[[2-(acetylamino)-2-deoxy-β-D-galactopyranosyl]oxy]-14,14-bis[[3-[[3-[[5-[[2-(acetylamino)-2-deoxy-β-D-galactopyranosyl]oxy]-1-oxopentyl]amino]propyl]amino]-3-oxopropoxy]methyl]-1,12,19,25-tetraoxo-16-oxa-13,20,24-triazanonacos-1-yl]-4-hydroxy-2-pyrrolidinyl]methyl hydrogen phosphate]

Table 39: Sequences in animal experiments

| Sequence ID | Sense strand 5'-3' | Modified sequence SEQ ID NO./ corresponding unmodified sequence SEQ ID NO. | Antisense strand 5'-3' | Modified sequence SEQ ID NO./ corresponding unmodified sequence SEQ ID NO. |
|---|---|---|---|---|
| D579-DV25PG5 | Cms-Cms-Um-Um-Um-Um-Cf-Um-Uf-Cf-Uf-Am-Am-Um-Gm-Am-Gf-Um-Cm-Gm-Am-G5 | 530/4 | UmsEVP-Cfs-Gm-Am-Cm-Uf-Cm-Am-Um-Um-Am-Gm-Am-Af-Gm-Af-Am-Am-Am-Gm-Gms-Ums-Gm | 457/17 |
| D579-DV25Pd7BG5 | Cms-Cms-Um-Um-Um-Um-Cf-Um-Uf-Cf-Uf-Am-Am-Um-Gd-Am-Gf-Um-Cm-Gm-Am-G5 | 531/4 | UmsEVP-Cfs-Gm-Am-Cm-Uf-Cd-Am-Um-Um-Am-Gm-Am-Af-Gm-Af-Am-Am-Am-Gm-Gms-Ums-Gm | 570/17 |
| D789-DV26PG5 | Gms-Ums-Um-Um-Gm-Um-Gf-Am-Af-Af-Cf-Am-Am-Am-Am-Am-Af-Gm-Um-Gm-Um-G5 | 533/6 | AmsEVP-Cfs-Af-Cf-Um-Uf-Um-Um-Um-Um-Gm-Um-Um-Uf-Cm-Af-Cm-Am-Am-Am-Cms-Ams-Am | 465/19 |
| D789-DV25PG5 | Gms-Ums-Um-Um-Gm-Um-Gf-Am-Af-Af-Cf-Am-Am-Am-Am-Am-Af-Gm-Um-Gm-Um-G5 | 533/6 | AmsEVP-Cfs-Am-Cm-Um-Uf-Um-Um-Um-Um-Gm-Um-Um-Uf-Cm-Af-Cm-Am-Am-Am-Cms-Ams-Am | 468/19 |
| D789-DV26Pd7BG5 | Gms-Ums-Um-Um-Gm-Um-Gf-Am-Af-Af-Cf-Am-Am-Am-Ad-Am-Af-Gm-Um-Gm-Um-G5 | 534/6 | AmsEVP-Cfs-Af-Cf-Um-Uf-Td-Um-Um-Um-Gm-Um-Um-Uf-Cm-Af-Cm-Am-Am-Am-Cms-Ams-Am | 572/19 |
| D789-DV25Pd7BG5 | Gms-Ums-Um-Um-Gm-Um-Gf-Am-Af-Af-Cf-Am-Am-Am-Ad-Am-Af-Gm-Um-Gm-Um-G5 | 534/6 | AmsEVP-Cfs-Am-Cm-Um-Uf-Td-Um-Um-Um-Gm-Um-Um-Uf-Cm-Af-Cm-Am-Am-Am-Cms-Ams-Am | 573/19 |
| D791-DV26PG5 | Ums-Ums-Gm-Um-Gm-Am-Af-Am-Cf-Af-Af-Am-Am-Am-Am-Gm-Uf-Gm-Um-Um-Am-G5 | 536/8 | UmsEVP-Afs-Af-Cf-Am-Cf-Um-Um-Um-Um-Um-Um-Gm-Uf-Um-Uf-Cm-Am-Cm-Am-Ams-Ams-Cm | 476/21 |
| D791-DV26Pd7BG5 | Ums-Ums-Gm-Um-Gm-Am-Af-Am-Cf-Af-Af-Am-Am-Am-Ad-Gm-Uf-Gm-Um-Um-Am-G5 | 537/8 | UmsEVP-Afs-Af-Cf-Am-Cf-Td-Um-Um-Um-Um-Um-Gm-Uf-Um-Uf-Cm-Am-Cm-Am-Ams-Ams-Cm | 576/21 |

(continued)

| Sequence ID | Sense strand 5'-3' | Modified sequence SEQ ID NO./ corresponding unmodified sequence SEQ ID NO. | Antisense strand 5'-3' | Modified sequence SEQ ID NO./ corresponding unmodified sequence SEQ ID NO. |
|---|---|---|---|---|
| D795-DV27PG5 | Gms-Ams-Am-Am-Cm-Am-Af-Am-Af-Af-Af-Gm-Um-Gm-Um-Um-Cf-Cm-Cm-Um-Um-G5 | 539/9 | AmsEVP-Afs-Gm-Gf-Gf-Af-Am-Cm-Am-Cm-Um-Um-Um-Uf-Um-Uf-Gm-Um-Um-Um-Cms-Ams-Cm | 479/22 |
| D812-DV25PG5 | Cms-Cms-Um-Um-Um-Um-Cf-Am-Af-Gf-Uf-Um-Gm-Am-Gm-Am-Af-Cm-Am-Am-Am-G5 | 541/3 | UmsEVP-Ufs-Um-Gm-Um-Uf-Cm-Um-Cm-Am-Am-Cm-Um-Uf-Gm-Af-Am-Am-Am-Gm-Gms-Gms-Am | 450/16 |
| D816-DV25PG5 | Ums-Ums-Cm-Am-Am-Gm-Uf-Um-Gf-Af-Gf-Am-Am-Cm-Am-Am-Af-Am-Am-Um-Um-G5 | 543/2 | AmsEVP-Afs-Um-Um-Um-Uf-Um-Gm-Um-Um-Cm-Um-Cm-Af-Am-Cf-Um-Um-Gm-Am-Ams-Ams-Am | 443/15 |
| D835-DV26PG5 | Ums-Ums-Gm-Gm-Gm-Um-Uf-Um-Uf-Af-Af-Am-Am-Um-Um-Am-Af-Am-Gm-Um-Am-G5 | 545/1 | UmsEVP-Afs-Cf-Uf-Um-Uf-Am-Am-Um-Um-Um-Um-Am-Af-Am-Af-Cm-Cm-Cm-Am-Ams-Ums-Um | 433/14 |
| D835-DV26Pd7BG5 | Ums-Ums-Gm-Gm-Gm-Um-Uf-Um-Uf-Af-Af-Am-Am-Um-Td-Am-Af-Am-Gm-Um-Am-G5 | 546/1 | UmsEVP-Afs-Cf-Uf-Um-Uf-Ad-Am-Um-Um-Um-Um-Am-Af-Am-Af-Cm-Cm-Cm-Am-Ams-Ums-Um | 565/14 |
| D838-DV29PG5 | Gms-Gms-Um-Um-Um-Um-Af-Am-Af-Am-Uf-Um-Am-Am-Am-Gm-Uf-Am-Um-Am-Am-G5 | 548/13 | UmsEVP-Ufs-Am-Uf-Af-Cf-Um-Um-Um-Am-Am-Um-Um-Uf-Um-Af-Am-Am-Am-Cm-Cms-Cms-Am | 496/26 |
| D838-DV29Pd7BG5 | Gms-Gms-Um-Um-Um-Um-Af-Am-Af-Am-Uf-Um-Am-Am-Ad-Gm-Uf-Am-Um-Am-Am-G5 | 549/13 | UmsEVP-Ufs-Am-Uf-Af-Cf-Td-Um-Um-Am-Am-Um-Um-Uf-Um-Af-Am-Am-Am-Cm-Cms-Cms-Am | 581/26 |
| D839-DV29PG5 | Gms-Ums-Um-Um-Um-Am-Af-Am-Af-Um-Uf-Am-Am-Am-Gm-Um-Af-Um-Am-Cm-Am-G5 | 551/7 | UmsEVP-Gfs-Um-Af-Uf-Af-Cm-Um-Um-Um-Am-Am-Um-Uf-Um-Uf-Am-Am-Am-Am-Cms-Cms-Cm | 471/20 |

(continued)

| Sequence ID | Sense strand 5'-3' | Modified sequence SEQ ID NO./ corresponding unmodified sequence SEQ ID NO. | Antisense strand 5'-3' | Modified sequence SEQ ID NO./ corresponding unmodified sequence SEQ ID NO. |
|---|---|---|---|---|
| D839-DV29Pd7BG5 | Gms-Ums-Um-Um-Um-Am-Af-Am-Af-Um-Uf-Am-Am-Am-Gd-Um-Af-Um-Am-Cm-Am-G5 | 552/7 | UmsEVP-Gfs-Um-Af-Uf-Af-Cd-Um-Um-Um-Am-Am-Um-Uf-Um-Uf-Am-Am-Am-Am-Cms-Cms-Cm | 583/20 |
| D576s-DV29PG5 | Cms-Cms-Am-Cm-Cm-Uf-Um-Uf-Um-Cf-Um-Um-Cm-Um-Am-Af-Um-Gm-Am-Am-G5 | 554/11 | UmsEVP-Ufs-Cm-Af-Uf-Uf-Am-Gm-Am-Am-Gm-Am-Am-Af-Am-Gf-Gm-Um-Gm-Gms-Gms-Am | 488/24 |
| D576s-DV29Pd7BG5 | Cms-Cms-Am-Cm-Cm-Uf-Um-Uf-Um-Cf-Um-Um-Cm-Td-Am-Af-Um-Gm-Am-Am-G5 | 555/11 | UmsEVP-Ufs-Cm-Af-Uf-Uf-Ad-Gm-Am-Am-Gm-Am-Am-Af-Am-Gf-Gm-Um-Gm-Gms-Gms-Am | 585/24 |
| D578s-DV29PG5 | Ams-Cms-Cm-Um-Um-Uf-Um-Cf-Um-Uf-Cm-Um-Am-Am-Um-Gf-Am-Gm-Um-Am-G5 | 557/12 | UmsEVP-Afs-Cm-Uf-Cf-Af-Um-Um-Am-Gm-Am-Am-Gm-Af-Am-Af-Am-Gm-Gm-Ums-Gms-Gm | 492/25 |
| D578s-DV29Pd7BG5 | Ams-Cms-Cm-Um-Um-Uf-Um-Cf-Um-Uf-Cm-Um-Am-Ad-Um-Gf-Am-Gm-Um-Am-G5 | 558/12 | UmsEVP-Afs-Cm-Uf-Cf-Af-Td-Um-Am-Gm-Am-Am-Gm-Af-Am-Af-Am-Gm-Gm-Ums-Gms-Gm | 569/25 |
| D585s-DV27PG5 | Cms-Ums-Um-Cm-Um-Af-Am-Uf-Gf-Af-Gm-Um-Cm-Gm-Am-Cf-Um-Um-Um-Am-G5 | 560/10 | UmsEVP-Afs-Am-Af-Gf-Uf-Cm-Gm-Am-Cm-Um-Cm-Am-Uf-Um-Af-Gm-Am-Am-Gms-Ams-Am | 483/23 |
| Zilebesiran | Gms-Ums-Cm-Am-Um-Cm-Cf-Am-Cf-Af-Af-Um-Gm-Am-Gm-Am-Gm-Um-Am-Cm-Am-GL | 561/507 | Ums-Gfs-Um-Am-Cm-Tgna-Cm-Um-Cm-Am-Um-Um-Gm-Uf-Gm-Gf-Am-Um-Gm-Am-Cms-Gms-Am | 587/520 |
| DNC-DV29PG5 | Gms-Ums-Gm-Am-Am-Am-Cf-Am-Af-Cm-Uf-Am-Gm-Um-Gm-Cm-Af-Cm-Cm-Um-Cm-G5 | 562/NA | UmsEVP-Afs-Gm-Af-Gf-Gf-Um-Gm-Cm-Am-Cm-Um-Am-Gf-Um-Uf-Gm-Um-Um-Um-Cms-Ams-Cm | 588/NA |

**Preparation of test articles:**

Drug vehicle: Saline
Preparation conditions: Aseptic environment
Labeling method: Each prepared dosing formulation was labeled with tags, with the outer packaging indicating the study number, name, concentration, quantity, preparation date, preparer, and storage conditions.
Storage conditions: Prepared immediately before use; remaining samples were stored at -80°C.

**Information on experimental animals:**

Species/Strain: hAGT transgenic mice
Grade: SPF
Sex: Male
Quantity: 150
Age: 6 to 8 weeks
Weight: 18 to 28 g
Source: Jiangsu GemPharmatech Co., Ltd.
Production license No.: SCXK (Su) 2018-0008

**Review by Institutional Animal Care and Use Committee (IACUC):**

After reception, the experimental animals were housed at Beijing BrightShines Technology Co., Ltd. under a project license No.: SYXK (Jing) 2022-0025. The project has been reviewed and approved by the IACUC of Beijing BrightShines Technology Co., Ltd. The experimental procedures were strictly conducted in compliance with IACUC requirements to ensure animal welfare.

**Housing and management:**

Housing conditions: After reception, the experimental animals were housed at Beijing BrightShines Technology Co., Ltd. under a project license No.: SYXK (Jing) 2022-0025. They were housed in rearing cages with dimensions of 29.0 cm (L) × 18.5 cm (W) × 13.0 cm (H). The environmental parameters were set as follows: a temperature ranging from 20°C to 26°C, a humidity ranging from 40% to 70%, a ventilation rate of at least 15 fresh air changes per hour, and a 12-hour light/12-hour dark cycle with artificial lighting.
The environmental conditions complied with the National Standard GB14925-2010 of the People's Republic of China, with environmental control provided by a modular air-conditioning system.
The animals had free access to food and water. Water bottles and drinking water in the bottles were replaced at least twice weekly. Used water bottles were sterilized in a pulse vacuum autoclave before reuse.
The cages and bedding were replaced at least once weekly. All cages and bedding were sterilized in a pulse vacuum autoclave before being introduced into a barrier environment. Cage racks were cleaned and disinfected at least once weekly.
Animal observation rooms were cleaned and disinfected daily, including shelves, floors, work surfaces, *etc.*
Disinfectants used in the barrier environment included: 6.67% benzalkonium bromide solution, 0.5% 84 disinfectant, 75% ethanol, and 0.08% didecyldimethylammonium bromide. These four disinfectants should be used alternately and should not be used in combination.
Animal feed: SPF rodent maintenance feed: produced by SPF (Beijing) Biotechnology Co., Ltd.; production license No.: SCXK (Jing) 2019-0010, issued by the Beijing Municipal Science and Technology Commission.
Feed testing: Each batch of feed had a quality certificate. Microbial testing was conducted quarterly by the company. A recent third-party feed inspection report was provided semiannually by the supplier. Nutrient composition testing complied with the National Standard GB14924.3-2010 of the People's Republic of China, while contaminant testing complied with the National Standard GB14924.2-2001 of the People's Republic of China.
Animal drinking water: Drinking water: sterile water prepared by a filtration system, directly filled into drinking bottles.
Drinking water testing: Microbial testing was conducted quarterly by the company. Water quality testing was conducted annually by a third-party testing agency. Drinking water testing complied with the National Standard

GB5749-2006 of the People's Republic of China.

Animal bedding: Corncob bedding: produced by SPF (Beijing) Biotechnology Co., Ltd.; production license No.: SCXK (Jing) 2019-0004, issued by the Beijing Municipal Science and Technology Commission.

Bedding testing: Microbial testing was conducted quarterly by the company. At least one recent third-party bedding inspection report was provided semiannually by the supplier. Bedding testing complied with the National Standard GB14924.2-2001 of the People's Republic of China.

### 2. Experimental methods

### Dose design and grouping

Definition of test date: The day on which the animals were administered with the vehicle or test article was defined as Day 0.

Grouping and dosing: After 3 days of acclimation, the experimental animals were randomly divided into a negative control group and a test article group based on AGT protein levels in serum, with 6 animals per group. A single subcutaneous injection was administered at a dose of 3 mg/kg, with a volume of 5 mL/kg and a concentration of 0.6 mg/mL. The day of dosing was recorded as Day 0.

Individual animals were identified by ear tags. Cages were identified by hanging cage cards. A laboratory signage was hung at the entrance of the laboratory. Grouping information is detailed in Table 40 below:

Table 40: Grouping information

| Group | Drug | Dose (mg/kg) | Mode/frequency of administration | Period (days) | Number of animals |
|---|---|---|---|---|---|
| Negative control group | Vehicle | / | / | 56 | 6 |
| Test article group | siRNA | 3 | s.c./once | 56 | 6 |

### Measurement indicators

### (1) General observation

Observations were conducted once daily from one week prior to dosing until the end of the experiment.

Observation items: cage-side observations for mortality or moribund state, mental status, behavioral activities, fecal characteristics, supply of feed and water, *etc.*

Test animals: all animals in both the negative control and test article groups.

### (2) Expression of AGT protein in serum

Time points for measurement: Day -3 (prior to dosing), Day 7 (1 week post-dosing), Day 14 (2 weeks post-dosing), Day 21 (3 weeks post-dosing), Day 28 (4 weeks post-dosing), and Day 35 (5 weeks post-dosing).

Method for AGT protein level measurement: measured using an ELISA kit; repeated freeze-thaw cycles of serum samples were avoided.

Test animals: all animals in both the negative control and test article groups.

### (3) Data processing and statistical analysis

Experimental data were expressed as mean ± standard deviation (Mean ± SD), and data analysis was performed using GraphPad Prism 8.3 software. Statistical analysis was conducted using two-way ANOVA and post hoc tests, with LSD test for homogeneity of variance and Dunnett T3 test for heterogeneity of variance. $P < 0.05$ was considered statistically significant.

### 3. Experimental results

The specific experimental results are shown in Table 41.

It can be seen that these sequences can continuously and significantly inhibit the expression of AGT protein in serum. For example, D576s-DV29PG5 exhibited inhibition rates of 84.09%, 82.11%, and 85.25% on Day 7, Day 14, and Day 28, respectively.

**(1) The** sequences **of the present disclosure, such as unmodified** sequences **1579, 1789, 1812, 1576s, 1578s, and 1585s with different modifications, exhibited significant inhibitory effects on the expression of AGT protein in serum. For example, D576s-DV29PG5 exhibited inhibition rates of 84.09%, 82.11%, and 85.25% on Day 7, Day 14, and Day 21, respectively (FIG. 8).**

Table 41: Inhibition rates of different sequences on AGT protein in serum

| **Unmodified sequence ID** | **Sequence ID** | **Inhibition rate (%)** | | | | | | | |
|---|---|---|---|---|---|---|---|---|---|
| | | **Day 7** | **Day 14** | **Day 21** | **Day 28** | **Day 35** | **Day 42** | **Day 49** | **Day 56** |
| | Saline | -12.05 | -4.17 | -14.50 | -17.18 | -6.21 | -5.65 | -13.98 | -8.02 |
| | DNC-DV29PG5 | 4.14 | -17.92 | 9.78 | 3.04 | | | | |
| | Zilebesiran | 72.52 | 67.96 | 74.49 | 70.53 | 57.13 | 55.50 | 40.41 | 29.90 |
| 1576s | D576s-DV29Pd7BG5 | 75.91 | 61.16 | 54.05 | 32.94 | | | | |
| 1576s | D576s-DV29PG5 | 84.09 | 82.11 | 85.25 | 84.61 | 83.67 | 82.63 | 76.08 | 74.43 |
| 1578s | D578s-DV29Pd7BG5 | 81.76 | 80.52 | 83.37 | 80.74 | 75.71 | 75.38 | 67.58 | 59.76 |
| 1578s | D578s-DV29PG5 | 84.16 | 81.73 | 85.02 | 83.70 | | | | |
| 1579 | D579-DV25PG5 | 80.18 | 82.30 | 80.62 | 78.42 | 78.42 | 79.22 | 74.17 | 70.95 |
| 1579 | D579-DV25Pd7BG5 | 82.45 | 83.37 | 81.28 | 80.19 | 76.74 | 76.95 | 69.05 | 64.51 |
| 1585s | D585s-DV27PG5 | 80.37 | 76.55 | 77.88 | 75.02 | 65.47 | 60.63 | 41.16 | 29.04 |
| 1789 | D789-DV25Pd7BG5 | 63.75 | 64.84 | 51.52 | 40.12 | | | | |
| 1789 | D789-DV26Pd7BG5 | 72.49 | 70.00 | 55.31 | 42.63 | | | | |
| 1789 | D789-DV25PG5 | 69.90 | 73.61 | 69.45 | 61.94 | | | | |
| 1789 | D789-DV26PG5 | 74.52 | 77.51 | 72.40 | 68.56 | 63.15 | 60.50 | 47.62 | 37.35 |
| 1791 | D791-DV26Pd7BG5 | 74.54 | 70.80 | 62.16 | 49.77 | | | | |
| 1791 | D791-DV26PG5 | 73.28 | 73.36 | 66.01 | 59.54 | | | | |
| 1795 | D795-DV27PG5 | 60.21 | 60.31 | 62.70 | 55.58 | | | | |
| 1812 | D812-DV25PG5 | 81.87 | 81.67 | 82.22 | 82.83 | 81.77 | 81.17 | 76.58 | 72.98 |
| 1816 | D816-DV25PG5 | 73.58 | 72.79 | 63.17 | 60.38 | | | | |
| 1835 | D835-DV26Pd7BG5 | 52.91 | 35.34 | 18.61 | 25.60 | | | | |

(continued)

| Unmodified sequence ID | Sequence ID | Inhibition rate (%) | | | | | | | |
|---|---|---|---|---|---|---|---|---|---|
| | | Day 7 | Day 14 | Day 21 | Day 28 | Day 35 | Day 42 | Day 49 | Day 56 |
| 1835 | D835-DV26PG5 | 65.38 | 63.25 | 59.95 | 59.09 | | | | |
| 1838 | D838-DV29Pd7BG5 | 69.83 | 64.03 | 59.38 | 56.69 | | | | |
| 1838 | D838-DV29PG5 | 69.85 | 73.02 | 73.01 | 70.69 | | | | |
| 1839 | D839-DV29PG5 | 67.89 | 62.97 | 65.43 | 63.36 | | | | |
| 1839 | D839-DV29Pd7BG5 | 75.62 | 72.52 | 73.96 | 67.02 | | | | |

As shown in Table 41, the conjugates formed by conjugation of these sequences with GalNAc compounds exhibited significant inhibitory effects on the expression of AGT protein in serum. For example, D576s-DV29PG5 exhibited inhibition rates of 84.09%, 82.11%, and 85.25% on Day 7, Day 14, and Day 21, respectively (FIG. 9).

The results demonstrated that the unmodified sequences designed in the present disclosure, such as 1579, 1789, 1812, 1576s, 1578s, and 1585s, when subjected to the template modifications of the present disclosure, optionally in combination with anti-off-target designs, and conjugated with GalNAc compounds, can be efficiently delivered to the liver in animals and can significantly inhibit AGT gene expression.

**Summary:**

The unmodified sequences designed in the present disclosure, such as 1579, 1789, 1812, 1576s, 1578s, and 1585s, when subjected to the template modifications of the present disclosure, optionally in combination with anti-off-target designs, and conjugated with GalNAc compounds, can be efficiently delivered to the liver in animals and can continuously and significantly inhibit AGT gene expression.

For example, D576s-DV29PG5 exhibited inhibition rates of 84.09%, 82.11%, and 85.25% on Day 7, Day 14, and Day 28, respectively.

**Conclusion:**

The present disclosure designed a series of siRNAs based on the AGT mRNA sequence, which were alternately modified or modified using a specific set of modification templates, and some of the sequences were subjected to anti-off-target designs. The results demonstrated that:

(1) A total of 13 unmodified sequences exhibited significant inhibitory effects on the AGT gene, with inhibition rates exceeding 45%, including 1576s, 1579, 1812, 1578s, 1789, 1835, 1838, 1795, 1585s, 1816, 1791, 1836, and 1839.
(2) The multiply modified sequences exhibit an inhibition rate of up to 70% or higher. Moreover, the modified sequences conjugated with GalNAc compounds can be efficiently delivered to the liver in animals and can significantly inhibit AGT gene expression.

The details are as follows:

**1. The alternately modified sequences of the present disclosure exhibited significant inhibitory effects on the AGT gene.**

(1) Among the 99 designed sequences, 33 sequences exhibited significant inhibitory effects on the AGT gene, with inhibition rates exceeding 40%. Specifically, 10 sequences exhibited inhibition rates exceeding 60%.
(2) A total of 23 sequences exhibited inhibition rates ranging from 25% to 40% on the AGT gene. For example, the

inhibition rates of B734-AL and B994-AL were 39.2% and 37.3%, respectively.
(3) A total of 43 sequences exhibited inhibition rates below 25% on the AGT gene. For example, the inhibition rate of B1017-AL was only 23.1%.
(4) siRNAs with similar sequences exhibited significant differences in activity. For example, the inhibition rate of B1365-AL was 40.4% higher than that of B1367-AL, indicating a significant improvement, while the two sequences differed by only 2 terminal bases; the inhibition rate of B 1816-AL was 30.5% higher than that of B1815-AL, indicating a significant improvement, while the two sequences differed by only 1 terminal base. Therefore, screening oligonucleotide sequences with significant inhibitory activity from a vast number of candidate sequences designed for AGT mRNA sequences is not straightforward and requires substantial creative effort.

**2. The unmodified sequences of the present disclosure exhibited significant inhibitory effects on the AGT gene.**

(1) A total of 13 unmodified sequences exhibited significant inhibitory effects on the AGT gene, with inhibition rates exceeding 45%, including 1576s, 1579, 1812, 1578s, 1789, 1835, 1838, 1795, 1585s, 1816, 1791, 1836, and 1839. Specifically, 1576s, 1578s, 1812, 1579, 1789, 1835, and 1838 exhibited inhibition rates exceeding 50%.
(2) The other unmodified sequences exhibited inhibition rates below 45% on the AGT gene. For example, the inhibition rates of 731 and 1011 were only 1.3% and 2.1%, respectively.
(3) siRNAs with similar sequences exhibited significant differences in activity. For example, the inhibition rate of unmodified sequence 734 was 41.7% higher than that of unmodified sequence 731, indicating a significant improvement.
(4) The effects of alternating modifications on activity varied across different sequences. For some sequences, the inhibition rate was significantly improved, e.g., unmodified sequence 731 exhibited a 25.1% increase in inhibition rate after alternating modification. For others, the improvement was minimal, e.g., unmodified sequences 994, 1279, and 1591 exhibited virtually no difference in inhibition rate between alternately modified and unmodified sequences.

**3. The sequences modified using the modification templates of the present disclosure exhibited significant inhibitory effects on the AGT gene.**

(1) The screened unmodified sequences 1576s, 1578s, 1838, 1835, 1816, 1812, 1579, 1836, 1789, 1839, 1791, 1795, and 1585s, when modified with templates DV25P-29P designed in the present disclosure, exhibited significant inhibitory effects on AGT gene expression, with inhibition rates of 40% or more. Specifically, C1812-DV25P, C1579-DV33P, and C1789-DV26P achieved inhibition rates of 74.4%, 74.0%, and 73.6%, respectively.
(2) The 12 sequences modified with templates DV25P-29P of the present disclosure showed a significant improvement in inhibition rate on AGT gene expression as compared to the alternately modified sequences. For example, the inhibition rate of unmodified sequence 1812 modified with template DV25P (C1579-DV33P) was 11.5% higher than that of the alternately modified sequence, while the inhibition rate of unmodified sequence 1791 modified with template DV26P (C1791-DV26P) was 8.5% higher than that of the alternately modified sequence.
(3) The same sequences modified with templates DV25-29P of the present disclosure exhibited significantly higher inhibition rates on AGT gene expression compared to modification with the templates disclosed in the prior art. For example, unmodified sequence 1579 modified with template DV25P of the present disclosure exhibited a 16.6% increase in inhibition rate compared to the sequence modified with the previously published Advanced ESC template DV22.
(4) The same sequences modified with templates DV25-29P of the present disclosure exhibited significantly higher inhibition rates on AGT gene expression compared to modification with templates DV32-34P of the present disclosure. For example, unmodified sequence 1812 modified with template DV25P of the present disclosure exhibited a 20.2% increase in inhibition rate compared to the sequence modified with template DV33P of the present disclosure.
(5) The activity of sequences modified with different templates varied greatly. For example, when unmodified sequence 1816 was modified with DV29P, the inhibition rate increased by 30.0% compared to modification with DV34; when unmodified sequence 1579 was modified with DV33P, the inhibition rate increased by 29.8% compared to modification with DV28P. Thus, it is uncertain which modification template would confer high activity to an siRNA sequence.
(6) The $IC_{50}$ values of these 24 sequences ranged from 1.884 pM to 61.498 pM. Specifically, the $IC_{50}$ values of C1789-

DV25P, C1812-DV25P, and C1789-DV26P were 1.884 pM, 2.160 pM, and 2.643 pM, respectively. The results demonstrated that these sequences effectively inhibited AGT gene expression at low concentrations.

**4. The sequences modified by alternating modifications and the modification templates of the present disclosure exhibited significantly enhanced AGT inhibitory activity compared to the unmodified sequences with minimal differences disclosed in the prior art.**

Compared to the unmodified sequences with minimal differences disclosed in the prior art, the unmodified sequences, alternately modified sequences, and sequences modified with specific modification templates of the present disclosure exhibited significantly higher inhibition rates on AGT gene expression, with an increase of up to 91%.

I. The unmodified sequences of the present disclosure exhibited significantly enhanced activity compared to the structurally similar unmodified sequences disclosed in the prior art. For example, unmodified sequence 1835 of the present disclosure exhibited a 37.7% increase in inhibition rate compared to the structurally similar 835P.

II. The alternately modified sequences of the present disclosure exhibited significantly enhanced activity compared to the structurally similar unmodified sequences disclosed in the prior art. For example, the alternately modified sequence B1812-AL of the present disclosure exhibited a 20.4% increase in inhibition rate compared to sequence 812P.

III. The sequences modified using the modification templates of the present disclosure exhibited significantly enhanced activity compared to the structurally similar unmodified sequences disclosed in the prior art. For example, the alternately modified sequence C1576s-DV29P of the present disclosure exhibited a 41.1% increase in inhibition rate compared to sequence 576P.

**5. The alternately modified sequences and template-modified sequences of the present disclosure, when incorporating specific anti-off-target designs, exhibited significant inhibitory effects on the AGT gene, thereby significantly reducing the inhibition of off-target genes.**

(1) All modified sequences exhibited significant inhibitory effects on the AGT gene. For example, the template-modified sequence D576s-DV29P exhibited an inhibition rate of 83.8%, while the sequence D576s-DV29Pd7B with both template and anti-off-target modifications exhibited an inhibition rate of 85.4%.

(2) After using any one or more of the various modification patterns of the present disclosure, followed by anti-off-target design, the sequences exhibited significant anti-off-target effects on certain off-target genes, thereby reducing the inhibition of off-target genes, *i.e.*, exhibiting anti-off-target effects, with a decrease of up to 72.535% in inhibition rate on off-target genes.

**6. The sequences modified using the modification templates of the present disclosure significantly inhibited the expression of AGT in mouse serum.**

The unmodified sequences designed in the present disclosure, such as 1579, 1789, 1812, 1576s, 1578s, and 1585s, when subjected to the template modifications of the present disclosure, optionally in combination with anti-off-target designs, and conjugated with GalNAc compounds, can be efficiently delivered to the liver in animals and can continuously and significantly inhibit AGT gene expression.

For example, D576s-DV29PG5 exhibited inhibition rates of 84.09%, 82.11%, and 85.25% on Day 7, Day 14, and Day 21, respectively.

**Sequence listing of the present disclosure**

| Sequence ID | Sense strand sequence 5'-3' | SEQ ID NO. | Antisense strand sequence 5'-3' | SEQ ID NO. |
|---|---|---|---|---|
| 1835 | UUGGGUUUUAAAAUUAAAGUA | 1 | UACUUUAAUUUUAAAACCCAAUU | 14 |
| 1816 | UUCAAGUUGAGAACAAAAAUU | 2 | AAUUUUUGUUCUCAACUUGAAAA | 15 |
| 1812 | CCUUUUCAAGUUGAGAACAAA | 3 | UUUGUUCUCAACUUGAAAAGGGA | 16 |
| 1579 | CCUUUUCUUCUAAUGAGUCGA | 4 | UCGACUCAUUAGAAGAAAAGGUG | 17 |
| 1836 | UGGGUUUUAAAAUUAAAGUAU | 5 | AUACUUUAAUUUUAAAACCCAAU | 18 |
| 1789 | GUUUGUGAAACAAAAAAGUGU | 6 | ACACUUUUUUGUUUCACAAACAA | 19 |
| 1839 | GUUUUAAAAUUAAAGUAUACA | 7 | UGUAUACUUUAAUUUUAAAACCC | 20 |
| 1791 | UUGUGAAACAAAAAAGUGUUA | 8 | UAACACUUUUUUGUUUCACAAAC | 21 |
| 1795 | GAAACAAAAAAGUGUUCCCUU | 9 | AAGGGAACACUUUUUUGUUUCAC | 22 |
| 1585s | CUUCUAAUGAGUCGACUUUA | 10 | UAAAGUCGACUCAUUAGAAGAA | 23 |
| 1576s | CCACCUUUUCUUCUAAUGAA | 11 | UUCAUUAGAAGAAAAGGUGGGA | 24 |
| 1578s | ACCUUUUCUUCUAAUGAGUA | 12 | UACUCAUUAGAAGAAAAGGUGG | 25 |
| 1838 | GGUUUUAAAAUUAAAGUAUAA | 13 | UUAUACUUUAAUUUUAAAACCCA | 26 |
| 1008 | CUUCACUGAGAGCGCCUGCCU | 27 | AGGCAGGCGCUCUCAGUGAAGGG | 113 |
| 1011 | CACUGAGAGCGCCUGCCUGCU | 28 | AGCAGGCAGGCGCUCUCAGUGAA | 114 |
| 1014 | UGAGAGCGCCUGCCUGCUGCU | 29 | AGCAGCAGGCAGGCGCUCUCAGU | 115 |
| 1016 | AGAGCGCCUGCCUGCUGCUGA | 30 | UCAGCAGCAGGCAGGCGCUCUCA | 116 |
| 1017 | GAGCGCCUGCCUGCUGCUGAU | 31 | AUCAGCAGCAGGCAGGCGCUCUC | 117 |
| 1020 | CGCCUGCCUGCUGCUGAUCCA | 32 | UGGAUCAGCAGCAGGCAGGCGCU | 118 |
| 1024 | UGCCUGCUGCUGAUCCAGCCU | 33 | AGGCUGGAUCAGCAGCAGGCAGG | 119 |
| 1026 | CCUGCUGCUGAUCCAGCCUCA | 34 | UGAGGCUGGAUCAGCAGCAGGCA | 120 |
| 1028 | UGCUGCUGAUCCAGCCUCACU | 35 | AGUGAGGCUGGAUCAGCAGCAGG | 121 |
| 1029 | GCUGCUGAUCCAGCCUCACUA | 36 | UAGUGAGGCUGGAUCAGCAGCAG | 122 |
| 1030 | CUGCUGAUCCAGCCUCACUAU | 37 | AUAGUGAGGCUGGAUCAGCAGCA | 123 |
| 1038 | CCAGCCUCACUAUGCCUCUGA | 38 | UCAGAGGCAUAGUGAGGCUGGAU | 124 |
| 1041 | GCCUCACUAUGCCUCUGACCU | 39 | AGGUCAGAGGCAUAGUGAGGCUG | 125 |
| 1044 | UCACUAUGCCUCUGACCUGGA | 40 | UCCAGGUCAGAGGCAUAGUGAGG | 126 |

(continued)

| Sequence ID | Sense strand sequence 5'-3' | SEQ ID NO. | Antisense strand sequence 5'-3' | SEQ ID NO. |
|---|---|---|---|---|
| 1046 | ACUAUGCCUCUGACCUGGACA | 41 | UGUCCAGGUCAGAGGCAUAGUGA | 127 |
| 1047 | CUAUGCCUCUGACCUGGACAA | 42 | UUGUCCAGGUCAGAGGCAUAGUG | 128 |
| 1053 | CUCUGACCUGGACAAGGUGGA | 43 | UCCACCUUGUCCAGGUCAGAGGC | 129 |
| 1057 | GACCUGGACAAGGUGGAGGGU | 44 | ACCCUCCACCUUGUCCAGGUCAG | 130 |
| 1061 | UGGACAAGGUGGAGGGUCUCA | 45 | UGAGACCCUCCACCUUGUCCAGG | 131 |
| 1065s | CAAGGUGGAGGGUCUCACUU | 46 | AAGUGAGACCCUCCACCUUGUC | 132 |
| 1071 | GGAGGGUCUCACUUUCCAGCA | 47 | UGCUGGAAAGUGAGACCCUCCAC | 133 |
| 1260 | CAGGGUGGGGGAGGUGCUGAA | 48 | UUCAGCACCUCCCCCACCCUGAU | 134 |
| 1262 | GGGUGGGGGAGGUGCUGAACA | 49 | UGUUCAGCACCUCCCCCACCCUG | 135 |
| 1265 | UGGGGGAGGUGCUGAACAGCA | 50 | UGCUGUUCAGCACCUCCCCCACC | 136 |
| 1266 | GGGGGAGGUGCUGAACAGCAU | 51 | AUGCUGUUCAGCACCUCCCCCAC | 137 |
| 1267 | GGGGAGGUGCUGAACAGCAUU | 52 | AAUGCUGUUCAGCACCUCCCCCA | 138 |
| 1268 | GGGAGGUGCUGAACAGCAUUU | 53 | AAAUGCUGUUCAGCACCUCCCCC | 139 |
| 1279 | AACAGCAUUUUUUUUGAGCUU | 54 | AAGCUCAAAAAAAAUGCUGUUCA | 140 |
| 1281 | CAGCAUUUUUUUUGAGCUUGA | 55 | UCAAGCUCAAAAAAAAUGCUGUU | 141 |
| 1282 | AGCAUUUUUUUUGAGCUUGAA | 56 | UUCAAGCUCAAAAAAAAUGCUGU | 142 |
| 1362 | GGUGACCCUGAACCGCCCAUU | 57 | AAUGGGCGGUUCAGGGUCACCUC | 143 |
| 1365 | GACCCUGAACCGCCCAUUCCU | 58 | AGGAAUGGGCGGUUCAGGGUCAC | 144 |
| 1367 | CCCUGAACCGCCCAUUCCUGU | 59 | ACAGGAAUGGGCGGUUCAGGGUC | 145 |
| 1369 | CUGAACCGCCCAUUCCUGUUU | 60 | AAACAGGAAUGGGCGGUUCAGGG | 146 |
| 1393 | GUGUAUGAUCAAAGCGCCACU | 61 | AGUGGCGCUUUGAUCAUACACAG | 147 |
| 1401 | UCAAAGCGCCACUGCCCUGCA | 62 | UGCAGGGCAGUGGCGCUUUGAUC | 148 |
| 1404 | AAGCGCCACUGCCCUGCACUU | 63 | AAGUGCAGGGCAGUGGCGCUUUG | 149 |
| 1407 | CGCCACUGCCCUGCACUUCCU | 64 | AGGAAGUGCAGGGCAGUGGCGCU | 150 |
| 1554 | GCGAUGUGUCACCCCCAGUCU | 65 | AGACUGGGGGUGACACAUCGCUG | 151 |
| 1558 | UGUGUCACCCCCAGUCUCCCA | 66 | UGGGAGACUGGGGGUGACACAUC | 152 |

(continued)

| Sequence ID | Sense strand sequence 5'-3' | SEQ ID NO. | Antisense strand sequence 5'-3' | SEQ ID NO. |
|---|---|---|---|---|
| 1561 | GUCACCCCCAGUCUCCCACCU | 67 | AGGUGGGAGACUGGGGGUGACAC | 153 |
| 1562 | UCACCCCCAGUCUCCCACCUU | 68 | AAGGUGGGAGACUGGGGGUGACA | 154 |
| 1564 | ACCCCCAGUCUCCCACCUUUU | 69 | AAAAGGUGGGAGACUGGGGGUGA | 155 |
| 1591 | AUGAGUCGACUUUGAGCUGGA | 70 | UCCAGCUCAAAGUCGACUCAUUA | 156 |
| 1592 | UGAGUCGACUUUGAGCUGGAA | 71 | UUCCAGCUCAAAGUCGACUCAUU | 157 |
| 1593 | GAGUCGACUUUGAGCUGGAAA | 72 | UUUCCAGCUCAAAGUCGACUCAU | 158 |
| 1596 | UCGACUUUGAGCUGGAAAGCA | 73 | UGCUUUCCAGCUCAAAGUCGACU | 159 |
| 1602 | UUGAGCUGGAAAGCAGCCGUU | 74 | AACGGCUGCUUUCCAGCUCAAAG | 160 |
| 1603 | UGAGCUGGAAAGCAGCCGUUU | 75 | AAACGGCUGCUUUCCAGCUCAAA | 161 |
| 1608 | UGGAAAGCAGCCGUUUCUCCU | 76 | AGGAGAAACGGCUGCUUUCCAGC | 162 |
| 1612 | AAGCAGCCGUUUCUCCUUGGU | 77 | ACCAAGGAGAAACGGCUGCUUUC | 163 |
| 1775 | CAACCGACCAGCUUGUUUGUA | 78 | UACAAACAAGCUGGUCGGUUGGA | 164 |
| 1790 | UUUGUGAAACAAAAAAGUGUU | 79 | AACACUUUUUUGUUUCACAAACA | 165 |
| 1796 | AAACAAAAAAGUGUUCCCUUU | 80 | AAAGGGAACACUUUUUUGUUUCA | 166 |
| 1798s | ACAAAAAAGUGUUCCCUUUU | 81 | AAAAGGGAACACUUUUUUGUUU | 167 |
| 1799 | CAAAAAAGUGUUCCCUUUUCA | 82 | UGAAAAGGGAACACUUUUUUGUU | 168 |
| 1810 | UCCCUUUUCAAGUUGAGAACA | 83 | UGUUCUCAACUUGAAAAGGGAAC | 169 |
| 1811 | CCCUUUUCAAGUUGAGAACAA | 84 | UUGUUCUCAACUUGAAAAGGGAA | 170 |
| 1814 | UUUUCAAGUUGAGAACAAAAA | 85 | UUUUUGUUCUCAACUUGAAAAGG | 171 |
| 1815 | UUUCAAGUUGAGAACAAAAAU | 86 | AUUUUUGUUCUCAACUUGAAAAG | 172 |
| 1817 | UCAAGUUGAGAACAAAAAUUG | 87 | CAAUUUUUGUUCUCAACUUGAAA | 173 |
| 1828 | ACAAAAAUUGGGUUUUAAAAU | 88 | AUUUUAAAACCCAAUUUUUGUUC | 174 |
| 1838s | GGUUUUAAAAUUAAAGUAUA | 89 | UAUACUUUAAUUUUAAAACCCA | 175 |
| 2018 | AUAGCUGGUUAUUUCUCCCUU | 90 | AAGGGAGAAAUAACCAGCUAUGG | 176 |
| 2020 | AGCUGGUUAUUUCUCCCUUGU | 91 | ACAAGGGAGAAAUAACCAGCUAU | 177 |
| 2025 | GUUAUUUCUCCCUUGUGUUAA | 92 | UUAACACAAGGGAGAAAUAACCA | 178 |

(continued)

| Sequence ID | Sense strand sequence 5'-3' | SEQ ID NO. | Antisense strand sequence 5'-3' | SEQ ID NO. |
|---|---|---|---|---|
| 2030 | UUCUCCCUUGUGUUAGUAAUA | 93 | UAUUACUAACACAAGGGAGAAAU | 179 |
| 2031 | UCUCCCUUGUGUUAGUAAUAA | 94 | UUAUUACUAACACAAGGGAGAAA | 180 |
| 2032 | CUCCCUUGUGUUAGUAAUAAA | 95 | UUUAUUACUAACACAAGGGAGAA | 181 |
| 2033 | UCCCUUGUGUUAGUAAUAAAA | 96 | UUUUAUUACUAACACAAGGGAGA | 182 |
| 729 | AGAACUGGAUGUUGCUGCUGA | 97 | UCAGCAGCAACAUCCAGUUCUGU | 183 |
| 731 | AACUGGAUGUUGCUGCUGAGA | 98 | UCUCAGCAGCAACAUCCAGUUCU | 184 |
| 732 | ACUGGAUGUUGCUGCUGAGAA | 99 | UUCUCAGCAGCAACAUCCAGUUC | 185 |
| 734 | UGGAUGUUGCUGCUGAGAAGA | 100 | UCUUCUCAGCAGCAACAUCCAGU | 186 |
| 734s | UGGAUGUUGCUGCUGAGAAA | 101 | UUUCUCAGCAGCAACAUCCAGU | 187 |
| 736 | GAUGUUGCUGCUGAGAAGAUU | 102 | AAUCUUCUCAGCAGCAACAUCCA | 188 |
| 743 | CUGCUGAGAAGAUUGACAGGU | 103 | ACCUGUCAAUCUUCUCAGCAGCA | 189 |
| 753s | GAUUGACAGGUUCAUGCAGA | 104 | UCUGCAUGAACCUGUCAAUCUU | 190 |
| 963 | GCACUGGAGUGACAUCCAGGA | 105 | UCCUGGAUGUCACUCCAGUGCUG | 191 |
| 966 | CUGGAGUGACAUCCAGGACAA | 106 | UUGUCCUGGAUGUCACUCCAGUG | 192 |
| 969 | GAGUGACAUCCAGGACAACUU | 107 | AAGUUGUCCUGGAUGUCACUCCA | 193 |
| 971 | GUGACAUCCAGGACAACUUCU | 108 | AGAAGUUGUCCUGGAUGUCACUC | 194 |
| 982 | GACAACUUCUCGGUGACUCAA | 109 | UUGAGUCACCGAGAAGUUGUCCU | 195 |
| 984 | CAACUUCUCGGUGACUCAAGU | 110 | ACUUGAGUCACCGAGAAGUUGUC | 196 |
| 992 | CGGUGACUCAAGUGCCCUUCA | 111 | UGAAGGGCACUUGAGUCACCGAG | 197 |
| 994 | GUGACUCAAGUGCCCUUCACU | 112 | AGUGAAGGGCACUUGAGUCACCG | 198 |
| B1835-AL | Ufs-Ums-Gf-Gm-Gf-Um-Uf-Um-Uf-Am-Af-Am-Af-Um-Uf-Am-Af-Am-Gf-Um-Af | 199 | Ums-Afs-Cm-Uf-Um-Uf-Am-Af-Um-Uf-Um-Uf-Am-Af-Am-Af-Cm-Cf-Cm-Af-Ams-Ufs-Um | 212 |
| B1816-AL | Ufs-Ums-Cf-Am-Af-Gm-Uf-Um-Gf-Am-Gf-Am-Af-Cm-Af-Am-Af-Am-Af-Um-Uf | 200 | Ams-Afs-Um-Uf-Um-Uf-Um-Gf-Um-Uf-Cm-Uf-Cm-Af-Am-Cf-Um-Uf-Gm-Af-Ams-Afs-Am | 213 |

(continued)

| Sequence ID | Sense strand sequence 5'-3' | SEQ ID NO. | Antisense strand sequence 5'-3' | SEQ ID NO. |
|---|---|---|---|---|
| B1812-AL | Cfs-Cms-Uf-Um-Uf-Um-Cf-Am-Af-Gm-Uf-Um-Gf-Am-Gf-Am-Af-Cm-Af-Am-Af | 201 | Ums-Ufs-Um-Gf-Um-Uf-Cm-Uf-Cm-Af-Am-Cf-Um-Uf-Gm-Af-Am-Af-Am-Gf-Gms-Gfs-Am | 214 |
| B1579-AL | Cfs-Cms-Uf-Um-Uf-Um-Cf-Um-Uf-Cm-Uf-Am-Af-Um-Gf-Am-Gf-Um-Cf-Gm-Af | 202 | Ums-Cfs-Gm-Af-Cm-Uf-Cm-Af-Um-Uf-Am-Gf-Am-Af-Gm-Af-Am-Af-Am-Gf-Gms-Ufs-Gm | 215 |
| B1836-AL | Ufs-Gms-Gf-Gm-Uf-Um-Uf-Um-Af-Am-Af-Am-Uf-Um-Af-Am-Af-Gm-Uf-Am-Uf | 203 | Ams-Ufs-Am-Cf-Um-Uf-Um-Af-Am-Uf-Um-Uf-Um-Af-Am-Af-Am-Cf-Cm-Cf-Ams-Afs-Um | 216 |
| B1789-AL | Gfs-Ums-Uf-Um-Gf-Um-Gf-Am-Af-Am-Cf-Am-Af-Am-Af-Am-Af-Gm-Uf-Gm-Uf | 204 | Ams-Cfs-Am-Cf-Um-Uf-Um-Uf-Um-Uf-Gm-Uf-Um-Uf-Cm-Af-Cm-Af-Am-Af-Cms-Afs-Am | 217 |
| B1839-AL | Gfs-Ums-Uf-Um-Uf-Am-Af-Am-Af-Um-Uf-Am-Af-Am-Gf-Um-Af-Um-Af-Cm-Af | 205 | Ums-Gfs-Um-Af-Um-Af-Cm-Uf-Um-Uf-Am-Af-Um-Uf-Um-Uf-Am-Af-Am-Af-Cms-Cfs-Cm | 218 |
| B1791-AL | Ufs-Ums-Gf-Um-Gf-Am-Af-Am-Cf-Am-Af-Am-Af-Am-Af-Gm-Uf-Gm-Uf-Um-Af | 206 | Ums-Afs-Am-Cf-Am-Cf-Um-Uf-Um-Uf-Um-Uf-Gm-Uf-Um-Uf-Cm-Af-Cm-Af-Ams-Afs-Cm | 219 |
| B 1795-AL | Gfs-Ams-Af-Am-Cf-Am-Af-Am-Af-Am-Af-Gm-Uf-Gm-Uf-Um-Cf-Cm-Cf-Um-Uf | 207 | Ams-Afs-Gm-Gf-Gm-Af-Am-Cf-Am-Cf-Um-Uf-Um-Uf-Um-Uf-Gm-Uf-Um-Uf-Cms-Afs-Cm | 220 |
| B1585sAL | Cms-Ufs-Um-Cf-Um-Af-Am-Uf-Gm-Af-Gm-Uf-Cm-Gf-Am-Cf-Um-Uf-Um-Af | 208 | Ums-Afs-Am-Af-Gm-Uf-Cm-Gf-Am-Cf-Um-Cf-Am-Uf-Um-Af-Gm-Af-Am-Gfs-Ams-Af | 221 |

(continued)

| Sequence ID | Sense strand sequence 5'-3' | SEQ ID NO. | Antisense strand sequence 5'-3' | SEQ ID NO. |
|---|---|---|---|---|
| B1576sAL | Cms-Cfs-Am-Cf-Cm-Uf-Um-Uf-Um-Cf-Um-Uf-Cm-Uf-Am-Af-Um-Gf-Am-Af | 209 | Ums-Ufs-Cm-Af-Um-Uf-Am-Gf-Am-Af-Gm-Af-Am-Af-Am-Gf-Gm-Uf-Gm-Gfs-Gms-Af | 222 |
| B1578sAL | Ams-Cfs-Cm-Uf-Um-Uf-Um-Cf-Um-Uf-Cm-Uf-Am-Af-Um-Gf-Am-Gf-Um-Af | 210 | Ums-Afs-Cm-Uf-Cm-Af-Um-Uf-Am-Gf-Am-Af-Gm-Af-Am-Af-Am-Gf-Gm-Ufs-Gms-Gf | 223 |
| B1838-AL | Gfs-Gms-Uf-Um-Uf-Um-Af-Am-Af-Am-Uf-Um-Af-Am-Af-Gm-Uf-Am-Uf-Am-Af | 211 | Ums-Ufs-Am-Uf-Am-Cf-Um-Uf-Um-Af-Am-Uf-Um-Uf-Um-Af-Am-Af-Am-Cf-Cms-Cfs-Am | 224 |
| B1008-AL | Cfs-Ums-Uf-Cm-Af-Cm-Uf-Gm-Af-Gm-Af-Gm-Cf-Gm-Cf-Cm-Uf-Gm-Cf-Cm-Uf | 225 | Ams-Gfs-Gm-Cf-Am-Gf-Gm-Cf-Gm-Cf-Um-Cf-Um-Cf-Am-Gf-Um-Gf-Am-Af-Gms-Gfs-Gm | 311 |
| B1011-AL | Cfs-Ams-Cf-Um-Gf-Am-Gf-Am-Gf-Cm-Gf-Cm-Cf-Um-Gf-Cm-Cf-Um-Gf-Cm-Uf | 226 | Ams-Gfs-Cm-Af-Gm-Gf-Cm-Af-Gm-Gf-Cm-Gf-Cm-Uf-Cm-Uf-Cm-Af-Gm-Uf-Gms-Afs-Am | 312 |
| B1014-AL | Ufs-Gms-Af-Gm-Af-Gm-Cf-Gm-Cf-Cm-Uf-Gm-Cf-Cm-Uf-Gm-Cf-Um-Gf-Cm-Uf | 227 | Ams-Gfs-Cm-Af-Gm-Cf-Am-Gf-Gm-Cf-Am-Gf-Gm-Cf-Gm-Cf-Um-Cf-Um-Cf-Ams-Gfs-Um | 313 |
| B1016-AL | Afs-Gms-Af-Gm-Cf-Gm-Cf-Cm-Uf-Gm-Cf-Cm-Uf-Gm-Cf-Um-Gf-Cm-Uf-Gm-Af | 228 | Ums-Cfs-Am-Gf-Cm-Af-Gm-Cf-Am-Gf-Gm-Cf-Am-Gf-Gm-Cf-Gm-Cf-Um-Cf-Ums-Cfs-Am | 314 |
| B1017-AL | Gfs-Ams-Gf-Cm-Gf-Cm-Cf-Um-Gf-Cm-Cf-Um-Gf-Cm-Uf-Gm-Cf-Um-Gf-Am-Uf | 229 | Ams-Ufs-Cm-Af-Gm-Cf-Am-Gf-Cm-Af-Gm-Gf-Cm-Af-Gm-Gf-Cm-Gf-Cm-Uf-Cms-Ufs-Cm | 315 |

(continued)

| Sequence ID | Sense strand sequence 5'-3' | SEQ ID NO. | Antisense strand sequence 5'-3' | SEQ ID NO. |
|---|---|---|---|---|
| B1020-AL | Cfs-Gms-Cf-Cm-Uf-Gm-Cf-Cm-Uf-Gm-Cf-Um-Gf-Cm-Uf-Gm-Af-Um-Cf-Cm-Af | 230 | Ums-Gfs-Gm-Af-Um-Cf-Am-Gf-Cm-Af-Gm-Cf-Am-Gf-Gm-Cf-Am-Gf-Gm-Cf-Gms-Cfs-Um | 316 |
| B1024-AL | Ufs-Gms-Cf-Cm-Uf-Gm-Cf-Um-Gf-Cm-Uf-Gm-Af-Um-Cf-Cm-Af-Gm-Cf-Cm-Uf | 231 | Ams-Gfs-Gm-Cf-Um-Gf-Gm-Af-Um-Cf-Am-Gf-Cm-Af-Gm-Cf-Am-Gf-Gm-Cf-Ams-Gfs-Gm | 317 |
| B1026-AL | Cfs-Cms-Uf-Gm-Cf-Um-Gf-Cm-Uf-Gm-Af-Um-Cf-Cm-Af-Gm-Cf-Cm-Uf-Cm-Af | 232 | Ums-Gfs-Am-Gf-Gm-Cf-Um-Gf-Gm-Af-Um-Cf-Am-Gf-Cm-Af-Gm-Cf-Am-Gf-Gms-Cfs-Am | 318 |
| B1028-AL | Ufs-Gms-Cf-Um-Gf-Cm-Uf-Gm-Af-Um-Cf-Cm-Af-Gm-Cf-Cm-Uf-Cm-Af-Cm-Uf | 233 | Ams-Gfs-Um-Gf-Am-Gf-Gm-Cf-Um-Gf-Gm-Af-Um-Cf-Am-Gf-Cm-Af-Gm-Cf-Ams-Gfs-Gm | 319 |
| B 1029-AL | Gfs-Cms-Uf-Gm-Cf-Um-Gf-Am-Uf-Cm-Cf-Am-Gf-Cm-Cf-Um-Cf-Am-Cf-Um-Af | 234 | Ums-Afs-Gm-Uf-Gm-Af-Gm-Gf-Cm-Uf-Gm-Gf-Am-Uf-Cm-Af-Gm-Cf-Am-Gf-Cms-Afs-Gm | 320 |
| B1030-AL | Cfs-Ums-Gf-Cm-Uf-Gm-Af-Um-Cf-Cm-Af-Gm-Cf-Cm-Uf-Cm-Af-Cm-Uf-Am-Uf | 235 | Ams-Ufs-Am-Gf-Um-Gf-Am-Gf-Gm-Cf-Um-Gf-Gm-Af-Um-Cf-Am-Gf-Cm-Af-Gms-Cfs-Am | 321 |
| B1038-AL | Cfs-Cms-Af-Gm-Cf-Cm-Uf-Cm-Af-Cm-Uf-Am-Uf-Gm-Cf-Cm-Uf-Cm-Uf-Gm-Af | 236 | Ums-Cfs-Am-Gf-Am-Gf-Gm-Cf-Am-Uf-Am-Gf-Um-Gf-Am-Gf-Gm-Cf-Um-Gf-Gms-Afs-Um | 322 |
| B1041-AL | Gfs-Cms-Cf-Um-Cf-Am-Cf-Um-Af-Um-Gf-Cm-Cf-Um-Cf-Um-Gf-Am-Cf-Cm-Uf | 237 | Ams-Gfs-Gm-Uf-Cm-Af-Gm-Af-Gm-Gf-Cm-Af-Um-Af-Gm-Uf-Gm-Af-Gm-Gf-Cms-Ufs-Gm | 323 |

(continued)

| Sequence ID | Sense strand sequence 5'-3' | SEQ ID NO. | Antisense strand sequence 5'-3' | SEQ ID NO. |
|---|---|---|---|---|
| B1044-AL | Ufs-Cms-Af-Cm-Uf-Am-Uf-Gm-Cf-Cm-Uf-Cm-Uf-Gm-Af-Cm-Cf-Um-Gf-Gm-Af | 238 | Ums-Cfs-Cm-Af-Gm-Gf-Um-Cf-Am-Gf-Am-Gf-Gm-Cf-Am-Uf-Am-Gf-Um-Gf-Ams-Gfs-Gm | 324 |
| B1046-AL | Afs-Cms-Uf-Am-Uf-Gm-Cf-Cm-Uf-Cm-Uf-Gm-Af-Cm-Cf-Um-Gf-Gm-Af-Cm-Af | 239 | Ums-Gfs-Um-Cf-Cm-Af-Gm-Gf-Um-Cf-Am-Gf-Am-Gf-Gm-Cf-Am-Uf-Am-Gf-Ums-Gfs-Am | 325 |
| B1047-AL | Cfs-Ums-Af-Um-Gf-Cm-Cf-Um-Cf-Um-Gf-Am-Cf-Cm-Uf-Gm-Gf-Am-Cf-Am-Af | 240 | Ums-Ufs-Gm-Uf-Cm-Cf-Am-Gf-Gm-Uf-Cm-Af-Gm-Af-Gm-Gf-Cm-Af-Um-Af-Gms-Ufs-Gm | 326 |
| B1053-AL | Cfs-Ums-Cf-Um-Gf-Am-Cf-Cm-Uf-Gm-Gf-Am-Cf-Am-Af-Gm-Gf-Um-Gf-Gm-Af | 241 | Ums-Cfs-Cm-Af-Cm-Cf-Um-Uf-Gm-Uf-Cm-Cf-Am-Gf-Gm-Uf-Cm-Af-Gm-Af-Gms-Gfs-Cm | 327 |
| B1057-AL | Gfs-Ams-Cf-Cm-Uf-Gm-Gf-Am-Cf-Am-Af-Gm-Gf-Um-Gf-Gm-Af-Gm-Gf-Gm-Uf | 242 | Ams-Cfs-Cm-Cf-Um-Cf-Cm-Af-Cm-Cf-Um-Uf-Gm-Uf-Cm-Cf-Am-Gf-Gm-Uf-Cms-Afs-Gm | 328 |
| B1061-AL | Ufs-Gms-Gf-Am-Cf-Am-Af-Gm-Gf-Um-Gf-Gm-Af-Gm-Gf-Gm-Uf-Cm-Uf-Cm-Af | 243 | Ums-Gfs-Am-Gf-Am-Cf-Cm-Cf-Um-Cf-Cm-Af-Cm-Cf-Um-Uf-Gm-Uf-Cm-Cf-Ams-Gfs-Gm | 329 |
| B1065sAL | Cms-Afs-Am-Gf-Gm-Uf-Gm-Gf-Am-Gf-Gm-Gf-Um-Cf-Um-Cf-Am-Cf-Um-Uf | 244 | Ams-Afs-Gm-Uf-Gm-Af-Gm-Af-Cm-Cf-Cm-Uf-Cm-Cf-Am-Cf-Cm-Uf-Um-Gfs-Ums-Cf | 330 |
| B1071-AL | Gfs-Gms-Af-Gm-Gf-Gm-Uf-Cm-Uf-Cm-Af-Cm-Uf-Um-Uf-Cm-Cf-Am-Gf-Cm-Af | 245 | Ums-Gfs-Cm-Uf-Gm-Gf-Am-Af-Am-Gf-Um-Gf-Am-Gf-Am-Cf-Cm-Cf-Um-Cf-Cms-Afs-Cm | 331 |

(continued)

| Sequence ID | Sense strand sequence 5'-3' | SEQ ID NO. | Antisense strand sequence 5'-3' | SEQ ID NO. |
|---|---|---|---|---|
| B1260-AL | Cfs-Ams-Gf-Gm-Gf-Um-Gf-Gm-Gf-Gm-Gf-Am-Gf-Gm-Uf-Gm-Cf-Um-Gf-Am-Af | 246 | Ums-Ufs-Cm-Af-Gm-Cf-Am-Cf-Cm-Uf-Cm-Cf-Cm-Cf-Cm-Af-Cm-Cf-Cm-Uf-Gms-Afs-Um | 332 |
| B1262-AL | Gfs-Gms-Gf-Um-Gf-Gm-Gf-Gm-Gf-Am-Gf-Gm-Uf-Gm-Cf-Um-Gf-Am-Af-Cm-Af | 247 | Ums-Gfs-Um-Uf-Cm-Af-Gm-Cf-Am-Cf-Cm-Uf-Cm-Cf-Cm-Cf-Cm-Af-Cm-Cf-Cms-Ufs-Gm | 333 |
| B 1265-AL | Ufs-Gms-Gf-Gm-Gf-Gm-Af-Gm-Gf-Um-Gf-Cm-Uf-Gm-Af-Am-Cf-Am-Gf-Cm-Af | 248 | Ums-Gfs-Cm-Uf-Gm-Uf-Um-Cf-Am-Gf-Cm-Af-Cm-Cf-Um-Cf-Cm-Cf-Cm-Cf-Ams-Cfs-Cm | 334 |
| B1266-AL | Gfs-Gms-Gf-Gm-Gf-Am-Gf-Gm-Uf-Gm-Cf-Um-Gf-Am-Af-Cm-Af-Gm-Cf-Am-Uf | 249 | Ams-Ufs-Gm-Cf-Um-Gf-Um-Uf-Cm-Af-Gm-Cf-Am-Cf-Cm-Uf-Cm-Cf-Cm-Cf-Cms-Afs-Cm | 335 |
| B1267-AL | Gfs-Gms-Gf-Gm-Af-Gm-Gf-Um-Gf-Cm-Uf-Gm-Af-Am-Cf-Am-Gf-Cm-Af-Um-Uf | 250 | Ams-Afs-Um-Gf-Cm-Uf-Gm-Uf-Um-Cf-Am-Gf-Cm-Af-Cm-Cf-Um-Cf-Cm-Cf-Cms-Cfs-Am | 336 |
| B1268-AL | Gfs-Gms-Gf-Am-Gf-Gm-Uf-Gm-Cf-Um-Gf-Am-Af-Cm-Af-Gm-Cf-Am-Uf-Um-Uf | 251 | Ams-Afs-Am-Uf-Gm-Cf-Um-Gf-Um-Uf-Cm-Af-Gm-Cf-Am-Cf-Cm-Uf-Cm-Cf-Cms-Cfs-Cm | 337 |
| B 1279-AL | Afs-Ams-Cf-Am-Gf-Cm-Af-Um-Uf-Um-Uf-Um-Uf-Um-Uf-Gm-Af-Gm-Cf-Um-Uf | 252 | Ams-Afs-Gm-Cf-Um-Cf-Am-Af-Am-Af-Am-Af-Am-Af-Um-Gf-Cm-Uf-Gm-Uf-Ums-Cfs-Am | 338 |
| B1281-AL | Cfs-Ams-Gf-Cm-Af-Um-Uf-Um-Uf-Um-Uf-Um-Uf-Gm-Af-Gm-Cf-Um-Uf-Gm-Af | 253 | Ums-Cfs-Am-Af-Gm-Cf-Um-Cf-Am-Af-Am-Af-Am-Af-Am-Af-Um-Gf-Cm-Uf-Gms-Ufs-Um | 339 |

(continued)

| Sequence ID | Sense strand sequence 5'-3' | SEQ ID NO. | Antisense strand sequence 5'-3' | SEQ ID NO. |
|---|---|---|---|---|
| B1282-AL | Afs-Gms-Cf-Am-Uf-Um-Uf-Um-Uf-Um-Uf-Um-Gf-Am-Gf-Cm-Uf-Um-Gf-Am-Af | 254 | Ums-Ufs-Cm-Af-Am-Gf-Cm-Uf-Cm-Af-Am-Af-Am-Af-Am-Af-Am-Uf-Gm-Cf-Ums-Gfs-Um | 340 |
| B1362-AL | Gfs-Gms-Uf-Gm-Af-Cm-Cf-Cm-Uf-Gm-Af-Am-Cf-Cm-Gf-Cm-Cf-Cm-Af-Um-Uf | 255 | Ams-Afs-Um-Gf-Gm-Gf-Cm-Gf-Gm-Uf-Um-Cf-Am-Gf-Gm-Gf-Um-Cf-Am-Cf-Cms-Ufs-Cm | 341 |
| B 1365-AL | Gfs-Ams-Cf-Cm-Cf-Um-Gf-Am-Af-Cm-Cf-Gm-Cf-Cm-Cf-Am-Uf-Um-Cf-Cm-Uf | 256 | Ams-Gfs-Gm-Af-Am-Uf-Gm-Gf-Gm-Cf-Gm-Gf-Um-Uf-Cm-Af-Gm-Gf-Gm-Uf-Cms-Afs-Cm | 342 |
| B1367-AL | Cfs-Cms-Cf-Um-Gf-Am-Af-Cm-Cf-Gm-Cf-Cm-Cf-Am-Uf-Um-Cf-Cm-Uf-Gm-Uf | 257 | Ams-Cfs-Am-Gf-Gm-Af-Am-Uf-Gm-Gf-Gm-Cf-Gm-Gf-Um-Uf-Cm-Af-Gm-Gf-Gms-Ufs-Cm | 343 |
| B 1369-AL | Cfs-Ums-Gf-Am-Af-Cm-Cf-Gm-Cf-Cm-Cf-Am-Uf-Um-Cf-Cm-Uf-Gm-Uf-Um-Uf | 258 | Ams-Afs-Am-Cf-Am-Gf-Gm-Af-Am-Uf-Gm-Gf-Gm-Cf-Gm-Gf-Um-Uf-Cm-Af-Gms-Gfs-Gm | 344 |
| B 1393-AL | Gfs-Ums-Gf-Um-Af-Um-Gf-Am-Uf-Cm-Af-Am-Af-Gm-Cf-Gm-Cf-Cm-Af-Cm-Uf | 259 | Ams-Gfs-Um-Gf-Gm-Cf-Gm-Cf-Um-Uf-Um-Gf-Am-Uf-Cm-Af-Um-Af-Cm-Af-Cms-Afs-Gm | 345 |
| B1401-AL | Ufs-Cms-Af-Am-Af-Gm-Cf-Gm-Cf-Cm-Af-Cm-Uf-Gm-Cf-Cm-Cf-Um-Gf-Cm-Af | 260 | Ums-Gfs-Cm-Af-Gm-Gf-Gm-Cf-Am-Gf-Um-Gf-Gm-Cf-Gm-Cf-Um-Uf-Um-Gf-Ams-Ufs-Cm | 346 |
| B1404-AL | Afs-Ams-Gf-Cm-Gf-Cm-Cf-Am-Cf-Um-Gf-Cm-Cf-Cm-Uf-Gm-Cf-Am-Cf-Um-Uf | 261 | Ams-Afs-Gm-Uf-Gm-Cf-Am-Gf-Gm-Gf-Cm-Af-Gm-Uf-Gm-Gf-Cm-Gf-Cm-Uf-Ums-Ufs-Gm | 347 |

(continued)

| Sequence ID | Sense strand sequence 5'-3' | SEQ ID NO. | Antisense strand sequence 5'-3' | SEQ ID NO. |
|---|---|---|---|---|
| B1407-AL | Cfs-Gms-Cf-Cm-Af-Cm-Uf-Gm-Cf-Cm-Cf-Um-Gf-Cm-Af-Cm-Uf-Um-Cf-Cm-Uf | 262 | Ams-Gfs-Gm-Af-Am-Gf-Um-Gf-Cm-Af-Gm-Gf-Gm-Cf-Am-Gf-Um-Gf-Gm-Cf-Gms-Cfs-Um | 348 |
| B1554-AL | Gfs-Cms-Gf-Am-Uf-Gm-Uf-Gm-Uf-Cm-Af-Cm-Cf-Cm-Cf-Cm-Af-Gm-Uf-Cm-Uf | 263 | Ams-Gfs-Am-Cf-Um-Gf-Gm-Gf-Gm-Gf-Um-Gf-Am-Cf-Am-Cf-Am-Uf-Cm-Gf-Cms-Ufs-Gm | 349 |
| B1558-AL | Ufs-Gms-Uf-Gm-Uf-Cm-Af-Cm-Cf-Cm-Cf-Cm-Af-Gm-Uf-Cm-Uf-Cm-Cf-Cm-Af | 264 | Ums-Gfs-Gm-Gf-Am-Gf-Am-Cf-Um-Gf-Gm-Gf-Gm-Gf-Um-Gf-Am-Cf-Am-Cf-Ams-Ufs-Cm | 350 |
| B1561-AL | Gfs-Ums-Cf-Am-Cf-Cm-Cf-Cm-Cf-Am-Gf-Um-Cf-Um-Cf-Cm-Cf-Am-Cf-Cm-Uf | 265 | Ams-Gfs-Gm-Uf-Gm-Gf-Gm-Af-Gm-Af-Cm-Uf-Gm-Gf-Gm-Gf-Gm-Uf-Gm-Af-Cms-Afs-Cm | 351 |
| B1562-AL | Ufs-Cms-Af-Cm-Cf-Cm-Cf-Cm-Af-Gm-Uf-Cm-Uf-Cm-Cf-Cm-Af-Cm-Cf-Um-Uf | 266 | Ams-Afs-Gm-Gf-Um-Gf-Gm-Gf-Am-Gf-Am-Cf-Um-Gf-Gm-Gf-Gm-Gf-Um-Gf-Ams-Cfs-Am | 352 |
| B1564-AL | Afs-Cms-Cf-Cm-Cf-Cm-Af-Gm-Uf-Cm-Uf-Cm-Cf-Cm-Af-Cm-Cf-Um-Uf-Um-Uf | 267 | Ams-Afs-Am-Af-Gm-Gf-Um-Gf-Gm-Gf-Am-Gf-Am-Cf-Um-Gf-Gm-Gf-Gm-Gf-Ums-Gfs-Am | 353 |
| B1591-AL | Afs-Ums-Gf-Am-Gf-Um-Cf-Gm-Af-Cm-Uf-Um-Uf-Gm-Af-Gm-Cf-Um-Gf-Gm-Af | 268 | Ums-Cfs-Cm-Af-Gm-Cf-Um-Cf-Am-Af-Am-Gf-Um-Cf-Gm-Af-Cm-Uf-Cm-Af-Ums-Ufs-Am | 354 |
| B1592-AL | Ufs-Gms-Af-Gm-Uf-Cm-Gf-Am-Cf-Um-Uf-Um-Gf-Am-Gf-Cm-Uf-Gm-Gf-Am-Af | 269 | Ums-Ufs-Cm-Cf-Am-Gf-Cm-Uf-Cm-Af-Am-Af-Gm-Uf-Cm-Gf-Am-Cf-Um-Cf-Ams-Ufs-Um | 355 |

(continued)

| Sequence ID | Sense strand sequence 5'-3' | SEQ ID NO. | Antisense strand sequence 5'-3' | SEQ ID NO. |
|---|---|---|---|---|
| B1593-AL | Gfs-Ams-Gf-Um-Cf-Gm-Af-Cm-Uf-Um-Uf-Gm-Af-Gm-Cf-Um-Gf-Gm-Af-Am-Af | 270 | Ums-Ufs-Um-Cf-Cm-Af-Gm-Cf-Um-Cf-Am-Af-Am-Gf-Um-Cf-Gm-Af-Cm-Uf-Cms-Afs-Um | 356 |
| B1596-AL | Ufs-Cms-Gf-Am-Cf-Um-Uf-Um-Gf-Am-Gf-Cm-Uf-Gm-Gf-Am-Af-Am-Gf-Cm-Af | 271 | Ums-Gfs-Cm-Uf-Um-Uf-Cm-Cf-Am-Gf-Cm-Uf-Cm-Af-Am-Af-Gm-Uf-Cm-Gf-Ams-Cfs-Um | 357 |
| B1602-AL | Ufs-Ums-Gf-Am-Gf-Cm-Uf-Gm-Gf-Am-Af-Am-Gf-Cm-Af-Gm-Cf-Cm-Gf-Um-Uf | 272 | Ams-Afs-Cm-Gf-Gm-Cf-Um-Gf-Cm-Uf-Um-Uf-Cm-Cf-Am-Gf-Cm-Uf-Cm-Af-Ams-Afs-Gm | 358 |
| B1603-AL | Ufs-Gms-Af-Gm-Cf-Um-Gf-Gm-Af-Am-Af-Gm-Cf-Am-Gf-Cm-Cf-Gm-Uf-Um-Uf | 273 | Ams-Afs-Am-Cf-Gm-Gf-Cm-Uf-Gm-Cf-Um-Uf-Um-Cf-Cm-Af-Gm-Cf-Um-Cf-Ams-Afs-Am | 359 |
| B1608-AL | Ufs-Gms-Gf-Am-Af-Am-Gf-Cm-Af-Gm-Cf-Cm-Gf-Um-Uf-Um-Cf-Um-Cf-Cm-Uf | 274 | Ams-Gfs-Gm-Af-Gm-Af-Am-Af-Cm-Gf-Gm-Cf-Um-Gf-Cm-Uf-Um-Uf-Cm-Cf-Ams-Gfs-Cm | 360 |
| B1612-AL | Afs-Ams-Gf-Cm-Af-Gm-Cf-Cm-Gf-Um-Uf-Um-Cf-Um-Cf-Cm-Uf-Um-Gf-Gm-Uf | 275 | Ams-Cfs-Cm-Af-Am-Gf-Gm-Af-Gm-Af-Am-Af-Cm-Gf-Gm-Cf-Um-Gf-Cm-Uf-Ums-Ufs-Cm | 361 |
| B 1775-AL | Cfs-Ams-Af-Cm-Cf-Gm-Af-Cm-Cf-Am-Gf-Cm-Uf-Um-Gf-Um-Uf-Um-Gf-Um-Af | 276 | Ums-Afs-Cm-Af-Am-Af-Cm-Af-Am-Gf-Cm-Uf-Gm-Gf-Um-Cf-Gm-Gf-Um-Uf-Gms-Gfs-Am | 362 |
| B1790-AL | Ufs-Ums-Uf-Gm-Uf-Gm-Af-Am-Af-Cm-Af-Am-Af-Am-Af-Am-Gf-Um-Gf-Um-Uf | 277 | Ams-Afs-Cm-Af-Cm-Uf-Um-Uf-Um-Uf-Um-Gf-Um-Uf-Um-Cf-Am-Cf-Am-Af-Ams-Cfs-Am | 363 |

(continued)

| Sequence ID | Sense strand sequence 5'-3' | SEQ ID NO. | Antisense strand sequence 5'-3' | SEQ ID NO. |
|---|---|---|---|---|
| B1796-AL | Afs-Ams-Af-Cm-Af-Am-Af-Am-Af-Am-Gf-Um-Gf-Um-Uf-Cm-Cf-Cm-Uf-Um-Uf | 278 | Ams-Afs-Am-Gf-Gm-Gf-Am-Af-Cm-Af-Cm-Uf-Um-Uf-Um-Uf-Um-Gf-Um-Uf-Ums-Cfs-Am | 364 |
| B1798sAL | Ams-Cfs-Am-Af-Am-Af-Am-Af-Gm-Uf-Gm-Uf-Um-Cf-Cm-Cf-Um-Uf-Um-Uf | 279 | Ams-Afs-Am-Af-Gm-Gf-Gm-Af-Am-Cf-Am-Cf-Um-Uf-Um-Uf-Um-Uf-Gm-Ufs-Ums-Uf | 365 |
| B1799-AL | Cfs-Ams-Af-Am-Af-Am-Af-Gm-Uf-Gm-Uf-Um-Cf-Cm-Cf-Um-Uf-Um-Uf-Cm-Af | 280 | Ums-Gfs-Am-Af-Am-Af-Gm-Gf-Gm-Af-Am-Cf-Am-Cf-Um-Uf-Um-Uf-Um-Uf-Gms-Ufs-Um | 366 |
| B1810-AL | Ufs-Cms-Cf-Cm-Uf-Um-Uf-Um-Cf-Am-Af-Gm-Uf-Um-Gf-Am-Gf-Am-Af-Cm-Af | 281 | Ums-Gfs-Um-Uf-Cm-Uf-Cm-Af-Am-Cf-Um-Uf-Gm-Af-Am-Af-Am-Gf-Gm-Gf-Ams-Afs-Cm | 367 |
| B1811-AL | Cfs-Cms-Cf-Um-Uf-Um-Uf-Cm-Af-Am-Gf-Um-Uf-Gm-Af-Gm-Af-Am-Cf-Am-Af | 282 | Ums-Ufs-Gm-Uf-Um-Cf-Um-Cf-Am-Af-Cm-Uf-Um-Gf-Am-Af-Am-Af-Gm-Gf-Gms-Afs-Am | 368 |
| B1814-AL | Ufs-Ums-Uf-Um-Cf-Am-Af-Gm-Uf-Um-Gf-Am-Gf-Am-Af-Cm-Af-Am-Af-Am-Af | 283 | Ums-Ufs-Um-Uf-Um-Gf-Um-Uf-Cm-Uf-Cm-Af-Am-Cf-Um-Uf-Gm-Af-Am-Af-Ams-Gfs-Gm | 369 |
| B1815-AL | Ufs-Ums-Uf-Cm-Af-Am-Gf-Um-Uf-Gm-Af-Gm-Af-Am-Cf-Am-Af-Am-Af-Am-Uf | 284 | Ams-Ufs-Um-Uf-Um-Uf-Gm-Uf-Um-Cf-Um-Cf-Am-Af-Cm-Uf-Um-Gf-Am-Af-Ams-Afs-Gm | 370 |
| B1817-AL | Ufs-Cms-Af-Am-Gf-Um-Uf-Gm-Af-Gm-Af-Am-Cf-Am-Af-Am-Af-Am-Uf-Um-Gf | 285 | Cms-Afs-Am-Uf-Um-Uf-Um-Uf-Gm-Uf-Um-Cf-Um-Cf-Am-Af-Cm-Uf-Um-Gf-Ams-Afs-Am | 371 |

(continued)

| Sequence ID | Sense strand sequence 5'-3' | SEQ ID NO. | Antisense strand sequence 5'-3' | SEQ ID NO. |
|---|---|---|---|---|
| B1828-AL | Afs-Cms-Af-Am-Af-Am-Af-Um-Uf-Gm-Gf-Gm-Uf-Um-Uf-Um-Af-Am-Af-Am-Uf | 286 | Ams-Ufs-Um-Uf-Um-Af-Am-Af-Am-Cf-Cm-Cf-Am-Af-Um-Uf-Um-Uf-Um-Gf-Ums-Ufs-Cm | 372 |
| B1838sAL | Gms-Gfs-Um-Uf-Um-Uf-Am-Af-Am-Af-Um-Uf-Am-Af-Am-Gf-Um-Af-Um-Af | 287 | Ums-Afs-Um-Af-Cm-Uf-Um-Uf-Am-Af-Um-Uf-Um-Uf-Am-Af-Am-Af-Cm-Cfs-Cms-Af | 373 |
| B2018-AL | Afs-Ums-Af-Gm-Cf-Um-Gf-Gm-Uf-Um-Af-Um-Uf-Um-Cf-Um-Cf-Cm-Cf-Um-Uf | 288 | Ams-Afs-Gm-Gf-Gm-Af-Gm-Af-Am-Af-Um-Af-Am-Cf-Cm-Af-Gm-Cf-Um-Af-Ums-Gfs-Gm | 374 |
| B2020-AL | Afs-Gms-Cf-Um-Gf-Gm-Uf-Um-Af-Um-Uf-Um-Cf-Um-Cf-Cm-Cf-Um-Uf-Gm-Uf | 289 | Ams-Cfs-Am-Af-Gm-Gf-Gm-Af-Gm-Af-Am-Af-Um-Af-Am-Cf-Cm-Af-Gm-Cf-Ums-Afs-Um | 375 |
| B2025-AL | Gfs-Ums-Uf-Am-Uf-Um-Uf-Cm-Uf-Cm-Cf-Cm-Uf-Um-Gf-Um-Gf-Um-Uf-Am-Af | 290 | Ums-Ufs-Am-Af-Cm-Af-Cm-Af-Am-Gf-Gm-Gf-Am-Gf-Am-Af-Am-Uf-Am-Af-Cms-Cfs-Am | 376 |
| B2030-AL | Ufs-Ums-Cf-Um-Cf-Cm-Cf-Um-Uf-Gm-Uf-Gm-Uf-Um-Af-Gm-Uf-Am-Af-Um-Af | 291 | Ums-Afs-Um-Uf-Am-Cf-Um-Af-Am-Cf-Am-Cf-Am-Af-Gm-Gf-Gm-Af-Gm-Af-Ams-Afs-Um | 377 |
| B2031-AL | Ufs-Cms-Uf-Cm-Cf-Cm-Uf-Um-Gf-Um-Gf-Um-Uf-Am-Gf-Um-Af-Am-Uf-Am-Af | 292 | Ums-Ufs-Am-Uf-Um-Af-Cm-Uf-Am-Af-Cm-Af-Cm-Af-Am-Gf-Gm-Gf-Am-Gf-Ams-Afs-Am | 378 |
| B2032-AL | Cfs-Ums-Cf-Cm-Cf-Um-Uf-Gm-Uf-Gm-Uf-Um-Af-Gm-Uf-Am-Af-Um-Af-Am-Af | 293 | Ums-Ufs-Um-Af-Um-Uf-Am-Cf-Um-Af-Am-Cf-Am-Cf-Am-Af-Gm-Gf-Gm-Af-Gms-Afs-Am | 379 |

(continued)

| Sequence ID | Sense strand sequence 5'-3' | SEQ ID NO. | Antisense strand sequence 5'-3' | SEQ ID NO. |
|---|---|---|---|---|
| B2033-AL | Ufs-Cms-Cf-Cm-Uf-Um-Gf-Um-Gf-Um-Uf-Am-Gf-Um-Af-Am-Uf-Am-Af-Am-Af | 294 | Ums-Ufs-Um-Uf-Am-Uf-Um-Af-Cm-Uf-Am-Af-Cm-Af-Cm-Af-Am-Gf-Gm-Gf-Ams-Gfs-Am | 380 |
| B729-AL | Afs-Gms-Af-Am-Cf-Um-Gf-Gm-Af-Um-Gf-Um-Uf-Gm-Cf-Um-Gf-Cm-Uf-Gm-Af | 295 | Ums-Cfs-Am-Gf-Cm-Af-Gm-Cf-Am-Af-Cm-Af-Um-Cf-Cm-Af-Gm-Uf-Um-Cf-Ums-Gfs-Um | 381 |
| B731-AL | Afs-Ams-Cf-Um-Gf-Gm-Af-Um-Gf-Um-Uf-Gm-Cf-Um-Gf-Cm-Uf-Gm-Af-Gm-Af | 296 | Ums-Cfs-Um-Cf-Am-Gf-Cm-Af-Gm-Cf-Am-Af-Cm-Af-Um-Cf-Cm-Af-Gm-Uf-Ums-Cfs-Um | 382 |
| B732-AL | Afs-Cms-Uf-Gm-Gf-Am-Uf-Gm-Uf-Um-Gf-Cm-Uf-Gm-Cf-Um-Gf-Am-Gf-Am-Af | 297 | Ums-Ufs-Cm-Uf-Cm-Af-Gm-Cf-Am-Gf-Cm-Af-Am-Cf-Am-Uf-Cm-Cf-Am-Gf-Ums-Ufs-Cm | 383 |
| B734-AL | Ufs-Gms-Gf-Am-Uf-Gm-Uf-Um-Gf-Cm-Uf-Gm-Cf-Um-Gf-Am-Gf-Am-Af-Gm-Af | 298 | Ums-Cfs-Um-Uf-Cm-Uf-Cm-Af-Gm-Cf-Am-Gf-Cm-Af-Am-Cf-Am-Uf-Cm-Cf-Ams-Gfs-Um | 384 |
| B734s-AL | Ums-Gfs-Gm-Af-Um-Gf-Um-Uf-Gm-Cf-Um-Gf-Cm-Uf-Gm-Af-Gm-Af-Am-Af | 299 | Ums-Ufs-Um-Cf-Um-Cf-Am-Gf-Cm-Af-Gm-Cf-Am-Af-Cm-Af-Um-Cf-Cm-Afs-Gms-Uf | 385 |
| B736-AL | Gfs-Ams-Uf-Gm-Uf-Um-Gf-Cm-Uf-Gm-Cf-Um-Gf-Am-Gf-Am-Af-Gm-Af-Um-Uf | 300 | Ams-Afs-Um-Cf-Um-Uf-Cm-Uf-Cm-Af-Gm-Cf-Am-Gf-Cm-Af-Am-Cf-Am-Uf-Cms-Cfs-Am | 386 |
| B743-AL | Cfs-Ums-Gf-Cm-Uf-Gm-Af-Gm-Af-Am-Gf-Am-Uf-Um-Gf-Am-Cf-Am-Gf-Gm-Uf | 301 | Ams-Cfs-Cm-Uf-Gm-Uf-Cm-Af-Am-Uf-Cm-Uf-Um-Cf-Um-Cf-Am-Gf-Cm-Af-Gms-Cfs-Am | 387 |

(continued)

| Sequence ID | Sense strand sequence 5'-3' | SEQ ID NO. | Antisense strand sequence 5'-3' | SEQ ID NO. |
|---|---|---|---|---|
| B753s-AL | Gms-Afs-Um-Uf-Gm-Af-Cm-Af-Gm-Gf-Um-Uf-Cm-Af-Um-Gf-Cm-Af-Gm-Af | 302 | Ums-Cfs-Um-Gf-Cm-Af-Um-Gf-Am-Af-Cm-Cf-Um-Gf-Um-Cf-Am-Af-Um-Cfs-Ums-Uf | 388 |
| B963-AL | Gfs-Cms-Af-Cm-Uf-Gm-Gf-Am-Gf-Um-Gf-Am-Cf-Am-Uf-Cm-Cf-Am-Gf-Gm-Af | 303 | Ums-Cfs-Cm-Uf-Gm-Gf-Am-Uf-Gm-Uf-Cm-Af-Cm-Uf-Cm-Cf-Am-Gf-Um-Gf-Cms-Ufs-Gm | 389 |
| B966-AL | Cfs-Ums-Gf-Gm-Af-Gm-Uf-Gm-Af-Cm-Af-Um-Cf-Cm-Af-Gm-Gf-Am-Cf-Am-Af | 304 | Ums-Ufs-Gm-Uf-Cm-Cf-Um-Gf-Gm-Af-Um-Gf-Um-Cf-Am-Cf-Um-Cf-Cm-Af-Gms-Ufs-Gm | 390 |
| B969-AL | Gfs-Ams-Gf-Um-Gf-Am-Cf-Am-Uf-Cm-Cf-Am-Gf-Gm-Af-Cm-Af-Am-Cf-Um-Uf | 305 | Ams-Afs-Gm-Uf-Um-Gf-Um-Cf-Cm-Uf-Gm-Gf-Am-Uf-Gm-Uf-Cm-Af-Cm-Uf-Cms-Cfs-Am | 391 |
| B971-AL | Gfs-Ums-Gf-Am-Cf-Am-Uf-Cm-Cf-Am-Gf-Gm-Af-Cm-Af-Am-Cf-Um-Uf-Cm-Uf | 306 | Ams-Gfs-Am-Af-Gm-Uf-Um-Gf-Um-Cf-Cm-Uf-Gm-Gf-Am-Uf-Gm-Uf-Cm-Af-Cms-Ufs-Cm | 392 |
| B982-AL | Gfs-Ams-Cf-Am-Af-Cm-Uf-Um-Cf-Um-Cf-Gm-Gf-Um-Gf-Am-Cf-Um-Cf-Am-Af | 307 | Ums-Ufs-Gm-Af-Gm-Uf-Cm-Af-Cm-Cf-Gm-Af-Gm-Af-Am-Gf-Um-Uf-Gm-Uf-Cms-Cfs-Um | 393 |
| B984-AL | Cfs-Ams-Af-Cm-Uf-Um-Cf-Um-Cf-Gm-Gf-Um-Gf-Am-Cf-Um-Cf-Am-Af-Gm-Uf | 308 | Ams-Cfs-Um-Uf-Gm-Af-Gm-Uf-Cm-Af-Cm-Cf-Gm-Af-Gm-Af-Am-Gf-Um-Uf-Gms-Ufs-Cm | 394 |
| B992-AL | Cfs-Gms-Gf-Um-Gf-Am-Cf-Um-Cf-Am-Af-Gm-Uf-Gm-Cf-Cm-Cf-Um-Uf-Cm-Af | 309 | Ums-Gfs-Am-Af-Gm-Gf-Gm-Cf-Am-Cf-Um-Uf-Gm-Af-Gm-Uf-Cm-Af-Cm-Cf-Gms-Afs-Gm | 395 |

(continued)

| Sequence ID | Sense strand sequence 5'-3' | SEQ ID NO. | Antisense strand sequence 5'-3' | SEQ ID NO. |
|---|---|---|---|---|
| B994-AL | Gfs-Ums-Gf-Am-Cf-Um-Cf-Am-Af-Gm-Uf-Gm-Cf-Cm-Cf-Um-Uf-Cm-Af-Cm-Uf | 310 | Ams-Gfs-Um-Gf-Am-Af-Gm-Gf-Gm-Cf-Am-Cf-Um-Uf-Gm-Af-Gm-Uf-Cm-Af-Cms-Cfs-Gm | 396 |
| C1835-DV29P | Ums-Ums-Gm-Gm-Gm-Um-Uf-Um-Uf-Am-Af-Am-Am-Um-Um-Am-Af-Am-Gm-Um-Am | 397 | UmsEVP-Afs-Cm-Uf-Uf-Uf-Am-Am-Um-Um-Um-Um-Am-Af-Am-Af-Cm-Cm-Cm-Am-Ams-Ums-Um | 432 |
| C1835-DV26P | Ums-Ums-Gm-Gm-Gm-Um-Uf-Um-Uf-Af-Af-Am-Am-Um-Um-Am-Af-Am-Gm-Um-Am | 398 | UmsEVP-Afs-Cf-Uf-Um-Uf-Am-Am-Um-Um-Um-Um-Am-Af-Am-Af-Cm-Cm-Cm-Am-Ams-Ums-Um | 433 |
| C1835-DV27P | Ums-Ums-Gm-Gm-Gm-Um-Uf-Um-Uf-Af-Af-Am-Am-Um-Um-Am-Af-Am-Gm-Um-Am | 398 | UmsEVP-Afs-Cm-Uf-Uf-Uf-Am-Am-Um-Um-Um-Um-Am-Af-Am-Af-Cm-Cm-Cm-Am-Ams-Ums-Um | 432 |
| C1835-DV28P | Ums-Ums-Gm-Gm-Gm-Um-Uf-Um-Uf-Am-Af-Am-Am-Um-Um-Am-Af-Am-Gm-Um-Am | 397 | UmsEVP-Afs-Cf-Uf-Um-Uf-Am-Am-Um-Um-Um-Um-Am-Af-Am-Af-Cm-Cm-Cm-Am-Ams-Ums-Um | 433 |
| C1835-DV32P | Ums-Ums-Gm-Gm-Gm-Um-Uf-Um-Uf-Am-Af-Am-Am-Um-Um-Am-Af-Am-Gm-Um-Am | 397 | UmsEVP-Afs-Cm-Um-Uf-Uf-Am-Am-Um-Um-Um-Um-Am-Af-Am-Af-Cm-Cm-Cm-Am-Ams-Ums-Um | 434 |
| C1835-DV34P | Ums-Ums-Gm-Gm-Gm-Um-Uf-Um-Uf-Am-Af-Am-Am-Um-Um-Am-Af-Am-Gm-Um-Am | 397 | UmsEVP-Afs-Cf-Um-Um-Uf-Am-Am-Um-Um-Um-Um-Am-Af-Am-Af-Cm-Cm-Cm-Am-Ams-Ums-Um | 435 |
| C1835-DV25P | Ums-Ums-Gm-Gm-Gm-Um-Uf-Um-Uf-Af-Af-Am-Am-Um-Um-Am-Af-Am-Gm-Um-Am | 398 | UmsEVP-Afs-Cm-Um-Um-Uf-Am-Am-Um-Um-Um-Um-Am-Af-Am-Af-Cm-Cm-Cm-Am-Ams-Ums-Um | 436 |

(continued)

| Sequence ID | Sense strand sequence 5'-3' | SEQ ID NO. | Antisense strand sequence 5'-3' | SEQ ID NO. |
|---|---|---|---|---|
| C1835-DV33P | Ums-Ums-Gm-Gm-Gm-Um-Uf-Um-Uf-Am-Af-Am-Am-Um-Um-Am-Af-Am-Gm-Um-Am | 397 | UmsEVP-Afs-Cf-Um-Uf-Uf-Am-Am-Um-Um-Um-Um-Am-Af-Am-Af-Cm-Cm-Cm-Am-Ams-Ums-Um | 437 |
| C1835-DV22 | Ums-Ums-Gm-Gm-Gm-Um-Uf-Um-Uf-Af-Af-Am-Am-Um-Um-Am-Am-Am-Gm-Um-Am | 399 | Ums-Afs-Cm-Um-Um-Uf-Am-Am-Um-Um-Um-Um-Am-Af-Am-Af-Cm-Cm-Cm-Am-Ams-Ums-Um | 438 |
| C1816-DV29P | Ums-Ums-Cm-Am-Am-Gm-Uf-Um-Gf-Am-Gf-Am-Am-Cm-Am-Am-Af-Am-Am-Um-Um | 400 | AmsEVP-Afs-Um-Uf-Uf-Uf-Um-Gm-Um-Um-Cm-Um-Cm-Af-Am-Cf-Um-Um-Gm-Am-Ams-Ams-Am | 439 |
| C1816-DV26P | Ums-Ums-Cm-Am-Am-Gm-Uf-Um-Gf-Af-Gf-Am-Am-Cm-Am-Am-Af-Am-Am-Um-Um | 401 | AmsEVP-Afs-Uf-Uf-Um-Uf-Um-Gm-Um-Um-Cm-Um-Cm-Af-Am-Cf-Um-Um-Gm-Am-Ams-Ams-Am | 440 |
| C1816-DV27P | Ums-Ums-Cm-Am-Am-Gm-Uf-Um-Gf-Af-Gf-Am-Am-Cm-Am-Am-Af-Am-Am-Um-Um | 401 | AmsEVP-Afs-Um-Uf-Uf-Uf-Um-Gm-Um-Um-Cm-Um-Cm-Af-Am-Cf-Um-Um-Gm-Am-Ams-Ams-Am | 439 |
| C1816-DV28P | Ums-Ums-Cm-Am-Am-Gm-Uf-Um-Gf-Am-Gf-Am-Am-Cm-Am-Am-Af-Am-Am-Um-Um | 400 | AmsEVP-Afs-Uf-Uf-Um-Uf-Um-Gm-Um-Um-Cm-Um-Cm-Af-Am-Cf-Um-Um-Gm-Am-Ams-Ams-Am | 440 |
| C1816-DV32P | Ums-Ums-Cm-Am-Am-Gm-Uf-Um-Gf-Am-Gf-Am-Am-Cm-Am-Am-Af-Am-Am-Um-Um | 400 | AmsEVP-Afs-Um-Um-Uf-Uf-Um-Gm-Um-Um-Cm-Um-Cm-Af-Am-Cf-Um-Um-Gm-Am-Ams-Ams-Am | 441 |
| C1816-DV34P | Ums-Ums-Cm-Am-Am-Gm-Uf-Um-Gf-Am-Gf-Am-Am-Cm-Am-Am-Af-Am-Am-Um-Um | 400 | AmsEVP-Afs-Uf-Um-Um-Uf-Um-Gm-Um-Um-Cm-Um-Cm-Af-Am-Cf-Um-Um-Gm-Am-Ams-Ams-Am | 442 |

(continued)

| Sequence ID | Sense strand sequence 5'-3' | SEQ ID NO. | Antisense strand sequence 5'-3' | SEQ ID NO. |
|---|---|---|---|---|
| C1816-DV25P | Ums-Ums-Cm-Am-Am-Gm-Uf-Um-Gf-Af-Gf-Am-Am-Cm-Am-Am-Af-Am-Am-Um-Um | 401 | AmsEVP-Afs-Um-Um-Um-Uf-Um-Gm-Um-Um-Cm-Um-Cm-Af-Am-Cf-Um-Um-Gm-Am-Ams-Ams-Am | 443 |
| C1816-DV33P | Ums-Ums-Cm-Am-Am-Gm-Uf-Um-Gf-Am-Gf-Am-Am-Cm-Am-Am-Af-Am-Am-Um-Um | 400 | AmsEVP-Afs-Uf-Um-Uf-Uf-Um-Gm-Um-Um-Cm-Um-Cm-Af-Am-Cf-Um-Um-Gm-Am-Ams-Ams-Am | 444 |
| C1816-DV22 | Ums-Ums-Cm-Am-Am-Gm-Uf-Um-Gf-Af-Gf-Am-Am-Cm-Am-Am-Am-Am-Am-Um-Um | 402 | Ams-Afs-Um-Um-Um-Uf-Um-Gm-Um-Um-Cm-Um-Cm-Af-Am-Cf-Um-Um-Gm-Am-Ams-Ams-Am | 445 |
| C1812-DV29P | Cms-Cms-Um-Um-Um-Um-Cf-Am-Af-Gm-Uf-Um-Gm-Am-Gm-Am-Af-Cm-Am-Am-Am | 403 | UmsEVP-Ufs-Um-Gf-Uf-Uf-Cm-Um-Cm-Am-Am-Cm-Um-Uf-Gm-Af-Am-Am-Am-Gm-Gms-Gms-Am | 446 |
| C1812-DV26P | Cms-Cms-Um-Um-Um-Um-Cf-Am-Af-Gf-Uf-Um-Gm-Am-Gm-Am-Af-Cm-Am-Am-Am | 404 | UmsEVP-Ufs-Uf-Gf-Um-Uf-Cm-Um-Cm-Am-Am-Cm-Um-Uf-Gm-Af-Am-Am-Am-Gm-Gms-Gms-Am | 447 |
| C1812-DV27P | Cms-Cms-Um-Um-Um-Um-Cf-Am-Af-Gf-Uf-Um-Gm-Am-Gm-Am-Af-Cm-Am-Am-Am | 404 | UmsEVP-Ufs-Um-Gf-Uf-Uf-Cm-Um-Cm-Am-Am-Cm-Um-Uf-Gm-Af-Am-Am-Am-Gm-Gms-Gms-Am | 446 |
| C1812-DV28P | Cms-Cms-Um-Um-Um-Um-Cf-Am-Af-Gm-Uf-Um-Gm-Am-Gm-Am-Af-Cm-Am-Am-Am | 403 | UmsEVP-Ufs-Uf-Gf-Um-Uf-Cm-Um-Cm-Am-Am-Cm-Um-Uf-Gm-Af-Am-Am-Am-Gm-Gms-Gms-Am | 447 |
| C1812-DV32P | Cms-Cms-Um-Um-Um-Um-Cf-Am-Af-Gm-Uf-Um-Gm-Am-Gm-Am-Af-Cm-Am-Am-Am | 403 | UmsEVP-Ufs-Um-Gm-Uf-Uf-Cm-Um-Cm-Am-Am-Cm-Um-Uf-Gm-Af-Am-Am-Am-Gm-Gms-Gms-Am | 448 |

(continued)

| Sequence ID | Sense strand sequence 5'-3' | SEQ ID NO. | Antisense strand sequence 5'-3' | SEQ ID NO. |
|---|---|---|---|---|
| C1812-DV34P | Cms-Cms-Um-Um-Um-Um-Cf-Am-Af-Gm-Uf-Um-Gm-Am-Gm-Am-Af-Cm-Am-Am-Am | 403 | UmsEVP-Ufs-Uf-Gm-Um-Uf-Cm-Um-Cm-Am-Am-Cm-Um-Uf-Gm-Af-Am-Am-Am-Gm-Gms-Gms-Am | 449 |
| C1812-DV25P | Cms-Cms-Um-Um-Um-Um-Cf-Am-Af-Gf-Uf-Um-Gm-Am-Gm-Am-Af-Cm-Am-Am-Am | 404 | UmsEVP-Ufs-Um-Gm-Um-Uf-Cm-Um-Cm-Am-Am-Cm-Um-Uf-Gm-Af-Am-Am-Am-Gm-Gms-Gms-Am | 450 |
| C1812-DV33P | Cms-Cms-Um-Um-Um-Um-Cf-Am-Af-Gm-Uf-Um-Gm-Am-Gm-Am-Af-Cm-Am-Am-Am | 403 | UmsEVP-Ufs-Uf-Gm-Uf-Uf-Cm-Um-Cm-Am-Am-Cm-Um-Uf-Gm-Af-Am-Am-Am-Gm-Gms-Gms-Am | 451 |
| C1812-DV22 | Cms-Cms-Um-Um-Um-Um-Cf-Am-Af-Gf-Uf-Um-Gm-Am-Gm-Am-Am-Cm-Am-Am-Am | 405 | Ums-Ufs-Um-Gm-Um-Uf-Cm-Um-Cm-Am-Am-Cm-Um-Uf-Gm-Af-Am-Am-Am-Gm-Gms-Gms-Am | 452 |
| C1579-DV29P | Cms-Cms-Um-Um-Um-Um-Cf-Um-Uf-Cm-Uf-Am-Am-Um-Gm-Am-Gf-Um-Cm-Gm-Am | 406 | UmsEVP-Cfs-Gm-Af-Cf-Uf-Cm-Am-Um-Um-Am-Gm-Am-Af-Gm-Af-Am-Am-Am-Gm-Gms-Ums-Gm | 453 |
| C1579-DV26P | Cms-Cms-Um-Um-Um-Um-Cf-Um-Uf-Cf-Uf-Am-Am-Um-Gm-Am-Gf-Um-Cm-Gm-Am | 407 | UmsEVP-Cfs-Gf-Af-Cm-Uf-Cm-Am-Um-Um-Am-Gm-Am-Af-Gm-Af-Am-Am-Am-Gm-Gms-Ums-Gm | 454 |
| C1579-DV27P | Cms-Cms-Um-Um-Um-Um-Cf-Um-Uf-Cf-Uf-Am-Am-Um-Gm-Am-Gf-Um-Cm-Gm-Am | 407 | UmsEVP-Cfs-Gm-Af-Cf-Uf-Cm-Am-Um-Um-Am-Gm-Am-Af-Gm-Af-Am-Am-Am-Gm-Gms-Ums-Gm | 453 |
| C1579-DV28P | Cms-Cms-Um-Um-Um-Um-Cf-Um-Uf-Cm-Uf-Am-Am-Um-Gm-Am-Gf-Um-Cm-Gm-Am | 406 | UmsEVP-Cfs-Gf-Af-Cm-Uf-Cm-Am-Um-Um-Am-Gm-Am-Af-Gm-Af-Am-Am-Am-Gm-Gms-Ums-Gm | 454 |

(continued)

| Sequence ID | Sense strand sequence 5'-3' | SEQ ID NO. | Antisense strand sequence 5'-3' | SEQ ID NO. |
|---|---|---|---|---|
| C1579-DV32P | Cms-Cms-Um-Um-Um-Um-Cf-Um-Uf-Cm-Uf-Am-Am-Um-Gm-Am-Gf-Um-Cm-Gm-Am | 406 | UmsEVP-Cfs-Gm-Am-Cf-Uf-Cm-Am-Um-Um-Am-Gm-Am-Af-Gm-Af-Am-Am-Am-Gm-Gms-Ums-Gm | 455 |
| C1579-DV34P | Cms-Cms-Um-Um-Um-Um-Cf-Um-Uf-Cm-Uf-Am-Am-Um-Gm-Am-Gf-Um-Cm-Gm-Am | 406 | UmsEVP-Cfs-Gf-Am-Cm-Uf-Cm-Am-Um-Um-Am-Gm-Am-Af-Gm-Af-Am-Am-Am-Gm-Gms-Ums-Gm | 456 |
| C1579-DV25P | Cms-Cms-Um-Um-Um-Um-Cf-Um-Uf-Cf-Uf-Am-Am-Um-Gm-Am-Gf-Um-Cm-Gm-Am | 407 | UmsEVP-Cfs-Gm-Am-Cm-Uf-Cm-Am-Um-Um-Am-Gm-Am-Af-Gm-Af-Am-Am-Am-Gm-Gms-Ums-Gm | 457 |
| C1579-DV33P | Cms-Cms-Um-Um-Um-Um-Cf-Um-Uf-Cm-Uf-Am-Am-Um-Gm-Am-Gf-Um-Cm-Gm-Am | 406 | UmsEVP-Cfs-Gf-Am-Cf-Uf-Cm-Am-Um-Um-Am-Gm-Am-Af-Gm-Af-Am-Am-Am-Gm-Gms-Ums-Gm | 458 |
| C1579-DV22 | Cms-Cms-Um-Um-Um-Um-Cf-Um-Uf-Cf-Uf-Am-Am-Um-Gm-Am-Gm-Um-Cm-Gm-Am | 408 | Ums-Cfs-Gm-Am-Cm-Uf-Cm-Am-Um-Um-Am-Gm-Am-Af-Gm-Af-Am-Am-Am-Gm-Gms-Ums-Gm | 459 |
| C1836-DV29P | Ums-Gms-Gm-Gm-Um-Um-Uf-Um-Af-Am-Af-Am-Um-Um-Am-Am-Af-Gm-Um-Am-Um | 409 | AmsEVP-Ufs-Am-Cf-Uf-Uf-Um-Am-Am-Um-Um-Um-Um-Af-Am-Af-Am-Cm-Cm-Cm-Ams-Ams-Um | 460 |
| C1836-DV26P | Ums-Gms-Gm-Gm-Um-Um-Uf-Um-Af-Af-Af-Am-Um-Um-Am-Am-Af-Gm-Um-Am-Um | 410 | AmsEVP-Ufs-Af-Cf-Um-Uf-Um-Am-Am-Um-Um-Um-Um-Af-Am-Af-Am-Cm-Cm-Cm-Ams-Ams-Um | 461 |
| C1836-DV27P | Ums-Gms-Gm-Gm-Um-Um-Uf-Um-Af-Af-Af-Am-Um-Um-Am-Am-Af-Gm-Um-Am-Um | 410 | AmsEVP-Ufs-Am-Cf-Uf-Uf-Um-Am-Am-Um-Um-Um-Um-Af-Am-Af-Am-Cm-Cm-Cm-Ams-Ams-Um | 460 |

(continued)

| Sequence ID | Sense strand sequence 5'-3' | SEQ ID NO. | Antisense strand sequence 5'-3' | SEQ ID NO. |
|---|---|---|---|---|
| C1836-DV28P | Ums-Gms-Gm-Gm-Um-Um-Uf-Um-Af-Am-Af-Am-Um-Um-Am-Am-Af-Gm-Um-Am-Um | 409 | AmsEVP-Ufs-Af-Cf-Um-Uf-Um-Am-Am-Um-Um-Um-Um-Af-Am-Af-Am-Cm-Cm-Cm-Ams-Ams-Um | 461 |
| C1836-DV32P | Ums-Gms-Gm-Gm-Um-Um-Uf-Um-Af-Am-Af-Am-Um-Um-Am-Am-Af-Gm-Um-Am-Um | 409 | AmsEVP-Ufs-Am-Cm-Uf-Uf-Um-Am-Am-Um-Um-Um-Um-Af-Am-Af-Am-Cm-Cm-Cm-Ams-Ams-Um | 462 |
| C1836-DV34P | Ums-Gms-Gm-Gm-Um-Um-Uf-Um-Af-Am-Af-Am-Um-Um-Am-Am-Af-Gm-Um-Am-Um | 409 | AmsEVP-Ufs-Af-Cm-Um-Uf-Um-Am-Am-Um-Um-Um-Um-Af-Am-Af-Am-Cm-Cm-Cm-Ams-Ams-Um | 463 |
| C1789-DV29P | Gms-Ums-Um-Um-Gm-Um-Gf-Am-Af-Am-Cf-Am-Am-Am-Am-Am-Af-Gm-Um-Gm-Um | 411 | AmsEVP-Cfs-Am-Cf-Uf-Uf-Um-Um-Um-Um-Gm-Um-Um-Uf-Cm-Af-Cm-Am-Am-Am-Cms-Ams-Am | 464 |
| C1789-DV26P | Gms-Ums-Um-Um-Gm-Um-Gf-Am-Af-Af-Cf-Am-Am-Am-Am-Am-Af-Gm-Um-Gm-Um | 412 | AmsEVP-Cfs-Af-Cf-Um-Uf-Um-Um-Um-Um-Gm-Um-Um-Uf-Cm-Af-Cm-Am-Am-Am-Cms-Ams-Am | 465 |
| C1789-DV27P | Gms-Ums-Um-Um-Gm-Um-Gf-Am-Af-Af-Cf-Am-Am-Am-Am-Am-Af-Gm-Um-Gm-Um | 412 | AmsEVP-Cfs-Am-Cf-Uf-Uf-Um-Um-Um-Um-Gm-Um-Um-Uf-Cm-Af-Cm-Am-Am-Am-Cms-Ams-Am | 464 |
| C1789-DV28P | Gms-Ums-Um-Um-Gm-Um-Gf-Am-Af-Am-Cf-Am-Am-Am-Am-Am-Af-Gm-Um-Gm-Um | 411 | AmsEVP-Cfs-Af-Cf-Um-Uf-Um-Um-Um-Um-Gm-Um-Um-Uf-Cm-Af-Cm-Am-Am-Am-Cms-Ams-Am | 465 |
| C1789-DV32P | Gms-Ums-Um-Um-Gm-Um-Gf-Am-Af-Am-Cf-Am-Am-Am-Am-Am-Af-Gm-Um-Gm-Um | 411 | AmsEVP-Cfs-Am-Cm-Uf-Uf-Um-Um-Um-Um-Gm-Um-Um-Uf-Cm-Af-Cm-Am-Am-Am-Cms-Ams-Am | 466 |

(continued)

| Sequence ID | Sense strand sequence 5'-3' | SEQ ID NO. | Antisense strand sequence 5'-3' | SEQ ID NO. |
|---|---|---|---|---|
| C1789-DV34P | Gms-Ums-Um-Um-Gm-Um-Gf-Am-Af-Am-Cf-Am-Am-Am-Am-Am-Af-Gm-Um-Gm-Um | 411 | AmsEVP-Cfs-Af-Cm-Um-Uf-Um-Um-Um-Um-Gm-Um-Um-Uf-Cm-Af-Cm-Am-Am-Am-Cms-Ams-Am | 467 |
| C1789-DV25P | Gms-Ums-Um-Um-Gm-Um-Gf-Am-Af-Af-Cf-Am-Am-Am-Am-Am-Af-Gm-Um-Gm-Um | 412 | AmsEVP-Cfs-Am-Cm-Um-Uf-Um-Um-Um-Um-Gm-Um-Um-Uf-Cm-Af-Cm-Am-Am-Am-Cms-Ams-Am | 468 |
| C1789-DV33P | Gms-Ums-Um-Um-Gm-Um-Gf-Am-Af-Am-Cf-Am-Am-Am-Am-Am-Af-Gm-Um-Gm-Um | 411 | AmsEVP-Cfs-Af-Cm-Uf-Uf-Um-Um-Um-Um-Gm-Um-Um-Uf-Cm-Af-Cm-Am-Am-Am-Cms-Ams-Am | 469 |
| C1789-DV22 | Gms-Ums-Um-Um-Gm-Um-Gf-Am-Af-Af-Cf-Am-Am-Am-Am-Am-Am-Gm-Um-Gm-Um | 413 | Ams-Cfs-Am-Cm-Um-Uf-Um-Um-Um-Um-Gm-Um-Um-Uf-Cm-Af-Cm-Am-Am-Am-Cms-Ams-Am | 470 |
| C1839-DV29P | Gms-Ums-Um-Um-Um-Am-Af-Am-Af-Um-Uf-Am-Am-Am-Gm-Um-Af-Um-Am-Cm-Am | 414 | UmsEVP-Gfs-Um-Af-Uf-Af-Cm-Um-Um-Um-Am-Am-Um-Uf-Um-Uf-Am-Am-Am-Am-Cms-Cms-Cm | 471 |
| C1839-DV26P | Gms-Ums-Um-Um-Um-Am-Af-Am-Af-Uf-Uf-Am-Am-Am-Gm-Um-Af-Um-Am-Cm-Am | 415 | UmsEVP-Gfs-Uf-Af-Um-Af-Cm-Um-Um-Um-Am-Am-Um-Uf-Um-Uf-Am-Am-Am-Am-Cms-Cms-Cm | 472 |
| C1839-DV27P | Gms-Ums-Um-Um-Um-Am-Af-Am-Af-Uf-Uf-Am-Am-Am-Gm-Um-Af-Um-Am-Cm-Am | 415 | UmsEVP-Gfs-Um-Af-Uf-Af-Cm-Um-Um-Um-Am-Am-Um-Uf-Um-Uf-Am-Am-Am-Am-Cms-Cms-Cm | 471 |
| C1839-DV28P | Gms-Ums-Um-Um-Um-Am-Af-Am-Af-Um-Uf-Am-Am-Am-Gm-Um-Af-Um-Am-Cm-Am | 414 | UmsEVP-Gfs-Uf-Af-Um-Af-Cm-Um-Um-Um-Am-Am-Um-Uf-Um-Uf-Am-Am-Am-Am-Cms-Cms-Cm | 472 |

(continued)

| Sequence ID | Sense strand sequence 5'-3' | SEQ ID NO. | Antisense strand sequence 5'-3' | SEQ ID NO. |
|---|---|---|---|---|
| C1839-DV32P | Gms-Ums-Um-Um-Um-Am-Af-Am-Af-Um-Uf-Am-Am-Am-Gm-Um-Af-Um-Am-Cm-Am | 414 | UmsEVP-Gfs-Um-Am-Uf-Af-Cm-Um-Um-Um-Am-Am-Um-Uf-Um-Uf-Am-Am-Am-Am-Cms-Cms-Cm | 473 |
| C1839-DV34P | Gms-Ums-Um-Um-Um-Am-Af-Am-Af-Um-Uf-Am-Am-Am-Gm-Um-Af-Um-Am-Cm-Am | 414 | UmsEVP-Gfs-Uf-Am-Um-Af-Cm-Um-Um-Um-Am-Am-Um-Uf-Um-Uf-Am-Am-Am-Am-Cms-Cms-Cm | 474 |
| C1791-DV29P | Ums-Ums-Gm-Um-Gm-Am-Af-Am-Cf-Am-Af-Am-Am-Am-Am-Gm-Uf-Gm-Um-Um-Am | 416 | UmsEVP-Afs-Am-Cf-Af-Cf-Um-Um-Um-Um-Um-Um-Gm-Uf-Um-Uf-Cm-Am-Cm-Am-Ams-Ams-Cm | 475 |
| C1791-DV26P | Ums-Ums-Gm-Um-Gm-Am-Af-Am-Cf-Af-Af-Am-Am-Am-Am-Gm-Uf-Gm-Um-Um-Am | 417 | UmsEVP-Afs-Af-Cf-Am-Cf-Um-Um-Um-Um-Um-Um-Gm-Uf-Um-Uf-Cm-Am-Cm-Am-Ams-Ams-Cm | 476 |
| C1791-DV27P | Ums-Ums-Gm-Um-Gm-Am-Af-Am-Cf-Af-Af-Am-Am-Am-Am-Gm-Uf-Gm-Um-Um-Am | 417 | UmsEVP-Afs-Am-Cf-Af-Cf-Um-Um-Um-Um-Um-Um-Gm-Uf-Um-Uf-Cm-Am-Cm-Am-Ams-Ams-Cm | 475 |
| C1791-DV28P | Ums-Ums-Gm-Um-Gm-Am-Af-Am-Cf-Am-Af-Am-Am-Am-Am-Gm-Uf-Gm-Um-Um-Am | 416 | UmsEVP-Afs-Af-Cf-Am-Cf-Um-Um-Um-Um-Um-Um-Gm-Uf-Um-Uf-Cm-Am-Cm-Am-Ams-Ams-Cm | 476 |
| C1791-DV32P | Ums-Ums-Gm-Um-Gm-Am-Af-Am-Cf-Am-Af-Am-Am-Am-Am-Gm-Uf-Gm-Um-Um-Am | 416 | UmsEVP-Afs-Am-Cm-Af-Cf-Um-Um-Um-Um-Um-Um-Gm-Uf-Um-Uf-Cm-Am-Cm-Am-Ams-Ams-Cm | 477 |
| C1791-DV34P | Ums-Ums-Gm-Um-Gm-Am-Af-Am-Cf-Am-Af-Am-Am-Am-Am-Gm-Uf-Gm-Um-Um-Am | 416 | UmsEVP-Afs-Af-Cm-Am-Cf-Um-Um-Um-Um-Um-Um-Gm-Uf-Um-Uf-Cm-Am-Cm-Am-Ams-Ams-Cm | 478 |

(continued)

| Sequence ID | Sense strand sequence 5'-3' | SEQ ID NO. | Antisense strand sequence 5'-3' | SEQ ID NO. |
|---|---|---|---|---|
| C1795-DV29P | Gms-Ams-Am-Am-Cm-Am-Af-Am-Af-Am-Af-Gm-Um-Gm-Um-Um-Cf-Cm-Cm-Um-Um | 418 | AmsEVP-Afs-Gm-Gf-Gf-Af-Am-Cm-Am-Cm-Um-Um-Um-Uf-Um-Uf-Gm-Um-Um-Um-Cms-Ams-Cm | 479 |
| C1795-DV26P | Gms-Ams-Am-Am-Cm-Am-Af-Am-Af-Af-Af-Gm-Um-Gm-Um-Um-Cf-Cm-Cm-Um-Um | 419 | AmsEVP-Afs-Gf-Gf-Gm-Af-Am-Cm-Am-Cm-Um-Um-Um-Uf-Um-Uf-Gm-Um-Um-Um-Cms-Ams-Cm | 480 |
| C1795-DV27P | Gms-Ams-Am-Am-Cm-Am-Af-Am-Af-Af-Af-Gm-Um-Gm-Um-Um-Cf-Cm-Cm-Um-Um | 419 | AmsEVP-Afs-Gm-Gf-Gf-Af-Am-Cm-Am-Cm-Um-Um-Um-Uf-Um-Uf-Gm-Um-Um-Um-Cms-Ams-Cm | 479 |
| C1795-DV28P | Gms-Ams-Am-Am-Cm-Am-Af-Am-Af-Am-Af-Gm-Um-Gm-Um-Um-Cf-Cm-Cm-Um-Um | 418 | AmsEVP-Afs-Gf-Gf-Gm-Af-Am-Cm-Am-Cm-Um-Um-Um-Uf-Um-Uf-Gm-Um-Um-Um-Cms-Ams-Cm | 480 |
| C1795-DV32P | Gms-Ams-Am-Am-Cm-Am-Af-Am-Af-Am-Af-Gm-Um-Gm-Um-Um-Cf-Cm-Cm-Um-Um | 418 | AmsEVP-Afs-Gm-Gm-Gf-Af-Am-Cm-Am-Cm-Um-Um-Um-Uf-Um-Uf-Gm-Um-Um-Um-Cms-Ams-Cm | 481 |
| C1795-DV34P | Gms-Ams-Am-Am-Cm-Am-Af-Am-Af-Am-Af-Gm-Um-Gm-Um-Um-Cf-Cm-Cm-Um-Um | 418 | AmsEVP-Afs-Gf-Gm-Gm-Af-Am-Cm-Am-Cm-Um-Um-Um-Uf-Um-Uf-Gm-Um-Um-Um-Cms-Ams-Cm | 482 |
| C1585s-DV29P | Cms-Ums-Um-Cm-Um-Af-Am-Uf-Gm-Af-Gm-Um-Cm-Gm-Am-Cf-Um-Um-Um-Am | 420 | UmsEVP-Afs-Am-Af-Gf-Uf-Cm-Gm-Am-Cm-Um-Cm-Am-Uf-Um-Af-Gm-Am-Am-Gms-Ams-Am | 483 |
| C1585s-DV26P | Cms-Ums-Um-Cm-Um-Af-Am-Uf-Gf-Af-Gm-Um-Cm-Gm-Am-Cf-Um-Um-Um-Am | 421 | UmsEVP-Afs-Af-Af-Gm-Uf-Cm-Gm-Am-Cm-Um-Cm-Am-Uf-Um-Af-Gm-Am-Am-Gms-Ams-Am | 484 |

(continued)

| Sequence ID | Sense strand sequence 5'-3' | SEQ ID NO. | Antisense strand sequence 5'-3' | SEQ ID NO. |
|---|---|---|---|---|
| C1585s-DV27P | Cms-Ums-Um-Cm-Um-Af-Am-Uf-Gf-Af-Gm-Um-Cm-Gm-Am-Cf-Um-Um-Um-Am | 421 | UmsEVP-Afs-Am-Af-Gf-Uf-Cm-Gm-Am-Cm-Um-Cm-Am-Uf-Um-Af-Gm-Am-Am-Gms-Ams-Am | 483 |
| C1585s-DV28P | Cms-Ums-Um-Cm-Um-Af-Am-Uf-Gm-Af-Gm-Um-Cm-Gm-Am-Cf-Um-Um-Um-Am | 420 | UmsEVP-Afs-Af-Af-Gm-Uf-Cm-Gm-Am-Cm-Um-Cm-Am-Uf-Um-Af-Gm-Am-Am-Gms-Ams-Am | 484 |
| C1585s-DV32P | Cms-Ums-Um-Cm-Um-Af-Am-Uf-Gm-Af-Gm-Um-Cm-Gm-Am-Cf-Um-Um-Um-Am | 420 | UmsEVP-Afs-Am-Am-Gf-Uf-Cm-Gm-Am-Cm-Um-Cm-Am-Uf-Um-Af-Gm-Am-Am-Gms-Ams-Am | 485 |
| C1585s-DV34P | Cms-Ums-Um-Cm-Um-Af-Am-Uf-Gm-Af-Gm-Um-Cm-Gm-Am-Cf-Um-Um-Um-Am | 420 | UmsEVP-Afs-Af-Am-Gm-Uf-Cm-Gm-Am-Cm-Um-Cm-Am-Uf-Um-Af-Gm-Am-Am-Gms-Ams-Am | 486 |
| C1585s-DV22 | Cms-Ums-Um-Cm-Um-Af-Am-Uf-Gf-Af-Gm-Um-Cm-Gm-Am-Cm-Um-Um-Um-Am | 422 | Ums-Afs-Am-Am-Gm-Uf-Cm-Gm-Am-Cm-Um-Cm-Am-Uf-Um-Af-Gm-Am-Am-Gms-Ams-Am | 487 |
| C1576s-DV29P | Cms-Cms-Am-Cm-Cm-Uf-Um-Uf-Um-Cf-Um-Um-Cm-Um-Am-Af-Um-Gm-Am-Am | 423 | UmsEVP-Ufs-Cm-Af-Uf-Uf-Am-Gm-Am-Am-Gm-Am-Am-Af-Am-Gf-Gm-Um-Gm-Gms-Gms-Am | 488 |
| C1576s-DV28P | Cms-Cms-Am-Cm-Cm-Uf-Um-Uf-Um-Cf-Um-Um-Cm-Um-Am-Af-Um-Gm-Am-Am | 423 | UmsEVP-Ufs-Cf-Af-Um-Uf-Am-Gm-Am-Am-Gm-Am-Am-Af-Am-Gf-Gm-Um-Gm-Gms-Gms-Am | 489 |
| C1576s-DV26P | Cms-Cms-Am-Cm-Cm-Uf-Um-Uf-Uf-Cf-Um-Um-Cm-Um-Am-Af-Um-Gm-Am-Am | 424 | UmsEVP-Ufs-Cf-Af-Um-Uf-Am-Gm-Am-Am-Gm-Am-Am-Af-Am-Gf-Gm-Um-Gm-Gms-Gms-Am | 489 |

(continued)

| Sequence ID | Sense strand sequence 5'-3' | SEQ ID NO. | Antisense strand sequence 5'-3' | SEQ ID NO. |
|---|---|---|---|---|
| C1576s-DV25P | Cms-Cms-Am-Cm-Cm-Uf-Um-Uf-Uf-Cf-Um-Um-Cm-Um-Am-Af-Um-Gm-Am-Am | 424 | UmsEVP-Ufs-Cm-Am-Um-Uf-Am-Gm-Am-Am-Gm-Am-Am-Af-Am-Gf-Gm-Um-Gm-Gms-Gms-Am | 490 |
| C1576s-DV27P | Cms-Cms-Am-Cm-Cm-Uf-Um-Uf-Uf-Cf-Um-Um-Cm-Um-Am-Af-Um-Gm-Am-Am | 424 | UmsEVP-Ufs-Cm-Af-Uf-Uf-Am-Gm-Am-Am-Gm-Am-Am-Af-Am-Gf-Gm-Um-Gm-Gms-Gms-Am | 488 |
| C1576s-DV22 | Cms-Cms-Am-Cm-Cm-Uf-Um-Uf-Uf-Cf-Um-Um-Cm-Um-Am-Am-Um-Gm-Am-Am | 425 | Ums-Ufs-Cm-Am-Um-Uf-Am-Gm-Am-Am-Gm-Am-Am-Af-Am-Gf-Gm-Um-Gm-Gms-Gms-Am | 491 |
| C1578s-DV29P | Ams-Cms-Cm-Um-Um-Uf-Um-Cf-Um-Uf-Cm-Um-Am-Am-Um-Gf-Am-Gm-Um-Am | 426 | UmsEVP-Afs-Cm-Uf-Cf-Af-Um-Um-Am-Gm-Am-Am-Gm-Af-Am-Af-Am-Gm-Gm-Ums-Gms-Gm | 492 |
| C1578s-DV28P | Ams-Cms-Cm-Um-Um-Uf-Um-Cf-Um-Uf-Cm-Um-Am-Am-Um-Gf-Am-Gm-Um-Am | 426 | UmsEVP-Afs-Cf-Uf-Cm-Af-Um-Um-Am-Gm-Am-Am-Gm-Af-Am-Af-Am-Gm-Gm-Ums-Gms-Gm | 493 |
| C1578s-DV26P | Ams-Cms-Cm-Um-Um-Uf-Um-Cf-Uf-Uf-Cm-Um-Am-Am-Um-Gf-Am-Gm-Um-Am | 427 | UmsEVP-Afs-Cf-Uf-Cm-Af-Um-Um-Am-Gm-Am-Am-Gm-Af-Am-Af-Am-Gm-Gm-Ums-Gms-Gm | 493 |
| C1578s-DV25P | Ams-Cms-Cm-Um-Um-Uf-Um-Cf-Uf-Uf-Cm-Um-Am-Am-Um-Gf-Am-Gm-Um-Am | 427 | UmsEVP-Afs-Cm-Um-Cm-Af-Um-Um-Am-Gm-Am-Am-Gm-Af-Am-Af-Am-Gm-Gm-Ums-Gms-Gm | 494 |
| C1578s-DV27P | Ams-Cms-Cm-Um-Um-Uf-Um-Cf-Uf-Uf-Cm-Um-Am-Am-Um-Gf-Am-Gm-Um-Am | 427 | UmsEVP-Afs-Cm-Uf-Cf-Af-Um-Um-Am-Gm-Am-Am-Gm-Af-Am-Af-Am-Gm-Gm-Ums-Gms-Gm | 492 |

(continued)

| Sequence ID | Sense strand sequence 5'-3' | SEQ ID NO. | Antisense strand sequence 5'-3' | SEQ ID NO. |
|---|---|---|---|---|
| C1578s-DV22 | Ams-Cms-Cm-Um-Um-Uf-Um-Cf-Uf-Uf-Cm-Um-Am-Am-Um-Gm-Am-Gm-Um-Am | 428 | Ums-Afs-Cm-Um-Cm-Af-Um-Um-Am-Gm-Am-Am-Gm-Af-Am-Af-Am-Gm-Gm-Ums-Gms-Gm | 495 |
| C1838-DV29P | Gms-Gms-Um-Um-Um-Um-Af-Am-Af-Am-Uf-Um-Am-Am-Am-Gm-Uf-Am-Um-Am-Am | 429 | UmsEVP-Ufs-Am-Uf-Af-Cf-Um-Um-Um-Am-Am-Um-Um-Uf-Um-Af-Am-Am-Am-Cm-Cms-Cms-Am | 496 |
| C1838-DV28P | Gms-Gms-Um-Um-Um-Um-Af-Am-Af-Am-Uf-Um-Am-Am-Am-Gm-Uf-Am-Um-Am-Am | 429 | UmsEVP-Ufs-Af-Uf-Am-Cf-Um-Um-Um-Am-Am-Um-Um-Uf-Um-Af-Am-Am-Am-Cm-Cms-Cms-Am | 497 |
| C1838-DV26P | Gms-Gms-Um-Um-Um-Um-Af-Am-Af-Af-Uf-Um-Am-Am-Am-Gm-Uf-Am-Um-Am-Am | 430 | UmsEVP-Ufs-Af-Uf-Am-Cf-Um-Um-Um-Am-Am-Um-Um-Uf-Um-Af-Am-Am-Am-Cm-Cms-Cms-Am | 497 |
| C1838-DV25P | Gms-Gms-Um-Um-Um-Um-Af-Am-Af-Af-Uf-Um-Am-Am-Am-Gm-Uf-Am-Um-Am-Am | 430 | UmsEVP-Ufs-Am-Um-Am-Cf-Um-Um-Um-Am-Am-Um-Um-Uf-Um-Af-Am-Am-Am-Cm-Cms-Cms-Am | 498 |
| C1838-DV27P | Gms-Gms-Um-Um-Um-Um-Af-Am-Af-Af-Uf-Um-Am-Am-Am-Gm-Uf-Am-Um-Am-Am | 430 | UmsEVP-Ufs-Am-Uf-Af-Cf-Um-Um-Um-Am-Am-Um-Um-Uf-Um-Af-Am-Am-Am-Cm-Cms-Cms-Am | 496 |
| C1838-DV22 | Gms-Gms-Um-Um-Um-Um-Af-Am-Af-Af-Uf-Um-Am-Am-Am-Gm-Um-Am-Um-Am-Am | 431 | Ums-Ufs-Am-Um-Am-Cf-Um-Um-Um-Am-Am-Um-Um-Uf-Um-Af-Am-Am-Am-Cm-Cms-Cms-Am | 499 |
| 579P | ACCUUUUCUUCUAAUGAGUCU | 500 | AGACUCAUUAGAAGAAAAGGUGG | 513 |
| 789P | UUUGUGAAACAAAAAAGUGA | 501 | UCACUUUUUUGUUUCACAAACA | 514 |
| 812P | UUGUUCUCAACUUGAAAAGGGA | 502 | CCUUUUCAAGUUGAGAACAA | 515 |
| 576P | CCACCUUUUCUUCUAAUGAGU | 503 | ACUCAUUAGAAGAAAAGGUGGGA | 516 |
| 578P | CACCUUUUCUUCUAAUGAGUA | 504 | UACUCAUUAGAAGAAAAGGUGGG | 517 |

(continued)

| Sequence ID | Sense strand sequence 5'-3' | SEQ ID NO. | Antisense strand sequence 5'-3' | SEQ ID NO. |
|---|---|---|---|---|
| 585P | UUCUAAUGAGUCGACUUUA | 505 | UAAAGUCGACUCAUUAGAA | 518 |
| AD-60771 | AGAACAAAAAUUGGGUUUUAA | 506 | UUAAAACCCAAUUUUUGUUCUCA | 519 |
| AD-85481 | GUCAUCCACAAUGAGAGUACA | 507 | UGUACUCUCAUUGUGGAUGACGA | 520 |
| 816P | UCAAGUUGAGAACAAAAAUA | 508 | UAUUUUUGUUCUCAACUUGAAA | 521 |
| 835P | GGGUUUUAAAAUUAAAGUAUA | 509 | UACUUUAAUUUUAAAACCCAA | 522 |
| 836P | GGUUUUAAAAUUAAAGUAUAC | 510 | AUACUUUAAUUUUAAAACCCA | 523 |
| 839P | GUUUUAAAAUUAAAGUAUACA | 511 | UAUACUUUAAUUUUAAAACCC | 524 |
| 791P | UUGUGAAACAAAAAAGUGUUU | 512 | ACACUUUUUUGUUUCACAAUU | 525 |
| D835-DV26P | Ums-Ums-Gm-Gm-Gm-Um-Uf-Um-Uf-Af-Af-Am-Am-Um-Um-Am-Af-Am-Gm-Um-Am | 398 | UmsEVP-Afs-Cf-Uf-Um-Uf-Am-Am-Um-Um-Um-Um-Am-Af-Am-Af-Cm-Cm-Cm-Am-Ams-Ums-Um | 433 |
| D835-DV26P+ | Ums-Ums-Gm-Gm-Gm-Um-Uf-Um-Uf-Af-Af-Am-Am-Um-Um-Am-Af-Am-Gm-Um-Am | 398 | UmsEVP-Afs-Cf-Uf-Um-Tgna-Am-Am-Um-Um-Um-Um-Am-Af-Am-Af-Cm-Cm-Cm-Am-Ams-Ums-Um | 564 |
| D835-DV26Pd7 B | Ums-Ums-Gm-Gm-Gm-Um-Uf-Um-Uf-Af-Af-Am-Am-Um-Td-Am-Af-Am-Gm-Um-Am | 526 | UmsEVP-Afs-Cf-Uf-Um-Uf-Ad-Am-Um-Um-Um-Um-Am-Af-Am-Af-Cm-Cm-Cm-Am-Ams-Ums-Um | 565 |
| D835-DV26Pd67 B | Ums-Ums-Gm-Gm-Gm-Um-Uf-Um-Uf-Af-Af-Am-Am-Um-Td-Ad-Af-Am-Gm-Um-Am | 527 | UmsEVP-Afs-Cf-Uf-Um-Td-Ad-Am-Um-Um-Um-Um-Am-Af-Am-Af-Cm-Cm-Cm-Am-Ams-Ums-Um | 566 |
| D578s-DV29P+ | Ams-Cms-Cm-Um-Um-Uf-Um-Cf-Um-Uf-Cm-Um-Am-Am-Um-Gf-Am-Gm-Um-Am | 426 | UmsEVP-Afs-Cm-Uf-Cf-Agna-Um-Um-Am-Gm-Am-Am-Gm-Af-Am-Af-Am-Gm-Gm-Ums-Gms-Gm | 567 |
| D578s-DV29Pd67 B | Ams-Cms-Cm-Um-Um-Uf-Um-Cf-Um-Uf-Cm-Um-Am-Ad-Td-Gf-Am-Gm-Um-Am | 528 | UmsEVP-Afs-Cm-Uf-Cf-Ad-Td-Um-Am-Gm-Am-Am-Gm-Af-Am-Af-Am-Gm-Gm-Ums-Gms-Gm | 568 |

(continued)

| Sequence ID | Sense strand sequence 5'-3' | SEQ ID NO. | Antisense strand sequence 5'-3' | SEQ ID NO. |
|---|---|---|---|---|
| D578s-DV29Pd7 B | Ams-Cms-Cm-Um-Um-Uf-Um-Cf-Um-Uf-Cm-Um-Am-Ad-Um-Gf-Am-Gm-Um-Am | 529 | UmsEVP-Afs-Cm-Uf-Cf-Af-Td-Um-Am-Gm-Am-Am-Gm-Af-Am-Af-Am-Gm-Gm-Ums-Gms-Gm | 569 |
| D579-DV25PG5 | Cms-Cms-Um-Um-Um-Um-Cf-Um-Uf-Cf-Uf-Am-Am-Um-Gm-Am-Gf-Um-Cm-Gm-Am-G5 | 530 | UmsEVP-Cfs-Gm-Am-Cm-Uf-Cm-Am-Um-Um-Am-Gm-Am-Af-Gm-Af-Am-Am-Am-Gm-Gms-Ums-Gm | 457 |
| D579-DV25Pd7 BG5 | Cms-Cms-Um-Um-Um-Um-Cf-Um-Uf-Cf-Uf-Am-Am-Um-Gd-Am-Gf-Um-Cm-Gm-Am-G5 | 531 | UmsEVP-Cfs-Gm-Am-Cm-Uf-Cd-Am-Um-Um-Am-Gm-Am-Af-Gm-Af-Am-Am-Am-Gm-Gms-Ums-Gm | 570 |
| D579-DV25Pd67 BG5 | Cms-Cms-Um-Um-Um-Um-Cf-Um-Uf-Cf-Uf-Am-Am-Um-Gd-Ad-Gf-Um-Cm-Gm-Am-G5 | 532 | UmsEVP-Cfs-Gm-Am-Cm-Td-Cd-Am-Um-Um-Am-Gm-Am-Af-Gm-Af-Am-Am-Am-Gm-Gms-Ums-Gm | 571 |
| D789-DV26PG5 | Gms-Ums-Um-Um-Gm-Um-Gf-Am-Af-Af-Cf-Am-Am-Am-Am-Am-Af-Gm-Um-Gm-Um-G5 | 533 | AmsEVP-Cfs-Af-Cf-Um-Uf-Um-Um-Um-Um-Gm-Um-Um-Uf-Cm-Af-Cm-Am-Am-Am-Cms-Ams-Am | 465 |
| D789-DV25PG5 | Gms-Ums-Um-Um-Gm-Um-Gf-Am-Af-Af-Cf-Am-Am-Am-Am-Am-Af-Gm-Um-Gm-Um-G5 | 533 | AmsEVP-Cfs-Am-Cm-Um-Uf-Um-Um-Um-Um-Gm-Um-Um-Uf-Cm-Af-Cm-Am-Am-Am-Cms-Ams-Am | 468 |
| D789-DV26Pd7 BG5 | Gms-Ums-Um-Um-Gm-Um-Gf-Am-Af-Af-Cf-Am-Am-Am-Ad-Am-Af-Gm-Um-Gm-Um-G5 | 534 | AmsEVP-Cfs-Af-Cf-Um-Uf-Td-Um-Um-Um-Gm-Um-Um-Uf-Cm-Af-Cm-Am-Am-Am-Cms-Ams-Am | 572 |
| D789-DV25Pd7 BG5 | Gms-Ums-Um-Um-Gm-Um-Gf-Am-Af-Af-Cf-Am-Am-Am-Ad-Am-Af-Gm-Um-Gm-Um-G5 | 534 | AmsEVP-Cfs-Am-Cm-Um-Uf-Td-Um-Um-Um-Gm-Um-Um-Uf-Cm-Af-Cm-Am-Am-Am-Cms-Ams-Am | 573 |

(continued)

| Sequence ID | Sense strand sequence 5'-3' | SEQ ID NO. | Antisense strand sequence 5'-3' | SEQ ID NO. |
|---|---|---|---|---|
| D789-DV26Pd67 BG5 | Gms-Ums-Um-Um-Gm-Um-Gf-Am-Af-Af-Cf-Am-Am-Am-Ad-Ad-Af-Gm-Um-Gm-Um-G5 | 535 | AmsEVP-Cfs-Af-Cf-Um-Td-Td-Um-Um-Um-Gm-Um-Um-Uf-Cm-Af-Cm-Am-Am-Am-Cms-Ams-Am | 574 |
| D789-DV25Pd67 BG5 | Gms-Ums-Um-Um-Gm-Um-Gf-Am-Af-Af-Cf-Am-Am-Am-Ad-Ad-Af-Gm-Um-Gm-Um-G5 | 535 | AmsEVP-Cfs-Am-Cm-Um-Td-Td-Um-Um-Um-Gm-Um-Um-Uf-Cm-Af-Cm-Am-Am-Am-Cms-Ams-Am | 575 |
| D791-DV26PG5 | Ums-Ums-Gm-Um-Gm-Am-Af-Am-Cf-Af-Af-Am-Am-Am-Am-Gm-Uf-Gm-Um-Um-Am-G5 | 536 | UmsEVP-Afs-Af-Cf-Am-Cf-Um-Um-Um-Um-Um-Um-Gm-Uf-Um-Uf-Cm-Am-Cm-Am-Ams-Ams-Cm | 476 |
| D791-DV26Pd7 BG5 | Ums-Ums-Gm-Um-Gm-Am-Af-Am-Cf-Af-Af-Am-Am-Am-Ad-Gm-Uf-Gm-Um-Um-Am-G5 | 537 | UmsEVP-Afs-Af-Cf-Am-Cf-Td-Um-Um-Um-Um-Um-Gm-Uf-Um-Uf-Cm-Am-Cm-Am-Ams-Ams-Cm | 576 |
| D791-DV26Pd67 BG5 | Ums-Ums-Gm-Um-Gm-Am-Af-Am-Cf-Af-Af-Am-Am-Am-Ad-Gd-Uf-Gm-Um-Um-Am-G5 | 538 | UmsEVP-Afs-Af-Cf-Am-Cd-Td-Um-Um-Um-Um-Um-Gm-Uf-Um-Uf-Cm-Am-Cm-Am-Ams-Ams-Cm | 577 |
| D795-DV27PG5 | Gms-Ams-Am-Am-Cm-Am-Af-Am-Af-Af-Af-Gm-Um-Gm-Um-Um-Cf-Cm-Cm-Um-Um-G5 | 539 | AmsEVP-Afs-Gm-Gf-Gf-Af-Am-Cm-Am-Cm-Um-Um-Um-Uf-Um-Uf-Gm-Um-Um-Um-Cms-Ams-Cm | 479 |
| D795-DV27Pd67 BG5 | Gms-Ams-Am-Am-Cm-Am-Af-Am-Af-Af-Af-Gm-Um-Gm-Td-Td-Cf-Cm-Cm-Um-Um-G5 | 540 | AmsEVP-Afs-Gm-Gf-Gf-Ad-Ad-Cm-Am-Cm-Um-Um-Um-Uf-Um-Uf-Gm-Um-Um-Um-Cms-Ams-Cm | 578 |
| D812-DV25PG5 | Cms-Cms-Um-Um-Um-Um-Cf-Am-Af-Gf-Uf-Um-Gm-Am-Gm-Am-Af-Cm-Am-Am-Am-G5 | 541 | UmsEVP-Ufs-Um-Gm-Um-Uf-Cm-Um-Cm-Am-Am-Cm-Um-Uf-Gm-Af-Am-Am-Am-Gm-Gms-Gms-Am | 450 |

(continued)

| Sequence ID | Sense strand sequence 5'-3' | SEQ ID NO. | Antisense strand sequence 5'-3' | SEQ ID NO. |
|---|---|---|---|---|
| D812-DV25Pd67 BG5 | Cms-Cms-Um-Um-Um-Um-Cf-Am-Af-Gf-Uf-Um-Gm-Am-Gd-Ad-Af-Cm-Am-Am-Am-G5 | 542 | UmsEVP-Ufs-Um-Gm-Um-Td-Cd-Um-Cm-Am-Am-Cm-Um-Uf-Gm-Af-Am-Am-Am-Gm-Gms-Gms-Am | 579 |
| D816-DV25PG5 | Ums-Ums-Cm-Am-Am-Gm-Uf-Um-Gf-Af-Gf-Am-Am-Cm-Am-Am-Af-Am-Am-Um-Um-G5 | 543 | AmsEVP-Afs-Um-Um-Um-Uf-Um-Gm-Um-Um-Cm-Um-Cm-Af-Am-Cf-Um-Um-Gm-Am-Ams-Ams-Am | 443 |
| D816-DV25Pd67 BG5 | Ums-Ums-Cm-Am-Am-Gm-Uf-Um-Gf-Af-Gf-Am-Am-Cm-Ad-Ad-Af-Am-Am-Um-Um-G5 | 544 | AmsEVP-Afs-Um-Um-Um-Td-Td-Gm-Um-Um-Cm-Um-Cm-Af-Am-Cf-Um-Um-Gm-Am-Ams-Ams-Am | 580 |
| D835-DV26PG5 | Ums-Ums-Gm-Gm-Gm-Um-Uf-Um-Uf-Af-Af-Am-Am-Um-Um-Am-Af-Am-Gm-Um-Am-G5 | 545 | UmsEVP-Afs-Cf-Uf-Um-Uf-Am-Am-Um-Um-Um-Um-Am-Af-Am-Af-Cm-Cm-Cm-Am-Ams-Ums-Um | 433 |
| D835-DV26Pd7 BG5 | Ums-Ums-Gm-Gm-Gm-Um-Uf-Um-Uf-Af-Af-Am-Am-Um-Td-Am-Af-Am-Gm-Um-Am-G5 | 546 | UmsEVP-Afs-Cf-Uf-Um-Uf-Ad-Am-Um-Um-Um-Um-Am-Af-Am-Af-Cm-Cm-Cm-Am-Ams-Ums-Um | 565 |
| D835-DV26Pd67 BG5 | Ums-Ums-Gm-Gm-Gm-Um-Uf-Um-Uf-Af-Af-Am-Am-Um-Td-Ad-Af-Am-Gm-Um-Am-G5 | 547 | UmsEVP-Afs-Cf-Uf-Um-Td-Ad-Am-Um-Um-Um-Um-Am-Af-Am-Af-Cm-Cm-Cm-Am-Ams-Ums-Um | 566 |
| D838-DV29PG5 | Gms-Gms-Um-Um-Um-Um-Af-Am-Af-Am-Uf-Um-Am-Am-Am-Gm-Uf-Am-Um-Am-Am-G5 | 548 | UmsEVP-Ufs-Am-Uf-Af-Cf-Um-Um-Um-Am-Am-Um-Um-Uf-Um-Af-Am-Am-Am-Cm-Cms-Cms-Am | 496 |
| D838-DV29Pd7 BG5 | Gms-Gms-Um-Um-Um-Um-Af-Am-Af-Am-Uf-Um-Am-Am-Ad-Gm-Uf-Am-Um-Am-Am-G5 | 549 | UmsEVP-Ufs-Am-Uf-Af-Cf-Td-Um-Um-Am-Am-Um-Um-Uf-Um-Af-Am-Am-Am-Cm-Cms-Cms-Am | 581 |

(continued)

| Sequence ID | Sense strand sequence 5'-3' | SEQ ID NO. | Antisense strand sequence 5'-3' | SEQ ID NO. |
|---|---|---|---|---|
| D838-DV29Pd67 BG5 | Gms-Gms-Um-Um-Um-Um-Af-Am-Af-Am-Uf-Um-Am-Am-Ad-Gd-Uf-Am-Um-Am-Am-G5 | 550 | UmsEVP-Ufs-Am-Uf-Af-Cd-Td-Um-Um-Am-Am-Um-Um-Uf-Um-Af-Am-Am-Am-Cm-Cms-Cms-Am | 582 |
| D839-DV29Pd7 BG5 | Gms-Ums-Um-Um-Um-Am-Af-Am-Af-Um-Uf-Am-Am-Am-Gd-Um-Af-Um-Am-Cm-Am-G5 | 552 | UmsEVP-Gfs-Um-Af-Uf-Af-Cd-Um-Um-Um-Am-Am-Um-Uf-Um-Uf-Am-Am-Am-Am-Cms-Cms-Cm | 583 |
| D839-DV29Pd67 BG5 | Gms-Ums-Um-Um-Um-Am-Af-Am-Af-Um-Uf-Am-Am-Am-Gd-Td-Af-Um-Am-Cm-Am-G5 | 553 | UmsEVP-Gfs-Um-Af-Uf-Ad-Cd-Um-Um-Um-Am-Am-Um-Uf-Um-Uf-Am-Am-Am-Am-Cms-Cms-Cm | 584 |
| D576s-DV29PG5 | Cms-Cms-Am-Cm-Cm-Uf-Um-Uf-Um-Cf-Um-Um-Cm-Um-Am-Af-Um-Gm-Am-Am-G5 | 554 | UmsEVP-Ufs-Cm-Af-Uf-Uf-Am-Gm-Am-Am-Gm-Am-Am-Af-Am-Gf-Gm-Um-Gm-Gms-Gms-Am | 488 |
| D576s-DV29Pd7 BG5 | Cms-Cms-Am-Cm-Cm-Uf-Um-Uf-Um-Cf-Um-Um-Cm-Td-Am-Af-Um-Gm-Am-Am-G5 | 555 | UmsEVP-Ufs-Cm-Af-Uf-Uf-Ad-Gm-Am-Am-Gm-Am-Am-Af-Am-Gf-Gm-Um-Gm-Gms-Gms-Am | 585 |
| D576s-DV29Pd67 BG5 | Cms-Cms-Am-Cm-Cm-Uf-Um-Uf-Um-Cf-Um-Um-Cm-Td-Ad-Af-Um-Gm-Am-Am-G5 | 556 | UmsEVP-Ufs-Cm-Af-Uf-Td-Ad-Gm-Am-Am-Gm-Am-Am-Af-Am-Gf-Gm-Um-Gm-Gms-Gms-Am | 586 |
| D578s-DV29PG5 | Ams-Cms-Cm-Um-Um-Uf-Um-Cf-Um-Uf-Cm-Um-Am-Am-Um-Gf-Am-Gm-Um-Am-G5 | 557 | UmsEVP-Afs-Cm-Uf-Cf-Af-Um-Um-Am-Gm-Am-Am-Gm-Af-Am-Af-Am-Gm-Gm-Ums-Gms-Gm | 492 |
| D578s-DV29Pd7 BG5 | Ams-Cms-Cm-Um-Um-Uf-Um-Cf-Um-Uf-Cm-Um-Am-Ad-Um-Gf-Am-Gm-Um-Am-G5 | 558 | UmsEVP-Afs-Cm-Uf-Cf-Af-Td-Um-Am-Gm-Am-Am-Gm-Af-Am-Af-Am-Gm-Gm-Ums-Gms-Gm | 569 |

(continued)

| Sequence ID | Sense strand sequence 5'-3' | SEQ ID NO. | Antisense strand sequence 5'-3' | SEQ ID NO. |
|---|---|---|---|---|
| D578s-DV29Pd67 BG5 | Ams-Cms-Cm-Um-Um-Uf-Um-Cf-Um-Uf-Cm-Um-Am-Ad-Td-Gf-Am-Gm-Um-Am-G5 | 559 | UmsEVP-Afs-Cm-Uf-Cf-Ad-Td-Um-Am-Gm-Am-Am-Gm-Af-Am-Af-Am-Gm-Gm-Ums-Gms-Gm | 568 |
| D585s-DV27PG5 | Cms-Ums-Um-Cm-Um-Af-Am-Uf-Gf-Af-Gm-Um-Cm-Gm-Am-Cf-Um-Um-Um-Am-G5 | 560 | UmsEVP-Afs-Am-Af-Gf-Uf-Cm-Gm-Am-Cm-Um-Cm-Am-Uf-Um-Af-Gm-Am-Am-Gms-Ams-Am | 483 |
| Zilebesiran | Gms-Ums-Cm-Am-Um-Cm-Cf-Am-Cf-Af-Af-Um-Gm-Am-Gm-Am-Gm-Um-Am-Cm-Am-GL | 561 | Ums-Gfs-Um-Am-Cm-Tgna-Cm-Um-Cm-Am-Um-Um-Gm-Uf-Gm-Gf-Am-Um-Gm-Am-Cms-Gms-Am | 587 |
| DNC-DV29PG5 | Gms-Ums-Gm-Am-Am-Am-Cf-Am-Af-Cm-Uf-Am-Gm-Um-Gm-Cm-Af-Cm-Cm-Um-Cm-G5 | 562 | UmsEVP-Afs-Gm-Af-Gf-Gf-Um-Gm-Cm-Am-Cm-Um-Am-Gf-Um-Uf-Gm-Um-Um-Um-Cms-Ams-Cm | 588 |
| APC-ZL | Gms-Ums-Cm-Am-Um-Cm-Cf-Am-Cf-Af-Af-Um-Gm-Am-Gm-Am-Gm-Um-Am-Cm-Am | 563 | Ums-Gfs-Um-Am-Cm-Tgna-Cm-Um-Cm-Am-Um-Um-Gm-Uf-Gm-Gf-Am-Um-Gm-Am-Cms-Gms-Am | 587 |

## Claims

1. A double-stranded RNAi agent, comprising any one oligonucleotide duplex selected from the following pairings of a sense strand and an antisense strand:

(1) the sense strand has the sequence shown in SEQ ID NO: 11 or a fragment thereof, or a modified sequence of the sequence or the fragment thereof; and the antisense strand has the sequence shown in SEQ ID NO: 24 or a fragment thereof, or a modified sequence of the sequence or the fragment thereof;
(2) the sense strand has the sequence shown in SEQ ID NO: 4 or a fragment thereof, or a modified sequence of the sequence or the fragment thereof; and the antisense strand has the sequence shown in SEQ ID NO: 17 or a fragment thereof, or a modified sequence of the sequence or the fragment thereof;
(3) the sense strand has the sequence shown in SEQ ID NO: 3 or a fragment thereof, or a modified sequence of the sequence or the fragment thereof; and the antisense strand has the sequence shown in SEQ ID NO: 16 or a fragment thereof, or a modified sequence of the sequence or the fragment thereof;
(4) the sense strand has the sequence shown in SEQ ID NO: 12 or a fragment thereof, or a modified sequence of the sequence or the fragment thereof; and the antisense strand has the sequence shown in SEQ ID NO: 25 or a fragment thereof, or a modified sequence of the sequence or the fragment thereof;
(5) the sense strand has the sequence shown in SEQ ID NO: 6 or a fragment thereof, or a modified sequence of the sequence or the fragment thereof; and the antisense strand has the sequence shown in SEQ ID NO: 19 or a fragment thereof, or a modified sequence of the sequence or the fragment thereof;
(6) the sense strand has the sequence shown in SEQ ID NO: 10 or a fragment thereof, or a modified sequence of the sequence or the fragment thereof; and the antisense strand has the sequence shown in SEQ ID NO: 23 or a fragment thereof, or a modified sequence of the sequence or the fragment thereof;
(7) the sense strand has the sequence shown in SEQ ID NO: 1 or a fragment thereof, or a modified sequence of the sequence or the fragment thereof; and the antisense strand has the sequence shown in SEQ ID NO: 14 or a fragment thereof, or a modified sequence of the sequence or the fragment thereof;
(8) the sense strand has the sequence shown in SEQ ID NO: 2 or a fragment thereof, or a modified sequence of the sequence or the fragment thereof; and the antisense strand has the sequence shown in SEQ ID NO: 15 or a fragment thereof, or a modified sequence of the sequence or the fragment thereof;
(9) the sense strand has the sequence shown in SEQ ID NO: 5 or a fragment thereof, or a modified sequence of the sequence or the fragment thereof; and the antisense strand has the sequence shown in SEQ ID NO: 18 or a fragment thereof, or a modified sequence of the sequence or the fragment thereof;
(10) the sense strand has the sequence shown in SEQ ID NO: 7 or a fragment thereof, or a modified sequence of the sequence or the fragment thereof; and the antisense strand has the sequence shown in SEQ ID NO: 20 or a fragment thereof, or a modified sequence of the sequence or the fragment thereof;
(11) the sense strand has the sequence shown in SEQ ID NO: 8 or a fragment thereof, or a modified sequence of the sequence or the fragment thereof; and the antisense strand has the sequence shown in SEQ ID NO: 21 or a fragment thereof, or a modified sequence of the sequence or the fragment thereof;
(12) the sense strand has the sequence shown in SEQ ID NO: 9 or a fragment thereof, or a modified sequence of the sequence or the fragment thereof; and the antisense strand has the sequence shown in SEQ ID NO: 22 or a fragment thereof, or a modified sequence of the sequence or the fragment thereof;
(13) the sense strand has the sequence shown in SEQ ID NO: 13 or a fragment thereof, or a modified sequence of the sequence or the fragment thereof; and the antisense strand has the sequence shown in SEQ ID NO: 26 or a fragment thereof, or a modified sequence of the sequence or the fragment thereof.

2. The double-stranded RNAi agent according to claim 1, wherein the double-stranded RNAi agent satisfies any one of the following conditions:

(1) the nucleotide sequence of the sense strand differs from any one of the sequences of SEQ ID NOs: 1 to 13 by 1 to 3 nucleotides; and
(2) the nucleotide sequence of the antisense strand differs from any one of the sequences of SEQ ID NOs: 14 to 26 by 1 to 3 nucleotides.

3. The double-stranded RNAi agent according to claim 1 or 2, wherein the sense strand or the antisense strand comprises at least one modified nucleotide selected from a deoxy-nucleotide, a 2'-deoxy-thymidine (dT) nucleotide, a 2'-O-methyl modified nucleotide, a 2'-fluoro modified nucleotide, a 2'-deoxy-modified nucleotide, a locked nucleotide, an unlocked nucleotide, a conformationally restricted nucleotide, a constrained ethyl nucleotide, an abasic nucleotide, a 2'-amino-modified nucleotide, a 2'-O-allyl-modified nucleotide, a 2'-C-alkyl-modified nucleotide, a 2'-hydroxy-modified nucleotide, a 2'-methoxyethyl modified nucleotide, a 2'-O-alkyl-modified nucleotide, a morpholino nucleotide, a phosphoramidate, a nucleotide comprising a non-natural base, a tetrahydropyran modified nucleotide, a 1,5-anhydrohexitol modified nucleotide, a cyclohexenyl modified nucleotide, a nucleotide comprising a phosphorothioate group, a nucleotide comprising a methylphosphonate group, a nucleotide comprising a 5'-phosphate group, and a nucleotide comprising a 5'-phosphate mimic; and combinations thereof;
or, nucleotide monomers are linked by a 3',5'-phosphodiester bond.

**4.** The double-stranded RNAi agent according to any one of claims 1 to 3, wherein the double-stranded RNAi agent satisfies one or more of the following conditions:

(1) at least one strand comprises a 3' overhang of at least 1 nucleotide or at least 2 nucleotide;
(2) the double-stranded RNAi agent has a duplex region of 15 to 30 nucleotide pairs in length, or a duplex region of 17 to 25 nucleotide pairs in length, or a duplex region of 19 to 23 nucleotide pairs in length, or a duplex region of 21 nucleotide pairs in length;
(3) each strand has 15 to 30 nucleotides, or 19 to 25 nucleotides;
(4) the sense strand has 21 nucleotides and the antisense strand has 23 nucleotides;
(5) all nucleotide modifications on the sense and antisense strands are chemical modifications at the 2' position of the nucleotide ribose; and
(6) the 3',5'-phosphodiester bond between nucleotide monomers is phosphorothioate-modified.

**5.** The double-stranded RNAi agent according to any one of claims 1 to 4, wherein the double-stranded RNAi agent satisfies one or more of the following conditions:

(1) the chemical modification at the 2' position of the nucleotide ribose is selected from any one of 2'-O-methyl, 2'-methoxyethyl, 2'-fluoro, 2'-benzyloxy, 2'-methylcarbonylamino, and 2'-pyridinylmethoxy, or a combination thereof;
(2) the chemical modification at the 2' position of each nucleotide ribose is selected from a combination of 2'-O-methyl and 2'-fluoro;
(3) the chemical modification at the 2' position of each nucleotide ribose is selected from an alternating combination of 2'-O-methyl and 2'-fluoro; and
(4) the chemical modification at the 2' position of each nucleotide ribose is as follows: on the antisense strand, all odd-numbered positions are modified with 2'-O-methyl, and all even-numbered positions are modified with 2'-fluoro; on the sense strand, positions corresponding to 2'-O-methyl modifications on the antisense strand are modified with 2'-fluoro, and positions corresponding to 2'-fluoro modifications on the antisense strand are modified with 2'-O-methyl.

**6.** The double-stranded RNAi agent according to any one of claims 1 to 5, further comprising the following modifications:

(1) the antisense strand is modified according to one of the modification patterns shown in Table 42;

the sense strand is modified according to one of the modification patterns shown in Table 43;
wherein the antisense strand is modified according to modification pattern A, and the sense strand is modified according to modification pattern a;
wherein the antisense strand is modified according to modification pattern B, and the sense strand is modified according to modification pattern a;
wherein the antisense strand is modified according to modification pattern C, and the sense strand is modified according to modification pattern a;
wherein the antisense strand is modified according to modification pattern B, and the sense strand is modified according to modification pattern b;
wherein the antisense strand is modified according to modification pattern C, and the sense strand is modified according to modification pattern b;
wherein the antisense strand is modified according to modification pattern D, and the sense strand is modified according to modification pattern b;
wherein the antisense strand is modified according to modification pattern E, and the sense strand is modified according to modification pattern b; or
wherein the antisense strand is modified according to modification pattern F, and the sense strand is modified according to modification pattern b; or

(2) the antisense strand is modified according to one of the modification patterns shown in Table 44;

the sense strand is modified according to one of the modification patterns shown in Table 45;
wherein the antisense strand is modified according to modification pattern A, and the sense strand is modified according to modification pattern a;
wherein the antisense strand is modified according to modification pattern B, and the sense strand is modified according to modification pattern a;
wherein the antisense strand is modified according to modification pattern C, and the sense strand is modified according to modification pattern a;
wherein the antisense strand is modified according to modification pattern B, and the sense strand is modified according to modification pattern b;
wherein the antisense strand is modified according to modification pattern C, and the sense strand is modified according to modification pattern b;
wherein the antisense strand is modified according to modification pattern D, and the sense strand is modified according to modification pattern b;
wherein the antisense strand is modified according to modification pattern E, and the sense strand is modified according to modification pattern b; or
wherein the antisense strand is modified according to modification pattern F, and the sense strand is modified according to modification pattern b.

**7.** The double-stranded RNAi agent according to any one of claims 1 to 6, wherein the double-stranded RNAi agent satisfies one or more of the following conditions:

(1) the nucleotides at positions 2 to 8 from the 5' end of the antisense strand are modified with a modification group, wherein the modification group is selected from UNA, GNA, and DNA, wherein the structures of UNA and GNA are as follows:

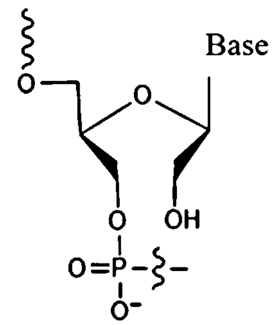

Unlocked nucleic acid (UNA)

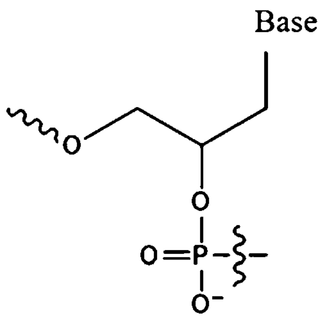

Glycol nucleic acid (GNA)

;

wherein the base is selected from adenine, guanine, cytosine, thymine, and uracil;
(2) the 5'-carbon atom of the 5'-terminal nucleotide glycoside of a modified antisense strand is phosphorylated, and the phosphorylation of the 5'-carbon atom is selected from the following 5'-phosphorylation groups: 5'-vinylphosphonate group (5'-E-VP), 5'-methylphosphonate group (5'-MP), 5'-C-methylphosphonate group, 5'-phosphorothioate group (5'-PS), and 5'-phosphate group (5'-P), the structures of which are shown below, respectively:

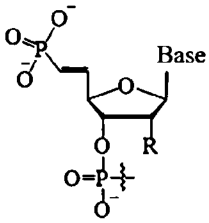

5'-vinylphosphonate group (5'-E-VP)

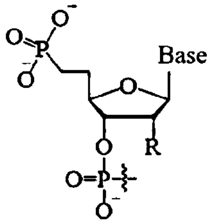

5'-methylphosphonate group (5'-MP)

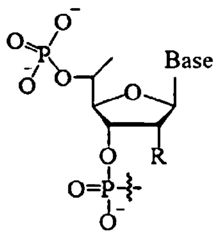

5'-C-methylphosphonate group

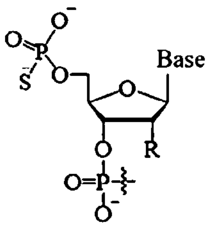

5'-phosphorothioate group (5'-PS)

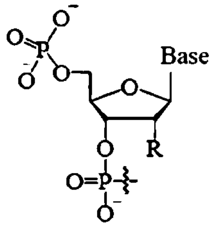

5'-phosphate group (5'-P)

;

wherein R is hydrogen, hydroxyl, amino, $C_{1-4}$ alkyl, aryl, $C_{1-4}$ alkoxy, $C_{1-4}$ alkylcarbonylamino, or halogen; and

the base is selected from adenine, guanine, cytosine, thymine, and uracil; and

(3) the 3',5'-phosphodiester bond between nucleotides at the terminus of the sequence is phosphorothioate-modified to form a chirally pure 3',5'-phosphorothioate bond, wherein the 5' ends of both the sense and antisense strands contain 1 to 3 phosphorothioate linkages, and the 3' end of the antisense strand contains 1 to 3 phosphorothioate linkages.

**8.** The double-stranded RNAi agent according to claim 1 or 2, wherein the double-stranded RNAi agent comprises any one oligonucleotide duplex selected from the following pairings of a sense strand and an antisense strand:

(1) the sense strand has the sequence shown in SEQ ID NO: 209; the antisense strand has the sequence shown in SEQ ID NO: 222;
(2) the sense strand has the sequence shown in SEQ ID NO: 202; the antisense strand has the sequence shown in SEQ ID NO: 215;
(3) the sense strand has the sequence shown in SEQ ID NO: 201; the antisense strand has the sequence shown in SEQ ID NO: 214;
(4) the sense strand has the sequence shown in SEQ ID NO: 210; the antisense strand has the sequence shown in SEQ ID NO: 223;
(5) the sense strand has the sequence shown in SEQ ID NO: 204; the antisense strand has the sequence shown in SEQ ID NO: 217;
(6) the sense strand has the sequence shown in SEQ ID NO: 208; the antisense strand has the sequence shown in SEQ ID NO: 221;
(7) the sense strand has the sequence shown in SEQ ID NO: 199; the antisense strand has the sequence shown in SEQ ID NO: 212;
(8) the sense strand has the sequence shown in SEQ ID NO: 200; the antisense strand has the sequence shown in SEQ ID NO: 213;
(9) the sense strand has the sequence shown in SEQ ID NO: 203; the antisense strand has the sequence shown in SEQ ID NO: 216;
(10) the sense strand has the sequence shown in SEQ ID NO: 205; the antisense strand has the sequence shown in SEQ ID NO: 218;
(11) the sense strand has the sequence shown in SEQ ID NO: 206; the antisense strand has the sequence shown in SEQ ID NO: 219;
(12) the sense strand has the sequence shown in SEQ ID NO: 207; the antisense strand has the sequence shown in SEQ ID NO: 220;
(13) the sense strand has the sequence shown in SEQ ID NO: 211; the antisense strand has the sequence shown in SEQ ID NO: 224.

**9.** A conjugate for reducing the expression of AGT, comprising the double-stranded RNAi agent according to any one of claims 1 to 8 and a ligand conjugated thereto.

**10.** The conjugate according to claim 9, wherein the conjugate satisfies one or more of the following conditions:

(1) the ligand is conjugated to the 3' end or the 5' end of the sense strand of the oligonucleotide;
(2) the ligand is a GalNAc derivative attached via a bivalent or trivalent branched linker;

(3) the ligand is as follows:

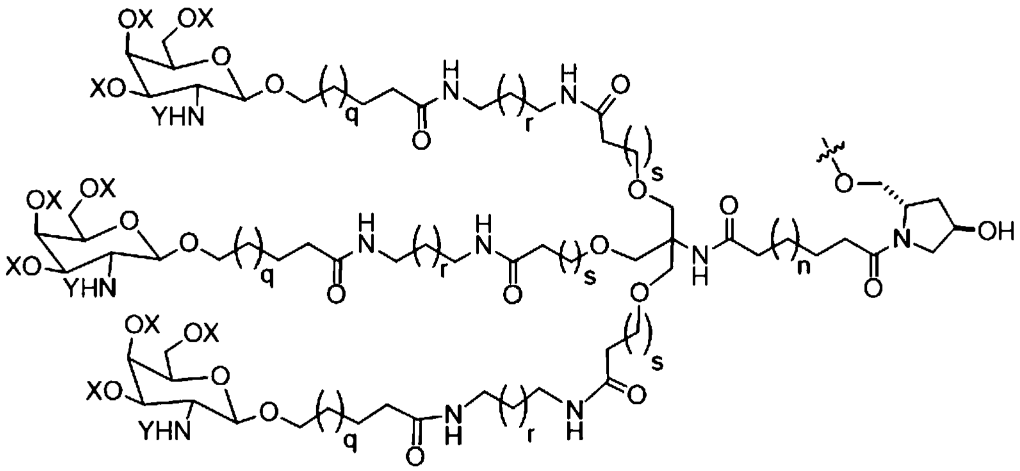

,

wherein X is hydrogen, a hydroxyl protecting group, or H, the hydroxyl protecting group being selected from acetyl, benzoyl, and isobutyryl; Y is an amino protecting group or H, the amino protecting group being selected from formyl, acetyl, propionyl, n-butyryl, and isobutyryl; n is an integer from 0 to 20; q, r, and s are independently integers from 1 to 7; and

(4) the ligand is as follows:

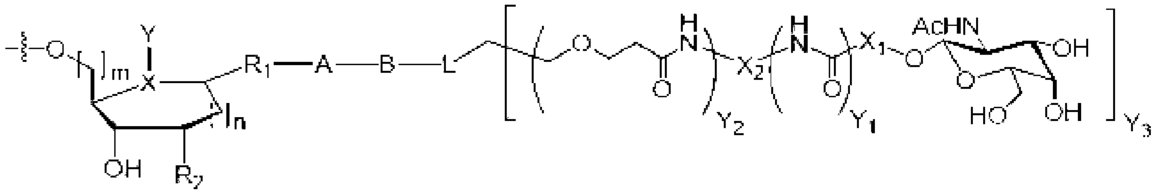

wherein X is oxygen, nitrogen, or sulfur;
Y is alkyl or aryl;
$R_1$ is oxygen or sulfur;
$R_2$ is hydrogen, amino, $C_{1-4}$ alkyl, aryl, $C_{1-4}$ alkoxy, or halogen;
A is $-(CH_2)_a-$, $-(CH_2CH_2O)_b-$, $-((CH_2)_cNHCO)_d-$, or $-((CH_2)_cCONH)_d-$, wherein a is an integer from 1 to 15, b is an integer from 1 to 7, c is an integer from 1 to 7, and d is an integer from 1 to 5;
B is $-(CH_2)_e-$, wherein e is an integer from 0 to 7;
L is -CONH- or -NHCO-;
$X_1$ is $-(CH_2)f-$ or $-(CH_2CH_2O)_fCH_2-$, wherein f is an integer from 1 to 5;
$X_2$ is $-(CH_2)_g-$, wherein g is an integer from 1 to 6;
$Y_1$ is 0 or 1;
$Y_2$ is 0, 1, or 2;
$Y_3$ is 1, 2, or 3;
m is an integer from 0 to 4;
n is an integer from 0 to 4.

**11.** The conjugate according to claim 9 or 10, wherein the conjugate satisfies one or more of the following conditions:

(1) the ligand is as follows:

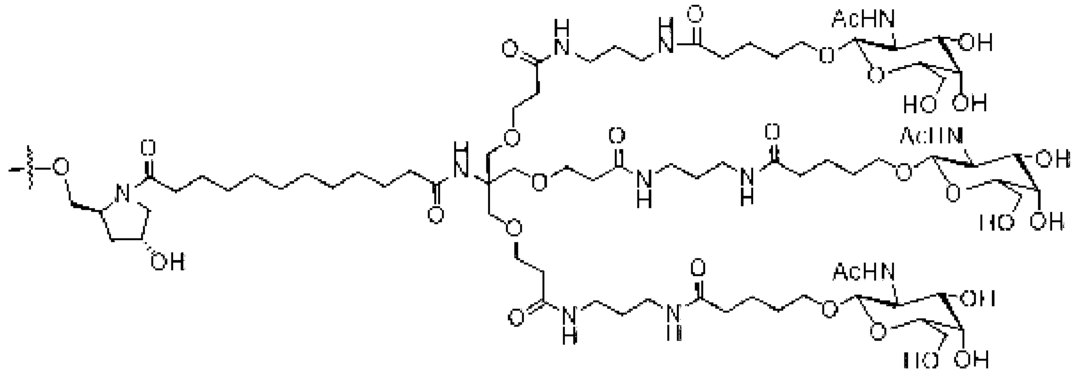

;

(2) the ligand is G4, G5, G6, or G7, with a structure as shown below:

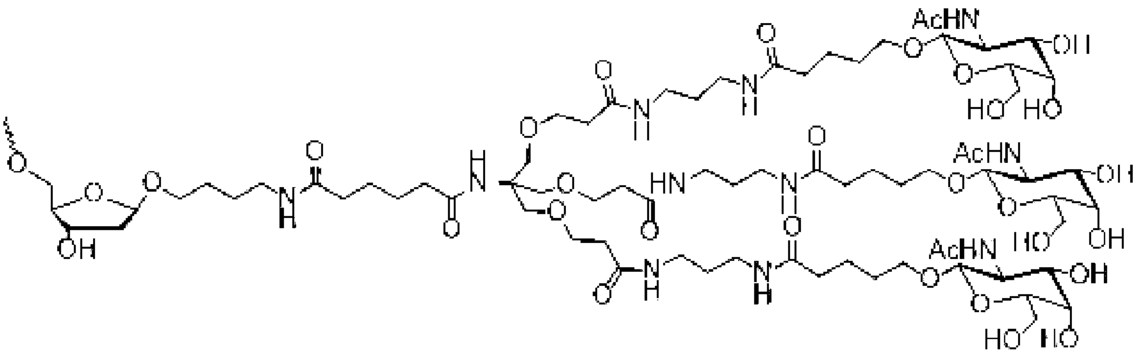

G4

,

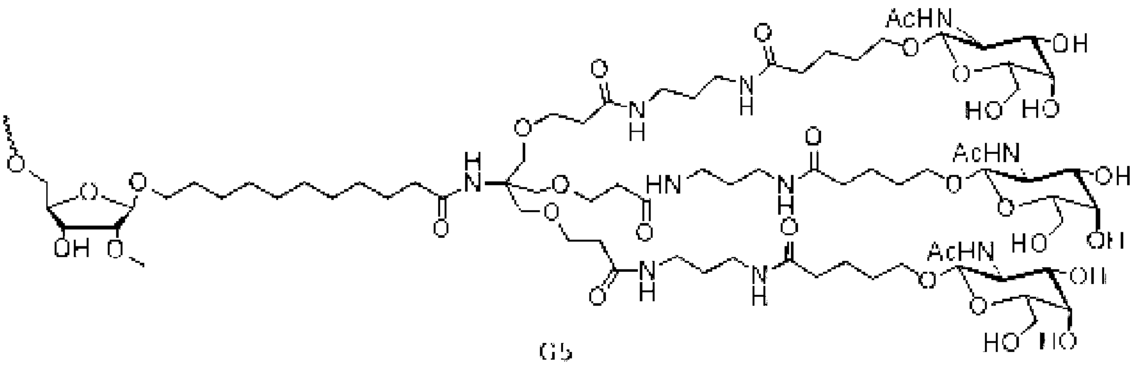

G5

,

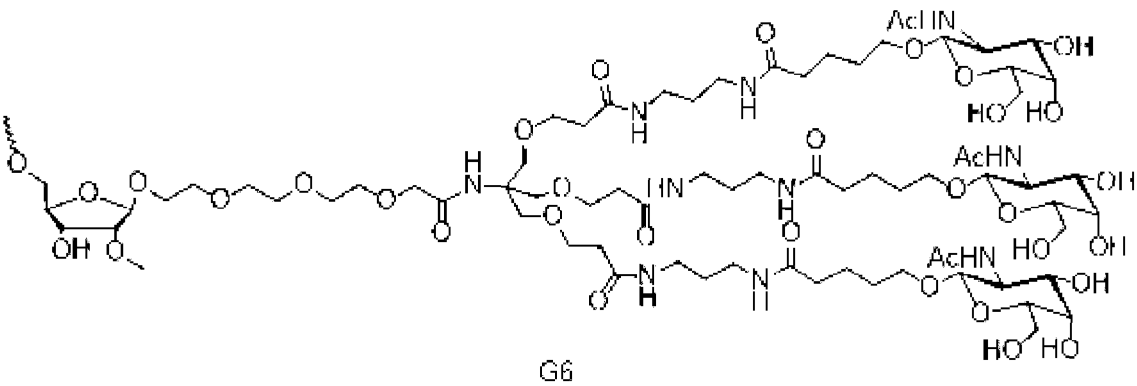

G6

,

or

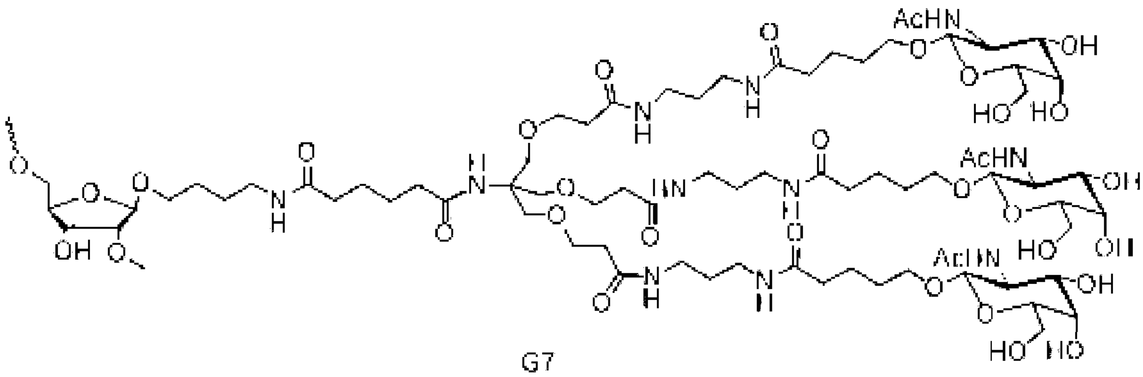

G7

;

and

(3) the conjugate has a structure as shown below:

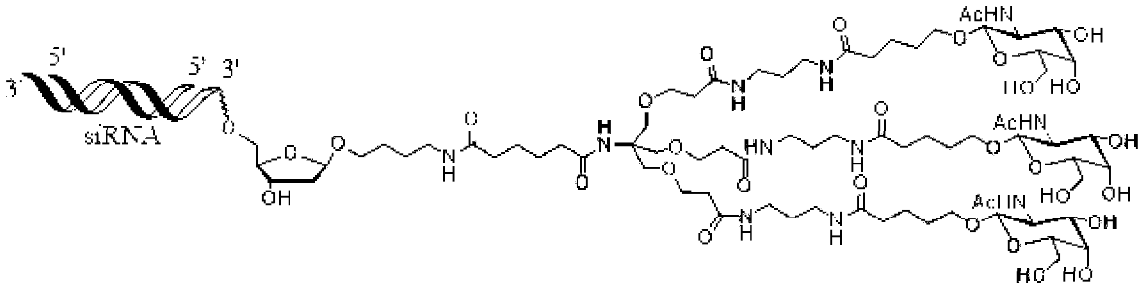

,

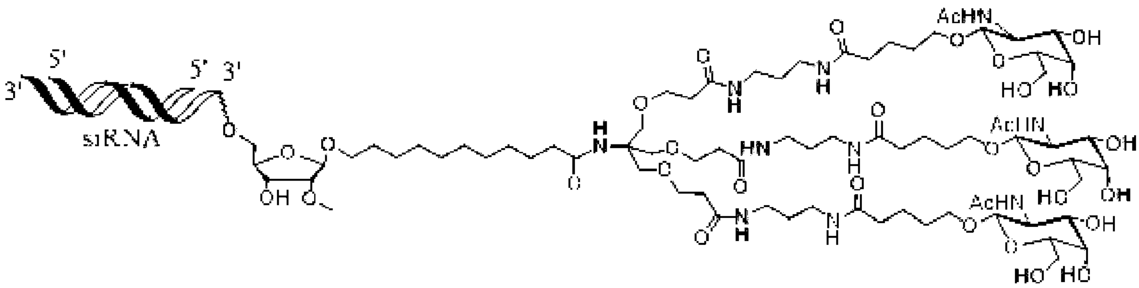

,

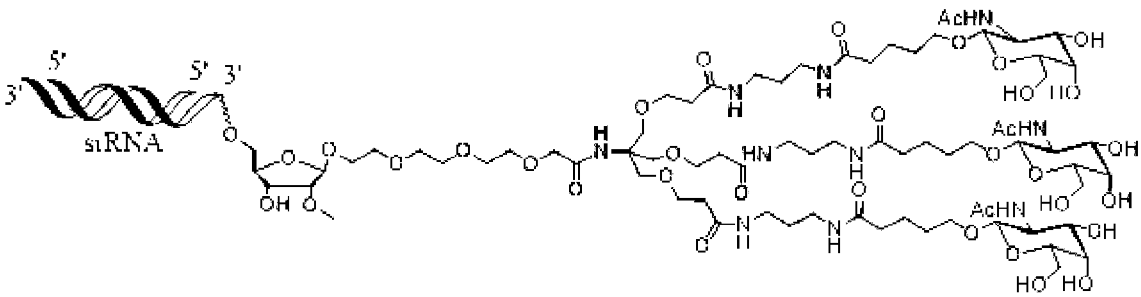

,

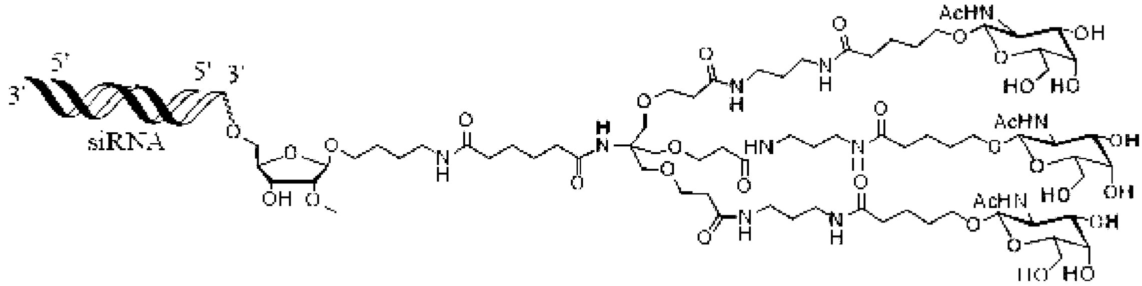

,

or

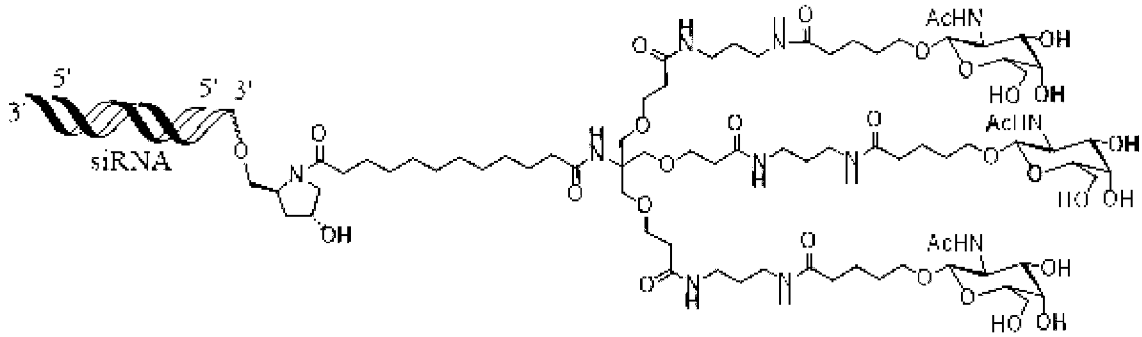

.

**12.** The conjugate according to any one of claims 9 to 11, wherein the double-stranded RNAi agent comprises any one oligonucleotide duplex selected from the following pairings of a sense strand and an antisense strand:

(1) the sense strand has the sequence shown in SEQ ID NO: 423; and the antisense strand has the sequence shown in SEQ ID NO: 488;
(2) the sense strand has the sequence shown in SEQ ID NO: 406; and the antisense strand has the sequence shown in SEQ ID NO: 453;
(3) the sense strand has the sequence shown in SEQ ID NO: 404; and the antisense strand has the sequence shown in SEQ ID NO: 450;
(4) the sense strand has the sequence shown in SEQ ID NO: 426; and the antisense strand has the sequence shown in SEQ ID NO: 492;
(5) the sense strand has the sequence shown in SEQ ID NO: 412; and the antisense strand has the sequence shown in SEQ ID NO: 465;
(6) the sense strand has the sequence shown in SEQ ID NO: 412; and the antisense strand has the sequence shown in SEQ ID NO: 468;
(7) the sense strand has the sequence shown in SEQ ID NO: 421; and the antisense strand has the sequence shown in SEQ ID NO: 483;
(8) the sense strand has the sequence shown in SEQ ID NO: 417; and the antisense strand has the sequence shown in SEQ ID NO: 476;
(9) the sense strand has the sequence shown in SEQ ID NO: 419; and the antisense strand has the sequence shown in SEQ ID NO: 479;
(10) the sense strand has the sequence shown in SEQ ID NO: 401; and the antisense strand has the sequence shown in SEQ ID NO: 443;
(11) the sense strand has the sequence shown in SEQ ID NO: 398; and the antisense strand has the sequence shown in SEQ ID NO: 433;
(12) the sense strand has the sequence shown in SEQ ID NO: 429; and the antisense strand has the sequence shown in SEQ ID NO: 496;
(13) the sense strand has the sequence shown in SEQ ID NO: 414; and the antisense strand has the sequence shown in SEQ ID NO: 471;

wherein the oligonucleotide duplex is conjugated with ligand G5;
or, the double-stranded RNAi agent comprises any one oligonucleotide duplex selected from the following pairings of a sense strand and an antisense strand:

(14) the sense strand has the sequence shown in SEQ ID NO: 554; and the antisense strand has the sequence shown in SEQ ID NO: 488;
(15) the sense strand has the sequence shown in SEQ ID NO: 530; and the antisense strand has the sequence shown in SEQ ID NO: 457;
(16) the sense strand has the sequence shown in SEQ ID NO: 531; and the antisense strand has the sequence shown in SEQ ID NO: 570;
(17) the sense strand has the sequence shown in SEQ ID NO: 541; and the antisense strand has the sequence shown in SEQ ID NO: 450;
(18) the sense strand has the sequence shown in SEQ ID NO: 557; and the antisense strand has the sequence shown in SEQ ID NO: 492;
(19) the sense strand has the sequence shown in SEQ ID NO: 533; and the antisense strand has the sequence shown in SEQ ID NO: 465;
(20) the sense strand has the sequence shown in SEQ ID NO: 560; and the antisense strand has the sequence shown in SEQ ID NO: 483;

wherein the oligonucleotide duplex is conjugated with ligand G5;
or, the double-stranded RNAi agent comprises an oligonucleotide duplex formed by pairing of the sense strand shown in SEQ ID NO: 423 and the antisense strand shown in SEQ ID NO: 488, and the oligonucleotide duplex is conjugated with ligand G5.

**13.** A nucleic acid-protein composition, comprising a duplex region of the double-stranded RNAi agent according to any one of claims 1 to 8, and a nuclease;
or, the nucleic acid-protein composition comprises an antisense strand of the duplex region of the double-stranded RNAi agent, and a nuclease.

**14.** A pharmaceutical composition, comprising the double-stranded RNAi agent according to any one of claims 1 to 8, the conjugate according to any one of claims 9 to 12, or the nucleic acid-protein composition according to claim 13, and a pharmaceutically acceptable carrier.

**15.** The double-stranded RNAi agent according to any one of claims 1 to 8, the conjugate according to any one of claims 9 to 12, the nucleic acid-protein composition according to claim 13, or the pharmaceutical composition according to claim 14 for use in treating a disease associated with AGT gene expression, or for use in treating hypertension and cardiovascular and cerebrovascular diseases.

Alternately modified sequences with inhibition rates above 40% against AGT

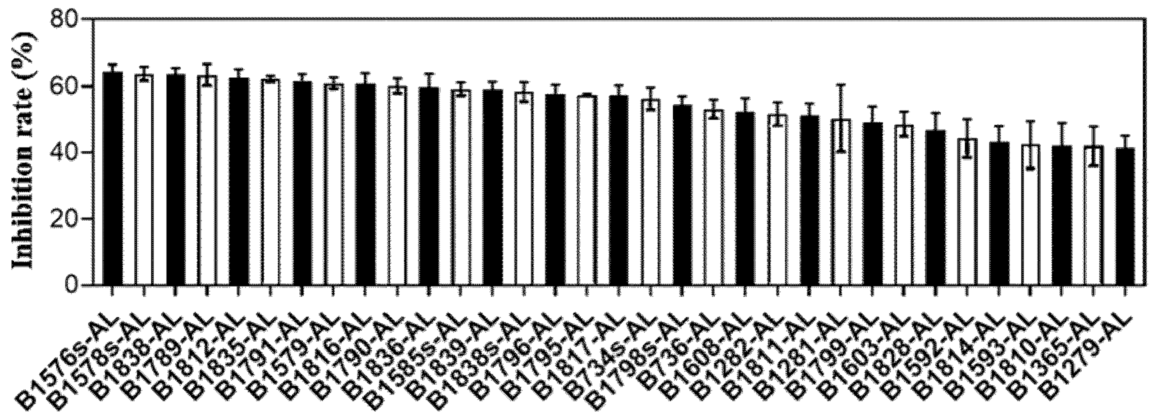


FIG. 1

Alternately modified sequences with inhibition rates of 25% to 40% against AGT

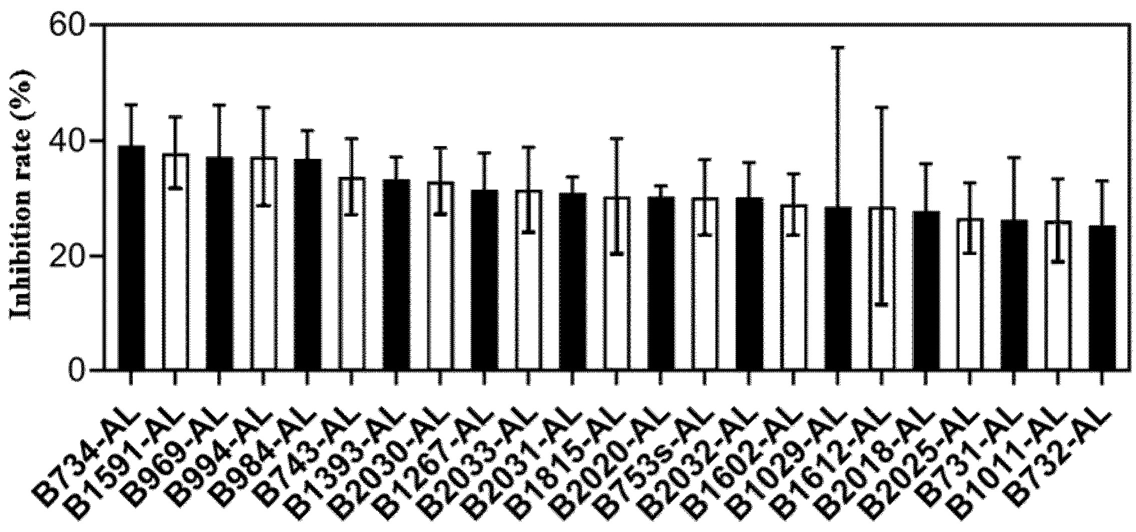


FIG. 2

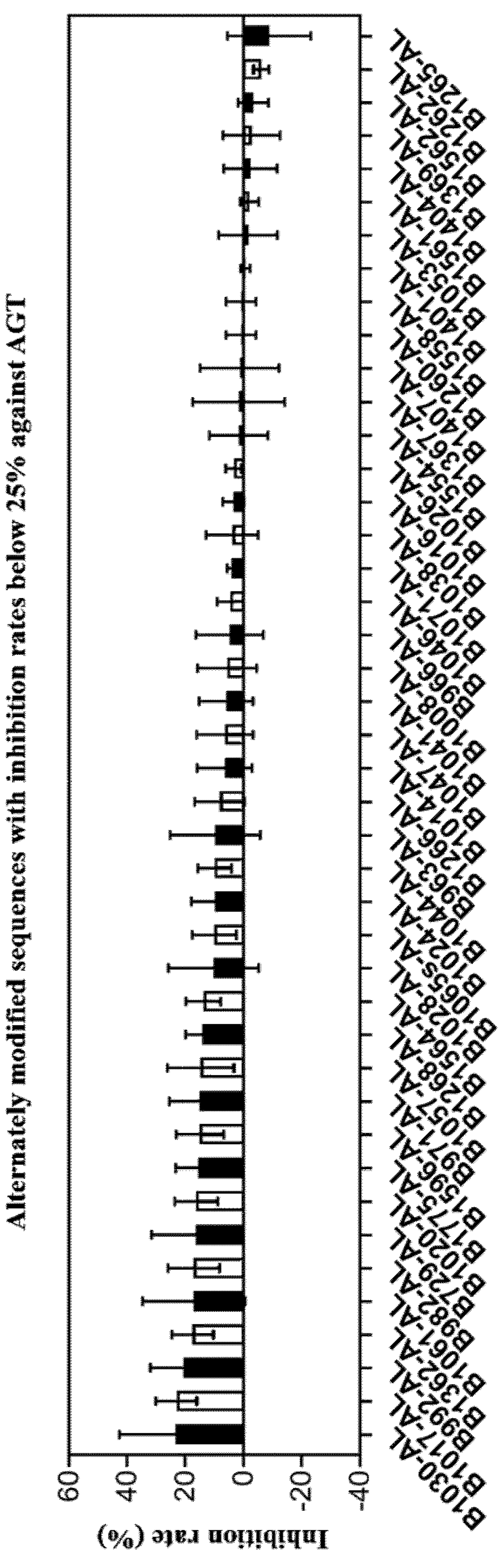
Alternately modified sequences with inhibition rates below 25% against AGT
Inhibition rate (%)
60
40
20
0
-20
-40

FIG. 3

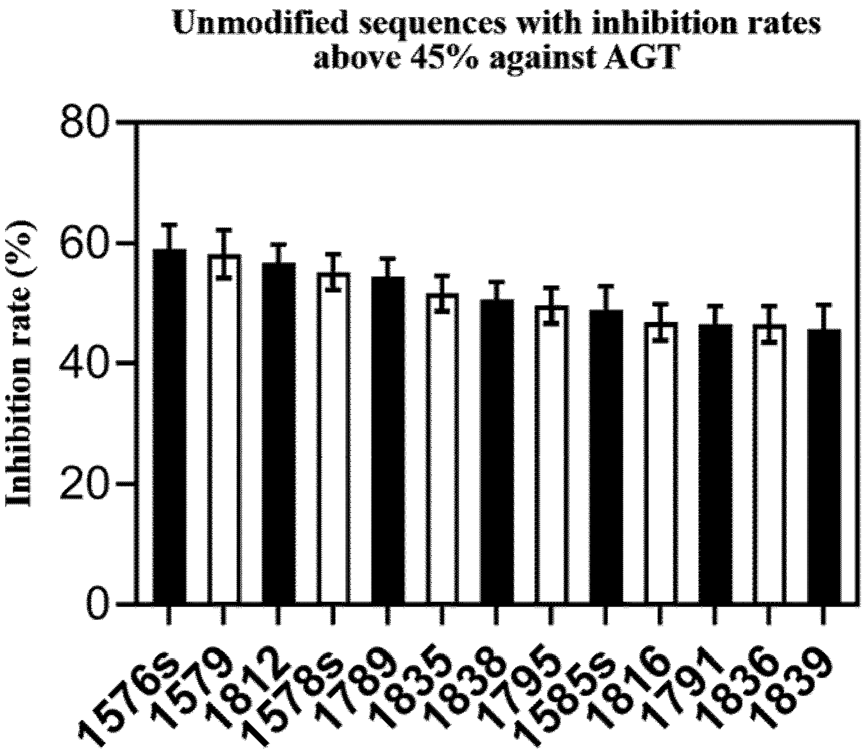
Unmodified sequences with inhibition rates
above 45% against AGT
Inhibition rate (%)
80
60
40
20
0
1576s
1579
1812
1578s
1789
1835
1838
1795
1585s
1816
1791
1836
1839

FIG. 4

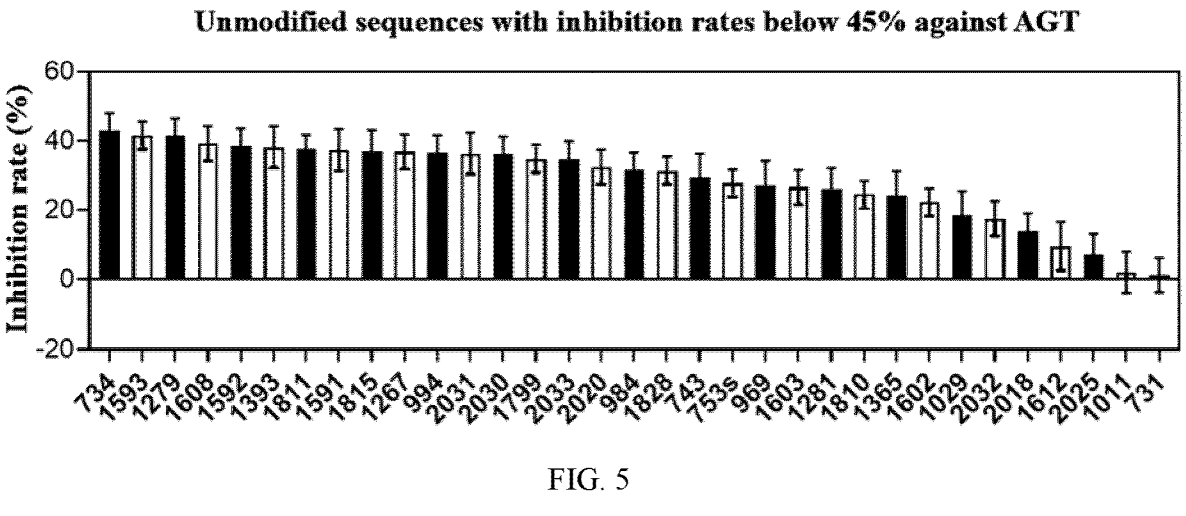
Unmodified sequences with inhibition rates below 45% against AGT
Inhibition rate (%)
60
40
20
0
-20
734
1593
1279
1608
1592
1393
1811
1591
1815
1267
994
2031
2030
1799
2033
2020
984
1828
743
753s
969
1603
1281
1810
1365
1602
1029
2032
2018
1612
2025
1011
731

FIG. 5

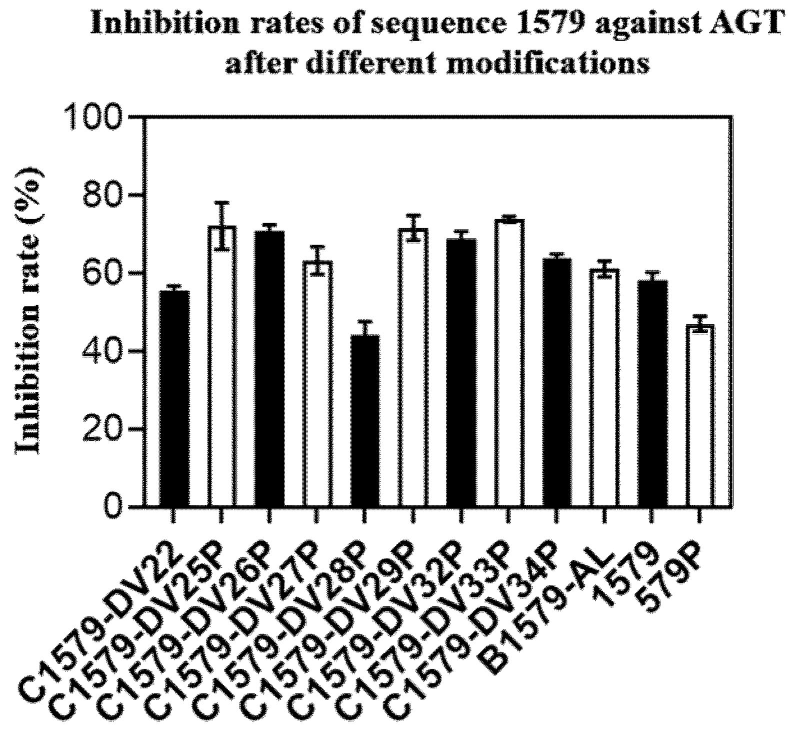
Inhibition rates of sequence 1579 against AGT
after different modifications
Inhibition rate (%)
100
80
60
40
20
0
C1579-DV22
C1579-DV25P
C1579-DV26P
C1579-DV27P
C1579-DV28P
C1579-DV29P
C1579-DV32P
C1579-DV33P
C1579-DV34P
B1579-AL
1579
579P

FIG. 6

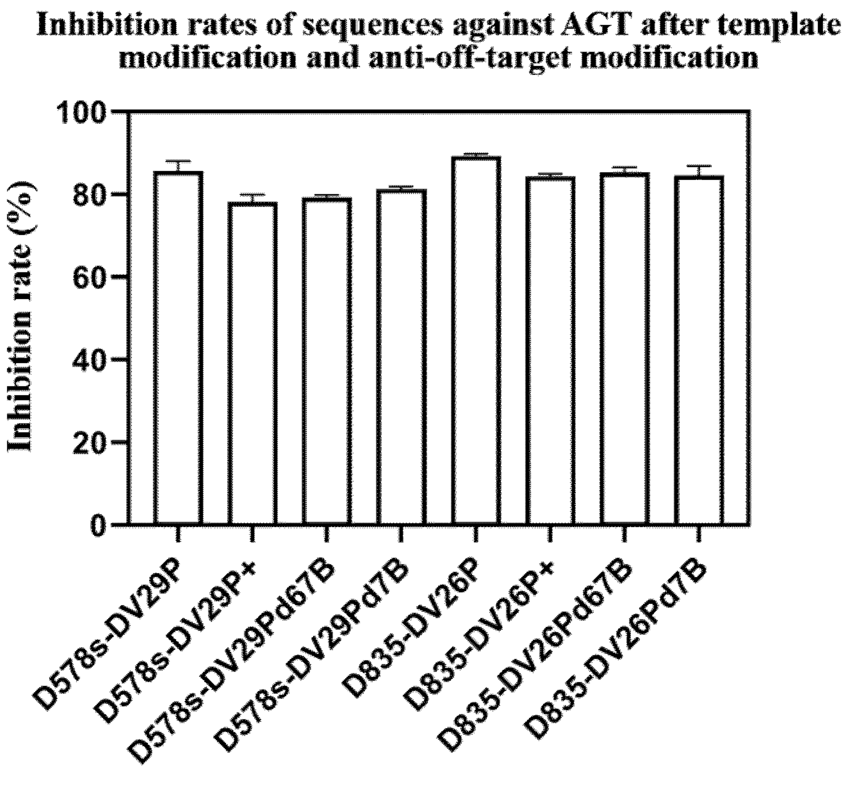
Inhibition rates of sequences against AGT after template
modification and anti-off-target modification
Inhibition rate (%)
100
80
60
40
20
0
D578s-DV29P
D578s-DV29P+
D578s-DV29Pd67B
D578s-DV29Pd7B
D835-DV26P
D835-DV26P+
D835-DV26Pd67B
D835-DV26Pd7B

FIG. 7

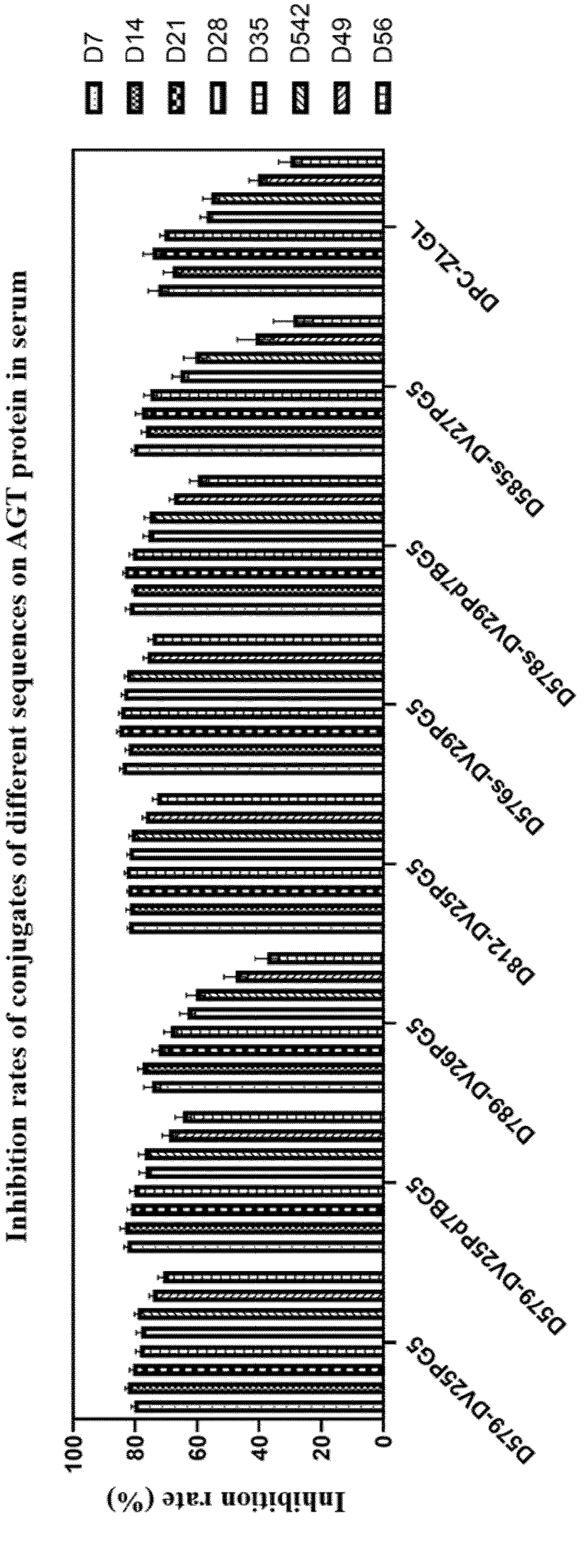
Inhibition rates of conjugates of different sequences on AGT protein in serum
Inhibition rate (%)
0
20
40
60
80
100
D579-DV25PG5
D579-DV25Pd7BG5
D789-DV26PG5
D812-DV25PG5
D576s-DV29PG5
D578s-DV29Pd7BG5
D585s-DV27PG5
DPC-ZLGL
D7
D14
D21
D28
D35
D542
D49
D56

FIG. 8

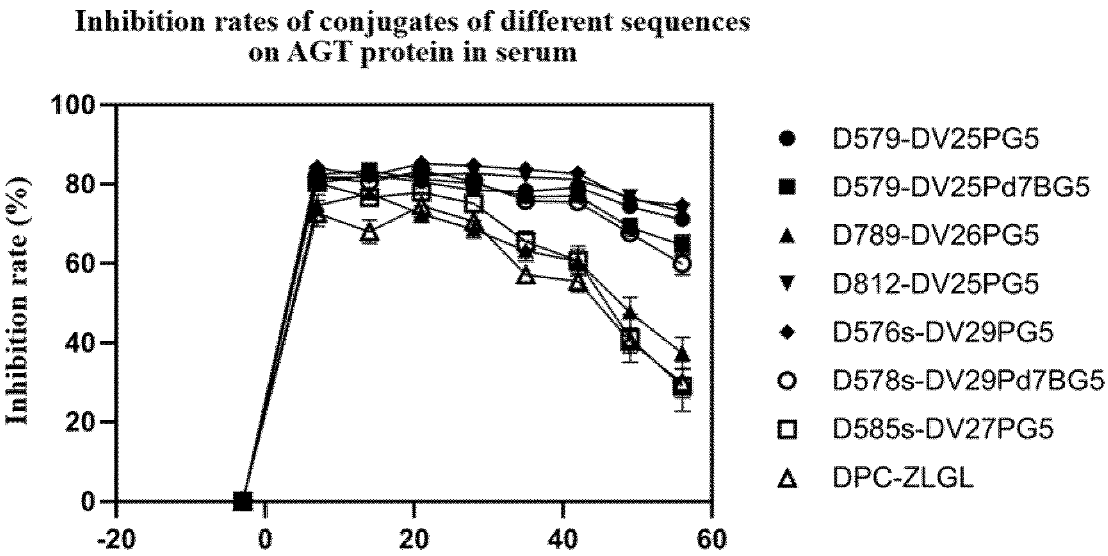
Inhibition rates of conjugates of different sequences
on AGT protein in serum
Inhibition rate (%)
100
80
60
40
20
0
-20
0
20
40
60
D579-DV25PG5
D579-DV25Pd7BG5
D789-DV26PG5
D812-DV25PG5
D576s-DV29PG5
D578s-DV29Pd7BG5
D585s-DV27PG5
DPC-ZLGL

FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8349809 B
- US 8513207 B
- CN 114044741 B
- CN 115784921 B
- CN 115677518 B
- CN 115745820 B
- CN 108368028 B
- CN 110520409 A
- CN 102625696 B
- CN 117751189 A
- WO 2024031101 A1
- CN 112313335 A
- CN 112852809 A
- CN 113862268 A
- CN 106574268 B
- CN 116854754 A
- GB 149252010 A
- GB 1492432010 A
- GB 1492422001 A
- GB 57492006 A


### Non-patent literature cited in the description


- **LIMA et al.** *Cell*, 2012, vol. 150, 883
- **MIYAO et al.** *DsRNA Res. Dev*, 1995, vol. 5, 115-121
- **TAKAKURA et al.** *DsRNA & Nucl. Acid Drug Dev.*, 1996, vol. 6, 177-183
- **FOSTER, D. J. et al.** Advanced siRNA Designs Further Improve In Vivo Performance of GalNAc-siRNA Conjugates. *Mol Ther*, 2018, vol. 26 (3), 708-717